(19) Europäisches Patentamt
European Patent Office
Office européen des brevets

(11) EP 1 847 530 A1

(12) **EUROPEAN PATENT APPLICATION**
published in accordance with Art. 158(3) EPC

(43) Date of publication:
**24.10.2007 Bulletin 2007/43**

(21) Application number: **06712670.6**

(22) Date of filing: **31.01.2006**

(51) Int Cl.:
***C07D 211/58*** *(2006.01)* ***A61K 31/4178*** *(2006.01)*
***A61K 31/4184*** *(2006.01)* ***A61K 31/427*** *(2006.01)*
***A61K 31/444*** *(2006.01)* ***A61K 31/4453*** *(2006.01)*
***A61K 31/4468*** *(2006.01)* ***A61K 31/4545*** *(2006.01)*
***A61K 31/4725*** *(2006.01)* ***A61K 31/496*** *(2006.01)*
***A61K 31/506*** *(2006.01)* ***A61K 31/5375*** *(2006.01)*
***A61K 31/5377*** *(2006.01)* ***A61K 31/541*** *(2006.01)*
***A61K 31/55*** *(2006.01)* ***A61P 1/04*** *(2006.01)*
***A61P 3/04*** *(2006.01)* ***A61P 9/00*** *(2006.01)*
***A61P 9/12*** *(2006.01)* ***A61P 25/04*** *(2006.01)*

(86) International application number:
**PCT/JP2006/301527**

(87) International publication number:
**WO 2006/080519 (03.08.2006 Gazette 2006/31)**

(84) Designated Contracting States:
**AT BE BG CH CY CZ DE DK EE ES FI FR GB GR HU IE IS IT LI LT LU LV MC NL PL PT RO SE SI SK TR**

(30) Priority: **31.01.2005 JP 2005022850**
**11.08.2005 JP 2005232988**

(71) Applicant: **KYOWA HAKKO KOGYO CO., LTD.**
**Chiyoda-ku,**
**Tokyo 100-8185 (JP)**

(72) Inventors:
- **SASHO, Setsuya**
**c/o BioFrontier Laboratories**
**Machida-shi, Tokyo 194-8533 (JP)**
- **SEISHI, Takashi**
**c/o Head Office,KYOWA HAKKO KOGYO CO., LTD.,**
**Tokyo 100-8185, (JP)**
- **ATSUMI, Eri**
**Nagaizumi-cho, Sunto-gun, Shizuoka 411-8731 (JP)**
- **OSAKADA, Mariko**
**Nagaizumi-cho, Sunto-gun, Shizuoka 411-8731 (JP)**
- **HIROSE, Ryo**
**c/o Head Office, KYOWA HAKKO KOGYO CO., LTD.,**
**Tokyo 100-8185, (JP)**
- **TOKI, Shinichiro**
**c/o Head Office,**
**Nagaizumi-cho, Sunto-gun, Shizuoka 411-8731 (JP)**
- **TSUKII, Katsuyoshi**
**Nagaizumi-cho, Sunto-gun, Shizuoka 411-8731 (JP)**
- **SHIRAKURA, Shiro**
**Nagaizumi-cho, Sunto-gun, Shizuoka 411-8731 (JP)**

(74) Representative: **Dossmann, Gérard et al**
**Bureau Casalonga & Josse**
**Bayerstrasse 71/73**
**80335 München (DE)**

# (54) DIAMINE DERIVATIVE

(57) The present invention provides a diamine derivative or the like represented by the general formula (I):

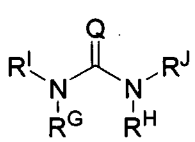

( I )

{wherein Q represents an oxygen atom or the like, $R^G$ represents a hydrogen atom or the like, $R^I$ represents

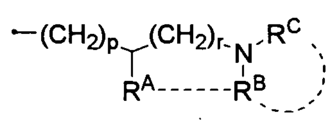

(wherein p and r may be the same or different, and each

represents 0 or the like, $R^A$ represents a hydrogen atom or the like, and $R^B$ and $R^c$ may be the same or different, and each represents a hydrogen atom or the like), $R^H$ represents a hydrogen atom or the like, and $R^J$ represents:

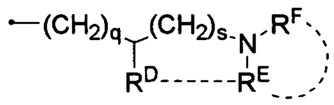

(wherein q and s may be the same or different, and each represents 0 or the like, $R^D$ represents a hydrogen atom or the like, and $R^E$ and $R^F$ may be the same or different, and each represents a hydrogen atom or the like) or the like}, etc.

## Description

Technical Field

The present invention relates to a diamine derivative or a pharmaceutically acceptable salt thereof, which has a histamine $H_3$ receptor antagonism and/or inverse agonism and is useful as a therapeutic agent and/or a preventive agent for allergy, inflammation, a cardiovascular disease, gastrointestinal disorder, central nervous system disorder, central nervous system psychiatric disorder, epilepsy, convulsion, obesity, sleep disorder, acute pain, chronic pain, neuropathic pain or the like.

Background Art

Most of the histamine in the body is distributed in mast cells in tissues and basophilic leukocytes in the blood except for a small part thereof, which is present in neurons, enterochromaffin-like cells, macrophages and the like. Histamine released from such cells interacts with histamine receptors on cell surface or in cell matrix, and exhibits its action. Histamine receptors are G protein-coupled receptors (GPCR), and 4 subtypes, $H_1$ to $H_4$ have been reported so far [Molecular Pharmacology, vol. 59, pp. 415-419 (2001)].

Among these subtypes, the histamine $H_3$ receptor exists in the presynaptic junction of histaminergic neurons in the central nervous system (CNS), and it was first identified as an autoreceptor that controls histamine synthesis and release in the nerve ending [Trends in Pharmacological Science, vol. 19, pp. 177-183, 1998; Annual Report in Medicinal Chemistry, vol. 33, pp. 31-39 (1998)].

In a mammalian brain, the histaminergic neurons project from the mammillary nucleus in the posterior hypothalamic area to a broad area of the brain tissues. Further, histamine controls the CNS, especially behaviors related to the hypothalamic area (for example, sleep, wakefulness rhythm, endocrine secretion, eating behavior, water drinking behavior, sexual behavior and the like). From this, it is considered that a ligand of the $H_3$ receptor which controls the amount of histamine influences largely on central activities, especially on behaviors related to the hypothalamic area, and it is considered to be effective in the prevention or treatment of sleep disorder characterized by dyssomnia or insomnia and arousal sensitive state such as epilepsy, convulsion or the like, and the maintenance of arousal state (for example, WO 2002/079168; WO 2002/072570; WO 00/06254; WO 99/24405; WO 96/38141; WO 99/24421 and the like). Further, the $H_3$ receptor is also expressed in endings of neurons other than the presynaptic junction of histaminergic neurons (for example, the presynaptic junction of neurons activated by neurotransmitters such as serotonin, noradrenaline, acetylcholine or dopamine) and controls the release of these neurotransmitters. Since or the like, these neurotransmitters are deeply relates to memory and recognition in the CNS, it is considered that an $H_3$ receptor antagonist and/or an $H_3$ receptor inverse agonist are/is effective for various CNS diseases characterized by memory impairment, recognition impairment and the like such as emotional instability, anxiety, Alzheimer's disease and attention-deficit hyperactivity disorder (ADHD) (for example, WO 2002/079168; WO 2002/072570; WO 00/06254 and the like). In addition, the expression of the $H_3$ receptor has been confirmed not only in the CNS but also in peripheral organs such as the digestive tract, the circulatory system and the respiratory tract or autonomic nerve endings which control the functions of these organs. Therefore, a possibility has been suggested that the $H_3$ receptor antagonist and/or inverse agonist are/is effective for asthma, inflammation, migraine, arrhythmia, septic shock or the like [for example, WO 2002/079168; WO 2002/072570; WO 00/06254; WO 99/24405; WO 96/38141; WO 99/24421; Trends in Pharmacological Science, vol. 19, pp. 177-183 (1998); Annual Report in Medicinal Chemistry, vol. 33, pp. 31-39 (1998) and the like]. Further, the relation between a histamine $H_3$ receptor antagonist and/or inverse agonist and acute nociceptive pain has been reported [British Journal of Pharmacology, vol. 111, pp. 1269-1279 (1994); Pharmacology, Biochemistry and Behavior, vol. 72, pp. 751-760 (2002)]. Further, it has recently been revealed that the $H_3$ receptor antagonist and/or inverse agonist improve (s) the neuropathic pain symptom , and the relation between the $H_3$ receptor and pain has been suggested (WO 2004/89410).

As the $H_3$ receptor antagonist and/or inverse agonist, a large number of compounds such as thioperamide, chlobenpropit and iodophenpropit have been reported.

On the other hand, as the diamine derivative, compounds represented by the following formulae (A) to (C) and the like are known (see Patent documents 1 and 2, and Non-patent document 1).

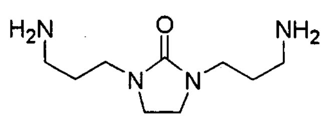
(A)

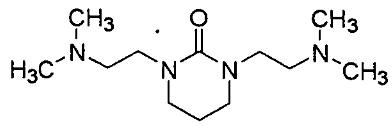
(B)

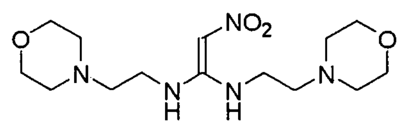
(C)

Further, as an α-unsaturated amine, compounds represented by the following formulae (D), (E), (F) and (G) are known as an insecticide or a miticide (see Patent document 3).

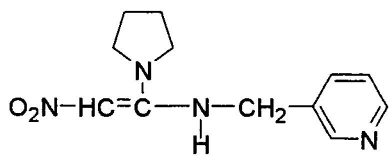
(D)

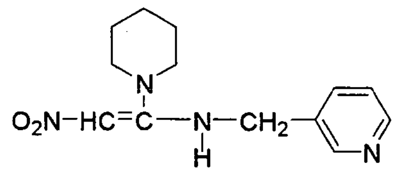
(E)

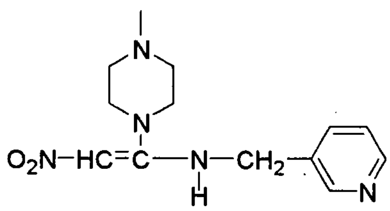
(F)

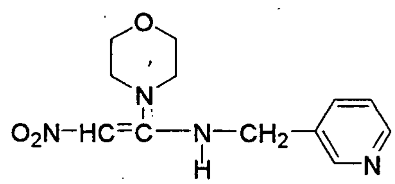
(G)

Patent document 1: Japanese Published Unexamined Patent Application No. 1992-226167
Patent document 2: Japanese Patent No. 3054654
Patent document 3: Japanese Published Examined Patent Application No. 1995-14916
Non-patent document 1: "Archiv der Pharmazie", vol. 313, pp. 471-476, 1980

Disclosure of the invention

Problems to be Solved by the Invention

An object of the present invention is to provide a diamine derivative or a pharmaceutically acceptable salt thereof, which has a histamine $H_3$ receptor antagonism and/or inverse agonism and is useful as a therapeutic agent and/or a preventive agent for allergy, inflammation, a cardiovascular disease (for example, hypertension, hypotension or the like), gastrointestinal disorder (for example, inappropriate secretion of acid or gastrointestinal hormones, movement disorder and the like), central nervous system disorder such as impaired attention or cognitive disability (for example, Alzheimer's disease, ADHD, memory function impairment due to aging or the like), central nervous system psychiatric disorder (for example, anxiety, schizophrenic disorder or the like), epilepsy, convulsion, obesity, sleep disorder (for example, sleep attack, sleep apnea, insomnia, disturbance of circadian rhythm or the like), acute pain, chronic pain, neuropathic pain or the like.

Means for Solving the Problems

The present invention relates to the following (1) to (27).

(1) A diamine derivative represented by the general formula (I) :

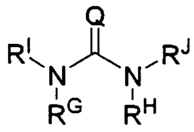

(I)

{wherein Q represents an oxygen atom, $=C(CN)_2$, $=CHNO_2$, =NCN, $=NSO_2NH_2$ or =C (CN) ($SO_2R^1$) (wherein $R^1$ represents substituted or unsubstituted lower alkyl or substituted or unsubstituted aryl),
$R^G$ represents a hydrogen atom or substituted or unsubstituted lower alkyl, or $R^G$ and $R^H$ are combined to represent substituted or unsubstituted alkylene or substituted or unsubstituted phenylene, and

(i) when $R^G$ is a hydrogen atom or substituted or unsubstituted lower alkyl,
$R^I$ represents:

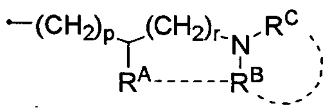

(wherein p and r may be the same or different, and each represents 0, 1, 2 or 3,
$R^A$ represents a hydrogen atom or substituted or unsubstituted lower alkyl, and
$R^B$ and $R^C$ may be the same or different, and each represents a hydrogen atom, substituted or unsubstituted lower alkyl, substituted or unsubstituted lower alkenyl, substituted or unsubstituted lower alkynyl, substituted or unsubstituted cycloalkyl, substituted or unsubstituted lower alkanoyl, substituted or unsubstituted cycloalkylcarbonyl, substituted or unsubstituted lower alkoxycarbonyl, substituted or unsubstituted aroyl, substituted or unsubstituted aryl, a substituted or unsubstituted aliphatic heterocyclic group or a substituted or unsubstituted aromatic heterocyclic group, or
$R^B$ and $R^C$ are combined together with the adjacent nitrogen atom thereto to form a substituted or unsubstituted nitrogen-containing heterocyclic group, or
$R^A$ and $R^B$ are combined to represent substituted or unsubstituted alkylene, and
$R^C$ represents a hydrogen atom, substituted or unsubstituted lower alkyl, substituted or unsubstituted lower alkenyl, substituted or unsubstituted lower alkynyl, substituted or unsubstituted cycloalkyl, substituted or unsubstituted lower alkanoyl, substituted or unsubstituted cycloalkylcarbonyl, substituted or unsubstituted lower alkoxycarbonyl, substituted or unsubstituted aroyl, substituted or unsubstituted aryl, a substituted or unsubstituted aliphatic heterocyclic group or a substituted or unsubstituted aromatic heterocyclic group),
$R^H$ represents a hydrogen atom or substituted or unsubstituted lower alkyl, and
$R^J$ represents:

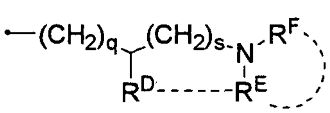

(wherein q and s may be the same or different, and each represents 0, 1, 2 or 3,
$R^D$ represents a hydrogen atom or substituted or unsubstituted lower alkyl, and
$R^E$ and $R^F$ may be the same or different, and each represents a hydrogen atom, substituted or unsubstituted lower alkyl, substituted or unsubstituted lower alkenyl, substituted or unsubstituted lower alkynyl, substituted or unsubstituted cycloalkyl, substituted or unsubstituted lower alkanoyl, substituted or unsubstituted cycloalkylcarbonyl, substituted or unsubstituted lower alkoxycarbonyl, substituted or unsubstituted aroyl, substituted or unsubstituted aryl, a substituted or unsubstituted aliphatic heterocyclic group or a substituted or unsubstituted aromatic heterocyclic group, or
$R^E$ and $R^F$ are combined together with the adjacent nitrogen atom thereto to form a substituted or unsubstituted nitrogen-containing heterocyclic group, or
$R^D$ and $R^E$ are combined to represent substituted or unsubstituted alkylene, and
$R^F$ represents a hydrogen atom, substituted or unsubstituted lower alkyl, substituted or unsubstituted lower

alkenyl, substituted or unsubstituted lower alkynyl, substituted or unsubstituted cycloalkyl, substituted or unsubstituted lower alkanoyl, substituted or unsubstituted cycloalkylcarbonyl, substituted or unsubstituted lower alkoxycarbonyl, substituted or unsubstituted aroyl, substituted or unsubstituted aryl, a substituted or unsubstituted aliphatic heterocyclic group or a substituted or unsubstituted aromatic heterocyclic group), or

$R^H$ and $R^J$ are combined to represent $-(CH_2)_t-X-(CH_2)_u-$[wherein t represents 0, 1, 2 or 3, and u represents 1, 2 or 3, and

X represents:

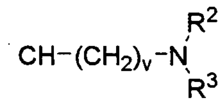

(wherein v represents 0 or 1, and

$R^2$ and $R^3$ may be the same or different, and each represents a hydrogen atom, substituted or unsubstituted lower alkyl, substituted or unsubstituted lower alkenyl, substituted or unsubstituted lower alkynyl, substituted or unsubstituted cycloalkyl, substituted or unsubstituted lower alkanoyl, substituted or unsubstituted cycloalkylcarbonyl, substituted or unsubstituted lower alkoxycarbonyl, substituted or unsubstituted aroyl, substituted or unsubstituted aryl, a substituted or unsubstituted aliphatic heterocyclic group or a substituted or unsubstituted aromatic heterocyclic group, or

$R^2$ and $R^3$ are combined together with the adjacent nitrogen atom thereto to form a substituted or unsubstituted nitrogen-containing heterocyclic group), or

$N-R^4$ (wherein $R^4$ represents a hydrogen atom, substituted or unsubstituted lower alkyl, substituted or unsubstituted lower alkenyl, substituted or unsubstituted lower alkynyl, substituted or unsubstituted cycloalkyl, substituted or unsubstituted lower alkanoyl, substituted or unsubstituted cycloalkylcarbonyl, substituted or unsubstituted lower alkoxycarbonyl, substituted or unsubstituted aroyl, substituted or unsubstituted aryl, a substituted or unsubstituted aliphatic heterocyclic group or a substituted or unsubstituted aromatic heterocyclic group)], and

(ii) when $R^G$ and $R^H$ are combined to form substituted or unsubstituted alkylene or substituted or unsubstituted phenylene,

$R^I$ is:

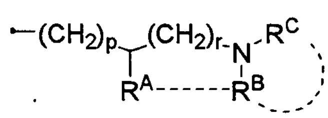

(wherein p, r, $R^A$, $R^B$ and $R^C$ have the same definitions as described above, respectively), and

$R^J$ is:

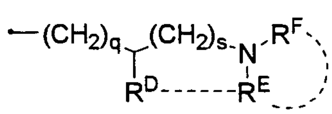

(wherein q, s, $R^D$, $R^E$ and $R^F$ have the same definitions as described above, respectively), with the proviso that when Q is an oxygen atom, at least $R^A$ and $R^B$, and/or $R^D$ and $R^E$ are respectively combined to represent substituted or unsubstituted alkylene, or $R^B$ and $R^C$, and/or $R^E$ and $R^F$ are respectively combined together with the adjacent nitrogen atom thereto to form a substituted or unsubstituted nitrogen-containing heterocyclic group, and

when Q is $=CHNO_2$, at least $R^G$ and $R^H$ are combined to represent substituted or unsubstituted alkylene or substituted or unsubstituted phenylene}, or a pharmaceutically acceptable salt thereof.

(2) The diamine derivative or the pharmaceutically acceptable salt thereof according to (1), wherein Q is an oxygen atom, $=C(CN)_2$, $=CHNO_2$, $=NCN$, $=NSO_2NH_2$ or $=C(CN)(SO_2R^{1A})$ (wherein $R^{1A}$ represents substituted or unsubstituted $C_{1-10}$ alkyl or substituted or unsubstituted $C_{6-14}$ aryl),

$R^G$ is a hydrogen atom or substituted or unsubstituted $C_{1-10}$ alkyl, or $R^G$ and $R^H$ are combined to form substituted or unsubstituted $C_{1-10}$ alkylene or substituted or unsubstituted phenylene, and

(i) when $R^G$ is a hydrogen atom or substituted or unsubstituted $C_{1-10}$ alkyl,
$R^I$ is:

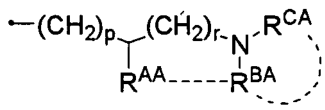

(wherein p and r have the same definitions as described above, respectively,
$R^{AA}$ represents a hydrogen atom or substituted or unsubstituted $C_{1-10}$ alkyl, and
$R^{BA}$ and $R^{CA}$ may be the same or different and each represents a hydrogen atom, substituted or unsubstituted $C_{1-10}$ alkyl, substituted or unsubstituted $C_{2-10}$ alkenyl, substituted or unsubstituted $C_{2-10}$ alkynyl, substituted or unsubstituted $C_{3-8}$ cycloalkyl, substituted or unsubstituted $C_{2-11}$ alkanoyl, substituted or unsubstituted $C_{3-8}$ cycloalkylcarbonyl, substituted or unsubstituted $C_{1-10}$ alkoxycarbonyl, substituted or unsubstituted $C_{7-15}$ aroyl, substituted or unsubstituted $C_{6-14}$ aryl, a substituted or unsubstituted aliphatic heterocyclic group or a substituted or unsubstituted aromatic heterocyclic group, or
$R^{BA}$ and $R^{CA}$ are combined together with the adjacent nitrogen atom thereto to form a substituted or unsubstituted nitrogen-containing heterocyclic group, or
$R^{AA}$ and $R^{BA}$ are combined to represent substituted or unsubstituted $C_{1-10}$ alkylene, and
$R^{CA}$ represents a hydrogen atom, substituted or unsubstituted $C_{1-10}$ alkyl, substituted or unsubstituted $C_{2-10}$ alkenyl, substituted or unsubstituted $C_{2-10}$ alkynyl, substituted or unsubstituted $C_{3-8}$ cycloalkyl, substituted or unsubstituted $C_{2-11}$ alkanoyl, substituted or unsubstituted $C_{3-8}$ cycloalkylcarbonyl, substituted or unsubstituted $C_{1-10}$ alkoxycarbonyl, substituted or unsubstituted $C_{7-15}$ aroyl, substituted or unsubstituted $C_{6-14}$ aryl, a substituted or unsubstituted aliphatic heterocyclic group or a substituted or unsubstituted aromatic heterocyclic group),
$R^H$ is a hydrogen atom or substituted or unsubstituted $C_{1-10}$ alkyl, and
$R^J$ is:

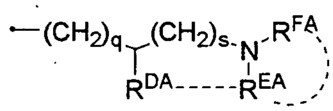

(wherein q and s have the same definitions as described above, respectively,
$R^{DA}$ represents a hydrogen atom or substituted or unsubstituted $C_{1-10}$ alkyl, and
$R^{EA}$ and $R^{FA}$ may be the same or different, and each represents a hydrogen atom, substituted or unsubstituted $C_{1-10}$ alkyl, substituted or unsubstituted $C_{2-10}$ alkenyl, substituted or unsubstituted $C_{2-10}$ alkynyl, substituted or unsubstituted $C_{3-8}$ cycloalkyl, substituted or unsubstituted $C_{2-11}$ alkanoyl, substituted or unsubstituted $C_{3-8}$ cycloalkylcarbonyl, substituted or unsubstituted $C_{1-10}$ alkoxycarbonyl, substituted or unsubstituted $C_{7-15}$ aroyl, substituted or unsubstituted $C_{6-14}$ aryl, a substituted or unsubstituted aliphatic heterocyclic group or a substituted or unsubstituted aromatic heterocyclic group, or
$R^{EA}$ and $R^{FA}$ are combined together with the adjacent nitrogen atom thereto to form a substituted or unsubstituted nitrogen-containing heterocyclic group), or
$R^{DA}$ and $R^{EA}$ are combined to represent substituted or unsubstituted $C_{1-10}$ alkylene, and
$R^{FA}$ represents a hydrogen atom, substituted or unsubstituted $C_{1-10}$ alkyl, substituted or unsubstituted $C_{2-10}$ alkenyl, substituted or unsubstituted $C_{2-10}$ alkynyl, substituted or unsubstituted $C_{3-8}$ cycloalkyl, substituted or unsubstituted $C_{2-11}$ alkanoyl, substituted or unsubstituted $C_{3-8}$ cycloalkylcarbonyl, substituted or unsubstituted $C_{1-10}$ alkoxycarbonyl, substituted or unsubstituted $C_{7-15}$ aroyl, substituted or unsubstituted $C_{6-14}$ aryl, a substituted or unsubstituted aliphatic heterocyclic group or a substituted or unsubstituted aromatic heterocyclic group), or
$R^H$ and $R^J$ are combined to form - $(CH_2)_t$-$X_A$- $(CH_2)_u$-[wherein t and u have the same definitions as described above, respectively, and
$X_A$ is:

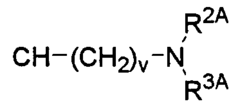

(wherein v represents 0 or 1, and
$R^{2A}$ and $R^{3A}$ may be the same or different, and each represents a hydrogen atom, substituted or unsubstituted $C_{1-10}$ alkyl, substituted or unsubstituted $C_{2-10}$ alkenyl, substituted or unsubstituted $C_{2-10}$ alkynyl, substituted or unsubstituted $C_{3-8}$ cycloalkyl, substituted or unsubstituted $C_{2-11}$ alkanoyl, substituted or unsubstituted $C_{3-8}$ cycloalkylcarbonyl, substituted or unsubstituted $C_{1-10}$ alkoxycarbonyl, substituted or unsubstituted $C_{7-15}$ aroyl, substituted or unsubstituted $C_{6-14}$ aryl, a substituted or unsubstituted aliphatic heterocyclic group or a substituted or unsubstituted aromatic heterocyclic group, or
$R^{2A}$ and $R^{3A}$ are combined together with the adjacent nitrogen atom thereto to form a substituted or unsubstituted nitrogen-containing heterocyclic group), or
N-$R^{4A}$ (wherein $R^{4A}$ represents a hydrogen atom, substituted or unsubstituted $C_{1-10}$ alkyl, substituted or unsubstituted $C_{2-10}$ alkenyl, substituted or unsubstituted $C_{2-10}$ alkynyl, substituted or unsubstituted $C_{3-8}$ cycloalkyl, substituted or unsubstituted $C_{2-11}$ alkanoyl, substituted or unsubstituted $C_{3-8}$ cycloalkylcarbonyl, substituted or unsubstituted $C_{1-10}$ alkoxycarbonyl, substituted or unsubstituted $C_{7-15}$ aroyl, substituted or unsubstituted $C_{4-14}$ aryl, a substituted or unsubstituted aliphatic heterocyclic group or a substituted or unsubstituted aromatic heterocyclic group)], and
(ii) when $R^G$ and $R^H$ are combined to form substituted or unsubstituted $C_{1-10}$ alkylene or substituted or unsubstituted phenylene,
$R^I$ is:

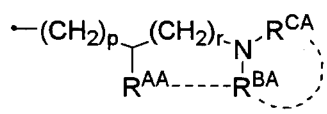

(wherein p, r, $R^{AA}$, $R^{BA}$ and $R^{CA}$ have the same definitions as described above, respectively), and
$R^J$ is:

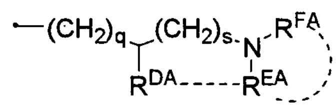

(wherein q, s, $R^{DA}$, $R^{EA}$ and $R^{FA}$ have the same definitions as described above, respectively).

(3) A diamine derivative represented by the general formula (II):

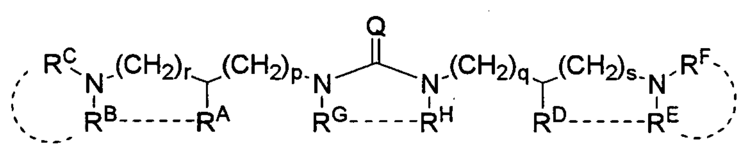

(II)

[wherein p, q, r and s may be the same or different, and each represents 0, 1, 2 or 3,
$R^A$ and $R^D$ may be the same or different, and each represents a hydrogen atom or substituted or unsubstituted lower alkyl,
$R^B$ and $R^E$ may be the same or different, and each represents a hydrogen atom, substituted or unsubstituted lower alkyl, substituted or unsubstituted lower alkenyl, substituted or unsubstituted lower alkynyl, substituted or unsubstituted cycloalkyl, substituted or unsubstituted lower alkanoyl, substituted or unsubstituted cycloalkylcarbonyl, substituted or unsubstituted lower alkoxycarbonyl, substituted or unsubstituted aroyl, substituted or unsubstituted aryl, a substituted or unsubstituted aliphatic heterocyclic group or a substituted or unsubstituted aromatic heterocyclic group, or

$R^A$ and $R^B$, and/or $R^D$ and $R^E$ are respectively combined to represent substituted or unsubstituted alkylene,

$R^C$ and $R^F$ may be the same or different, and each represents a hydrogen atom, substituted or unsubstituted lower alkyl, substituted or unsubstituted lower alkenyl, substituted or unsubstituted lower alkynyl, substituted or unsubstituted cycloalkyl, substituted or unsubstituted lower alkanoyl, substituted or unsubstituted cycloalkylcarbonyl, substituted or unsubstituted lower alkoxycarbonyl, substituted or unsubstituted aroyl, substituted or unsubstituted aryl, a substituted or unsubstituted aliphatic heterocyclic group or a substituted or unsubstituted aromatic heterocyclic group, or

$R^B$ and $R^c$, and/or $R^E$ and $R^F$ are respectively combined together with the adjacent nitrogen atom thereto to form a substituted or unsubstituted nitrogen-containing heterocyclic group,

$R^G$ and $R^H$ may be the same or different, and each represents a hydrogen atom or substituted or unsubstituted lower alkyl, or

$R^G$ and $R^H$ are combined to represent substituted or unsubstituted alkylene or substituted or unsubstituted phenylene, and

Q represents an oxygen atom, $=C(CN)_2$, $=CHNO_2$, =NCN, $=NSO_2NH_2$ or $=C(CN)(SO_2R^1)$ (wherein $R^1$ represents substituted or unsubstituted lower alkyl or substituted or unsubstituted aryl), with the proviso that

when Q is an oxygen atom, at least $R^A$ and $R^B$, and/or $R^D$ and $R^E$ are respectively combined to represent substituted or unsubstituted alkylene, or $R^B$ and $R^C$, and/or $R^E$ and $R^F$ are respectively combined together with the adjacent nitrogen atom thereto to form a substituted or unsubstituted nitrogen-containing heterocyclic group, and

when Q is $=CHNO_2$, at least $R^G$ and $R^H$ are combined to represent substituted or unsubstituted alkylene or substituted or unsubstituted phenylene], or a pharmaceutically acceptable salt thereof.

(4) The diamine derivative or the pharmaceutically acceptable salt thereof according to (2), wherein Q is $=C(CN)_2$, =NCN or $=C(CN)SO_2R^{1A}$ (wherein $R^{1A}$ has the same definition as described above).

(5) The diamine derivative or the pharmaceutically acceptable salt thereof according to any one of (1) to (3), wherein Q is $=C(CN)_2$.

(6) The diamine derivative or the pharmaceutically acceptable salt thereof according to any one of (1) to (5), wherein p and q are 0.

(7) The diamine derivative or the pharmaceutically acceptable salt thereof according to any one of (1) to (6), wherein r and s may be the same or different, and each is 1 or 2.

(8) The diamine derivative or the pharmaceutically acceptable salt thereof according to any one of (1) to (7), wherein $R^G$ and $R^H$ are combined to form substituted or unsubstituted $C_{1-10}$ alkylene.

(9) The diamine derivative or the pharmaceutically acceptable salt thereof according to (8), wherein the substituted or unsubstituted $C_{1-10}$ alkylene is substituted or unsubstituted methylene, substituted or unsubstituted ethylene or substituted or unsubstituted trimethylene.

(10) The diamine derivative or the pharmaceutically acceptable salt thereof according to (8), wherein the substituted or unsubstituted $C_{1-10}$ alkylene is ethylene.

(11) The diamine derivative or the pharmaceutically acceptable salt thereof according to any one of (1) to (7), wherein $R^G$ and $R^H$ are combined to form substituted or unsubstituted 1,2-phenylene.

(12) The diamine derivative or the pharmaceutically acceptable salt thereof according to any one of (1) to (11), wherein $R^B$ and $R^C$, and/or $R^E$ and $R^F$ are respectively combined together with the adjacent nitrogen atom thereto to form a substituted or unsubstituted nitrogen-containing heterocyclic group.

(13) The diamine derivative or the pharmaceutically acceptable salt thereof according to any one of (1), (2) and (4) to (5), wherein $R^H$ and $R^J$ are combined to form $-(CH_2)_t-X_A-(CH_2)_u-$ (wherein t, u, and $X_A$ have the same definitions as described above, respectively).

(14) The diamine derivative or the pharmaceutically acceptable salt thereof according to (13), wherein $X_A$ is:

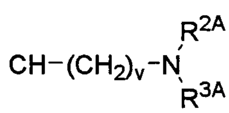

(wherein v, $R^{2A}$ and $R^{3A}$ have the same definitions as described above, respectively).

(15) The diamine derivative or the pharmaceutically acceptable salt thereof according to (13) or (14), wherein p is 0.

(16) The diamine derivative or the pharmaceutically acceptable salt thereof according to any one of (13) to (15), wherein r is 1 or 2.

(17) The diamine derivative or the pharmaceutically acceptable salt thereof according to any one of (1) to (5), wherein $R^A$ or $R^{AA}$ is a hydrogen atom and $R^G$ and $R^H$ are combined to form substituted or unsubstituted $C_{1-10}$ alkylene.

(18) The diamine derivative or the pharmaceutically acceptable salt thereof according to any one of (1) to (5) and (17), wherein $R^B$ and $R^C$, or $R^{BA}$ and $R^{CA}$ are combined together with the adjacent nitrogen atom thereto to form a substituted or unsubstituted nitrogen-containing heterocyclic group.

(19) The diamine derivative or the pharmaceutically acceptable salt thereof according to (17) or (18), wherein $R^D$ or $R^{DA}$ is a hydrogen atom.

(20) The diamine derivative or the pharmaceutically acceptable salt thereof according to any one of (17) to (19), wherein $R^E$ and $R^F$, or $R^{EA}$ and $R^{FA}$ are combined together with the adjacent nitrogen atom thereto to form a substituted or unsubstituted nitrogen-containing heterocyclic group.

(21) The diamine derivative or the pharmaceutically acceptable salt thereof according to any one of (1) to (5), (17) and (18), wherein $R^D$ and $R^E$, or $R^{DA}$ and $R^{EA}$ are combined to form substituted or unsubstituted $C_{1-10}$ alkylene and q is 0.

(22) The diamine derivative or the pharmaceutically acceptable salt thereof according to (21), wherein s is 2, and $R^D$ and $R^E$, or $R^{DA}$ and $R^{EA}$ are combined to form ethylene.

(23) A pharmaceutical composition which comprises, as an active ingredient, the diamine derivative or the pharmaceutically acceptable salt thereof recited in any one of (1) to (22).

(24) A histamine $H_3$ receptor antagonist and/or inverse agonist which comprises, as an active ingredient, the diamine derivative or the pharmaceutically acceptable salt thereof recited in any one of (1) to (22).

(25) Use of the diamine derivative or the pharmaceutically acceptable salt thereof recited in any one of (1) to (22) for the manufacture of a pharmaceutical composition.

(26) A method of antagonizing and/or inversely agonizing a histamine $H_3$ receptor, which comprises, as an active ingredient, administering the diamine derivative or the pharmaceutically acceptable salt thereof recited in any one of (1) to (22).

(27) Use of the diamine derivative or the pharmaceutically acceptable salt thereof recited in any one of (1) to (22) for the manufacture of a histamine $H_3$ receptor antagonist and/or inverse agonist.

## Effect of the Invention

According to the present invention, a diamine derivative or a pharmaceutically acceptable salt thereof, which has a histamine $H_3$ receptor antagonism and/or inverse agonism and is useful as a therapeutic agent and/or a preventive agent for allergy, inflammation, a cardiovascular disease (for example, hypertension, hypotension or the like), gastrointestinal disorder (for example, inappropriate secretion of acid or gastrointestinal hormones, movement disorder or the like), central nervous system disorder such as impaired attention or cognitive disability (for example, Alzheimer's disease, ADHD, memory function impairment due to aging or the like), central nervous system psychiatric disorder (for example, anxiety, schizophrenic disorder or the like), epilepsy, convulsion, obesity, sleep disorder (for example, sleep attack, sleep apnea, insomnia, disturbance of circadian rhythm or the like), acute pain, chronic pain, neuropathic pain or the like can be provided.

## Best Mode for Carrying Out the Invention

Hereinafter, a compound represented by the general formula (I) is referred to as Compound (I). The compounds having the other formula numbers are referred to in the same manner.

In the definitions of the respective groups in the general formulae (I) and (II), examples of the lower alkyl, the lower alkyl moiety of the lower alkanoyl and lower alkoxycarbonyl, the $C_{1-10}$ alkyl and the $C_{1-10}$ alkyl moiety of the $C_{2-11}$ alkanoyl and $C_{1-10}$ alkoxycarbonyl include linear or branched alkyl having 1 to 10 carbon atoms.

Specific examples thereof include methyl, ethyl, propyl, isopropyl, butyl, isobutyl, sec-butyl, tert-butyl, pentyl, isopentyl, neopentyl, hexyl, heptyl, octyl, nonyl, decyl and the like.

Examples of the lower alkenyl and $C_{2-10}$ alkenyl include linear or branched alkenyl having 2 to 10 carbon atoms. Specific examples thereof include vinyl, allyl, 1-propenyl, butenyl, pentenyl, hexenyl, heptenyl, octenyl, nonenyl, decenyl and the like.

Examples of the lower alkynyl and $C_{2-10}$ alkynyl include linear or branched alkynyl having 2 to 10 carbon atoms. Specific examples thereof include ethynyl, propynyl, butynyl, pentynyl, hexynyl, heptynyl, octynyl, nonynyl, decynyl and the like.

Examples of the cycloalkyl, the cycloalkyl moiety of the cycloalkylcarbonyl, the $C_{3-8}$ cycloalkyl and the $C_{3-8}$ cycloalkyl moiety of the $C_{3-8}$ cycloalkylcarbonyl include cycloalkyl having 3 to 8 carbon atoms. Specific examples thereof include cyclopropyl, cyclobutyl, cyclopentyl, cyclohexyl, cycloheptyl, cyclooctyl and the like.

Examples of the aryl, the aryl moiety of the aroyl, the $C_{6-14}$ aryl and the $C_{6-14}$ aryl moiety of the $C_{7-15}$ aroyl

include aryl having 6 to 14 carbon atoms. Specific examples thereof include phenyl, naphthyl, anthranil and the like.

Examples of the alkylene and $C_{1-10}$ alkylene include linear or branched alkylene having 1 to 10 carbon atoms. Specific examples thereof include methylene, ethylene, trimethylene, tetramethylene, pentamethylene, hexamethylene, heptamethylene, octamethylene, nonamethylene, decamethylene, propylene, ethylethylene, methylmethylene, dimethylmethylene and the like.

Examples of the aliphatic heterocyclic group include a 5- or 6-membered monocyclic aliphatic heterocyclic group which contains at least one atom selected from a nitrogen atom, an oxygen atom and a sulfur atom, a bicyclic or tricyclic fused ring aliphatic heterocyclic group in which 3- to 8-membered rings are condensed containing at least one atom selected from a nitrogen atom, an oxygen atom and a sulfur atom. Specific examples thereof include aziridinyl, oxyranyl, azetidinyl, tetrahydrofuranyl, thioranyl, pyrrolidinyl, dioxoranyl, imidazolidinyl, pyrazolydinyl, tetrahydropyranyl, tetrahydrothiopyranyl, piperidinyl, piperidino, piperazinyl, morpholinyl, morpholino, thiomorpholinyl, thiomorpholino, perhydroazepinyl, homopiperazinyl, perhydroazocinyl, tetrahydroquinolinyl, tetrahydroisoquinolinyl, dihydrobenzofuranyl, dihydrobenzothienyl, dihydroindolinyl, dihydropyridyl and the like.

Examples of the aromatic heterocyclic group include a 5- or 6-membered monocyclic aromatic heterocyclic group containing at least one atom selected from a nitrogen atom, an oxygen atom and a sulfur atom, a bicyclic or tricyclic fused ring aromatic heterocyclic group in which 3- to 8-membered rings are condensed containing at least one atom selected from a nitrogen atom, an oxygen atom and a sulfur atom. Specific examples thereof include furyl, thienyl, pyrrolyl, oxazolyl, isooxazolyl, thiazolyl, isothiazolyl, imidazolyl, pyrazolyl, triazolyl, tetrazolyl, pyridyl, pyrazinyl, pyrimidinyl, pyridazinyl, benzooxazolyl, benzothienyl, indolyl, indazolyl, benzimidazolyl, benzotriazolyl, purinyl, quinolyl, isoquinolyl, quinazolinyl, phthalazinyl, cinnolinyl, quinoxalinyl, naphthyridinyl, puteridinyl, furazanyl, carbazolyl and the like.

Examples of the nitrogen-containing heterocyclic group combined together with the adjacent nitrogen atom include a 5- or 6-membered monocyclic aliphatic heterocyclic group or monocyclic aromatic heterocyclic group containing at least one nitrogen atom (the monocyclic aliphatic heterocyclic group and the monocyclic aromatic heterocyclic group may contain another nitrogen atom, an oxygen atom or a sulfur atom), a bicyclic or tricyclic fused ring aliphatic heterocyclic group or fused ring aromatic heterocyclic group in which 3- to 8-membered rings are condensed containing at least one nitrogen atom (the fused ring aliphatic heterocyclic group and the fused ring aromatic heterocyclic group may contain another nitrogen atom, an oxygen atom or a sulfur atom) and the like. Specific examples thereof include aziridinyl, azetidinyl, pyrrolidinyl, piperidino, perhydroazepinyl, azepinyl, perhydroazocinyl, 1-pyrrolyl, imidazolyl, pyrazolyl, triazolyl, tetrazolyl, imidazolidinyl, pyrazolidinyl, piperazinyl, morpholino, thiomorpholino, homopiperazinyl, dihydropyridyl, tetrahydropyridyl, tetrahydroquinolinyl, tetrahydroisoquinolinyl, indolinyl, dihydroindolinyl, dihydroisoindolinyl, benzimidazolyl, benzotriazolyl, purinyl, carbazolyl, thiazolidinyl and the like.

Examples of the substituents of the substituted lower alkyl, substituted lower alkenyl, substituted lower alkynyl, substituted lower alkanoyl, substituted lower alkoxycarbonyl, substituted alkylene, substituted $C_{1-10}$ alkyl, substituted $C_{2-10}$ alkenyl, substituted $C_{2-10}$ alkynyl, substituted $C_{2-11}$ alkanoyl, substituted $C_{1-10}$ alkoxycarbonyl and substituted $C_{1-10}$ alkylene, which may be the same or different, and in number of, for example, 1 to a substitutable number, preferably 1 to 3, include halogen, hydroxy, mercapto, azido, nitro, cyano, carboxy, carbamoyl, $C_{3-8}$ cycloalkyl optionally having 1 to 3 substituents selected from Substituent group C described below, $C_{6-14}$ aryl optionally having 1 to 3 substituents selected from Substituent group B described below, an aliphatic heterocyclic group optionally having 1 to 3 substituents selected from Substituent group C described below, an aromatic heterocyclic group optionally having 1 to 3 substituents selected from Substituent group C described below,

$C_{1-10}$ alkoxy optionally having 1 to 3 substituents selected from Substituent group A described below, $C_{3-8}$ cycloalkyloxy optionally having 1 to 3 substituents selected from Substituent group C described below, $C_{6-14}$ aryloxy optionally having 1 to 3 substituents selected from Substituent group B described below, aromatic heterocyclic oxy optionally having 1 to 3 substituents selected from Substituent group C described below, $C_{2-11}$ alkanoyloxy optionally having 1 to 3 substituents selected from Substituent group A described below, $C_{7-15}$ aroyloxy optionally having 1 to 3 substituents selected from Substituent group B described below,

$C_{1-10}$ alkylthio optionally having 1 to 3 substituents selected from Substituent group A described below, $C_{6-14}$ arylthio optionally having 1 to 3 substituents selected from Substituent group B described below, $-NR^xR^y$ (wherein $R^x$ and $R^y$ may be the same or different, and each represents a hydrogen atom, $C_{1-10}$ alkyl optionally having 1 to 3 substituents selected from Substituent group A described below, $C_{3-8}$ cycloalkyl optionally having 1 to 3 substituents selected from Substituent group C described below, $C_{6-14}$ aryl optionally having 1 to 3 substituents selected from Substituent group B described below, an aromatic heterocyclic group optionally having 1 to 3 substituents selected from Substituent group C described below, $C_{2-11}$ alkanoyl optionally having 1 to 3 substituents selected from Substituent group A described below, $C_{7-15}$ aroyl optionally having 1 to 3 substituents selected from Substituent group B described below, $C_{1-10}$ alkoxycarbonyl optionally having 1 to 3 substituents selected from Substituent group A described below, $C_{1-10}$ alkylsulfonyl optionally having 1 to 3 substituents selected from Substituent group A described below, or $C_{6-14}$ arylsulfonyl optionally having 1 to 3 substituents selected from Substituent group B described below),

$C_{2-11}$ alkanoyl optionally having 1 to 3 substituents selected from Substituent group A described below, $C_{3-8}$ cycloalkyl-

carbonyl optionally having 1 to 3 substituents selected from Substituent group C described below, $C_{7-15}$ aroyl optionally having 1 to 3 substituents selected from Substituent group B described below, aliphatic heterocyclic carbonyl optionally having 1 to 3 substituents selected from Substituent group C described below, aromatic heterocyclic carbonyl optionally having 1 to 3 substituents selected from Substituent group C described below, $C_{1-10}$ alkoxycarbonyl optionally having 1 to 3 substituents selected from Substituent group A described below, $C_{6-14}$ aryloxycarbonyl optionally having 1 to 3 substituents selected from Substituent group B described below, $C_{1-10}$ alkylcarbamoyl optionally having 1 to 3 substituents selected from Substituent group A described below, di-$C_{1-10}$ alkylcarbamoyl optionally having 1 to 3 substituents selected from Substituent group A described below, $C_{6-14}$ arylcarbamoyl optionally having 1 to 3 substituents selected from Substituent group B described below,

$C_{1-10}$ alkylsulfonyl optionally having 1 to 3 substituents selected from Substituent group A described below, $C_{6-14}$ arylsulfonyl optionally having 1 to 3 substituents selected from Substituent group B described below, $C_{1-10}$ alkylsulfamoyl optionally having 1 to 3 substituents selected from Substituent group A described below, $C_{6-14}$ arylsulfamoyl optionally having 1 to 3 substituents selected from Substituent group B described below and the like.

Examples of the substituents of the substituted aryl, substituted aroyl, substituted phenylene, substituted $C_{6-14}$ aryl, and substituted $C_{7-15}$ aroyl include $C_{1-10}$ alkyl optionally having 1 to 3 substituents selected from Substituent group A described below, $C_{2-10}$ alkenyl optionally having 1 to 3 substituents selected from Substituent group A described below and the like in addition to the groups exemplified as examples of the substituents of the substituted lower alkyl described above.

Examples of the substituents of the substituted cycloalkyl, substituted cycloalkylcarbonyl, substituted $C_3$-8 cycloalkyl, substituted $C_{3-8}$ cycloalkylcarbonyl, substituted aliphatic heterocyclic group, substituted aromatic heterocyclic group and nitrogen-containing heterocyclic group formed together with the adjacent nitrogen atom include oxo and the like in addition to the groups exemplified as examples of the substituents of the substituted aryl described above.

Substituent group A means a group consisting of halogen, hydroxy, mercapto, amino, azido, nitro, cyano, carboxy, carbamoyl, $C_{6-14}$ aryl, an aliphatic heterocyclic group, an aromatic heterocyclic group,

$C_{1-10}$ alkoxy, $C_{6-14}$ aryloxy, aromatic heterocyclic oxy, $C_{7-15}$ aralkyloxy, $C_{2-11}$ alkanoyloxy, $C_{7-15}$ aroyloxy, hydroxy-substituted $C_{1-10}$ alkoxy, $C_{1-10}$ alkoxy-substituted $C_{1-10}$ alkoxy, amino- substituted $C_{1-10}$ alkoxy, $C_{1-10}$ alkylamino-substituted $C_{1-10}$ alkoxy, (di-$C_{1-10}$ alkylamino)-substituted $C_{1-10}$ alkoxy,

$C_{1-10}$ alkylthio,

-$NR^{xx}R^{yy}$ (wherein $R^{xx}$ and $R^{yy}$ may be the same or different, and each represents a hydrogen atom, $C_{1-10}$ alkyl, $C_{6-14}$ aryl, $C_{7-15}$ aralkyl, $C_{2-11}$ alkanoyl, $C_{7-15}$ aroyl, $C_{1-10}$ alkoxycarbonyl, $C_{7-15}$ aralkyloxycarbonyl, hydroxy-substituted $C_{1-10}$ alkyl, $C_{1-10}$ alkoxy-substituted $C_{1-10}$ alkyl, amino-substituted $C_{1-10}$ alkyl, $C_{1-10}$ alkylamino-substituted $C_{1-10}$ alkyl or (di-$C_{1-10}$ alkylamino)-substituted $C_{1-10}$ alkyl),

$C_{2-11}$ alkanoyl, $C_{7-15}$ aroyl, $C_{1-10}$ alkoxycarbonyl, $C_{6-14}$ aryloxycarbonyl

$C_{1-10}$ alkylsulfonyl and $C_{6-14}$ arylsulfonyl.

Substituent group B means a group in which $C_{1-10}$ alkyl and $C_{2-10}$ alkenyl are added to the Substituent group A described above.

Substituent group C means a group in which oxo is added to the Substituent group B described above.

The halogen as used herein means each atom of fluorine, chlorine, bromine and iodine.

Further, examples of the $C_{1-10}$ alkyl and the $C_{1-10}$ alkyl moiety of the $C_{1-10}$ alkoxy, $C_{2-11}$ alkanoyloxy, $C_{1-10}$ alkylthio, $C_{2-11}$ alkanoyl, $C_{1-10}$ alkoxycarbonyl, $C_{1-10}$ alkylsulfonyl, $C_{1-10}$ alkylcarbamoyl, di-$C_{1-10}$ alkylcarbamoyl, $C_{1-10}$ alkylsulfamoyl, $C_{1-10}$ alkoxy-substituted $C_{1-10}$ alkoxy, $C_{1-10}$ alkylamino-substituted $C_{1-10}$ alkoxy, (di-$C_{1-10}$ alkylamino)-substituted $C_{1-10}$ alkoxy, $C_{1-10}$ alkoxy-substituted $C_{1-10}$ alkyl, $C_{1-10}$ alkylamino-substituted $C_{1-10}$ alkyl and (di-$C_{1-10}$ alkylamino) -substituted $C_{1-10}$ alkyl as used herein include the groups exemplified as examples of the lower alkyl described above. Examples of the $C_{1-10}$ alkylene moiety of the hydroxy-substituted $C_{1-10}$ alkoxy, $C_1$-$_{10}$ alkoxy-substituted $C_{1-10}$ alkoxy, amino-substituted $C_{1-10}$ alkoxy, $C_{1-10}$ alkylamino-substituted $C_{1-10}$ alkoxy, (di-$C_{1-10}$ alkylamino) -substituted $C_{1-10}$ alkoxy, hydroxy-substituted $C_{1-10}$ alkyl, $C_{1-10}$ alkoxy-substituted $C_{1-10}$ alkyl, amino-substituted $C_{1-10}$ alkyl, $C_{1-10}$ alkylamino-substituted $C_{1-10}$ alkyl and (di-$C_{1-10}$ alkylamino)-substituted $C_{1-10}$ alkyl include the groups exemplified as examples of the alkylene described above.

Examples of the $C_{2-10}$ alkenyl; the $C_{3-8}$ cycloalkyl and the $C_{3-8}$ cycloalkyl moiety of the $C_{3-8}$ cycloalkyloxy and $C_{3-8}$ cycloalkylcarbonyl; the $C_{6-14}$ aryl and the $C_{6-14}$ aryl moiety of the $C_{6-14}$ aryloxy, $C_{7-15}$ aroyloxy, $C_{6-14}$ arylthio, $C_{7-15}$ aroyl, $C_{6-14}$ arylsulfonyl, $C_{6-14}$ aryloxycarbonyl, $C_{6-14}$ arylcarbamoyl and $C_{6-14}$ arylsulfamoyl; the aliphatic heterocyclic group and the aliphatic heterocyclic moiety of the aliphatic heterocyclic carbonyl; the aromatic heterocyclic group and the aromatic heterocyclic moiety of the aromatic heterocyclic oxy and aromatic heterocyclic carbonyl include the groups exemplified as examples of the lower alkenyl, cycloalkyl, aryl, aliphatic heterocyclic group and aromatic heterocyclic group described above, respectively. Examples of the $C_{7-15}$ aralkyl and the aralkyl moiety of the $C_{7-15}$ aralkyloxy and $C_{7-15}$ aralkyloxycarbonyl include benzyl, phenethyl, 3-phenylpropyl, 4-phenylbutyl, 5-phenylpentyl, 6-phenylhexyl, naphthylmethyl, naphthylethyl, anthranilmethyl and the like.

In addition, the two alkyl moieties in the di-$C_{1-10}$ alkylcarbamoyl, (di-$C_{1-10}$ alkylamino)-substituted $C_{1-10}$ alkoxy

and (di-$C_{1-10}$ alkylamino)-substituted $C_{1-10}$ alkyl may be the same or different.

The pharmaceutically acceptable salts of Compounds (I) and (II) include, for example, acid addition salts, metal salts, ammonium salts, organic amine addition salts, amino acid addition salts and the like that are pharmaceutically acceptable. Examples of the pharmaceutically acceptable acid addition salts of Compounds (I) and (II) include inorganic acid salts such as hydrochloride, hydrobromate, sulfate and phosphate; organic acid salts such as acetate, tartrate, gluconate, lactate, malate, oxalate, maleate, fumarate, citrate, benzoate, benzenesulfonate and methanesulfonate, and the like; and the like. Examples of the pharmaceutically acceptable metal salts include alkali metal salts such as sodium salts and potassium salts; alkaline earth metal salts such as magnesium salts and calcium salts; aluminum salts, zinc salts, and the like. Examples of the pharmaceutically acceptable ammonium salts include salts of ammonium, tetramethylammonium and the like. Examples of the pharmaceutically acceptable organic amine addition salts include addition salts of morpholine, piperidine and the like. Examples of the pharmaceutically acceptable amino acid addition salts include addition salts of lysine, glycine, phenylalanine, aspartic acid, glutamic acid and the like.

Next, a process for producing Compound (I) will be described.

Incidentally, in a production process described below, when the defined groups change under the conditions in the production process or are not suitable for carrying out the process, a subject compound can be produced by employing the methods of introducing or removing a protecting group which are generally used in synthetic organic chemistry, [for example, Protective Groups in Organic Synthesis, third edition, T. W. Greene, John Wiley & Sons Inc. (1999)] and the like. If necessary, the order of the reaction steps such as introduction of a substituent may be changed.

Compounds (I) can be produced according to the following steps.

Production process 1

Among the Compounds (I) in which $R^I$ is

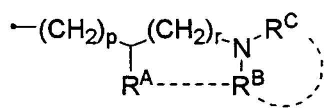

(wherein $R^A$, $R^B$, $R^C$, p and r have the same definitions as described above, respectively), and $R^J$ is

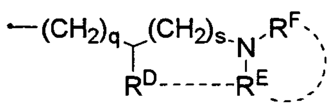

(wherein $R^D$, $R^E$, $R^F$, q and s have the same definitions as described above, respectively),

Compound (Ia-1) in which $R^G$ and $R^H$ may be the same or different, and each represents a hydrogen atom or substituted or unsubstituted lower alkyl can be produced according to the following steps.

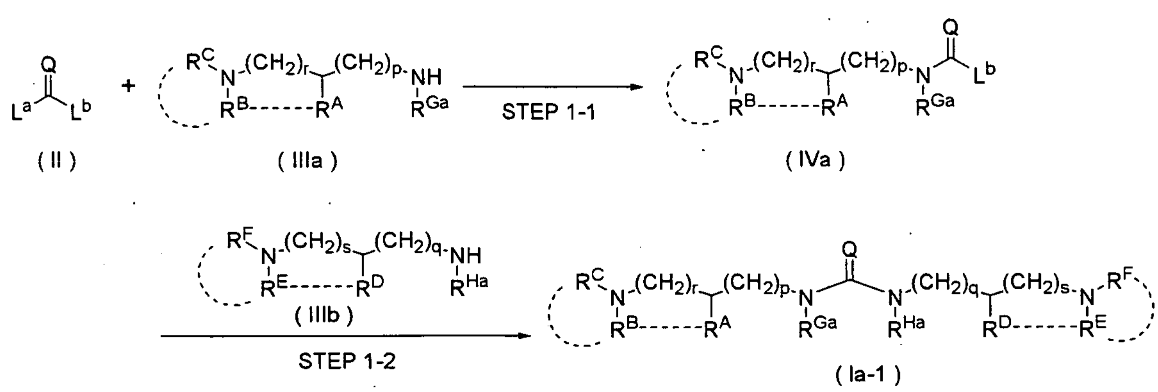


( II ) ( IIIa ) ( IVa ) ( IIIb ) ( Ia-1 )

[In the formulae, $L^a$ and $L^b$ may be the same or different, and each represents a leaving group, for example, a halogen atom such as a chlorine atom, a bromine atom or an iodine atom; an aromatic heterocyclic group such as imidazolyl,

triazolyl, tetrazolyl, benzoimidazolyl or benzotriazolyl; $C_{1-10}$ alkoxy such as methoxy, ethoxy, or propoxy; $C_{1-10}$ alkylthio (for example, methylthio, ethylthio, propylthio or the like) which may be substituted by a fluorine atom, a chlorine atom, a bromine atom, an iodine atom, hydroxy, nitro, aryl or the like; $C_{1-10}$ alkoxy (for example, methoxy, ethoxy, propoxy or the like) which may be substituted by a fluorine atom, a chlorine atom, a bromine atom, an iodine atom, hydroxy, nitro, methoxy, ethoxy, propoxy, isopropoxy, butoxy, phenyl or the like; $C_{6-14}$ aryloxy such as phenoxy which may be substituted by a fluorine atom, a chlorine atom, a bromine atom, an iodine atom, hydroxy, nitro, methoxy, ethoxy, propoxy, isopropoxy, butoxy or the like, $R^{Ga}$ and $R^{Ha}$ may be the same or different, and each represents a hydrogen atom or substituted or unsubstituted lower alkyl among the above-described definitions of $R^G$ and $R^H$, and $R^A$, $R^B$ $R^C$, $R^D$, $R^E$, $R^F$, p, q, r, s and Q have the same definitions as described above, respectively.]

Step 1-1

Compound (IVa) can be produced by reacting Compound (II) with 0.5 to 1.5 equivalents of, preferably 0.8 to 1.0 equivalent of Compound (IIIa) without solvent or in a suitable solvent at a temperature between -30˚C and 150˚C for 5 minutes to 72 hours.

Examples of the solvent include tetrahydrofuran (THF), 1,4-dioxane, N,N-dimethyl formamide (DMF), N,N-dimethyl acetamide (DMA), N-methylpyrrolidinone (NMP), dimethylsulfoxide (DMSO), methanol, ethanol, acetonitrile, dichloromethane, chloroform, 1,2-dichloroethane, ethyl acetate, toluene, pyridine and the like, and these can be used alone or as a mixture.

Compound (II) can be obtained, for example, as a commercially available product or in a similar manner to the method described in Example 136. Compound (IIIa) can be obtained, for example, as a commercially available product or in a similar manner to the method described in Jikken Kagaku Koza 4th edition, vol. 20, pp. 279-372, Maruzen Co., Ltd., (1992) or the like.

Step 1-2

Compound (Ia-1) can be produced by reacting Compound (IVa) obtained in Step 1-1 described above with preferably 1 equivalent to a large excess amount of Compound (IIIb) without solvent or in a suitable solvent at a temperature between 0˚C and 200˚C, preferably at a temperature between room temperature and 200˚C for 5 minutes to 130 hours.

Examples of the solvent include THF, 1,4-dioxane, DMF, DMA, NMP, DMSO, methanol, ethanol, acetonitrile, dichloromethane, chloroform, 1,2-dichloroethane, ethyl acetate, toluene, pyridine and the like, and these can be used alone or as a mixture.

Compound (IIIb) can be obtained, for example, as a commercially available product or in a similar manner to the method described in Jikken Kagaku Koza 4th edition, vol. 20, pp. 279-372, Maruzen Co., Ltd., (1992) or the like.

Production process 2

Among the Compounds (I) in which $R^I$ is

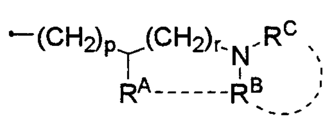

(wherein $R^A$, $R^B$, $R^C$, p and r have the same definitions as described above, respectively), and $R^J$ is

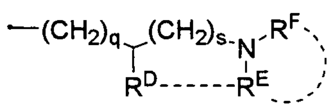

(wherein $R^D$, $R^E$ $R^F$, q and s have the same definitions as described above, respectively),

Compound (Ia-2) in which $R^G$ and $R^H$ are the same, and each is a hydrogen atom or substituted or unsubstituted lower alkyl, p and q, r and s, $R^A$ and $R^D$, $R^B$ and $R^E$, and $R^C$ and $R^F$ are the same, respectively, and Q has the same definition as described above can be produced in a similar manner the following step.

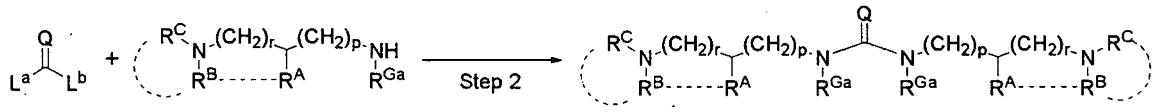


( II ) ( IIIa ) ( Ia-2 )

(In the formulae, $L^a$, $L^b$, $R^A$, $R^B$, $R^C$, $R^{Ga}$, p, r, and Q have the same definitions as described above, respectively.)

Compound (Ia-2) can be produced by reacting Compound (II) with preferably 2 equivalents to a large excess amount of Compound (IIIa) without solvent or in a suitable solvent at a temperature between 0°C and 200°C, preferably at a temperature between room temperature and 200°C for 5 minutes to 130 hours.

Examples of the solvent include THF, 1,4-dioxane, DMF, DMA, NMP, DMSO, methanol, ethanol, acetonitrile, dichloromethane, chloroform, 1,2-dichloroethane, ethyl acetate, toluene, pyridine and the like, and these can be used alone or as a mixture.

Production process 3

Among the Compounds (I) in which $R^I$ is

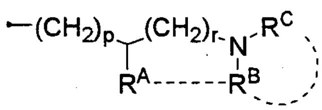

(wherein $R^A$, $R^B$, $R^C$, p and r have the same definitions as described above, respectively), and $R^J$ is

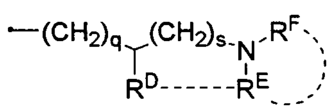

(wherein $R^D$, $R^E$, $R^F$, q and s have the same definitions as described above, respectively),

Compound (Ib-1) in which $R^G$ and $R^H$ are combined to represent substituted or unsubstituted alkylene or substituted or unsubstituted phenylene can be produced according to the following steps.

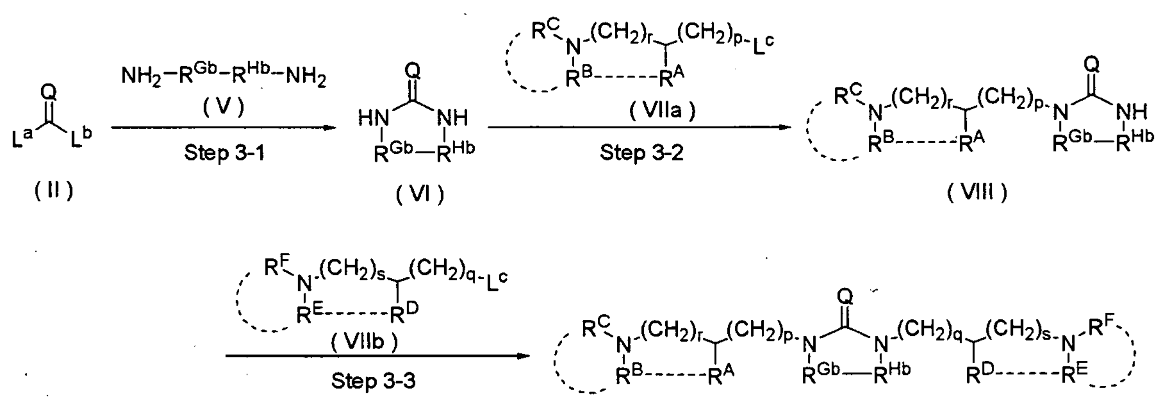


( II ) ( VI ) ( VIII )

( Ib-1 )

(In the formulae, $R^{Gb}$ and $R^{Hb}$ are combined to represent substituted or unsubstituted alkylene or substituted or unsubstituted phenylene as formed by combining $R^G$ and $R^H$ in the above-described definitions of $R^G$ and $R^H$, $L^C$ represents hydroxy or a leaving group such as a chlorine atom, a bromine atom, an iodine atom, methanesulfonyloxy, trifluoromethanesulfonyloxy, benzenesulfonyloxy or p-toluenesulfonyloxy, and $L^a$, $L^b$, $R^A$, $R^B$, $R^C$, $R^D$, $R^E$, $R^F$, p, q, r, s and Q have the same definitions as described above, respectively.)

Step 3-1

Compound (VI) can be produced by reacting Compound (II) with preferably 1 equivalent to a large excess amount of Compound (V) without solvent or in a suitable solvent at a temperature between -30˚C and 150˚C for 5 minutes to 130 hours.

Examples of the solvent include THF, 1,4-dioxane, DMF, DMA, NMP, DMSO, methanol, ethanol, acetonitrile, dichloromethane, chloroform, 1,2-dichloroethane, ethyl acetate, toluene, pyridine and the like, and these can be used alone or as a mixture.

Compound (V) can be obtained, for example, as a. commercially available product or in a similar manner to the method described in Jikken Kagaku Koza 4th edition, vol. 20, pp. 279-372, Maruzen Co., Ltd., (1992) or the like.

Step 3-2

When $L^c$ is a leaving group such as a chlorine atom, a bromine atom, an iodine atom, methanesulfonyloxy, trifluoromethanesulfonyloxy, benzenesulfonyloxy or p-toluenesulfonyloxy, Compound (VIII) can be produced by reacting Compound (VI) obtained in Step 3-1 described above with 0.5 to 1.5 equivalents of, preferably 0.8 to 1.0 equivalent of Compound (VIIa) in a suitable solvent, in the presence of preferably 1 to 10 equivalents of a suitable base, further, if necessary, in the presence of preferably a catalytic amount to 10 equivalents of an additive such as sodium bromide, potassium bromide, sodium iodide or potassium iodide at a temperature between -30˚C and the boiling point of a solvent to be used for 5 minutes to 72 hours.

Examples of the solvent include THF, 1,4-dioxane, DMF, DMA, NMP, DMSO, acetonitrile, dichloromethane, chloroform, 1,2-dichloroethane, ethyl acetate, toluene, pyridine and the like, and these can be used alone or as a mixture.

Examples of the base include potassium carbonate, sodium carbonate, potassium hydroxide, sodium hydroxide, sodium hydride, potassium hydride, potassium tert-butoxide, triethylamine, diisopropylamine, pyridine and the like.

When $L^c$ is hydroxy, Compound (VIII) can be produced by reacting Compound (VI) with preferably 1 to 50 equivalents of Compound (VIIa) in a suitable solvent, in the presence of preferably 1 to 50 equivalents of a phosphine compound and preferably 1 to 50 equivalents of an azo compound at a temperature between -78˚C and the boiling point of a solvent to be used for 5 minutes to 72 hours.

Examples of the phosphine compound include triphenylphosphine, tributylphosphine and the like.

Examples of the azo compound include diethyl azodicarboxylate (DEAD), di-tert-butylazadicarboxylate and the like.

As a combination of the phosphine compound and the azo compound to be used, for example, a combination of triphenylphosphine and DEAD and the like are preferred.

Examples of the solvent include dichloromethane, chloroform, 1,2-dichloroethane, toluene, ethyl acetate, acetonitrile, diethyl ether, THF, 1,2-dimethoxyethane, 1,4-dioxane, DMF, DMA, NMP, and the like, and these can be used alone or as a mixture.

Compound (VIIa) can be obtained, for example, as a commercially available product or in a similar manner to the method described in Jikken Kagaku Koza 4th edition, vol. 19, pp. 363-482, Maruzen Co., Ltd., (1992) or the like.

Step 3-3

Compound (Ib-1) can be produced in the same manner as in Step 3-2 described above by using Compound (VIII) obtained in Step 3-2 described above and Compound (VIIb). It is preferred that Compound (VIIb) is used in an amount of 1 to 30 equivalents at this time.

Compound (VIIb) can be obtained, for example, as a commercially available product or in a similar manner to the method described in Jikken Kagaku Koza 4th edition, vol. 19, pp. 363-482, Maruzen Co., Ltd., (1992) or the like.

Further, Compound (VIII) can also be produced according to the following steps.

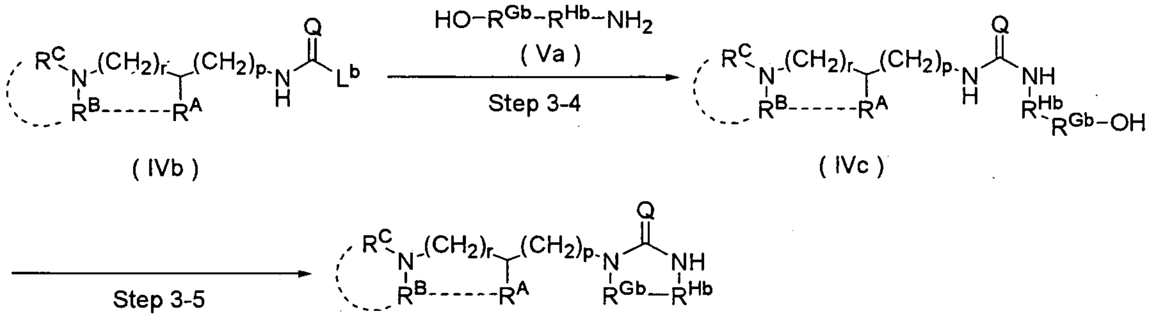


( IVb ) ( IVc ) ( VIII )

(In the formulae, $L^b$, $R^A$, $R^B$, $R^c$, $R^{Gb}$, $R^{Hb}$, p, r and Q have the same definitions as described above, respectively.)

Step 3-4

Compound (IVc) can be produced, for example, in the same manner as the method described in Japanese Published Unexamined Patent Application No. 1993-17471 or the like by using Compound (IVb) in which $R^{Ga}$ is a hydrogen atom among the Compounds (IVa) obtained in Step 1-1 of Production process 1 and Compound (Va).

Compound (Va) can be obtained, for example, as a commercially available product or according to the method described in Jikken Kagaku Koza 4th edition, vol. 20, pp. 1-110, pp. 279-372, Maruzen Co., Ltd., (1992) or the like.

Step 3-5

Compound (VIII) can be produced, for example, in the same manner as the method described in Japanese Published Unexamined Patent Application No. 1993-17471 or the like by using Compound (IVc) obtained in Step 3-4 described above.

Further, in the same manner as the method described in Japanese Published Unexamined Patent Application No. 1993-17471 or the like, Compound (VIII) can also be produced by isolating an intermediate in which a hydroxyl group of Compound (IVc) is converted into a leaving group such as sulfonate or halogen and then reacting the intermediate in the presence of preferably 1 to 20 equivalents of a base such as potassium carbonate, sodium carbonate, potassium tert-butoxide, sodium hydride or diazabicycloundecene at a temperature between room temperature and the boiling point of a solvent to be used for 5 minutes to 12 hours.

Production process 4

Among the Compounds (I) in which $R^I$ is

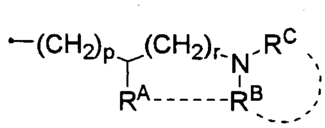

(wherein $R^A$, $R^B$, $R^C$, P and r have the same definitions as described above, respectively), and $R^J$ is

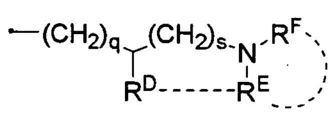

(wherein $R^D$, $R^E$, $R^F$, q and s have the same definitions as described above, respectively), Compound (Ib-2) in which $R^G$ and $R^H$ are combined to form substituted or unsubstituted alkylene or substituted or unsubstituted phenylene, p and q, r and s, $R^A$ and $R^D$, $R^B$ and $R^E$, and $R^C$ and $R^F$ are the same, respectively, and Q has the same definition as described above can be produced according to the following step.

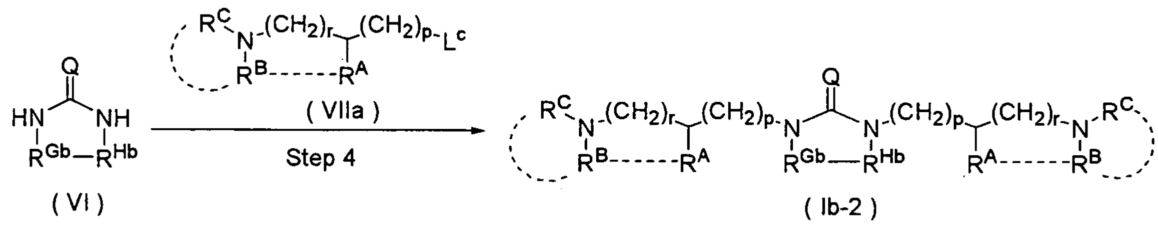


(VI) (VIIa) (Ib-2)

(In the formulae, $L^c$, $R^A$, $R^B$, $R^C$, $R^{Gb}$, $R^{Hb}$, p, r and Q have the same definitions as described above, respectively.)

Step 4

When $L^c$ is a leaving group such as a chlorine atom, a bromine atom, an iodine atom, methanesulfonyloxy, trifluoromethanesulfonyloxy, benzenesulfonyloxy or p-toluenesulfonyloxy, Compound (Ib-2) can be produced by reacting Compound (VI) obtained in Step 3-1 of Production process 3 described above with preferably 2 to 50 equivalents of Compound (VIIa) in a suitable solvent, in the presence of preferably 2 to 50 equivalents of a suitable base, further, if necessary, in the presence of preferably a catalytic amount to 10 equivalents of an additive such as sodium bromide, potassium bromide, sodium iodide or potassium iodide at a temperature between -30°C and the boiling point of a solvent to be used for 5 minutes to 130 hours.

Examples of the solvent include THF, 1,4-dioxane, DMF, DMA, NMP, DMSO, acetonitrile, dichloromethane, chloroform, 1,2-dichloroethane, ethyl acetate, toluene, pyridine and the like, and these can be used alone or as a mixture.

Examples of the base include potassium carbonate, sodium carbonate, potassium hydroxide, sodium hydroxide, sodium hydride, potassium hydride, potassium tert-butoxide, triethylamine, diisopropylamine, pyridine and the like.

When $L^c$ is hydroxy, Compound (Ib-2) can be produced by reacting Compound (VI) with preferably 2 to 50 equivalents of Compound (VIIa) in a suitable solvent, in the presence of preferably 2 to 50 equivalents of a phosphine compound and preferably 2 to 50 equivalents of an azo compound at a temperature between -78°C and the boiling point of a solvent to be used for 5 minutes to 72 hours.

Examples of the phosphine compound include triphenylphosphine, tributylphosphine and the like.

Examples of the azo compound include DEAD, di-tert-butylazadicarboxylate and the like.

As a combination of the phosphine compound and the azo compound to be used, for example, a combination of triphenylphosphine and DEAD and the like are preferred.

Examples of the solvent include dichloromethane, chloroform, 1,2-dichloroethane, toluene, ethyl acetate, acetonitrile, diethyl ether, THF, 1,2-dimethoxyethane, 1,4-dioxane, DMF, DMA, NMP, and the like, and these can be used alone or as a mixture.

Production process 5

Compound (Ib-1) can also be produced according to the following step.

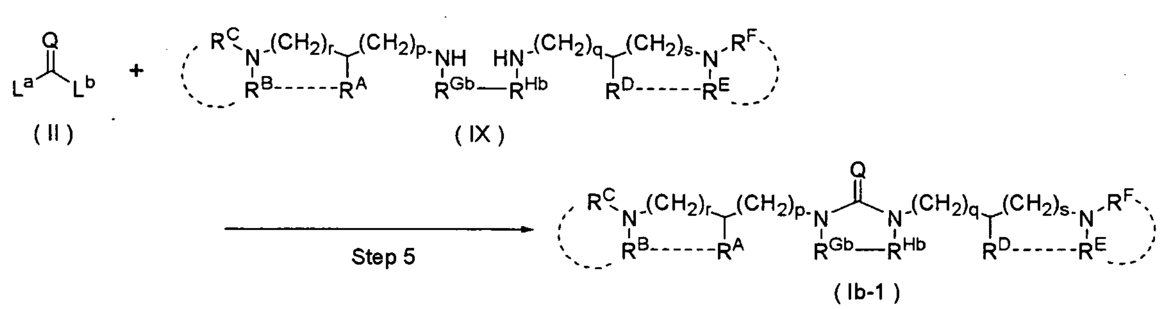


(II) (IX) (Ib-1)

(In the formulae, $L^a$, $L^b$, $R^A$, $R^B$, $R^C$, $R^D$, $R^E$, $R^F$, $R^{Gb}$, $R^{Hb}$, p, q, r, s and Q have the same definitions as described above, respectively.)

Step 5

Compound (Ib-1) can be produced in the same manner as in Step 3-1 of Production process 3 by using

Compound (II) and Compound (IX).

Compound (IX) can be obtained, for example, as a commercially available product or in a similar manner to the method described in German Patent No. 2628347 or the like, or the method described in Example 135 or the like.

Production process 6

Among the Compounds (I) in which $R^I$ is

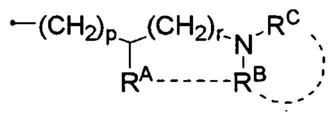

(wherein $R^A$, $R^B$, $R^C$, p and r have the same definitions as described above, respectively), and $R^J$ is

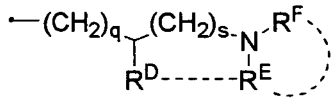

(wherein $R^D$, $R^E$, $R^F$, q and s have the same definitions as described above, respectively),

Compound (Ic-1) in which $R^A$ and $R^D$ are the same, and each represents a hydrogen atom or substituted or unsubstituted lower alkyl, $R^G$ and $R^H$ are combined to form substituted or unsubstituted alkylene or substituted or unsubstituted phenylene, $R^B$ and $R^C$ may be the same or different, and each represents a hydrogen atom, substituted or unsubstituted lower alkyl, substituted or unsubstituted lower alkenyl, substituted or unsubstituted lower alkynyl, substituted or unsubstituted cycloalkyl, substituted or unsubstituted lower alkanoyl, substituted or unsubstituted cycloalkylcarbonyl, substituted or unsubstituted lower alkoxycarbonyl, substituted or unsubstituted aroyl, substituted or unsubstituted aryl, a substituted or unsubstituted aliphatic heterocyclic group or a substituted or unsubstituted aromatic heterocyclic group, or $R^B$ and $R^c$ are combined together with the adjacent nitrogen atom thereto to form a substituted or unsubstituted nitrogen-containing heterocyclic group, $R^E$, $R^F$, p and r are the same as $R^E$, $R^c$, q and s, respectively, and have the same definitions as described above, respectively, and Q has the same definition as described above can be produced according to the following steps.

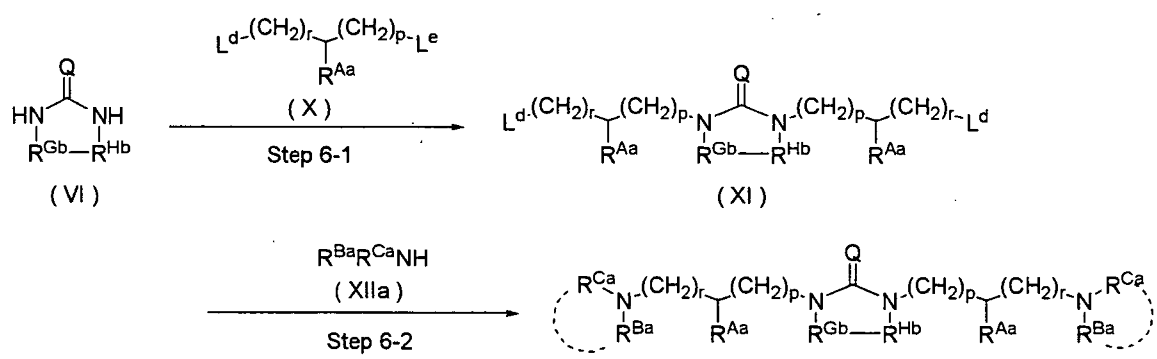


( VI ) ( XI ) ( Ic-1 )

(In the formulae, $L^d$ represents a leaving group such as a chlorine atom, a bromine atom, an iodine atom, methanesulfonyloxy, trifluoromethanesulfonyloxy, benzenesulfonyloxy or p-toluenesulfonyloxy, $L^e$ has the same definition as $L^c$ described above, $R^{Aa}$ represents a hydrogen atom or substituted or unsubstituted lower alkyl in the above-described definition of $R^A$, $R^{Ba}$ and $R^{Ca}$ may be the same or different, and each represents a hydrogen atom, substituted or unsubstituted lower alkyl, substituted or unsubstituted lower alkenyl, substituted or unsubstituted lower alkynyl, substituted or unsubstituted cycloalkyl, substituted or unsubstituted lower alkanoyl, substituted or unsubstituted cycloalkylcarbonyl, substituted or unsubstituted lower alkoxycarbonyl, substituted or unsubstituted aroyl, substituted or unsubstituted aryl, a substituted or unsubstituted aliphatic heterocyclic group or a substituted or unsubstituted aromatic heterocyclic group, or a substituted or unsubstituted nitrogen-containing heterocyclic group as formed by combining $R^B$ and

$R^c$ together with the adjacent nitrogen atom thereto in the above-described definition of $R^B$ and $R^C$, and $R^{Gb}$, $R^{Hb}$, p, r and Q have the same definitions as described above, respectively.)

Step 6-1

When $L^e$ is a leaving group such as a chlorine atom, a bromine atom, an iodine atom, methanesulfonyloxy, trifluoromethanesulfonyloxy, benzenesulfonyloxy or p-toluenesulfonyloxy, Compound (XI) can be produced by reacting Compound (VI) obtained in Step 3-1 of Production process 3 with preferably 2 to 50 equivalents of Compound (X) in a suitable solvent, in the presence of preferably 2 to 50 equivalents of a suitable base, further, if necessary, in the presence of preferably a catalytic amount to 10 equivalents of an additive such as sodium bromide, potassium bromide, sodium iodide or potassium iodide at a temperature between -30˚C and the boiling point of a solvent to be used for 5 minutes to 100 hours.

Examples of the solvent include THF, 1,4-dioxane, DMF, DMA, NMP, DMSO, acetonitrile, dichloromethane, chloroform, 1,2-dichloroethane, ethyl acetate, toluene, pyridine and the like, and these can be used alone or as a mixture.

Examples of the base include potassium carbonate, sodium carbonate, potassium hydroxide, sodium hydroxide, sodium hydride, potassium hydride, potassium tert-butoxide, triethylamine, diisopropylethylamine, N-methylmorpholine, pyridine and the like.

When $L^e$ is hydroxy, Compound (XI) can be produced by reacting Compound (VI) obtained in Step 3-1 of Production process 3 with preferably 2 to 50 equivalents of Compound (X) in a suitable solvent, in the presence of preferably 2 to 50 equivalents of a phosphine compound and preferably 2 to 50 equivalents of an azo compound at a temperature between -78˚C and the boiling point of a solvent to be used for 5 minutes to 72 hours.

Examples of the phosphine compound include triphenylphosphine, tributylphosphine and the like.

Examples of the azo compound include DEAD, di-tert-butylazadicarboxylate and the like.

As a combination of the phosphine compound and the azo compound to be used, for example, a combination of triphenylphosphine and DEAD and the like are preferred.

Examples of the solvent include dichloromethane, chloroform, 1,2-dichloroethane, toluene, ethyl acetate, acetonitrile, diethyl ether, THF, 1,2-dimethoxyethane, 1,4-dioxane, DMF, DMA, NMP, and the like, and these can be used alone or as a mixture.

Compound (X) can be obtained, for example, as a commercially available product or in a similar manner to the method described in Jikken Kagaku Koza 4th edition, vol. 19, pp. 363-482, Maruzen Co., Ltd., (1992) or the like.

Step 6-2

Compound (Ic-1) can be produced by reacting Compound (XI) obtained in Step 6-1 described above with preferably 2 equivalents to a large excess amount of Compound (XIIa) without solvent or in a suitable solvent, and if necessary, in the presence of preferably 2 to 10 equivalents of a suitable base at a temperature between -30˚C and 150˚C for 5 minutes to 100 hours.

Examples of the solvent include THF, 1,4-dioxane, DMF, DMA, NMP, DMSO, acetonitrile, dichloromethane, chloroform, 1,2-dichloroethane, ethyl acetate, toluene, pyridine and the like, and these can be used alone or as a mixture.

Examples of the base include potassium carbonate, sodium carbonate, potassium hydroxide, sodium hydroxide, sodium hydride, potassium hydride, potassium tert-butoxide, triethylamine, diisopropylethylamine, N-methylmorpholine, pyridine and the like.

Compound (XIIa) can be obtained, for example, as a commercially available product or according to the method described in Jikken Kagaku Koza 4th edition, vol. 20, pp. 279-372, Maruzen Co., Ltd., (1992) or the like.

Production process 7

Among the Compounds (I) in which $R^I$ is

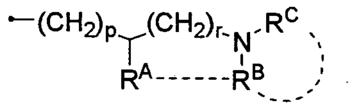

(wherein $R^A$, $R^B$, $R^C$, p and r have the same definitions as described above, respectively), and $R^J$ is

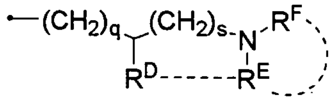

(wherein $R^D$, $R^E$, $R^F$, q and s have the same definitions as described above, respectively),

Compound (Ic-2) in which $R^A$ and $R^D$ may be the same or different, and each represents a, hydrogen atom or substituted or unsubstituted lower alkyl, $R^G$ and $R^H$ are combined to form substituted or unsubstituted alkylene or substituted or unsubstituted phenylene, $R^B$, $R^C$, $R^E$ and $R^F$ may be the same or different, and each represents a hydrogen atom, substituted or unsubstituted lower alkyl, substituted or unsubstituted lower alkenyl, substituted or unsubstituted lower alkynyl, substituted or unsubstituted cycloalkyl, substituted or unsubstituted lower alkanoyl, substituted or unsubstituted cycloalkylcarbonyl, substituted or unsubstituted lower alkoxycarbonyl, substituted or unsubstituted aroyl, substituted or unsubstituted aryl, a substituted or unsubstituted aliphatic heterocyclic group or a substituted or unsubstituted aromatic heterocyclic group, or $R^B$ and $R^C$ and/or $R^E$ and $R^F$ are combined together with the adjacent nitrogen atom thereto to form a substituted or unsubstituted nitrogen-containing heterocyclic group, respectively, and p, q, r, s and Q have the same definitions as described above, respectively can be produced according to the following steps.

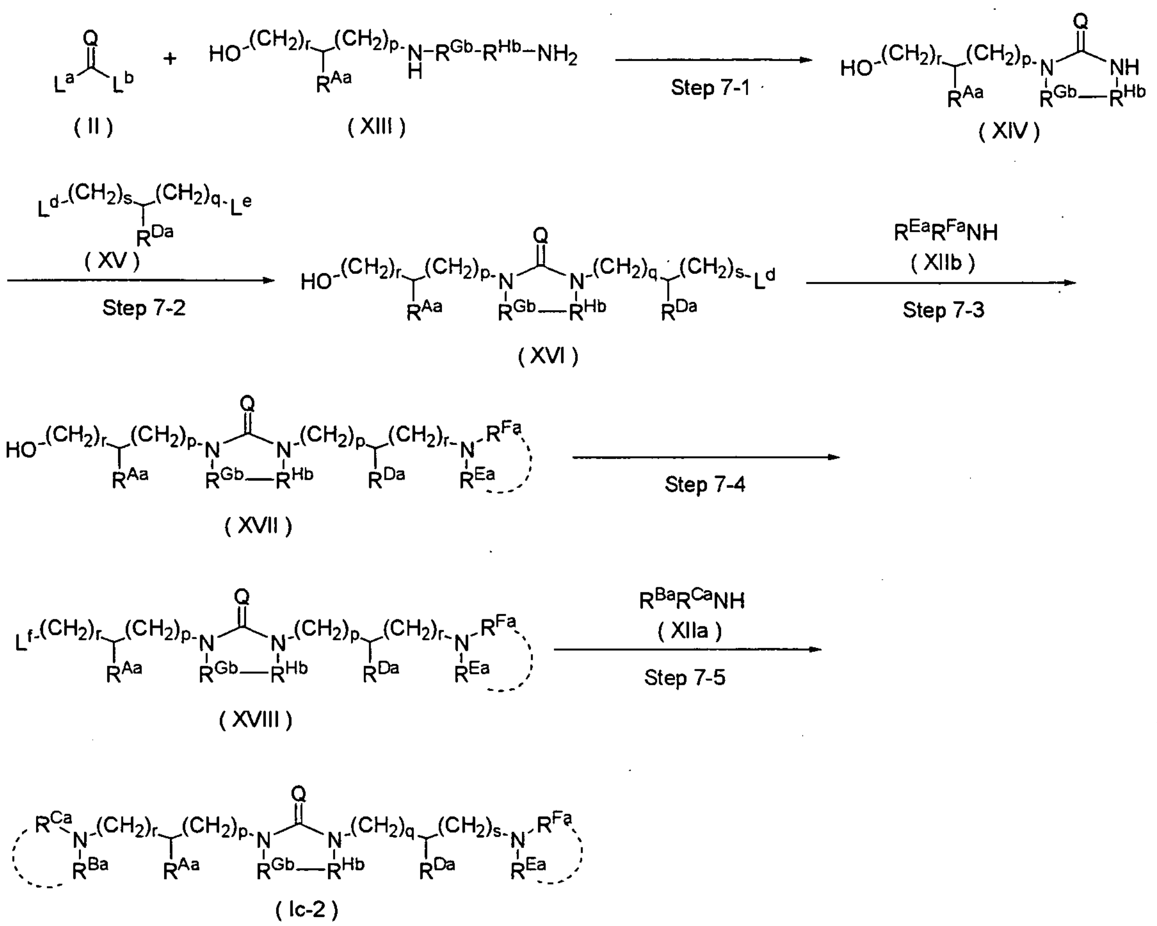


( II ) ( XIII ) ( XIV ) ( XV ) ( XVI ) ( XIIb ) ( XVII ) ( XVIII ) ( XIIa ) ( Ic-2 )

(In the formulae, $R^{Ba}$, $R^{Ca}$, $R^{Ea}$ and $R^{Fa}$ may be the same or different, and each represents a hydrogen atom, substituted or unsubstituted lower alkyl, substituted or unsubstituted lower alkenyl, substituted or unsubstituted lower alkynyl, substituted or unsubstituted cycloalkyl, substituted or unsubstituted lower alkanoyl, substituted or unsubstituted cycloalkylcarbonyl, substituted or unsubstituted lower alkoxycarbonyl, substituted or unsubstituted aroyl, substituted or unsubstituted aryl, a substituted or unsubstituted aliphatic heterocyclic group or a substituted or unsubstituted aromatic heterocyclic group in the above-described definitions of $R^B$, $R^C$, $R^E$ and $R^F$, or a substituted or unsubstituted nitrogen-containing heterocyclic group as formed by combining $R^B$ and $R^C$ and/or $R^E$ and $R^F$ together with the adjacent nitrogen atom thereto, respectively, $R^{Da}$ represents a hydrogen atom or substituted or unsubstituted lower alkyl in the above-described definition of $R^D$, $L^f$ has the same definition as $L^d$ described above, $R^{Aa}$, $R^{Gb}$, $R^{Hb}$, $L^a$, $L^b$, $L^d$, $L^e$, p, q, r, s and

Q have the dame definitions as described above, respectively.)

Step 7-1

Compound (XIV) can be produced in the same manner as in Step 3-1 of Production process 3 by using Compound (II) and Compound (XIII).

Compound (XIII) can be obtained, for example, as a commercially available product or in a similar manner to the method described in J. Am. Chem. Soc., vol. 72, pp. 1814-1815 (1950) or the like.

Step 7-2

Compound (XVI) can be produced in the same manner as in Step 6-1 of Production process 6 by using Compound (XIV) obtained in Step 7-1 described above and Compound (XV). It is preferred that Compound (XV) is used in an amount of 1 to 50 equivalents at this time.

Compound (XV) can be obtained, for example, as a commercially available product or in a similar manner to the method described in Jikken Kagaku Koza 4th edition, vol. 19, pp. 363-482, Maruzen Co., Ltd., (1992) or the like.

Step 7-3

Compound (XVII) can be produced in the same manner as in Step 6-2 of Production process 6 by using Compound (XVI) obtained in Step 7-2 described above and Compound (XIIb). It is preferred that Compound (XIIb) is used in an amount of 1 equivalent to a large excess amount at this time.

Compound (XIIb) can be obtained, for example, as a commercially available product or in a similar manner to the method described in Jikken Kagaku Koza 4th edition, vol. 20, pp. 279-372, Maruzen Co., Ltd., (1992) or the like.

Step 7-4

Compound (XVIII) can be produced in a similar manner to, for example, the method described in Jikken Kagaku Koza 4th edition, vol. 19, pp. 363-482, Maruzen Co., Ltd., (1992) or the like by using Compound (XVII) obtained in Step 7-3 described above.

Step 7-5

Compound (Ic-2) can be produced in the same manner as in Step 6-2 in Production process 6 by using Compound (XVIII) obtained in Step 7-4 described above and Compound (XIIa). It is preferred that Compound (XIIa) is used in an amount of 1 equivalent to a large excess amount at this time.

Production process 8

Among the Compounds (I) in which $R^I$ is

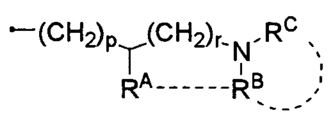

(wherein $R^A$, $R^B$, $R^C$, p and r have the same definitions as described above, respectively), and $R^J$ is

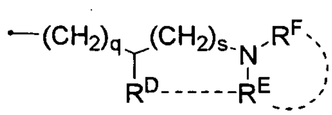

(wherein $R^D$, $R^E$, $R^F$, q and s have the same definitions as described above, respectively),

Compound (Ic-3) in which $R^A$ and $R^D$ may be the same or different, and each represents a hydrogen atom or substituted or unsubstituted lower alkyl, $R^G$ and $R^H$ are combined to form substituted or unsubstituted alkylene or

substituted or unsubstituted phenylene, $R^B$ and $R^C$ may be the same or different, and each represents a hydrogen atom, substituted or unsubstituted lower alkyl, substituted or unsubstituted lower alkenyl, substituted or unsubstituted lower alkynyl, substituted or unsubstituted cycloalkyl, substituted or unsubstituted lower alkanoyl, substituted or unsubstituted cycloalkylcarbonyl, substituted or unsubstituted lower alkoxycarbonyl, substituted or unsubstituted aroyl, substituted or unsubstituted aryl, a substituted or unsubstituted aliphatic heterocyclic group or a substituted or unsubstituted aromatic heterocyclic group, or $R^B$ and $R^C$ are combined together with the adjacent nitrogen atom thereto to form a substituted or unsubstituted nitrogen-containing heterocyclic group, $R^E$ and $R^F$ are the same as $R^B$ and $R^C$, respectively, and p, q, r, s and Q have the same definitions as described above, respectively, can be produced according to the following steps.

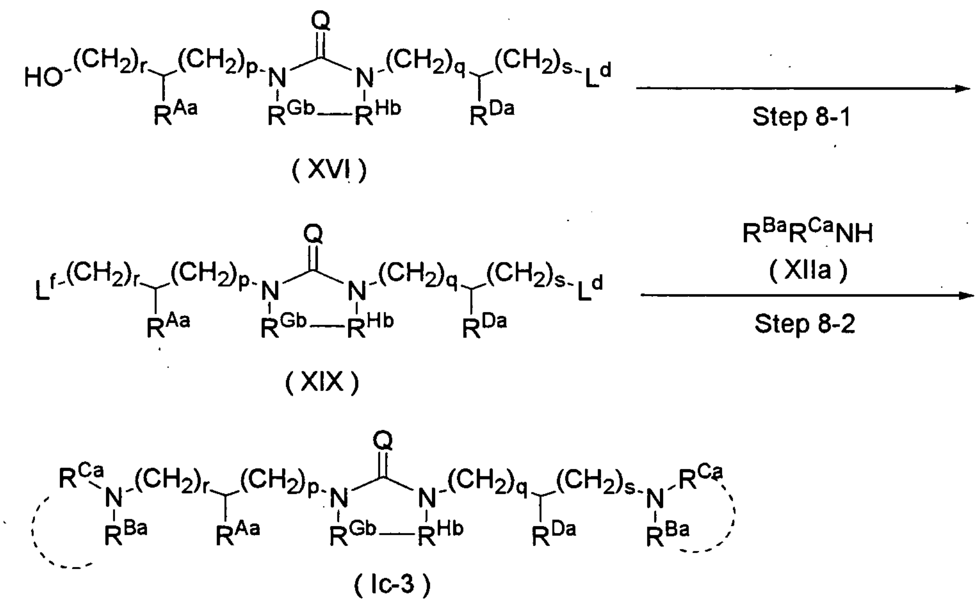


( XVI )

( XIX )

( Ic-3 )

(In the formulae, $R^{Aa}$, $R^{Ba}$, $R^{Ca}$, $R^{Da}$, $R^{Gb}$, $R^{Hb}$, $L^d$, $L^f$, p, q, r, s and Q have the same definitions as described above, respectively.)

Step 8-1

Compound (XIX) can be produced in a similar manner to, for example, the method described in Jikken Kagaku Koza 4th edition, vol. 19, pp. 363-482, Maruzen Co., Ltd., (1992) or the like by using Compound (XVI) obtained in Step 7-2 of Production process 7.

Step 8-2

Compound (Ic-3) can be produced in the same manner as in Step 6-2 in Production process 6 by using Compound (XIX) obtained in Step 8-1 described above and Compound (XIIa). It is preferred that Compound (XIIa) is used in an amount of 2 to 50 equivalents at this time.

Production process 9

Among the Compounds (I), Compound (Id) in which $R^D$ is a hydrogen atom or substituted or unsubstituted lower alkyl, $R^G$ and $R^H$ are combined to form substituted or unsubstituted alkylene or substituted or unsubstituted phenylene, $R^E$ and $R^F$ may be the same or different, and each represents a hydrogen atom, substituted or unsubstituted lower alkyl, substituted or unsubstituted lower alkenyl, substituted or unsubstituted lower alkynyl, substituted or unsubstituted cycloalkyl, substituted or unsubstituted lower alkanoyl, substituted or unsubstituted cycloalkylcarbonyl, substituted or unsubstituted lower alkoxycarbonyl, substituted or unsubstituted aroyl, substituted or unsubstituted aryl, a substituted or unsubstituted aliphatic heterocyclic group or a substituted or unsubstituted aromatic heterocyclic group, or $R^E$ and $R^F$ are combined together with the adjacent nitrogen atom thereto to form a substituted or unsubstituted nitrogen-containing heterocyclic group, and $R^A$, $R^B$, $R^C$, p, q, r, s and Q have the same definitions as described above, respectively, can be produced according to the following steps.

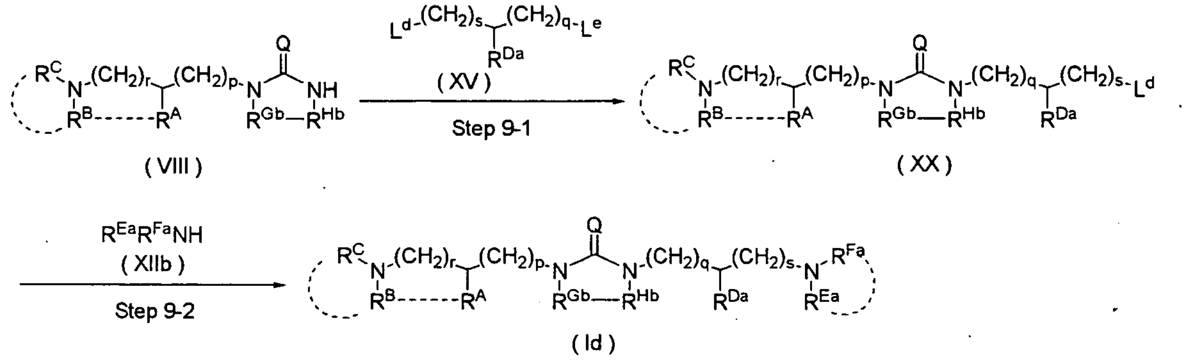


(In the formulae, $R^A$, $R^B$, $R^C$, $R^{Da}$, $R^{Ea}$, $R^{Fa}$, $R^{Gb}$, $R^{Hb}$ $L^d$, $L^e$, p, q, r, s and Q have the same definitions as described above, respectively.)

Step 9-1

Compound (XX) can be produced in the same manner as in Step 6-1 of Production process 6 by using Compound (VIII) obtained in Step 3-2 of Production process 3 and Compound (XV). It is preferred that Compound (XV) is used in an amount of 1 to 50 equivalents at this time.

Step 9-2

Compound (Id) can be produced in the same manner as in Step 6-2 of Production process 6 by using Compound (XX) obtained in Step 9-1 described above and Compound (XIIb). It is preferred that Compound (XIIb) is used in an amount of 1 equivalent to a large excess amount at this time.

Production process 10

Among the Compounds (I), Compound (Ie) in which $R^G$ is a hydrogen atom or substituted or unsubstituted lower alkyl, $R^H$ and $R^J$ are combined to form $-(CH_2)_t-X-(CH_2)_u-$(wherein t, u and X have the same definitions as described above, respectively) can be produced according to the following steps.

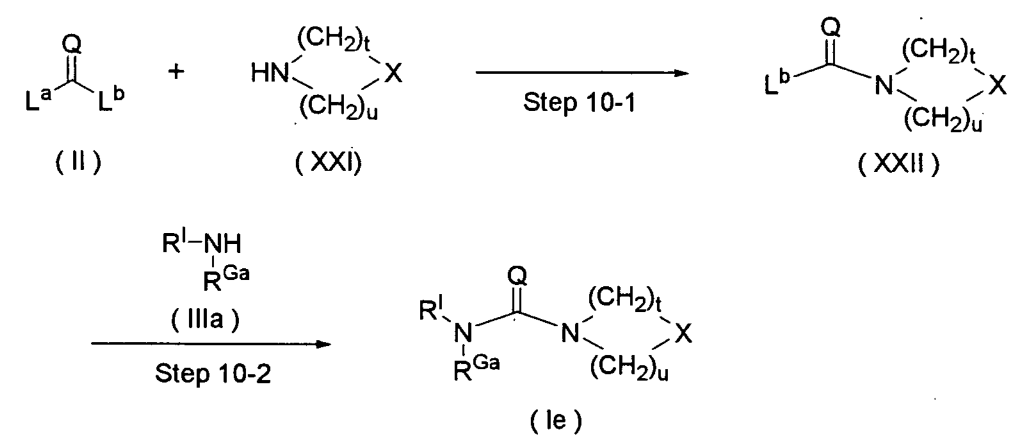


(In the formulae, Q, $L^a$, $L^b$, t, u, X, $R^I$ and $R^{Ga}$ have the same definitions as described above, respectively.)

Step 10-1

Compound (XXII) can be produced by reacting Compound (II) with preferably 1 to 1.5 equivalents of Compound (XXI) without solvent or in a suitable solvent, and if necessary, in the presence of preferably 1 to 10 equivalents of a base at a temperature between -30˚C and 150˚C for 5 minutes to 72 hours.

Examples of the solvent include THF, 1,4-dioxane, DMF, DMA, NMP, DMSO, methanol, ethanol, acetonitrile, dichloromethane, chloroform, 1,2-dichloroethane, ethyl acetate, toluene, pyridine and the like, and these can be used alone or as a mixture.

Examples of the base include potassium carbonate, sodium carbonate, potassium hydroxide, sodium hydroxide, triethylamine, diisopropylethylamine, pyridine and the like.

Compound (XXI) can be obtained, for example, as a commercially available product or in a similar manner to the method described in Reference example 1, 2, 3 or 6 or the like.

Step 10-2

Compound (Ie) can be produced by reacting Compound (XXII) obtained in Step 10-1 described above with preferably 1 equivalent to a large excess amount of Compound (IIIa) without solvent or in a suitable solvent at a temperature between 0˚C and 200˚C, and if necessary, in the presence of preferably 1 to 10 equivalents of a base at a temperature, preferably between room temperature and 200˚C for 5 minutes to 130 hours.

Examples of the solvent include THF, 1,4-dioxane, DMF, DMA, NMP, DMSO, methanol, ethanol, acetonitrile, dichloromethane, chloroform, 1,2-dichloroethane, ethyl acetate, toluene, pyridine and the like, and these can be used alone or as a mixture.

Examples of the base include potassium carbonate, sodium carbonate, potassium hydroxide, sodium hydride, triethylamine, diisopropylethylamine, pyridine and the like.

Compound (IIIa) can be obtained, for example, as a commercially available product or in a similar manner to the method described in Reference example 4, 5 or 7 or the like.

Further, Compound (Ie) can also be produced by carrying out Step 1 and Step 2 successively without isolating Compound (XXII).

Production process 11

Among the Compounds (Ie) in which $R^I$ is

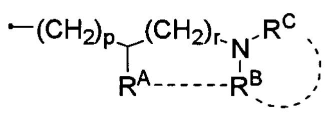

(wherein $R^A$, $R^B$, $R^C$, p and r have the same definitions as described above, respectively),

Compound (If) in which $R^A$ is a hydrogen atom or substituted or unsubstituted lower alkyl, $R^B$ and $R^C$ may be the same or different, and each represents a hydrogen atom, substituted or unsubstituted lower alkyl, substituted or unsubstituted lower alkenyl, substituted or unsubstituted lower alkynyl, substituted or unsubstituted cycloalkyl, substituted or unsubstituted lower alkanoyl, substituted or unsubstituted cycloalkylcarbonyl, substituted or unsubstituted lower alkoxycarbonyl, substituted or unsubstituted aroyl, substituted or unsubstituted aryl, a substituted or unsubstituted aliphatic heterocyclic group or a substituted or unsubstituted aromatic heterocyclic group, or $R^B$ and $R^C$ are combined together with the adjacent nitrogen atom thereto to form a substituted or unsubstituted nitrogen-containing heterocyclic group, can be produced according to the following steps.

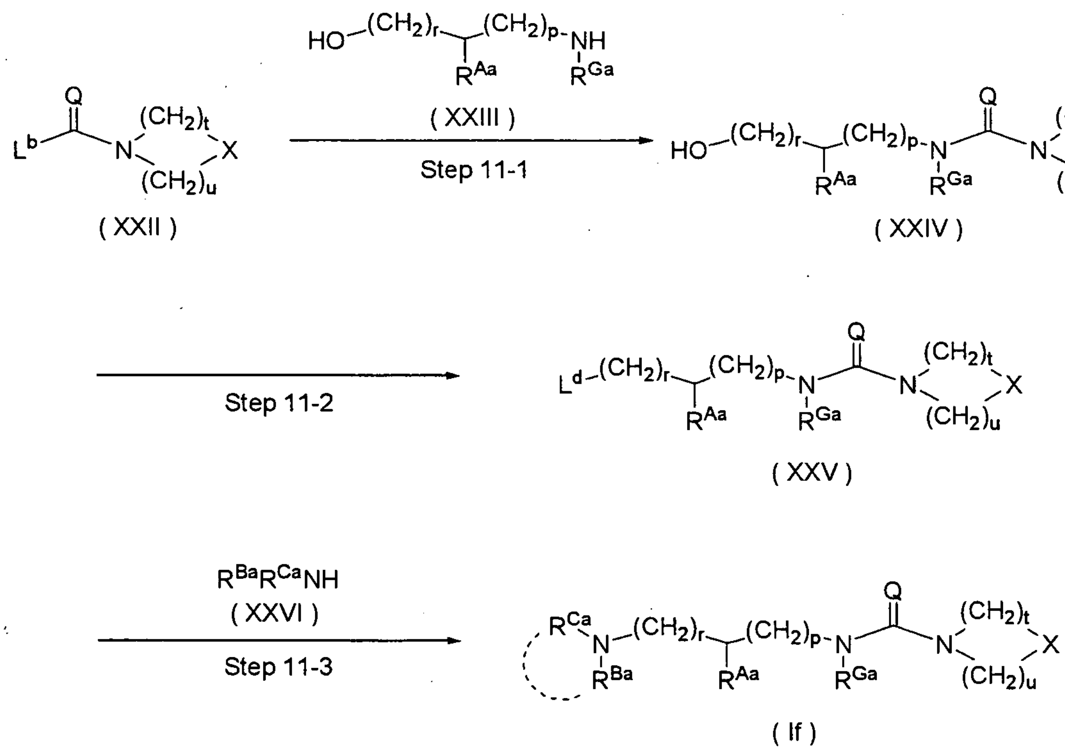


(XXII) (XXIV) (XXV) (If)

(In the formulae, Q, X, p, r, t, u, $L^b$, $L^d$, $R^{Aa}$, $R^Ba$, $R^{Ca}$ and $R^{Ga}$ have the same definitions as described above, respectively.)

Step 11-1

Compound (XXIV), can be produced in the same manner as in Step 10-2 of Production process 10 by using Compound (XXII) and Compound (XXIII).

Compound (XXIII) can be obtained, for example, as a commercially available product or in a similar manner to the method described in Jikken Kagaku Koza 4th edition, vol. 20, pp. 1-110, pp. 279-372, Maruzen Co., Ltd., (1992) or the like.

Step 11-2

Compound (XXV) can be obtained in a similar manner to, for example, the method described in J. Org. Chem., vol. 51, pp. 713-717, (1986) or the like or the method described in Jikken Kagaku Koza 4th edition, vol. 19, pp. 363-482, Maruzen Co., Ltd., (1992) or the like by using Compound (XXIV) obtained in Step 11-1 described above.

Step 11-3

Compound (If) can be produced by reacting Compound (XXV) obtained in Step 11-2 described above with preferably 1 equivalent to a large excess amount of Compound (XXVI) in a suitable solvent, and if necessary, in the presence of preferably 1 to 10 equivalents of a suitable base, further, if necessary, in the presence of preferably a catalytic amount to 10 equivalents of an additive such as sodium bromide, potassium bromide, sodium iodide or potassium iodide at a temperature between -30°C and the boiling point of a solvent to be used for 5 minutes to 100 hours.

Examples of the solvent include THF, 1,4-dioxane, DMF, DMA, NMP, DMSO, methanol, ethanol, acetonitrile, dichloromethane, chloroform, 1,2-dichloroethane, ethyl acetate, toluene, pyridine and the like, and these can be used alone or as a mixture.

Examples of the base include potassium carbonate, sodium carbonate, potassium hydroxide, sodium hydroxide, potassium hydride, potassium tert-butoxide, triethylamine, diisopropylethylamine, N-methylmorpholine, pyridine and the like.

Compound (XXVI) can be obtained, for example, as a commercially available product or in a similar manner to the method described in Jikken Kagaku Koza 4th edition, vol. 20, pp. 279-372, Maruzen Co., Ltd., (1992) or the like.

Production process 12

Among the Compounds (Ie), Compound (Ig) in which $R^{Ga}$ is substituted or unsubstituted lower alkyl can be produced according to the following step.

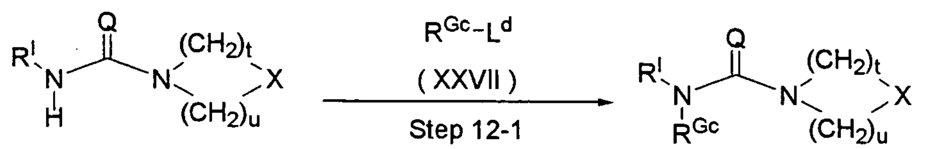


( Ih ) ( Ig )

(In the formulae, Q, t, u, X, $L^d$ and $R^I$ have the same definitions as described above, respectively, and $R^{Gc}$ represents substituted or unsubstituted lower alkyl in the above-described definition of $R^G$.)

Step 12-1

Compound (Ig) can be produced by reacting Compound (Ih) obtained in Step 10-2 of Production process 10 with preferably 1 equivalent to 10 equivalents of Compound (XXVII) in a suitable solvent, and if necessary, in the presence of preferably 1 to 10 equivalents of a suitable base at a temperature between -30˚C and the boiling point of a solvent to be used for 5 minutes to 100 hours.

Examples of the solvent include THF, 1,4-dioxane, DMF, DMA, NMP, DMSO, toluene, pyridine and the like, and these can be used alone or as a mixture.

Examples of the base include potassium carbonate, potassium hydroxide, potassium hydride, sodium hydride, potassium tert-butoxide, triethylamine, diisopropylethylamine, and the like.

Compound (XXVII) can be obtained, for example, as a commercially available product or in a similar manner to the method described in Jikken Kagaku Koza 4th edition, vol. 19, pp. 363-482, Maruzen Co., Ltd., (1992) or the like.

Production process 13

Compound (Id) can also be produced according to the following steps.

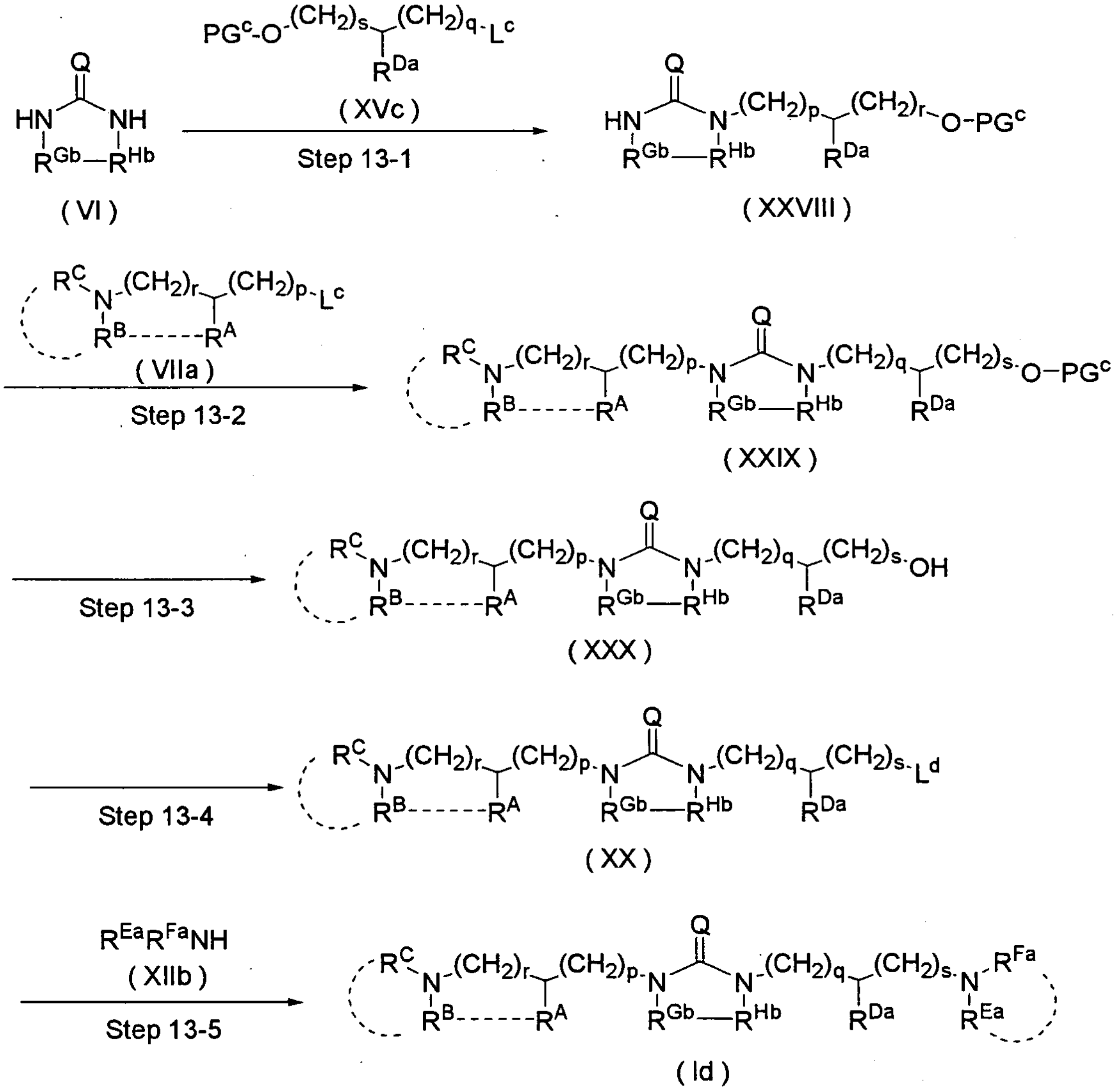


(VI) (XXVIII) (XXIX) (XXX) (XX) (Id)

(In the formulae, $PG^c$ represents a protecting group for a hydroxyl group which is stable under a basic condition such as a tert-butyldimethylsilyl group, a tert-butyldiphenylsilyl group or a tetrahydropyranyl group, and $R^A$, $R^B$ $R^C$, $R^{Da}$, $R^{Ea}$, $R^{Fa}$, $R^{Gb}$, $R^{Hb}$, $L^c$, $L^d$, p, q, r and s have the same definitions as described above, respectively.)

Step 13-1

Compound (XXVIII) can be produced in the same manner as in Step 3-2 of Production process 3 by reacting Compound (VI) obtained in Step 3-1 of Production process 3 with Compound (XVc).

Compound (XVc) can be obtained, for example, as a commercially available product or in a similar manner to the method described in Jikken Kagaku Koza 4th edition, vol. 19, pp. 363-482, Maruzen Co., Ltd., (1992); Protective Groups in Organic Synthesis, third edition, T. W. Greene, John Wiley & Sons Inc. (1999) or the like.

Step 13-2

Compound (XXIX) can be produced in the same manner as in Step 3-3 of Production process 3 by reacting Compound (XXVIII) obtained in Step 13-1 described above with Compound (VIIa).

Step 13-3

Compound (XXX) can be produced by removing a protecting group $PG^c$ for a hydroxyl group of Compound (XXIX) obtained in Step 13-2 described above in a similar manner to the method described in Protective Groups in

Organic Synthesis, third edition, T. W. Greene, John Wiley & Sons Inc. (1999) or the like.

Step 13-4

Compound (XX) can be produced, for example, in a similar manner to the method described in Jikken Kagaku Koza 4th edition, vol. 19, pp. 363-482, Maruzen Co., Ltd., (1992) or the like by using Compound (XXX) obtained in Step 13-3 described above.

Step 13-5

Compound (Id) can be produced in the same manner as in Step 9-2 of Production process 9.

Production process 14

Among the Compounds (I) in which $R^I$ is

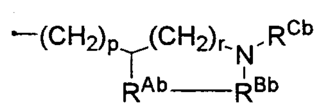

(wherein $R^{Ab}$ and $R^{Bb}$ are substituted or unsubstituted alkylene as formed by combining $R^A$ and $R^B$ in the above-described definitions of $R^A$ and $R^B$, $R^{Cb}$ represents substituted or unsubstituted lower alkyl, substituted or unsubstituted lower alkenyl, substituted or unsubstituted lower alkynyl, substituted or unsubstituted cycloalkyl, substituted or unsubstituted lower alkanoyl, substituted or unsubstituted cycloalkylcarbonyl, substituted or unsubstituted lower alkoxycarbonyl, substituted or unsubstituted aroyl, substituted or unsubstituted aryl, a substituted or unsubstituted aliphatic heterocyclic group or a substituted or unsubstituted aromatic heterocyclic group in the above-described definition of $R^C$, and p and r have the same definitions as described above, respectively), and $R^J$ is

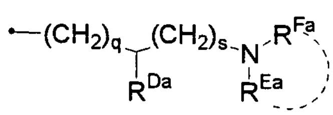

(wherein $R^{Da}$, $R^{Ea}$, $R^{Fa}$, q and s have the same definitions as described above, respectively),

Compound (Id-2) in which $R^G$ and $R^H$ are combined to form substituted or unsubstituted alkylene or substituted or unsubstituted phenylene can also be produced according to the following steps.

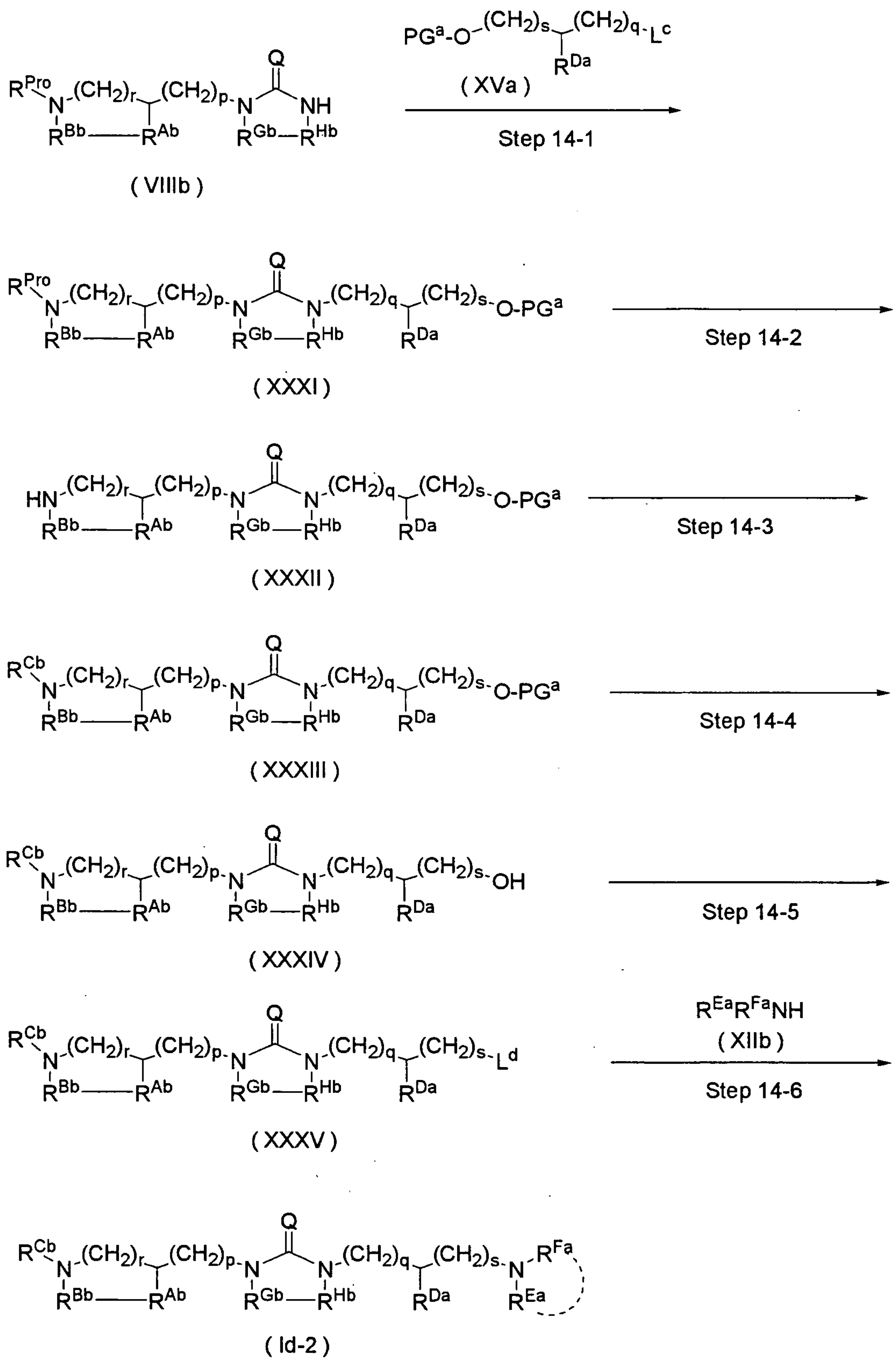


(VIIIb)

(XXXI)

(XXXII)

(XXXIII)

(XXXIV)

(XXXV)

(Id-2)

(In the formulae, $R^{Pro}$ represents a protecting group for an amino group such as a tert-butoxycarbonyl group or a benzyl group, $PG^a$ represents a protecting group for a hydroxyl group which is not removed under a condition in which $R^{Pro}$ which is a protecting group for an amino group is removed (for example, in the case where $R^{Pro}$ is a tert-butoxycarbonyl group, $PG^a$ represents a benzyl group, a 4-methoxybenzyl group, 3,4-dimethoxybenzyl group or the like), and $R^{Ab}$,

$R^{Bb}$, $R^{Cb}$, $R^{Da}$, $R^{Ea}$, $R^{Fa}$, $R^{Gb}$, $R^{Hb}$, $L^c$, $L^d$, p, q, r, s and Q have the same definitions as described above, respectively.)

Step 14-1

Compound (XXXI) can be produced in the same manner as in Step 3-3 of Production process 3 by reacting Compound (VIIIb) obtained by the method described in Step 3-2 or Step 3-5 of Production process 3 with Compound (XVa).

Compound (XVa) can be obtained, for example, as a commercially available product or in a similar manner to the method described in Jikken Kagaku Koza 4th edition, vol. 19, pp. 363-482, Maruzen Co., Ltd., (1992); Protective Groups in Organic Synthesis, third edition, T. W. Greene, John Wiley & Sons Inc. (1999) or the like.

Step 14-2

Compound (XXXII) can be produced by removing $R^{Pro}$ which is a protecting group for an amino group of Compound (XXXI) obtained in Step 14-1 described above under a condition in which $PG^a$ which is a protecting group for a hydroxyl group is not removed. As the method of removing $R^{Pro}$, for example, a known method can be used depending on the type of the protecting group, and specific examples thereof include the method described in Protective Groups in Organic Synthesis, third edition, T. W. Greene, John Wiley & Sons Inc. (1999) or the like and similar methods thereto. More specifically, for example, in the case where $R^{Pro}$ is a tert-butoxycarbonyl group and $PG^a$ is a benzyl group, Compound (XXXII) can be produced by treating Compound (XXXI) without solvent or in a suitable solvent, in the presence of preferably 1 equivalent to a large excess amount of a suitable acid, and if necessary, in the presence of preferably 1 equivalent to 5 equivalents of a cation trapping agent such as thiophenol or anisole at a temperature between -20˚C and the boiling point of a solvent to be used for 5 minutes to 72 hours.

Examples of the solvent at this time include methylene chloride, 1,2-dichloroethane, ethyl acetate, water and the like, and these can be used alone or as a mixture. Examples of the acid include hydrochloric acid, trifluoroacetic acid and the like.

Step 14-3

Compound (XXXIII) can be produced by reacting Compound (XXXII) obtained in Step 14-2 described above with 1 to 10 equivalents of $R^{Cb}$-$L^d$ (wherein $R^{Cb}$ and $L^d$ have the same definitions as described above, respectively) without solvent or in a suitable solvent, and if necessary, in the presence of preferably 1 to 10 equivalents of sodium iodide, potassium iodide or the like, and/or if necessary, in the presence of preferably 1 to 10 equivalents of a base at a temperature between -20˚C and 150˚C for 5 minutes to 120 hours.

Examples of the base include potassium carbonate, potassium hydroxide, sodium hydroxide, sodium methoxide, potassium tert-butoxide, triethylamine, diisopropylethylamine, N-methylmorpholine, pyridine, DBU and the like.

Examples of the solvent include methanol, ethanol, dichloromethane, chloroform, 1,2-dichloroethane, toluene, ethyl acetate, acetonitrile, diethyl ether, THF, DME, 1,4-dioxane, DMF, DMA, NMP, pyridine, water and the like, and these can be used alone or as a mixture.

Further, Compound (XXXIII) can also be produced by reacting Compound (XXXII) obtained in Step 14-2 described above with preferably 1 to 10 equivalents of a corresponding carbonyl compound or a equivalent thereof in a suitable solvent, in the presence of preferably 1 to 10 equivalents of a reducing agent and preferably 1 to 10 equivalents of an acid at a temperature between -20˚C and 150˚C for 5 minutes to 72 hours.

Examples of the reducing agent at this time include triacetoxy sodium borohydride, sodium borohydride cyanide and the like. Examples of the acid include hydrochloric acid, sulfuric acid, acetic acid, trifluoroacetic acid and the like. Examples of the solvent include methanol, ethanol, dichloromethane, chloroform, 1,2-dichloroethane, toluene, ethyl acetate, acetonitrile, diethyl ether, THF, DME, 1,4-dioxane, DMF, DMA, NMP, water and the like, and these can be used alone or as a mixture.

Step 14-4

Compound (XXXIV) can be produced by removing a protecting group $PG^a$ for a hydroxyl group of Compound (XXXIII) obtained in Step 14-3 described above. As the method of removing $RG^a$, for example, a known method can be used depending on the type of the protecting group, and specific examples thereof include the method described in Protective Groups in Organic Synthesis, third edition, T. W. Greene, John Wiley & Sons Inc. (1999) or the like and similar methods thereto. More specifically, for example, in the case where $PG^a$ is a benzyl group, Compound (XXXIV) can be produced by treating Compound (XXXIII) in a suitable solvent in a hydrogen atmosphere or in the presence of a suitable hydrogen source and in the presence of a suitable catalyst at a temperature between -20˚C and the boiling point of a

solvent to be used at normal pressure or under pressure for 5 minutes to 72 hours.

Examples of the catalyst at this time include palladium carbon, palladium, palladium hydroxide, palladium acetate, palladium black, platinum black and the like, and these can be used preferably in an amount of 0.01 to 50% by weight. Examples of the hydrogen source include formic acid, ammonium formate, sodium formate and the like, and these can be used preferably in an amount of 2 equivalents to a large excess amount. Examples of the solvent include methanol, ethanol, toluene, ethyl acetate, acetonitrile, diethyl ether, THF, DME, 1,4-dioxane, DMF, DMA, NMP, water and the like, and these can be used alone or as a mixture.

Further, in the case where $PG^a$ is a benzyl group, Compound (XXXIV) can also be produced by treating Compound (XXXIII) in a suitable solvent, in the presence of preferably a catalytic amount to 10 equivalents of a suitable Lewis acid such as boron trifluoride, boron trichloride, boron tribromide or tin tetrachloride at a temperature between -78°C and the boiling point of a solvent to be used for 5 minutes to 72 hours.

Examples of the solvent at this time include acetonitrile, diethyl ether, THF, DME, 1,4-dioxane, methylene chloride, 1,2-dichloroethane and the like, and these can be used alone or as a mixture.

Step 14-5

Compound (XXXV) can be produced in the same manner as in Step 7-4 of Production process 7 by using Compound (XXXIV) obtained in Step 14-4 described above, for example, in a similar manner to the method described in Jikken Kagaku Koza 4th edition, vol. 19, pp. 363-482, Maruzen Co., Ltd., (1992) or the like.

Step 14-6

Compound (Id-2) can be produced in the same manner as in Step 7-5 of Production process 7 by reacting Compound (XXXV) obtained in Step 14-5 described above with preferably 2 equivalents to a large excess amount of Compound (XIIb) without solvent or in a suitable solvent, and if necessary, in the presence of preferably 2 to 10 equivalents of a suitable base at a temperature between -30°C and 150°C for 5 minutes to 100 hours.

Examples of the solvent include THF, 1,4-dioxane, DMF, DMA, NMP, DMSO, acetonitrile, dichloromethane, chloroform, 1,2-dichloroethane, ethyl acetate, toluene, pyridine and the like, and these can be used alone or as a mixture.

Examples of the base include potassium carbonate, sodium carbonate, potassium hydroxide, sodium hydroxide, sodium hydride, potassium hydride, potassium tert-butoxide, triethylamine, diisopropylethylamine, N-methylmorpholine, pyridine and the like.

Compound (XIIb) can be obtained, for example, as a commercially available product or in a similar manner to the method described in Jikken Kagaku Koza 4th edition, vol. 20, pp. 279-372, Maruzen Co., Ltd., (1992) or the like.

Production process 15

Further, Compound (Id-2) can also be produced according to the following steps.

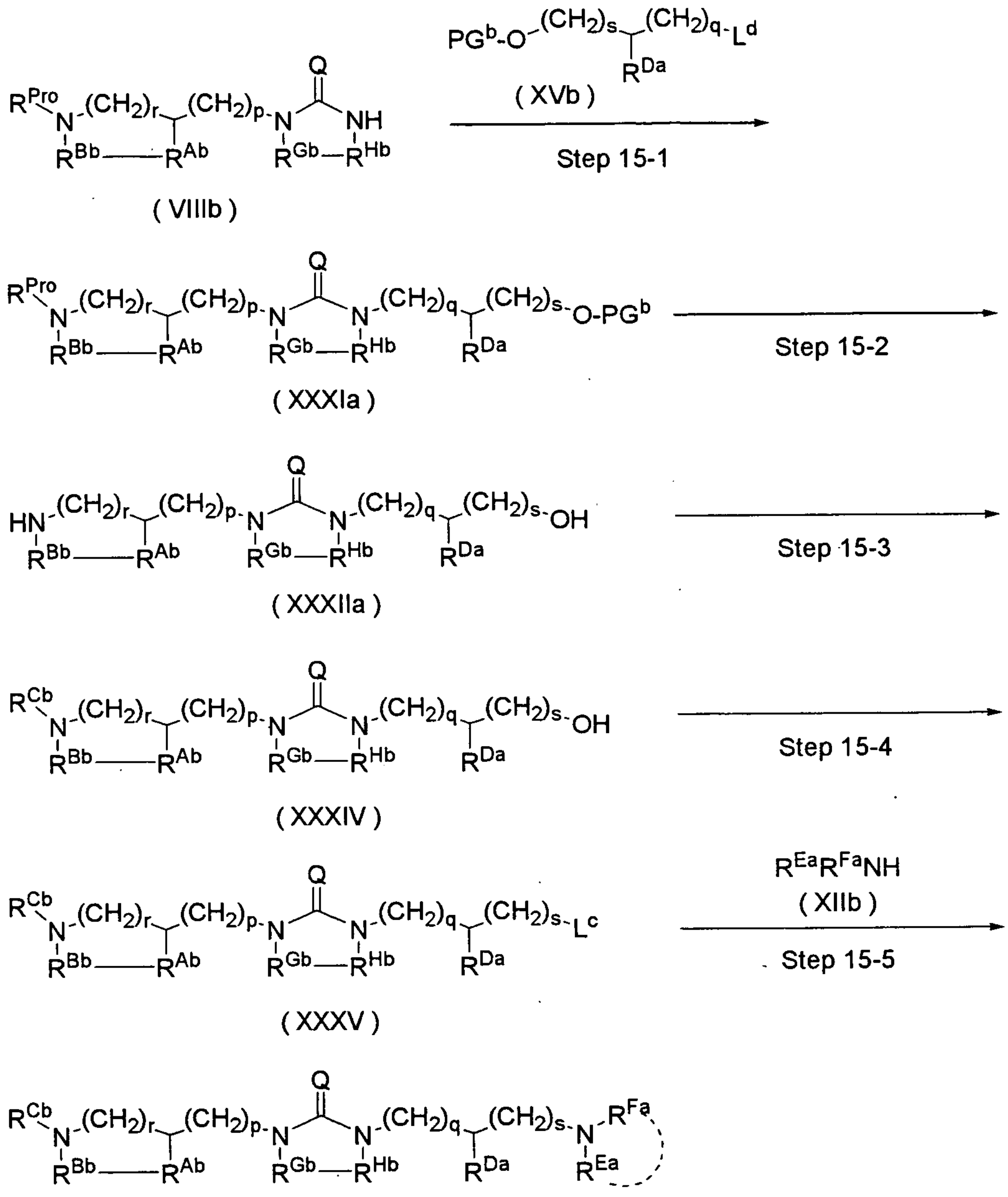


(VIIIb)

(XXXIa)

(XXXIIa)

(XXXIV)

(XXXV)

(Id-2)

(In the formulae, $PG^b$ represents a protecting group for a hydroxyl group which can be removed under a condition in which $R^{Pro}$ which is a protecting group for an amino group is removed (for example, $PG^b$ is the same as $R^{Pro}$, or in the case where $R^{Pro}$ is a tert-butoxycarbonyl group, $PG^b$ represents a tert-butyldimethylsilyl group or the like), and $R^{Ab}$, $R^{Bb}$, $R^{Cb}$, $R^{Da}$, $R^{Ea}$, $R^{Ea}$, $R^{Gb}$, $R^{Hb}$, $R^{Pro}$, $L^c$, $L^d$, p, q, r, s and Q have the same definitions as described above, respectively.)

Step 15-1

Compound (XXXIa) can be produced in the same manner as in Step 14-1 of Production process 14 by reacting Compound (VIIIb) with Compound (XVb).

Compound (XVb) can be obtained, for example, as a commercially available product or in a similar manner to the method described in Jikken Kagaku Koza 4th edition, vol. 19, pp. 363-482, Maruzen Co., Ltd., (1992); Protective Groups in Organic Synthesis, third edition, T. W. Greene, John Wiley & Sons Inc. (1999) or the like.

Step 15-2

Compound (XXXIIa) can be produced by removing a protecting group for an amino group, $R^{Cb}$ and a protecting group for a hydroxyl group, $PG^b$ of Compound (XXXIa) obtained in Step 15-1 described above. As the method of removing such protecting groups, for example, a suitable known method can be used depending on the type of $R^{Cb}$ and $PG^b$, and specific examples thereof include the method described in Protective Groups in Organic Synthesis, third edition, T. W. Greene, John Wiley & Sons Inc. (1999) or the like and similar methods thereto. More specifically, for example, in the case where $R^{Cb}$ is a tert-butoxycarbonyl group and $PG^b$ is a tert-butyldimethylsilyl group, Compound (XXXIIa) can be produced by treating Compound (XXXIa) without solvent or in a suitable solvent, in the presence of preferably 1 equivalent to a large excess amount of a suitable acid, and if necessary, in the presence of preferably 1 equivalent to 5 equivalents of a cation trapping agent such as thiophenol or anisole at a temperature between -20˚C and the boiling point of a solvent to be used for 5 minutes to 72 hours.

Examples of the solvent at this time include dichloromethane, 1,2-dichloroethane, ethyl acetate, water and the like, and these can be used alone or as a mixture. Examples of the acid include hydrochloric acid, trifluoroacetic acid and the like.

Step 15-3

Compound (XXXIV) can be produced in the same manner as in Step 14-3 of Production process 14 by using Compound (XXXIIa) obtained in Step 15-2 described above.

Step 15-4 and Step 15-5

Compound (Id-2) can be produced in the same manner as in Step 14-5 and Step 14-6 of Production process 14.

Conversion of a functional group contained in $R^A$, $R^E$, $R^C$, $R^D$, $R^E$, $R^F$, $R^G$, $R^H$ or Q in Compound (I) can be carried out by a known method [for example, the method described in Comprehensive Organic Transformations, R. C. Larock (1989) or the like], or a similar method thereto.

Further, by combining the above-described methods appropriately and carrying them out, Compound (I) having a desired functional group at a desired position can be obtained.

The intermediates and subject compounds in the respective production processes described above can be isolated and purified by carrying out an isolation and purification method commonly used in synthetic organic chemistry, for example, filtration, extraction, washing, drying, concentration, recrystallization, various types of chromatography or the like. The intermediates can also be used to the subsequent reactions without further purification.

Among the Compounds (I), there are also compounds which exist as stereoisomers such as tautomers, regioisomers, geometric isomers and optical isomers, and all possible isomers including these and mixtures thereof are included in the present invention.

When it is desired to obtain a salt of Compound (I) in the case where Compound (I) is obtained in the form of a salt, it can be purified as it is. Further, when it is obtained in a free form, it can be converted into a salt by dissolving or suspending Compound (I) in a suitable solvent, and then adding an acid or a base. Then, the resulting salt may be isolated and purified.

Further, Compound (I) or a pharmaceutically acceptable salt thereof may be present in the form of adducts with water or various solvents, and these adducts are also included in the diamine derivative or a pharmaceutically acceptable salt thereof of the present invention.

Specific examples of Compound (I) obtained according to the present invention are shown in Table 1 to Table 9. However, the compound of the present invention is not limited to these.

Table 1

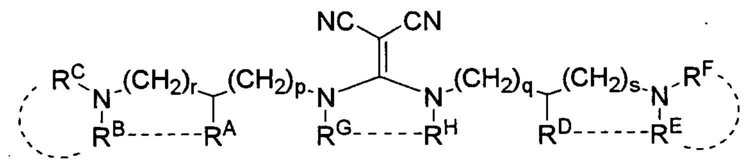

| Example No. | Compound No. | 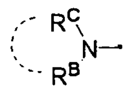 | r | $R^A$ | p | $R^G$ | $R^H$ | q | $R^D$ | s | 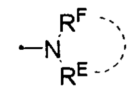 |
|---|---|---|---|---|---|---|---|---|---|---|---|
| 1 | 1 | 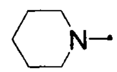 | 2 | H | 0 | H | H | 0 | H | 1 | 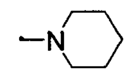 |
| 2 | 2 | 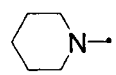 | 2 | H | 0 | H | H | 0 | H | 2 | 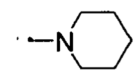 |
| 3 | 3 | 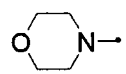 | 2 | H | 0 | H | H | 0 | H | 2 | 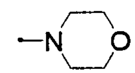 |

Table 2

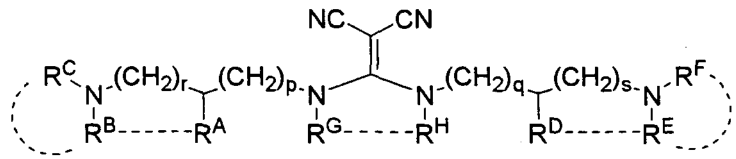

| Example No. | Compound No. | $R^C$ | $R^B$ | $R^A$ | r | p | $R^G$ | $R^H$ | q | s | $R^D$ | $R^E$ | $R^F$ |
|---|---|---|---|---|---|---|---|---|---|---|---|---|---|
| 4 | 4 | $CH_3$ | $-CH_2CH_2CH_2-$ | | 0 | 2 | $-CH_2CH_2-$ | | 2 | 0 | $-CH_2CH_2CH_2-$ | | $CH_3$ |

Table 3

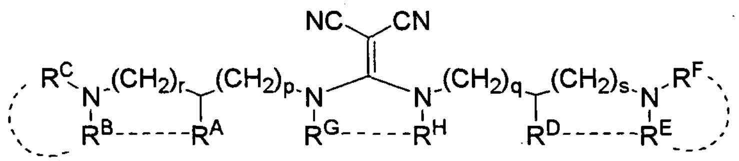

| Example No. | Compound No. | 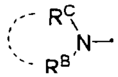 | r | $R^A$ | p | $R^G$ | $R^H$ | q | $R^D$ | s | 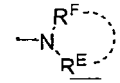 |
|---|---|---|---|---|---|---|---|---|---|---|---|
| 5 | 5 | 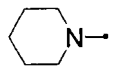 | 1 | H | 0 | $-CH_2CH_2-$ | | 0 | H | 1 | 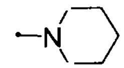 |
| 6 | 6 | 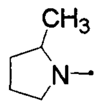 | 1 | H | 0 | $-CH_2CH_2-$ | | 0 | H | 1 | 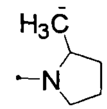 |
| 7 | 7 | 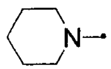 | 3 | H | 0 | $-CH_2CH_2-$ | | 0 | H | 1 | 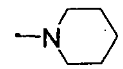 |
| 8 | 8 | 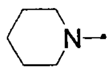 | 3 | H | 0 | $-CH_2CH_2-$ | | 0 | H | 2 | 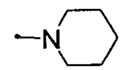 |
| 9 | 9 | 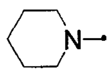 | 3 | H | 0 | $-CH_2CH_2-$ | | 0 | H | 3 | 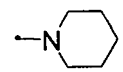 |
| 10 | 10 | 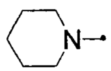 | 2 | H | 0 | $-CH_2CH_2-$ | | 0 | H | 1 | 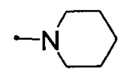 |
| 11 | 11 | 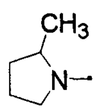 | 2 | H | 0 | $-CH_2CH_2-$ | | 0 | H | 1 | 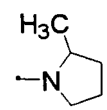 |

(continued)

| Example No. | Compound No. | 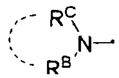 | r | $R^A$ | p | $R^G$ | $R^H$ | q | $R^D$ | s | 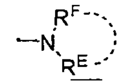 |
|---|---|---|---|---|---|---|---|---|---|---|---|
| 12 | 12 | 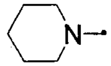 | 2 | H | 0 | $-CH_2CH_2-$ | | 0 | H | 1 | 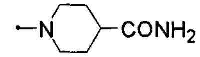 |
| 13 | 13 | 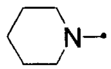 | 2 | H | 0 | $-CH_2CH_2-$ | | 0 | H | 1 | 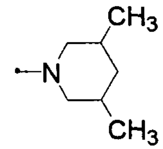 |
| 14 | 14 | 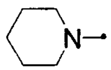 | 2 | H | 0 | $-CH_2CH_2-$ | | 0 | H | 1 | 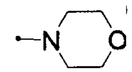 |
| 15 | 15 | 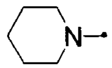 | 2 | H | 0 | $-CH_2CH_2-$ | | 0 | H | 1 | 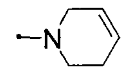 |
| 16 | 16 | 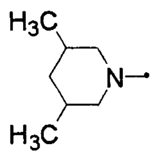 | 2 | H | 0 | $-CH_2CH_2-$ | | 0 | H | 1 | 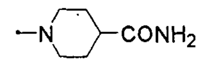 |
| 17 | 17 | 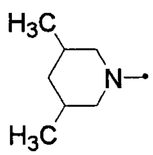 | 2 | H | 0 | $-CH_2CH_2-$ | | 0 | H | 1 | 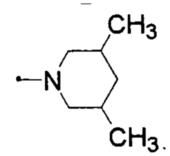 |
| 18 | 18 | 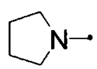 | 2 | H | 0 | $-CH_2CH_2-$ | | 0 | H | 1 | 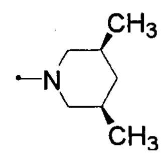 |

(continued)

| Example No. | Compound No. | 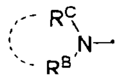 | r | $R^A$ | p | $R^G$ | $R^H$ | q | $R^D$ | s | 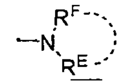 |
|---|---|---|---|---|---|---|---|---|---|---|---|
| 19 | 19 | 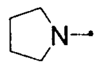 | 2 | H | 0 | $-CH_2CH_2-$ | | 0 | H | 1 | 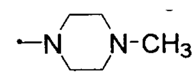 |
| 20 | 20 | 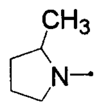 | 2 | H | 0 | $-CH_2CH_2-$ | | 0 | H | 1 | 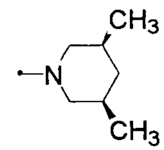 |
| 21 | 21 | 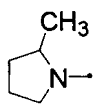 | 2 | H | 0 | $-CH_2CH_2-$ | | 0 | H | 1 | 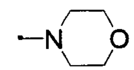 |
| 22 | 22 | 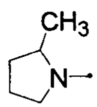 | 2 | H | 0 | $-CH_2CH_2-$ | | 0 | H | 1 | 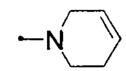 |
| 23 | 23 | 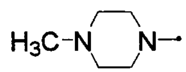 | 2 | H | 0 | $-CH_2CH_2-$ | | 0 | H | 1 | 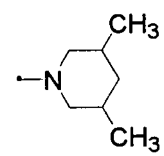 |
| 24 | 24 | 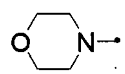 | 2 | H | 0 | $-CH_2CH_2-$ | | 0 | H | 1 | 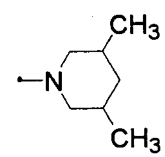 |

(continued)

| Example No. | Compound No. | 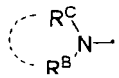 | r | $R^A$ | p | $R^G$ | $R^H$ | q | $R^D$ | s | 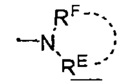 |
|---|---|---|---|---|---|---|---|---|---|---|---|
| 25 | 25 | 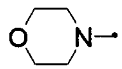 | 2 | H | 0 | $-CH_2CH_2-$ | | 0 | H | 1 | 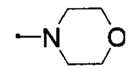 |
| 26 | 26 | 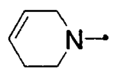 | 2 | H | 0 | $-CH_2CH_2-$ | | 0 | H | 1 | 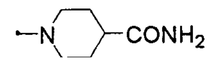 |
| 27 | 27 | 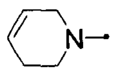 | 2 | H | 0 | $-CH_2CH_2-$ | | 0 | H | 1 | 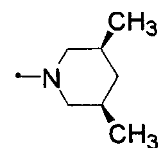 |
| 28 | 28 | 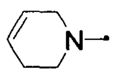 | 2 | H | 0 | $-CH_2CH_2-$ | | 0 | H | 1 | 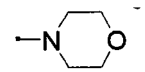 |
| 29 | 29 | 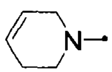 | 2 | H | 0 | $-CH_2CH_2-$ | | 0 | H | 1 | 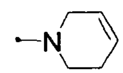 |
| 30 | 30 | 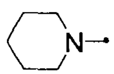 | 2 | H | 0 | $-CH_2CH_2-$ | | 0 | H | 1 | 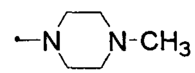 |
| 31 | 31 | 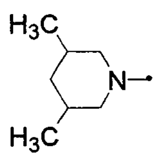 | 2 | H | 0 | $-CH_2CH_2-$ | | 0 | H | 1 | 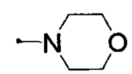 |
| 32 | 32 | 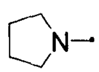 | 2 | H | 0 | $-CH_2CH_2-$ | | 0 | H | 1 | 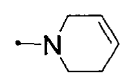 |

(continued)

| Example No. | Compound No. | 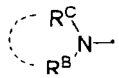 | r | $R^A$ | p | $R^G$ | $R^H$ | q | $R^D$ | s | 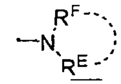 |
|---|---|---|---|---|---|---|---|---|---|---|---|
| 33 | 33 | 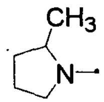 | 2 | H | 0 | $-CH_2CH_2-$ | | 0 | H | 1 | 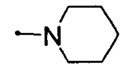 |
| 34 | 34 | 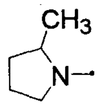 | 2 | H | 0 | $-CH_2CH_2-$ | | 0 | H | 1 | 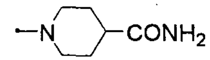 |
| 35 | 35 | 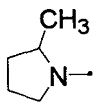 | 2 | H | 0 | $-CH_2CH_2-$ | | 0 | H | 1 | 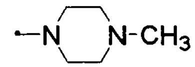 |
| 36 | 36 | 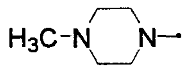 | 2 | H | 0 | $-CH_2CH_2-$ | | 0 | H | 1 | 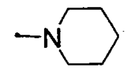 |
| 37 | 37 | 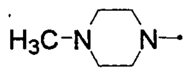 | 2 | H | 0 | $-CH_2CH_2-$ | | 0 | H | 1 | 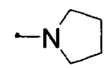 |
| 38 | 38 | 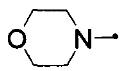 | 2 | H | 0 | $-CH_2CH_2-$ | | 0 | H | 1 | 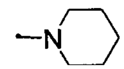 |
| 39 | 39 | 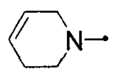 | 2 | H | 0 | $-CH_2CH_2-$ | | 0 | H | 1 | 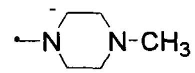 |
| 40 | 40 | 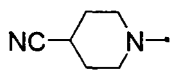 | 2 | H | 0 | $-CH_2CH_2-$ | | 0 | H | 1 | 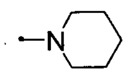 |
| 41 | 41 | 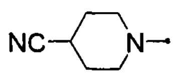 | 2 | H | 0 | $-CH_2CH_2-$ | | 0 | H | 1 | 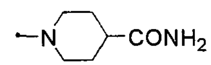 |

(continued)

| Example No. | Compound No. | 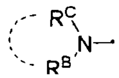 | r | $R^A$ | p | $R^G$ $R^H$ | q | $R^D$ | s | 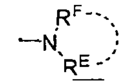 |
|---|---|---|---|---|---|---|---|---|---|---|
| 42 | 42 | 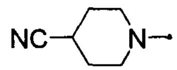 | 2 | H | 0 | $-CH_2CH_2-$ | 0 | H | 1 | 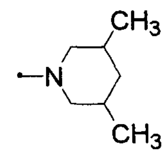 |
| 43 | 43 | 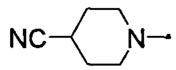 | 2 | H | 0 | $-CH_2CH_2-$ | 0 | H | 1 | 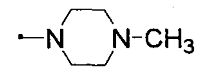 |
| 44 | 44 | 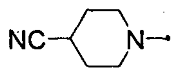 | 2 | H | 0 | $-CH_2CH_2-$ | 0 | H | 1 | 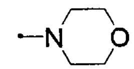 |
| 45 | 45 | 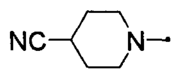 | 2 | H | 0 | $-CH_2CH_2-$ | 0 | H | 1 | 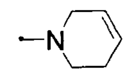 |
| 46 | 46 | 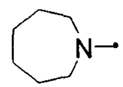 | 2 | H | 0 | $-CH_2CH_2-$ | 0 | H | 1 | 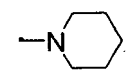 |
| 47 | 47 | 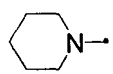 | 2 | H | 0 | $-CH_2CH_2-$ | 0 | H | 1 | 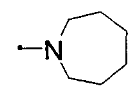 |
| 48 | 48 | 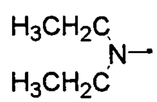 | 2 | H | 0 | $-CH_2CH_2-$ | 0 | H | 1 | 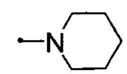 |
| 49 | 49 | 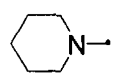 | 2 | H | 0 | $-CH_2CH_2-$ | 0 | H | 1 | 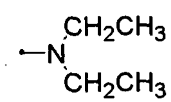 |

(continued)

| Example No. | Compound No. | 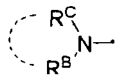 | r | $R^A$ | p | $R^G$ | $R^H$ | q | $R^D$ | s | 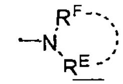 |
|---|---|---|---|---|---|---|---|---|---|---|---|
| 50 | 50 | 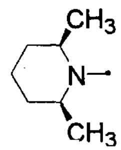 | 2 | H | 0 | $-CH_2CH_2-$ | | 0 | H | 1 | 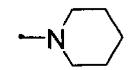 |
| 51 | 51 | 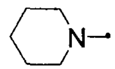 | 2 | H | 0 | $-CH_2CH_2-$ | | 0 | H | 1 | 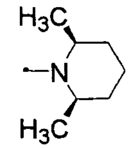 |
| 52 | 52 | 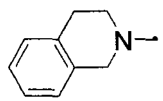 | 2 | H | 0 | $-CH_2CH_2-$ | | 0 | H | 1 | 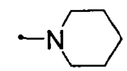 |
| 53 | 53 | 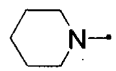 | 2 | H | 0 | $-CH_2CH_2-$ | | 0 | H | 1 | 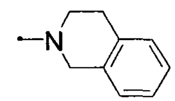 |
| 54 | 54 | 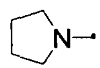 | 2 | H | 0 | $-CH_2CH_2-$ | | 0 | H | 1 | 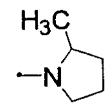 |
| 55 | 55 | 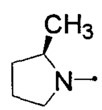 | 2 | H | 0 | $-CH_2CH_2-$ | | 0 | H | 1 | 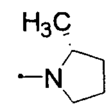 |
| 56 | 56 | 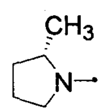 | 2 | H | 0 | $-CH_2CH_2-$ | | 0 | H | 1 | 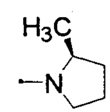 |

(continued)

| Example No. | Compound No. | 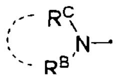 | r | $R^A$ | p | $R^G$ | $R^H$ | q | $R^D$ | s | 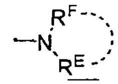 |
|---|---|---|---|---|---|---|---|---|---|---|---|
| 57 | 57 | 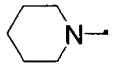 | 2 | H | 0 | $-CH_2CH_2-$ | | 0 | H | 1 | 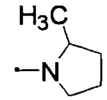 |
| 58 | 58 | 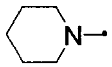 | 2 | H | 0 | $-CH_2CH_2-$ | | 0 | H | 1 | 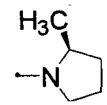 |
| 59 | 59 | 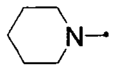 | 2 | H | 0 | $-CH_2CH_2-$ | | 0 | H | 1 | 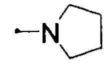 |
| 60 | 60 | 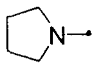 | 2 | H | 0 | $-CH_2CH_2-$ | | 0 | H | 1 | 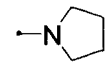 |
| 61 | 61 | 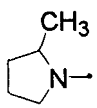 | 2 | H | 0 | $-CH_2CH_2-$ | | 0 | H | 1 | 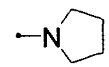 |
| 62 | 62 | 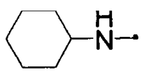 | 2 | H | 0 | $-CH_2CH_2-$ | | 0 | H | 1 | 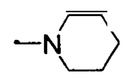 |
| 63 | 63 | 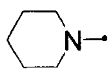 | 2 | H | 0 | $-CH_2CH_2-$ | | 0 | H | 1 | 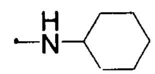 |
| 64 | 64 | 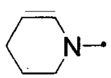 | 2 | H | 0 | $-CH_2CH_2-$ | | 0 | H | 2 | 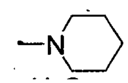 |
| 65 | 65 | 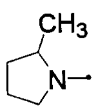 | 2 | H | 0 | $-CH_2CH_2-$ | | 0 | H | 2 | 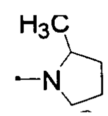 |

(continued)

| Example No. | Compound No. | 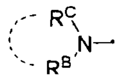 | r | $R^A$ | p | $R^G$ | $R^H$ | q | $R^D$ | s | 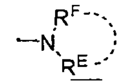 |
|---|---|---|---|---|---|---|---|---|---|---|---|
| 66 | 66 | 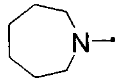 | 2 | H | 0 | $-CH_2CH_2-$ | | 0 | H | 2 | 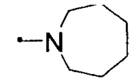 |
| 67 | 67 | 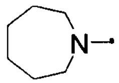 | 2 | H | 0 | $-CH_2CH_2-$ | | 0 | H | 2 | 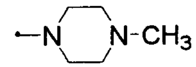 |
| 68 | 68 | 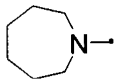 | 2 | H | 0 | $-CH_2CH_2-$ | | 0 | H | 2 | 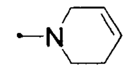 |
| 69 | 69 | 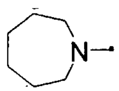 | 2 | H | 0 | $-CH_2CH_2-$ | | 0 | H | 2 | 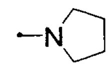 |
| 70 | 70 | 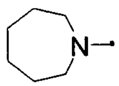 | 2 | H | 0 | $-CH_2CH_2-$ | | 0 | H | 2 | 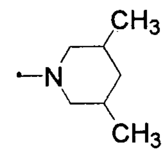 |
| 71 | 71 | 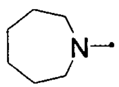 | 2 | H | 0 | $-CH_2CH_2-$ | | 0 | H | 2 | 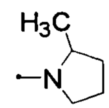 |
| 72 | 72 | 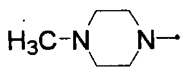 | 2 | H | 0 | $-CH_2CH_2-$ | | 0 | H | 2 | 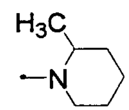 |
| 73 | 73 | 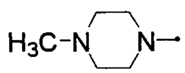 | 2 | H | 0 | $-CH_2CH_2-$ | | 0 | H | 2 | 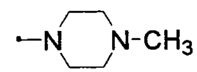 |

(continued)

| Example No. | Compound No. | 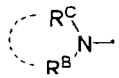 | r | $R^A$ | p | $R^G$ $R^H$ | q | $R^D$ | s | 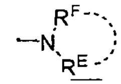 |
|---|---|---|---|---|---|---|---|---|---|---|
| 74 | 74 | 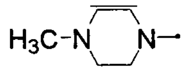 | 2 | H | 0 | $-CH_2CH_2-$ | 0 | H | 2 | 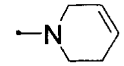 |
| 75 | 75 | 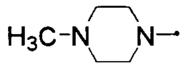 | 2 | H | 0 | $-CH_2CH_2-$ | 0 | H | 2 | 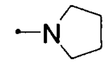 |
| 76 | 76 | 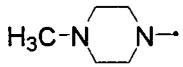 | 2 | H | 0 | $-CH_2CH_2-$ | 0 | H | 2 | 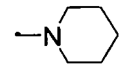 |
| 77 | 77 | 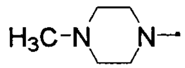 | 2 | H | 0 | $-CH_2CH_2-$ | 0 | H | 2 | 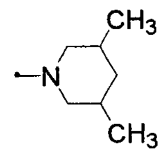 |
| 78 | 78 | 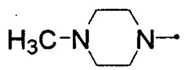 | 2 | H | 0 | $-CH_2CH_2-$ | 0 | H | 2 | 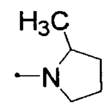 |
| 79 | 79 | 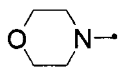 | 2 | H | 0 | $-CH_2CH_2-$ | 0 | H | 2 | 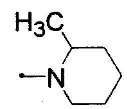 |
| 80 | 80 | 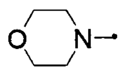 | 2 | H | 0 | $-CH_2CH_2-$ | 0 | H | 2 | 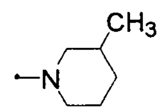 |
| 81 | 81 | 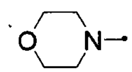 | 2 | H | 0 | $-CH_2CH_2-$ | 0 | H | 2 | 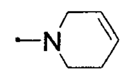 |
| 82 | 82 | 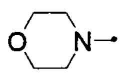 | 2 | H | 0 | $-CH_2CH_2-$ | 0 | H | 2 | 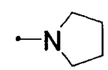 |

(continued)

| Example No. | Compound No. | 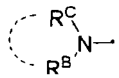 | r | $R^A$ | p | $R^G$ | $R^H$ | q | $R^D$ | s | 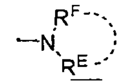 |
|---|---|---|---|---|---|---|---|---|---|---|---|
| 83 | 83 | 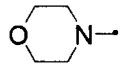 | 2 | H | 0 | $-CH_2CH_2-$ | | 0 | H | 2 | 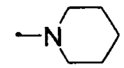 |
| 84 | 84 | 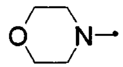 | 2 | H | 0 | $-CH_2CH_2-$ | | 0 | H | 2 | 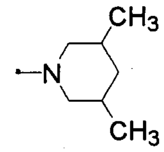 |
| 85 | 85 | 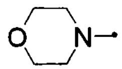 | 2 | H | 0 | $-CH_2CH_2-$ | | 0 | H | 2 | 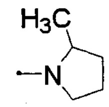 |
| 86 | 86 | 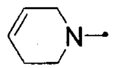 | 2 | H | 0 | $-CH_2CH_2-$ | | 0 | H | 2 | 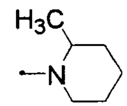 |
| 87 | 87 | 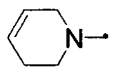 | 2 | H | 0 | $-CH_2CH_2-$ | | 0 | H | 2 | 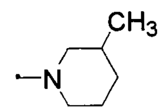 |
| 88 | 88 | 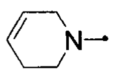 | 2 | H | 0 | $-CH_2CH_2-$ | | 0 | H | 2 | 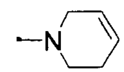 |
| 89 | 89 | 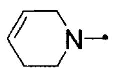 | 2 | H | 0 | $-CH_2CH_2-$ | | 0 | H | 2 | 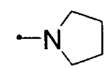 |
| 90 | 90 | 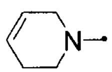 | 2 | H | 0 | $-CH_2CH_2-$ | | 0 | H | 2 | 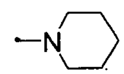 |

(continued)

| Example No. | Compound No. | 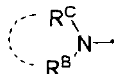 | r | $R^A$ | p | $R^G$ | $R^H$ | q | $R^D$ | s | 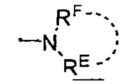 |
|---|---|---|---|---|---|---|---|---|---|---|---|
| 91 | 91 | 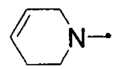 | 2 | H | 0 | $-CH_2CH_2-$ | | 0 | H | 2 | 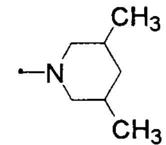 |
| 92 | 92 | 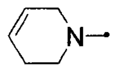 | 2 | H | 0 | $-CH_2CH_2-$ | | 0 | H | 2 | 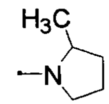 |
| 93 | 93 | 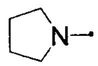 | 2 | H | 0 | $-CH_2CH_2-$ | | 0 | H | 2 | 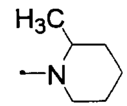 |
| 94 | 94 | 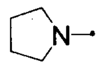 | 2 | H | 0 | $-CH_2CH_2-$ | | 0 | H | 2 | 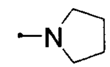 |
| 95 | 95 | 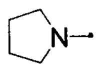 | 2 | H | 0 | $-CH_2CH_2-$ | | 0 | H | 2 | 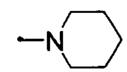 |
| 96 | 96 | 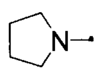 | 2 | H | 0 | $-CH_2CH_2-$ | | 0 | H | 2 | 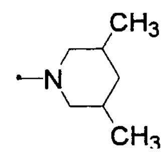 |
| 97 | 97 | 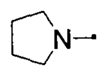 | 2 | H | 0 | $-CH_2CH_2-$ | | 0 | H | 2 | 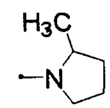 |
| 98 | 98 | 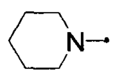 | 2 | H | 0 | $-CH_2CH_2-$ | | 0 | H | 2 | 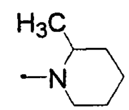 |

(continued)

| Example No. | Compound No. | 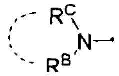 | r | $R^A$ | p | $R^G$ | $R^H$ | q | $R^D$ | s | 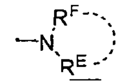 |
|---|---|---|---|---|---|---|---|---|---|---|---|
| 99 | 99 | 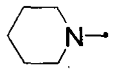 | 2 | H | 0 | | $-CH_2CH_2-$ | 0 | H | 2 | 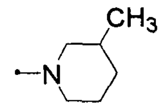 |
| 100 | 100 | 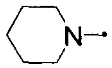 | 2 | H | 0 | | $-CH_2CH_2-$ | 0 | H | 2 | 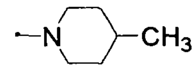 |
| 101 | 101 | 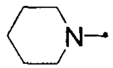 | 2 | H | 0 | | $-CH_2CH_2-$ | 0 | H | 2 | 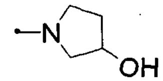 |
| 102 | 102 | 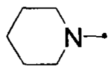 | 2 | H | 0 | | $-CH_2CH_2-$ | 0 | H | 2 | 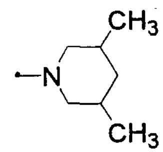 |
| 103 | 103 | 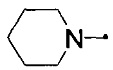 | 2 | H | 0 | | $-CH_2CH_2-$ | 0 | H | 2 | 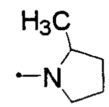 |
| 104 | 104 | 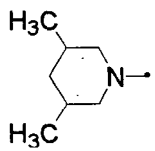 | 2 | H | 0 | | $-CH_2CH_2-$ | 0 | H | 2 | 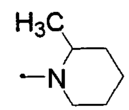 |
| 105 | 105 | 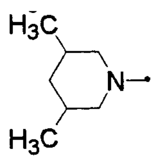 | 2 | H | 0 | | $-CH_2CH_2-$ | 0 | H | 2 | 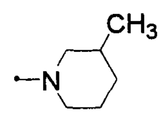 |

(continued)

| Example No. | Compound No. | 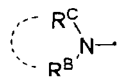 | r | $R^A$ | p | $R^G$ | $R^H$ | q | $R^D$ | s | 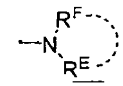 |
|---|---|---|---|---|---|---|---|---|---|---|---|
| 106 | 106 | 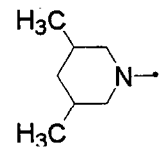 | 2 | H | 0 | $-CH_2CH_2-$ | | 0 | H | 2 | 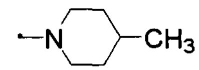 |
| 107 | 107 | 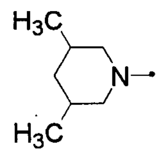 | 2 | H | 0 | $-CH_2CH_2-$ | | 0 | H | 2 | 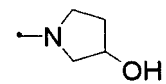 |
| 108 | 108 | 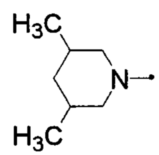 | 2 | H | 0 | $-CH_2CH_2-$ | | 0 | H | 2 | 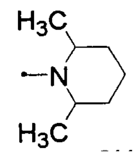 |
| 109 | 109 | 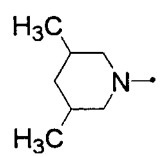 | 2 | H | 0 | $-CH_2CH_2-$ | | 0 | H | 2 | 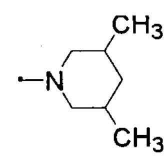 |
| 110 | 110 | 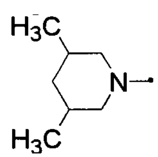 | 2 | H | 0 | $-CH_2CH_2-$ | | 0 | H | 2 | 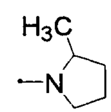 |
| 111 | 111 | 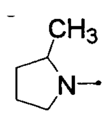 | 2 | H | 0 | $-CH_2CH_2-$ | | 0 | H | 2 | 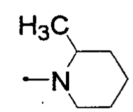 |

(continued)

| Example No. | Compound No. | 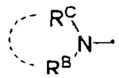 | r | $R^A$ | p | $R^G$ | $R^H$ | q | $R^D$ | s | 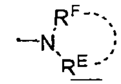 |
|---|---|---|---|---|---|---|---|---|---|---|---|
| 112 | 112 | 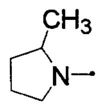 | 2 | H | 0 | $-CH_2CH_2-$ | | 0 | H | 2 | 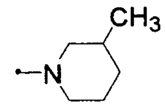 |
| 113 | 113 | 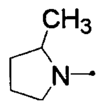 | 2 | H | 0 | $-CH_2CH_2-$ | | 0 | H | 2 | 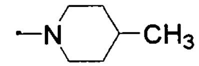 |
| 114 | 114 | 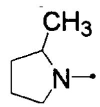 | 2 | H | 0 | $-CH_2CH_2-$ | | 0 | H | 2 | 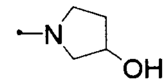 |
| 115 | 115 | 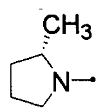 | 2 | H | 0 | $-CH_2CH_2-$ | | 0 | H | 2 | 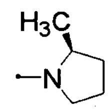 |
| 116 | 116 | 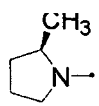 | 2 | H | 0 | $-CH_2CH_2-$ | | 0 | H | 2 | 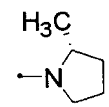 |
| 117 | 117 | 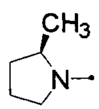 | 2 | H | 0 | $-CH_2CH_2-$ | | 0 | H | 2 | 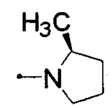 |
| 118 | 118 | 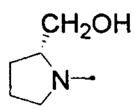 | 2 | H | 0 | $-CH_2CH_2-$ | | 0 H | H | 2 | 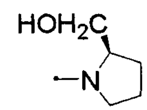 |

(continued)

| Example No. | Compound No. | 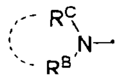 | r | $R^A$ | p | $R^G$ | $R^H$ | q | $R^D$ | s | 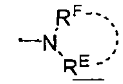 |
|---|---|---|---|---|---|---|---|---|---|---|---|
| 119 | 119 | 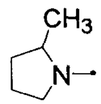 | 2 | H | 0 | $-CH_2CH_2-$ | | 0 H | H | 2 | 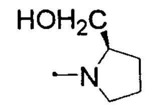 |
| 120 | 120 | 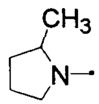 | 2 | H | 0 | $-CH_2CH_2-$ | | 0 H | H | 2 | 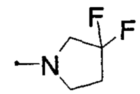 |
| 121 | 121 | 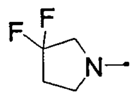 | 2 | H | 0 | $-CH_2CH_2-$ | | 0 H | H | 2 | 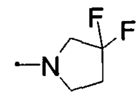 |
| 122 | 122 | 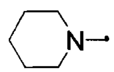 | 2 | H | 0 | $-CH_2CH_2CH_2-$ | | 0 H | H | 2 | 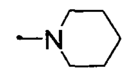 |
| 123 | 123 | 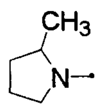 | 2 | H | 0 | $-CH_2CH_2CH_2-$ | | 0 | H | 2 | 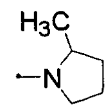 |
| 124 | 124 | 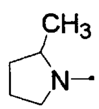 | 2 | H | 0 | 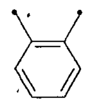 | | 0 | H | 2 | 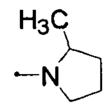 |
| 125 | 125 | 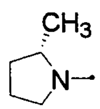 | 2 | H | 0 | 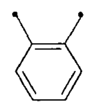 | | 0 | H | 2 | 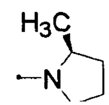 |

Table 4

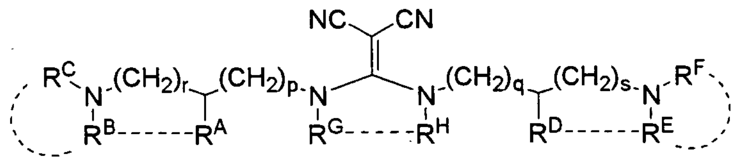

| Example No. | Compound No. | 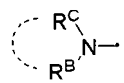 | r | $R^A$ | p | $R^G$ | $R^H$ | q | s | $R^D$ | $R^E$ | $R^F$ |
|---|---|---|---|---|---|---|---|---|---|---|---|---|
| 126 | 126 | 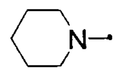 | 2 | H | 0 | $-CH_2CH_2-$ | | 0 | 2 | $-CH_2CH_2-$ | | 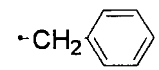 |
| 127 | 127 | 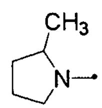 | 2 | H | 0 | $-CH_2CH_2-$ | | 0 | 2 | $-CH_2CH_2-$ | | 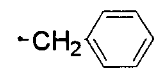 |
| 128 | 128 | 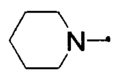 | 2 | H | 0 | $-CH_2CH_2-$ | | 0 | 2 | $-CH_2CH_2-$ | | H |
| 129 | 129 | 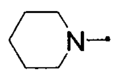 | 2 | H | 0 | $-CH_2CH_2-$ | | 0 | 2 | $-CH_2CH_2-$ | | $CH_3$ |
| 130 | 130 | 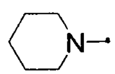 | 2 | H | 0 | $-CH_2CH_2-$ | | 0 | 2 | $-CH_2CH_2-$ | | 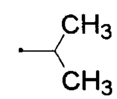 |
| 131 | 131 | 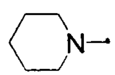 | 2 | H | 0 | $-CH_2CH_2-$ | | 0 | 2 | $-CH_2CH_2-$ | | 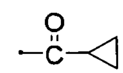 |
| 132 | 132 | 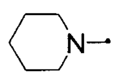 | 2 | H | 0 | $-CH_2CH_2-$ | | 0 | 2 | $-CH_2CH_2-$ | | 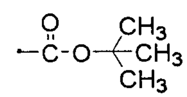 |

(continued)

| Example No. | Compound No. | 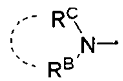 | r | $R^A$ | p | $R^G$ | $R^H$ | q | s | $R^D$ | $R^E$ | $R^F$ |
|---|---|---|---|---|---|---|---|---|---|---|---|---|
| 133 | 133 | 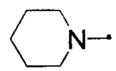 | 2 | H | 0 | $-CH_2CH_2-$ | | 1 | 2 | $-CH_2CH_2-$ | | 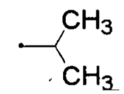 |

Table 5

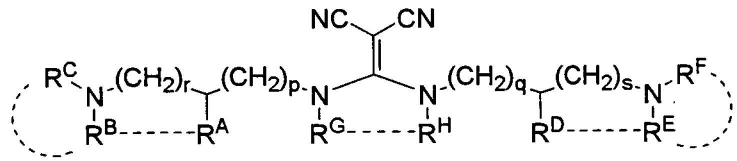

| Example No. | Compound No. | $R^C$ | $R^B$ | $R^A$ | r | p | $R^G$ $R^H$ | q | s | $R^D$ | $R^E$ | $R^F$ |
|---|---|---|---|---|---|---|---|---|---|---|---|---|
| 134 | 134 | 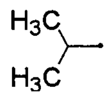 | $-CH_2CH_2-$ | | 2 | 1 | $-CH_2CH_2-$ | 1 | 2 | $-CH_2CH_2-$ | | 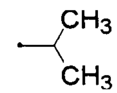 |

Table 6

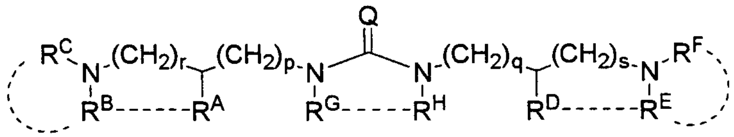

| Example No. | Compound No. | 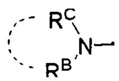 | r | $R^A$ | p | $R^G$ | $R^H$ | Q | q | $R^D$ | s | 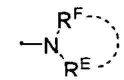 |
|---|---|---|---|---|---|---|---|---|---|---|---|---|
| 135 | 135 | 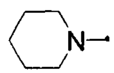 | 2 | H | 0 | $-CH_2CH_2-$ | | 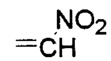 | 0 | H | 2 | 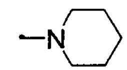 |
| 136 | 136 | 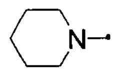 | 2 | H | 0 | $-CH_2CH_2-$ | | 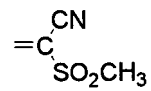 | 0 | H | 2 | 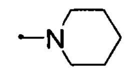 |
| 137 | 137 | 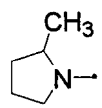 | 2 | H | 0 | $-CH_2CH_2-$ | | 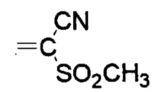 | 0 | H | 2 | 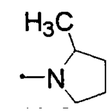 |
| 138 | 138 | 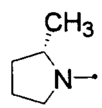 | 2 | H | 0 | $-CH_2CH_2-$ | | 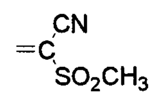 | 0 | H | 2 | 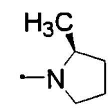 |
| 139 | 139 | 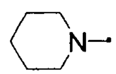 | 2 | H | 0 | $-CH_2CH_2-$ | | 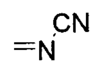 | 0 | H | 2 | 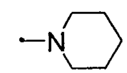 |
| 140 | 140 | 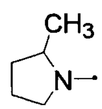 | 2 | H | 0 | $-CH_2CH_2-$ | | 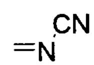 | 0 | H | 2 | 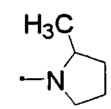 |

(continued)

| Example No. | Compound No. | 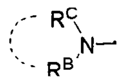 | r | $R^A$ | p | $R^G$ | $R^H$ | Q | q | $R^D$ | s | 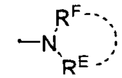 |
|---|---|---|---|---|---|---|---|---|---|---|---|---|
| 141 | 141 | 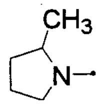 | 2 | H | 0 | $-CH_2CH_2$ | | =O | 0 | H | 2 | 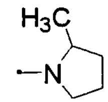 |
| 142 | 142 | 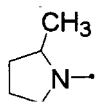 | 2 | H | 0 | $-CH_2CH_2$ | | 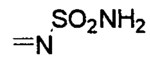 | 0 | H | 2 | 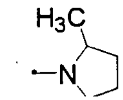 |

Table 7

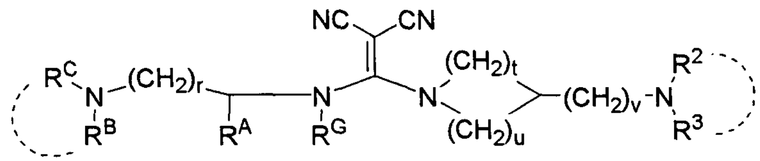

| Example No. | Compound No. | 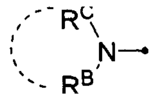 | r | $R^A$ | $R^G$ | t | u | v | 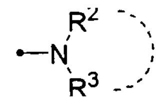 |
|---|---|---|---|---|---|---|---|---|---|
| 143 | 143 | 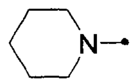 | 1 | H | H | 2 | 2 | 0 | 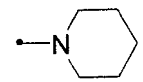 |
| 144 | 144 | 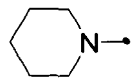 | 1 | H | $CH_3$ | 2 | 2 | 0 | 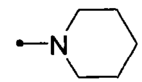 |
| 145 | 145 | 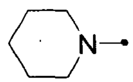 | 1 | H | H | 2 | 2 | 0 | 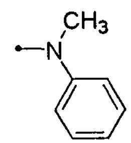 |
| 146 | 146 | 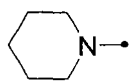 | 1 | H | $CH_3$ | 2 | 2 | 0 | 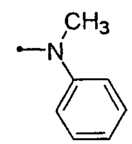 |
| 147 | 147 | 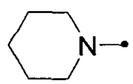 | 1 | H | H | 2 | 2 | 1 | 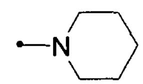 |
| 148 | 148 | 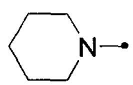 | 1 | H | H | 2 | 2 | 1 | 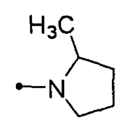 |

(continued)

| Example No. | Compound No. | 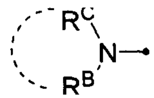 | r | $R^A$ | $R^G$ | t | u | v | 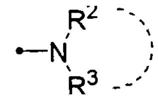 |
|---|---|---|---|---|---|---|---|---|---|
| 149 | 149 | 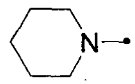 | 1 | H | $CH_3$ | 2 | 2 | 1 | 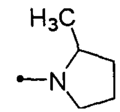 |
| 150 | 150 | 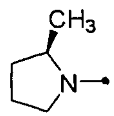 | 1 | H | H | 2 | 2 | 1 | 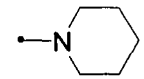 |
| 151 | 151 | 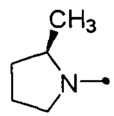 | 1 | H | $CH_3$ | 2 | 2 | 1 | 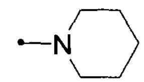 |
| 152 | 152 | 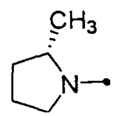 | 1 | H | H | 2 | 2 | 1 | 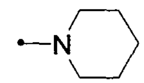 |
| 153 | 153 | 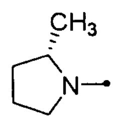 | 1 | H | $CH_3$ | 2 | 2 | 1 | 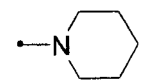 |
| 154 | 154 | 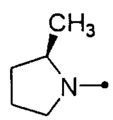 | 1 | H | H | 2 | 2 | 1 | 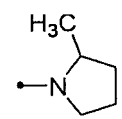 |
| 155 | 155 | 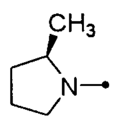 | 1 | H | $CH_3$ | 2 | 2 | 1 | 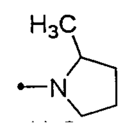 |

(continued)

| Example No. | Compound No. | 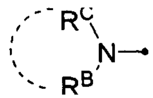 | r | $R^A$ | $R^G$ | t | u | v | 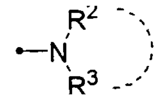 |
|---|---|---|---|---|---|---|---|---|---|
| 156 | 156 | 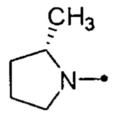 | 1 | H | H | 2 | 2 | 1 | 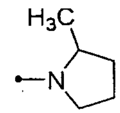 |
| 157 | 157 | 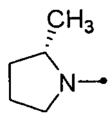 | 1 | H | $CH_3$ | 2 | 2 | 1 | 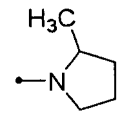 |
| 158 | 158 | 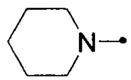 | 2 | H | H | 2 | 2 | 0 | 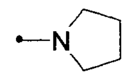 |
| 159 | 159 | 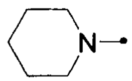 | 2 | H | $CH_3$ | 2 | 2 | 0 | 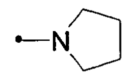 |
| 160 | 160 | 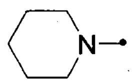 | 2 | H | H | 2 | 2 | 0 | 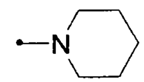 |
| 161 | 161 | 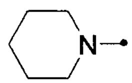 | 2 | H | $CH_3$ | 2 | 2 | 0 | 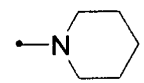 |
| 162 | 162 | 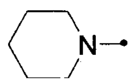 | 2 | H | $CH_2CH_3$ | 2 | 2 | 0 | 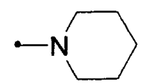 |
| 163 | 163 | 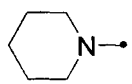 | 2 | H | H | 2 | 2 | 0 | 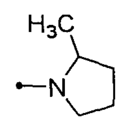 |

(continued)

| Example No. | Compound No. | 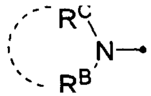 | r | $R^A$ | $R^G$ | t | u | v | 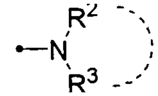 |
|---|---|---|---|---|---|---|---|---|---|
| 164 | 164 | 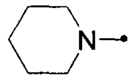 | 2 | H | H | 2 | 2 | 0 | 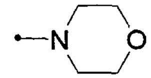 |
| 165 | 165 | 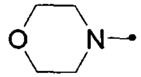 | 2 | H | H | 2 | 2 | 0 | 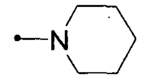 |
| 166 | 166 | 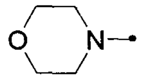 | 2 | H | $CH_3$ | 2 | 2 | 0 | 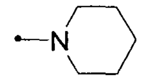 |
| 167 | 167 | 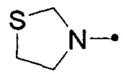 | 2 | H | H | 2 | 2 | 0 | 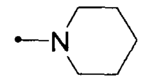 |
| 168 | 168 | 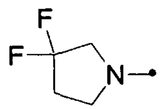 | 2 | H | H | 2 | 2 | 0 | 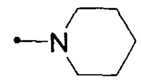 |
| 169 | 169 | 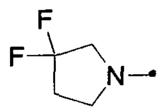 | 2 | H | $CH_3$ | 2 | 2 | 0 | 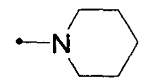 |
| 170 | 170 | 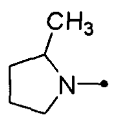 | 2 | H | H | 2 | 2 | 0 | 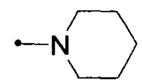 |
| 171* | 171* | 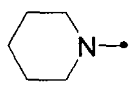 | 2 | H | H | 0 | 3 | 1 | 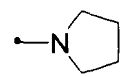 |

(continued)

| Example No. | Compound No. | 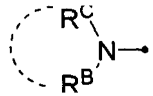 | r | $R^A$ | $R^G$ | t | u | v | 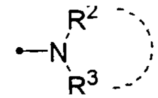 |
|---|---|---|---|---|---|---|---|---|---|
| 172 | 172 | 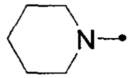 | 2 | .H | H | 2 | 2 | 1 | 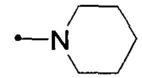 |
| *171: | 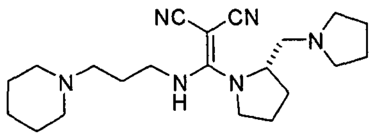 | | | | | | | | |
| 173 | 173 | 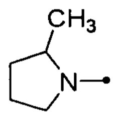 | 2 | H | H | 2 | 2 | 1 | 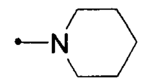 |
| 174 | 174 | 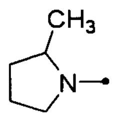 | 2 | H | H | 2 | 2 | 1 | 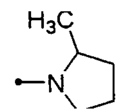 |

Table 8

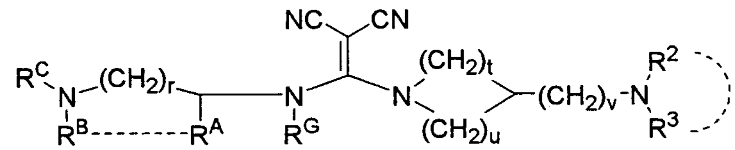

| Example No. | Compound No. | $R^C$ | $R^B$ | $R^A$ | r | $R^G$ | t | u | v | 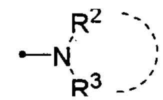 |
|---|---|---|---|---|---|---|---|---|---|---|
| 175 | 175 | 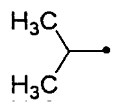 | $CH_2CH_2$ | | 2 | H | 2 | 2 | 0 | 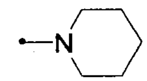 |
| 176 | 176 | 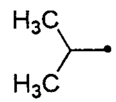 | $CH_2CH_2$ | | 2 | $CH_3$ | 2 | 2 | 0 | 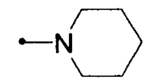 |
| 177 | 177 | 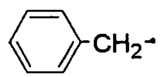 | $CH_2CH_2$ | | 2 | H | 2 | 2 | 0 | 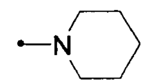 |
| 178 | 178 | 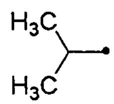 | $CH_2CH_2$ | | 2 | H | 2 | 2 | 1 | 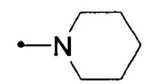 |
| 179 | 179 | 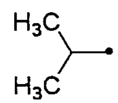 | $CH_2CH_2$ | | 2 | $CH_3$ | 2 | 2 | 1 | 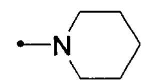 |
| 180 | 180 | 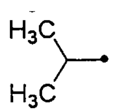 | $CH_2CH_2$ | | 2 | H | 2 | 2 | 1 | 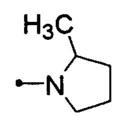 |

(continued)

| Example No. | Compound No. | $R^C$ | $R^B$ | $R^A$ | r | $R^G$ | t | u | v | 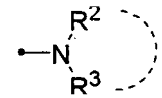 |
|---|---|---|---|---|---|---|---|---|---|---|
| 181 | 181 | 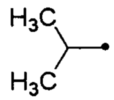 | $CH_2CH_2$ | | 2 | $CH_3$ | 2 | 2 | 1 | 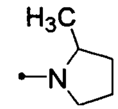 |
| 182 | 182 | 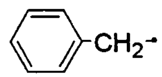 | $CH_2CH_2$ | | 2 | H | 2 | 2 | 1 | 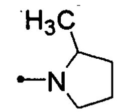 |

Table 9

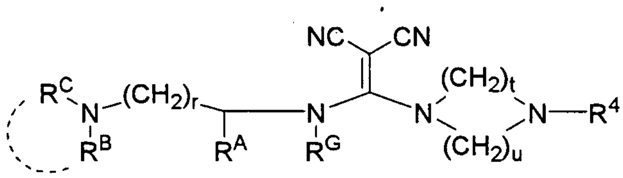

| Example No. | Compound No. | 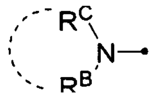 | r | $R^A$ | $R^G$ | t | u | $R^4$ |
|---|---|---|---|---|---|---|---|---|
| 183 | 183 | 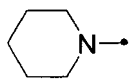 | 2 | H | H | 2 | 2 | 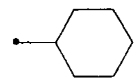 |

Table 10

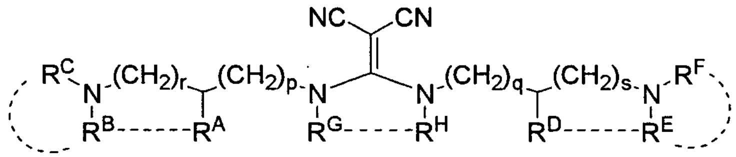

| Example No. | Compound No. | $R^C$ | $R^B$ $R^A$ | r | | p $R^G$ $R^H$ | q | s | $R^D$ $R^E$ | $E^F$ |
|---|---|---|---|---|---|---|---|---|---|---|
| 184 | 184 | $CH_3$ | $-CH_2CH_2CH_2-$ | 0 | 2 | $-CH_2CH_2-$ 0 | 0 | 2 | $-CH_2CH_2-$ | 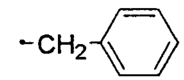 |
| 185 | 185 | $CH_3$ | $-CH_2CH_2CH_2-$ | 0 | 2 | $-CH_2CH_2-$ | 0 | 2 | $-CH_2CH_2-$ | H |
| 186 | 186 | $CH_3$ | $-CH_2CH_2CH_2-$ | 0 | 2 | $-CH_2CH_2-$ | 0 | 2 | $-CH_2CH_2-$ | 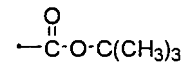 |
| 187 | 187 | $CH_3$ | $-CH_2CH_2CH_2-$ | 0 | 2 | $-CH_2CH_2-$ | 0 | 2 | $-CH_2CH_2-$ | 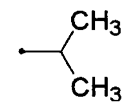 |

Table 11

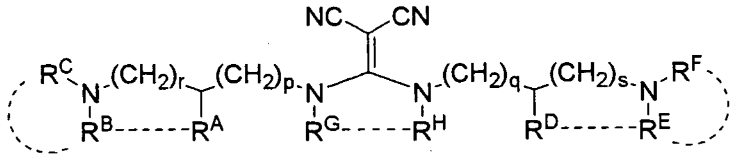

| Example No. | Compound No. | 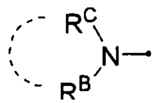 | r | $R^A$ | p | $R^G$ | $R^H$ | q | $R_D$ | s | 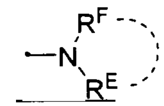 |
|---|---|---|---|---|---|---|---|---|---|---|---|
| 188 | 188 | 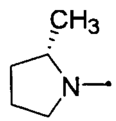 | 1 | 0 | 0 | $-CH_2CH_2-$ | | 0 | H | 1 | 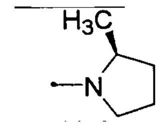 |
| 189 | 189 | 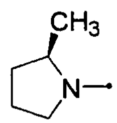 | 1 | H | 0 | $-CH_2CH_2-$ | | 0 | H | 1 | 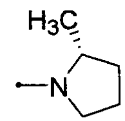 |
| 190 | 190 | 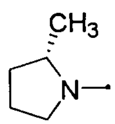 | 3 | H | 0 | $-CH_2CH_2-$ | | 0 | H | 1 | 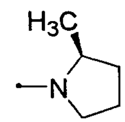 |
| 191 | 191 | 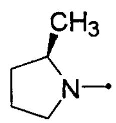 | 3 | H | 0 | $-CH_2CH_2-$ | | 0 | H | 1 | 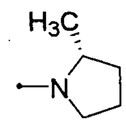 |
| 192 | 192 | 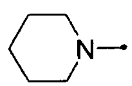 | 1 | H | 0 | 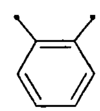 | | 0 | H | 1 | 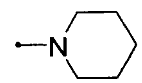 |

(continued)

| Example No. | Compound No. | 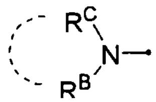 | r | $R^A$ | p | $R^G$ | $R^H$ | q | $R_D$ | s | 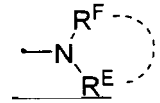 |
|---|---|---|---|---|---|---|---|---|---|---|---|
| 193 | 193 | 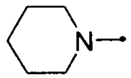 | 2 | H | 0 | $-CH_2CH_2-$ | | 0 | H | 1 | 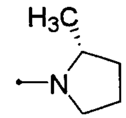 |
| 194 | 194 | 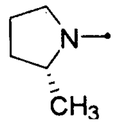 | 2 | H | 0 | $-CH_2CH_2-$ | | 0 | H | 1 | 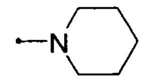 |
| 195 | 195 | 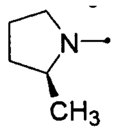 | 2 | H | 0 | $-CH_2CH_2-$ | | 0 | H | 1 | 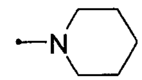 |
| 196 | 196 | 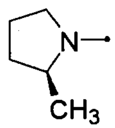 | 2 | H | 0 | $-CH_2CH_2-$ | | 0 | H | 1 | 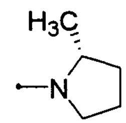 |
| 197 | 197 | 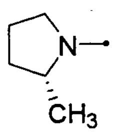 | 2 | H | 0 | $-CH_2CH_2-$ | | 0 | H | 1 | 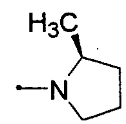 |
| 198 | 198 | 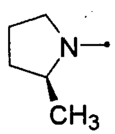 | 2 | H | 0 | $-CH_2CH_2-$ | | 0 | H | 1 | 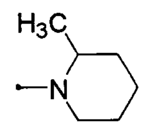 |

(continued)

| Example No. | Compound No. | 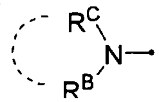 | r | $R^A$ | p | $R^G$ | $R^H$ | q | $R_D$ | s | 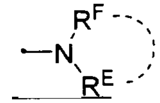 |
|---|---|---|---|---|---|---|---|---|---|---|---|
| 199 | 199 | 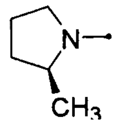 | 2 | H | 0 | $-CH_2CH_2-$ | | 0 | H | 1 | 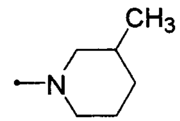 |
| 200 | 200 | 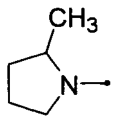 | 2 | H | 0 | 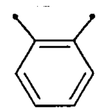 | | 0 | H | 1 | 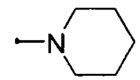 |
| 201 | 201 | 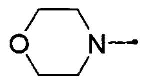 | 2 | H | 0 | $-CH_2CH_2-$ | | 0 | H | 2 | 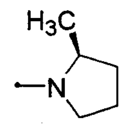 |
| 202 | 202 | 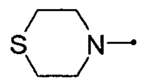 | 2 | H | | $-CH_2CH_2-$ | | 0 | H | 2 | 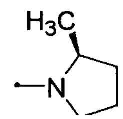 |
| 203 | 203 | 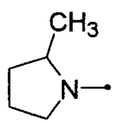 | 2 | H | 0 | $-CH_2CH_2-$ | | 0 | H | 2 | 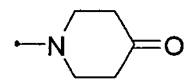 |
| 204 | 204 | 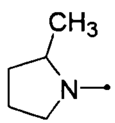 | 2 | H | | $-CH_2CH_2-$ | | 0 | H | 2 | 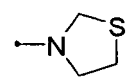 |

(continued)

| Example No. | Compound No. | 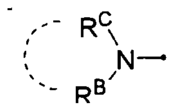 | r | $R^A$ | p | $R^G$ | $R^H$ | q | $R_D$ | s | 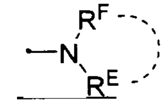 |
|---|---|---|---|---|---|---|---|---|---|---|---|
| 205 | 205 | 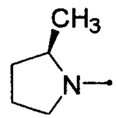 | 2 | H | C | 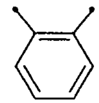 | | 0 | H | 2 | 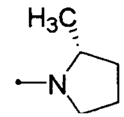 |

Table 12

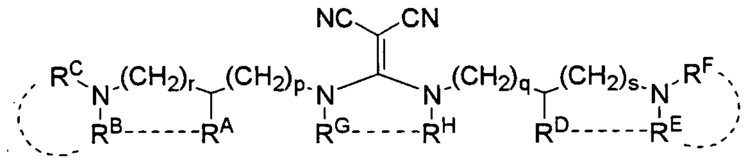

| Example No. | Compound No. | 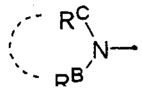 | r | $R^A$ | p | $R^G$ | $R^H$ | q | s | $R^D$ | $R^E$ | $R^F$ |
|---|---|---|---|---|---|---|---|---|---|---|---|---|
| 206 | 206 | 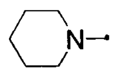 | | H | 0 | $-CH_2CH_2-$ | | 0 | 2 | $-CH_2CH_2-$ | | 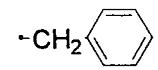 |
| 207 | 207 | 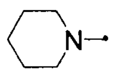 | | H | 0 | $-CH_2CH_2-$ | | 0 | 2 | $-CH_2CH_2-$ | | 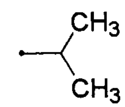 |
| 208 | 208 | 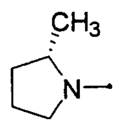 | | H | 0 | $-CH_2CH_2-$ | | 0 | 2 | $-CH_2CH_2-$ | | 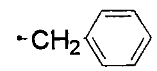 |
| 209 | 209 | 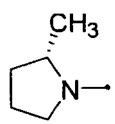 | | H | 0 | $-CH_2CH_2-$ | | 0 | 2 | $-CH_2CH_2-$ | | H |
| 210 | 210 | 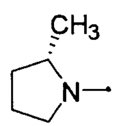 | | H | 0 | $-CH_2CH_2-$ | | 0 | 2 | $-CH_2CH_2-$ | | 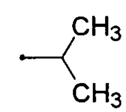 |
| 211 | 211 | 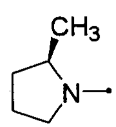 | | H | 0 | $-CH_2CH_2-$ | | 0 | 2 | $-CH_2CH_2-$ | | 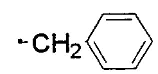 |

(continued)

| Example No. | Compound No. | 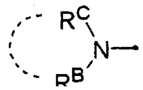 | r | $R^A$ | p | $R^G$ | $R^H$ | q | s | $R^D$ | $R^E$ | $R^F$ |
|---|---|---|---|---|---|---|---|---|---|---|---|---|
| 212 | 212 | 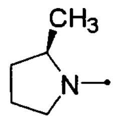 | 1 | H | 0 | $-CH_2CH_2-$ | | 0 | 2 | $-CH_2CH_2-$ | | H |
| 213 | 213 | 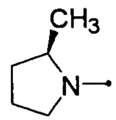 | 1 | H | 0 | $-CH_2CH_2-$ | | 0 | 2 | $-CH_2CH_2-$ | | 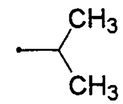 |
| 214 | 214 | 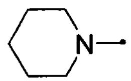 | 2 | H | 0 | $-CH_2CH_2-$ | | 0 | 2 | $-CH_2CH_2-$ | | $-CH_2CN$ |
| 215 | 215 | 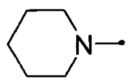 | 2 | H | 0 | $-CH_2CH_2-$ | | 0 | 2 | $-CH_2CH_2-$ | | $-CH_2CH_2F$ |
| 216 | 216 | 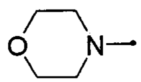 | 2 | H | 0 | $-CH_2CH_2-$ | | 0 | 2 | $-CH_2CH_2-$ | | 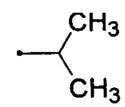 |
| 217 | 217 | 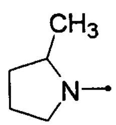 | 2 | H | 0 | $-CH_2CH_2-$ | | 0 | 2 | $-CH_2CH_2-$ | | 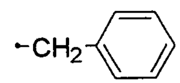 |
| 218 | 218 | 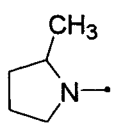 | 2 | H | 0 | $-CH_2CH_2-$ | | 0 | 2 | $-CH_2CH_2-$ | | H |

(continued)

| Example No. | Compound No. | 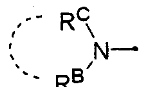 | r | $R^A$ | p | $R^G$ | $R^H$ | q | s | $R^D$ | $R^E$ | $R^F$ |
|---|---|---|---|---|---|---|---|---|---|---|---|---|
| 219 | 219 | 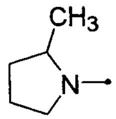 | 2 | H | 0 | $-CH_2CH_2-$ | | 0 | 2 | **-CH2CH2-** | | 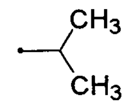 |
| 220 | 220 | 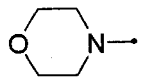 | 2 | H | 0 | $-CH_2CH_2-$ | | 0 | 2 | $-CH_2CH_2-$ | | 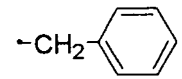 |
| 221 | 221 | 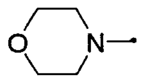 | 2 | H | 0 | $-CH_2CH_2-$ | | 0 | 2 | $-CH_2CH_2-$ | | H |
| 222 | 222 | 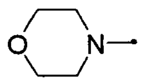 | 2 | H | 0 | $-CH_2CH_2-$ | | 0 | 2 | $-CH_2CH_2-$ | | $-CH_2CH_2F$ |
| 223 | 223 | 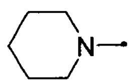 | 2 | H | 0 | $-CH_2CH_2-$ | | 0 | 2 | $-CH_2CH_2-$ | | 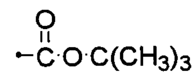 |
| 224 | 224 | 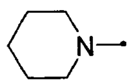 | 2 | H | 0 | $-CH_2CH_2-$ | | 0 | 2 | $-CH_2CH_2-$ | | H |
| 225 | 225 | 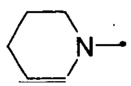 | 2 | H | 0 | $-CH_2CH_2-$ | | 0 | 2 | $-CH_2CH_2-$ | | $-CH_2CF_3$ |
| 226 | 226 | 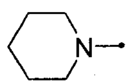 | 2 | H | 0 | $-CH_2CH_2-$ | | 0 | 2 | $-CH_2CH_2-$ | | 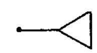 |
| 227 | 227 | 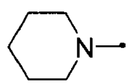 | 2 | H | 0 | $-CH_2CH_2-$ | | 0 | 2 | $-CH_2CH_2-$ | | 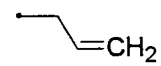 |

(continued)

| Example No. | Compound No. | 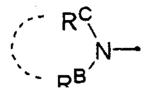 | r | $R^A$ | p | $R^G$ | $R^H$ | q | s | $R^D$ | $R^E$ | $R^F$ |
|---|---|---|---|---|---|---|---|---|---|---|---|---|
| 228 | 228 | 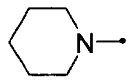 | 2 | H | 0 | $-CH_2CH_2-$ | | 0 | 2 | $-CH_2CH_2-$ | | 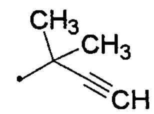 |
| 229 | 229 | 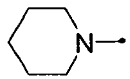 | 2 | H | 0 | $-CH_2CH_2-$ | | 0 | 2 | $-CH_2CH_2-$ | | 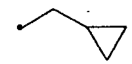 |
| 230 | 230 | 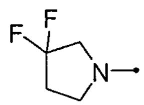 | 2 | H | 0 | $-CH_2CH_2-$ | | 0 | 2 | $-CH_2CH_2-$ | | 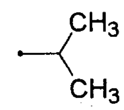 |
| 231 | 231 | 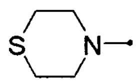 | 2 | H | 0 | $-CH_2CH_2-$ | | 0 | 2 | $-CH_2CH_2-$ | | 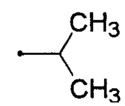 |
| 232 | 232 | 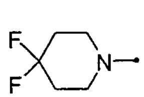 | 2 | H | 0 | $-CH_2CH_2-$ | | 0 | 2 | $-CH_2CH_2-$ | | 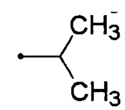 |
| 233 | 233 | 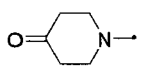 | 2 | H | 0 | $-CH_2CH_2-$ | | 0 | 2 | $-CH_2CH_2-$ | | 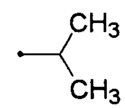 |
| 234 | 234 | 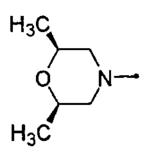 | 2 | H | 0 | $-CH_2CH_2-$ | | 0 | 2 | $-CH_2CH_2-$ | | 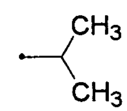 |

(continued)

| Example No. | Compound No. | 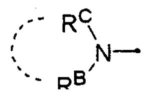 | r | $R^A$ | p | $R^G$ $R^H$ | q | s | $R^D$ $R^E$ | $R^F$ |
|---|---|---|---|---|---|---|---|---|---|---|
| 235 | 235 | 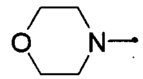 | 2 | H | 0 | $-CH_2CH_2-$ | 0 | 2 | $-CH_2CH_2-$ | 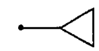 |
| 236 | 236 | 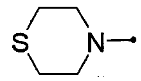 | 2 | H | 0 | $-CH_2CH_2-$ | 0 | 2 | $-CH_2CH_2-$ | 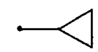 |
| 237 | 237 | 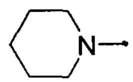 | 2 | H | 0 | $-CH_2CH_2CH_2-$ | 0 | 2 | $-CH_2CH_2-$ | 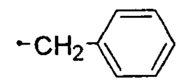 |
| 238 | 238 | 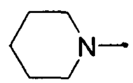 | 2 | H | 0 | $-CH_2CH_2CH_2-$ | 0 | 2 | $-CH_2CH_2-$ | 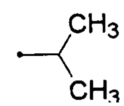 |

Table 13

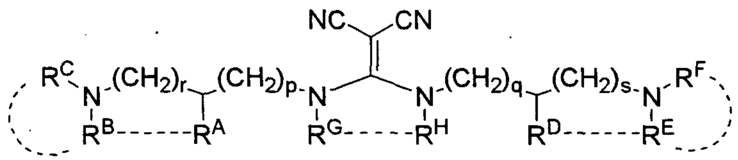

| Example No. | Compound No. | $R^C$ | $R^B$ | $R^A$ | r | p | $R^G$ | $R^H$ | q | s | $R^D$ $R^E$ | $R^F$ |
|---|---|---|---|---|---|---|---|---|---|---|---|---|
| 239 | 239 | 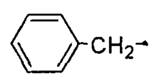 | $-CH_2CH_2-$ | | 2 | 0 | H | H | 0 | 2 | $-CH_2CH_2-$ | 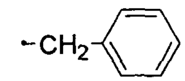 |
| 240 | 240 | 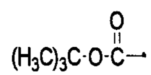 | $-CH_2CH_2-$ | | 2 | 0 | H | H | 0 | 2 | $-CH_2CH_2-$ | 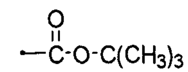 |
| 241 | 241 | 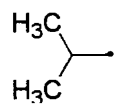 | $-CH_2CH_2-$ | | 2 | 0 | H | H | 0 | 2 | $-CH_2CH_2-$ | 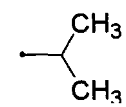 |

Table 14

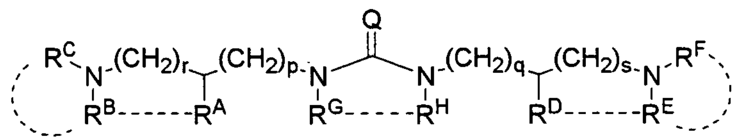

| Example No. | Compound No. | 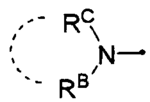 | r | $R^A$ | p | $R^G$ | $R^H$ | Q | q | $R^D$ | s | 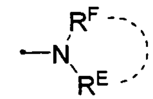 |
|---|---|---|---|---|---|---|---|---|---|---|---|---|
| 242 | 242 | 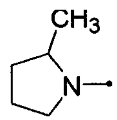 | 2 | H | 0 | $-CH_2CH_2-$ | | 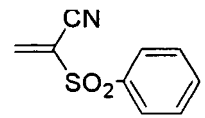 | 0 | H | 1 | 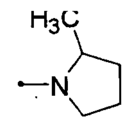 |
| 243 | 243 | 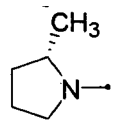 | 2 | H | 0 | $-CH_2CH_2-$ | | 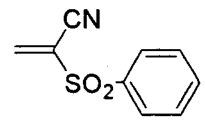 | 0 | H | 1 | 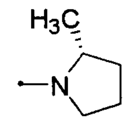 |
| 244 | 244 | 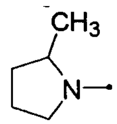 | 2 | H | 0 | $-CH_2CH_2-$ | | 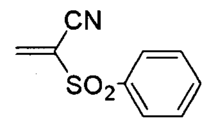 | 0 | H | 2 | 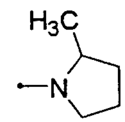 |
| 245 | 245 | 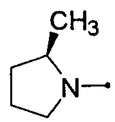 | 2 | H | 0 | $-CH_2CH_2-$ | | 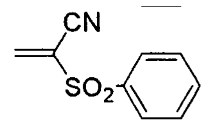 | 0 | H | 2 | 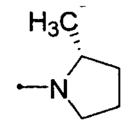 |
| 246 | 246 | 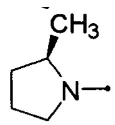 | 2 | H | 0 | $-CH_2CH_2-$ | | 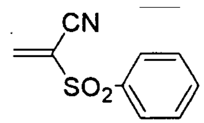 | 0 | H | 2 | 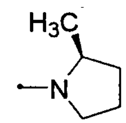 |
| 247 | 247 | 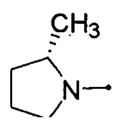 | 2 | H | 0 | $-CH_2CH_2-$ | | 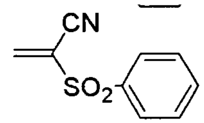 | 0 | H | 2 | 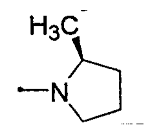 |

Table 15

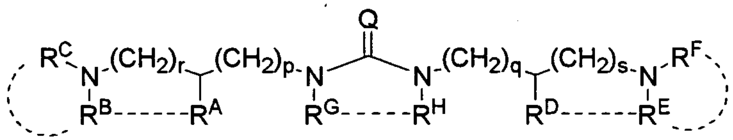

| Example No. | Compounc No. | 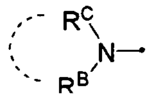 | r | $R^A$ | p | $R^G$ | $R^H$ | Q | q | s | $R^D$ $R^E$ | $R^F$ |
|---|---|---|---|---|---|---|---|---|---|---|---|---|
| 248 | 248 | 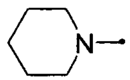 | 2 | H | 0 | $-CH_2CH_2-$ | | 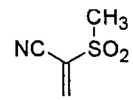 | 0 | 2 | $-CH_2CH_2-$ | 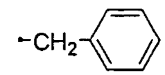 |
| 249 | 249 | 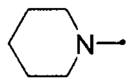 | 2 | H | 0 | $-CH_2CH_2-$ | | 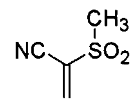 | 0 | 2 | $-CH_2CH_2-$ | H |
| 250 | 250 | 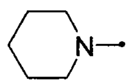 | 2 | H | 0 | $-CH_2CH_2-$ | | 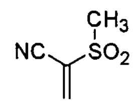 | 0 | 2 | $-CH_2CH_2-$ | 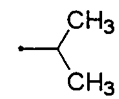 |

Among the compounds as illustrated in Table 1 to Table 6, preferred examples of the compound include the compounds 4, 5, 6, 7, 10, 11, 13, 15, 17, 18, 20, 27, 33, 46, 47, 51, 52, 54, 55, 56, 57, 58, 59, 60, 61, 62, 63, 64, 65, 66, 68, 69, 70, 71, 78, 85, 87, 88, 89, 90, 91, 92, 96, 97, 98, 99, 100, 102, 103, 104, 105, 108, 110, 111, 112, 113, 114, 115, 116, 117, 118, 119, 122, 123, 124, 125, 126, 129, 130, 133, 134, 136, 137, 138, 139, 140, 141, 142 and the like. The chemical structures of these compounds are shown below. More preferred examples of the compound include compounds 4, 5, 6, 7, 10, 11, 20, 27, 33, 46, 47, 51, 54, 55, 56, 57, 58, 61, 64, 65, 71, 97, 103, 110, 111, 112, 113, 115, 116, 117, 119, 122, 123, 124, 125, 126, 129, 130, 133, 136, 137, 138, 139, 140 and the like.

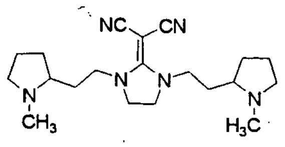
4

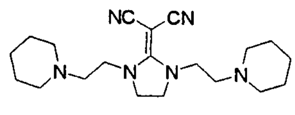
5

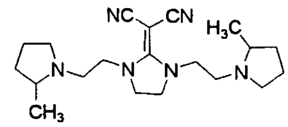
6

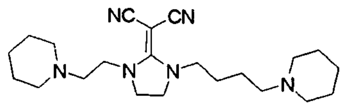
7

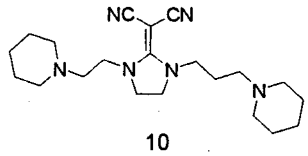
10

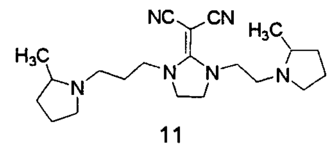
11

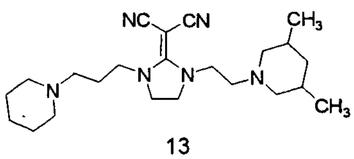
13

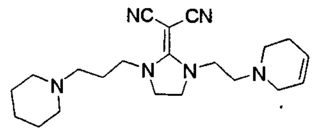
15

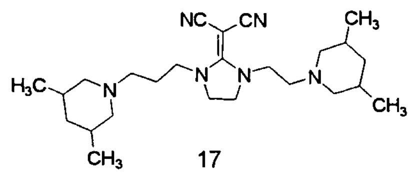
17

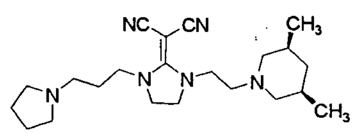
18

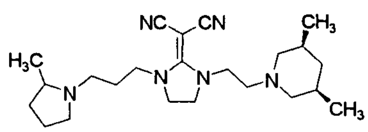
20

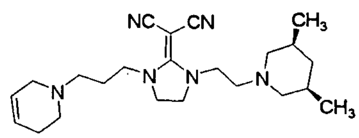
27

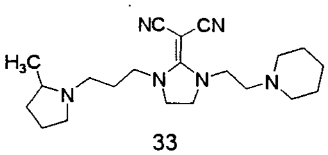
33

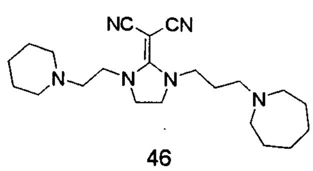
46

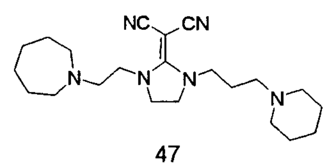
47

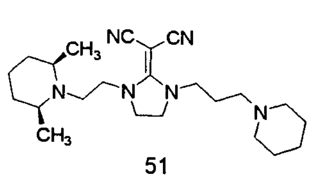
51

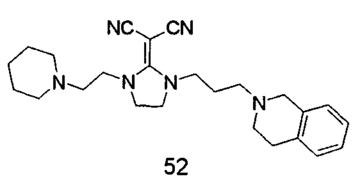
52

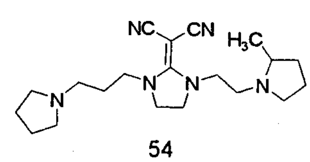
54

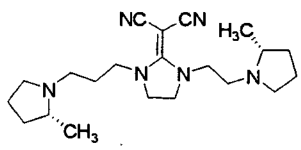
55

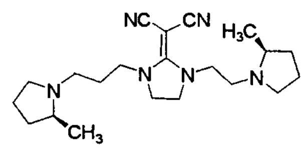
56

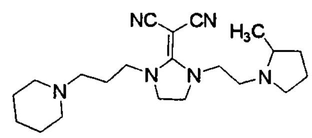
57

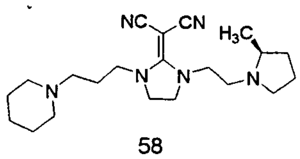
58

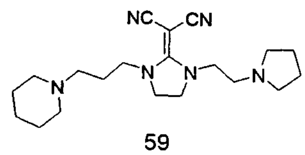
59

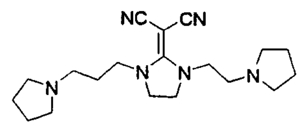
60

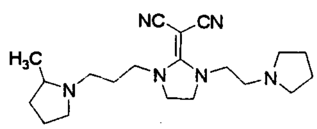
61

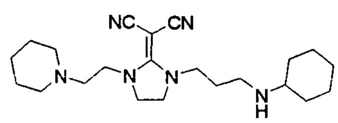
62

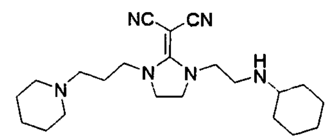
63

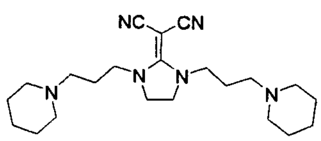
64

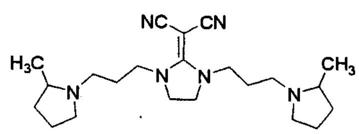
65

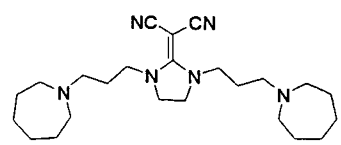
66

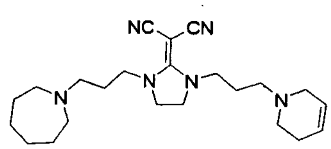
68

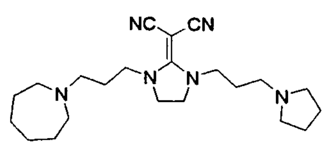
69

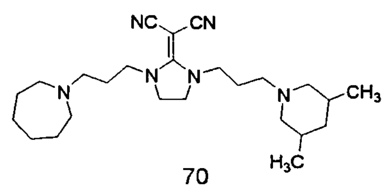
70

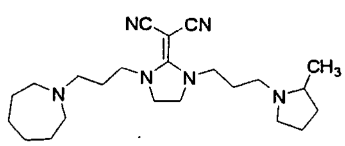
71

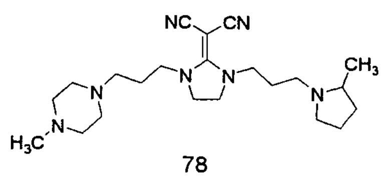
78

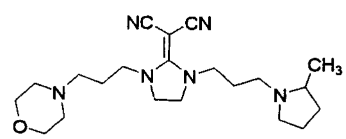
85

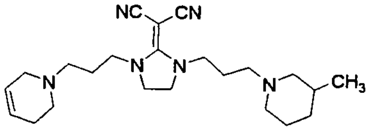
87

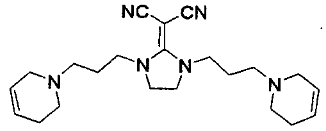
88

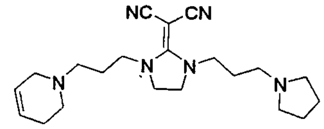
89

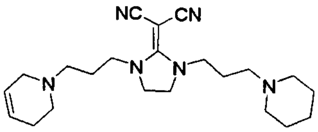
90

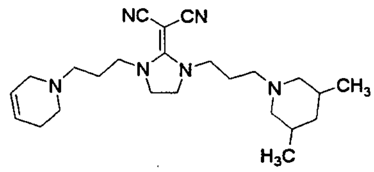
91

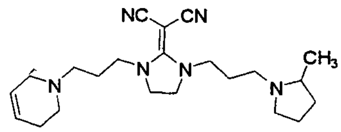
92

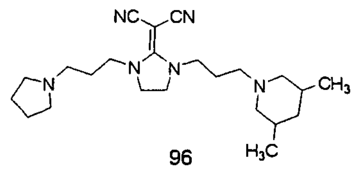
96

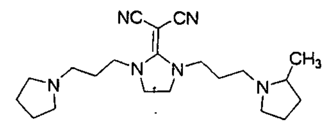
97

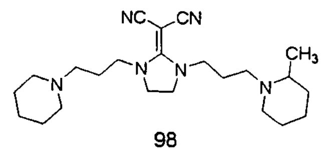
98

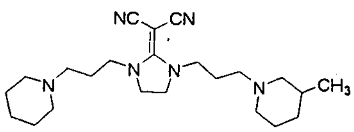
99

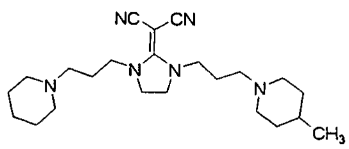
100

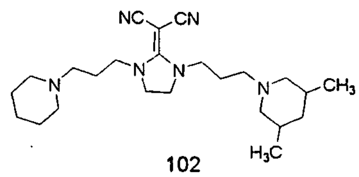
102

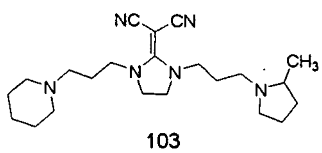
103

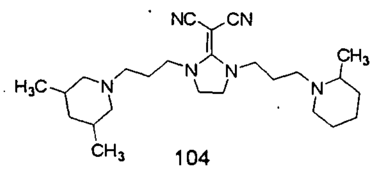
104

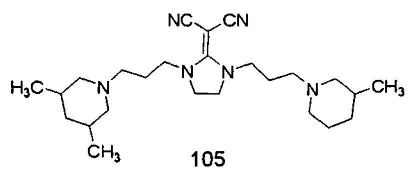
105

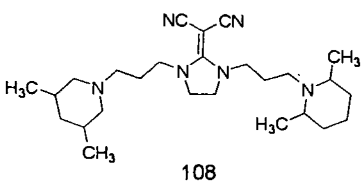
108

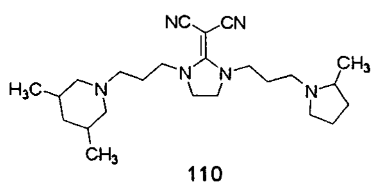
110

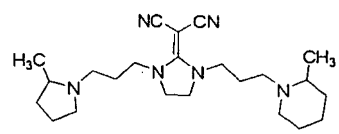
111

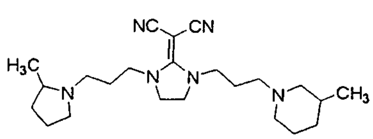
112

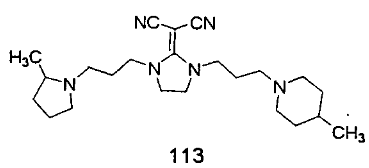
113

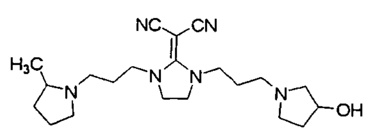
114

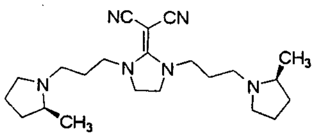
115

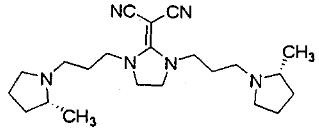
116

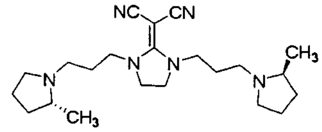
117

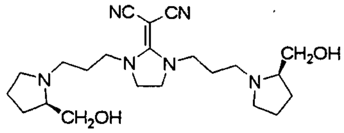

118

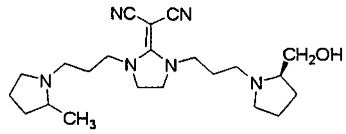

119

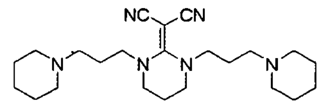

122

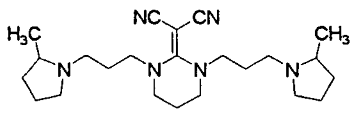

123

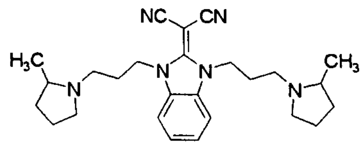

124

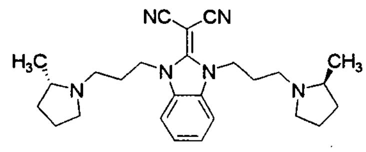

125

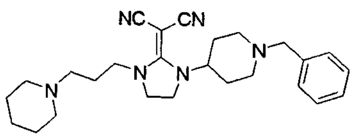

126

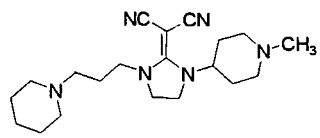

129

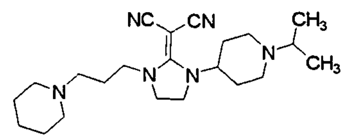

130

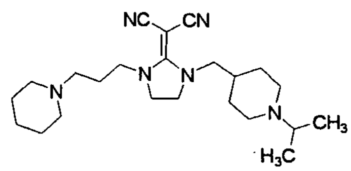

133

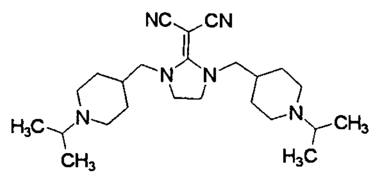

134

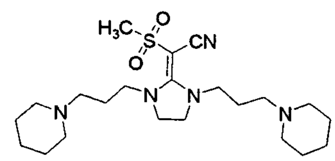

136

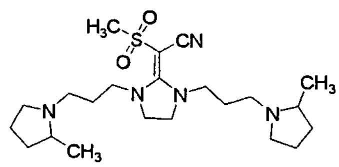

137

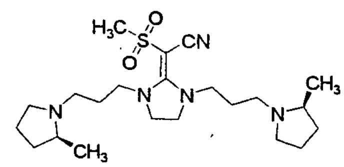

138

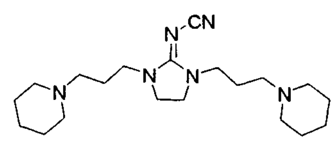

139

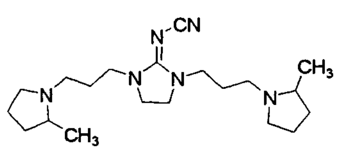

140

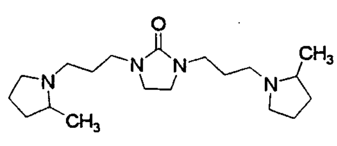

141

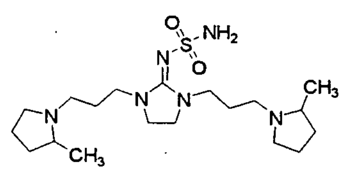

142

Further, among the compounds as illustrated in Table 7 to Table 9, preferred examples of the compound include compounds 143, 144, 147, 148, 149, 151, 153, 155, 157, 158, 159, 160, 161, 162, 163, 165, 166, 168, 169, 170, 172, 173, 174, 175, 176, 178, 179, 180, 181 and the like. The chemical structures of these compounds are shown below. More preferred examples of the compound include compounds 143, 144, 149, 159, 160, 161, 165, 166, 175, 176, 181 and the like.

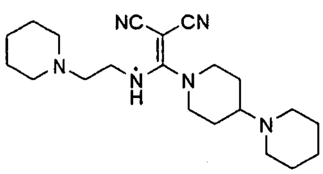

143

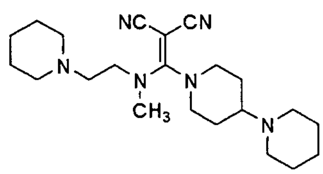

144

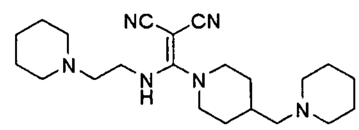

147

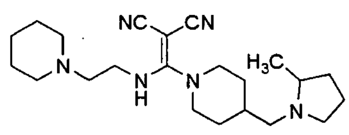

148

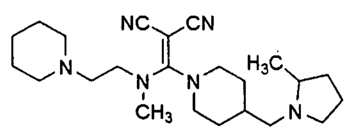

149

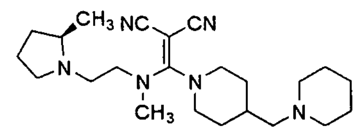

151

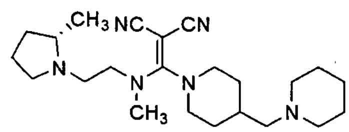

153

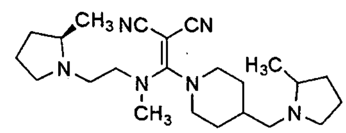

155

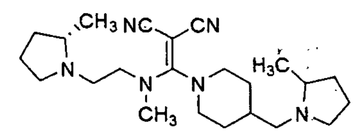

157

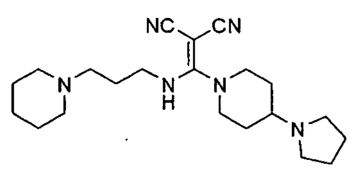

158

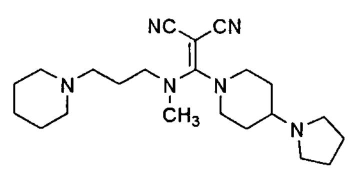

159

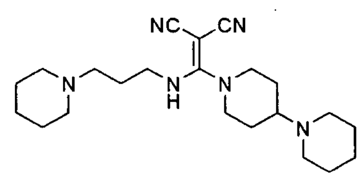

160

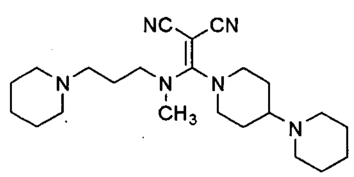

161

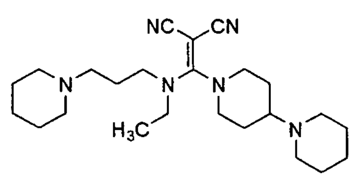

162

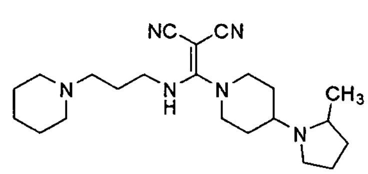

163

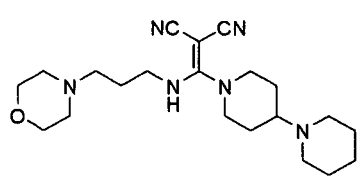

165

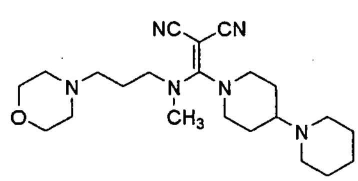

166

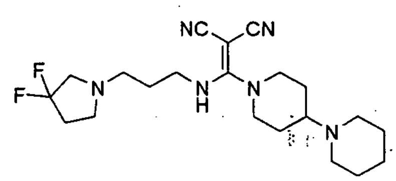

168

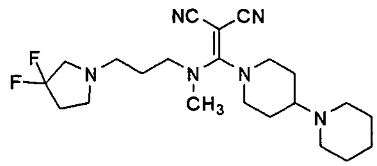

169

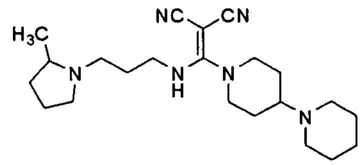

170

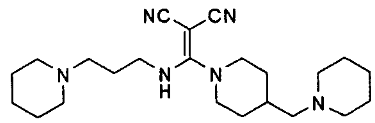

172

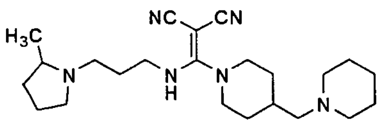

173

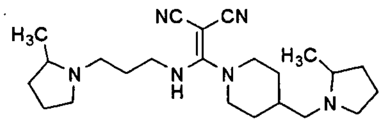

174

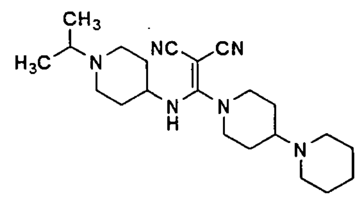

175

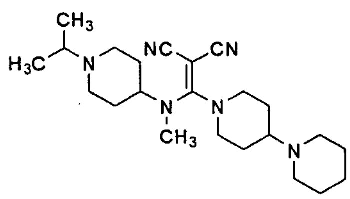

176

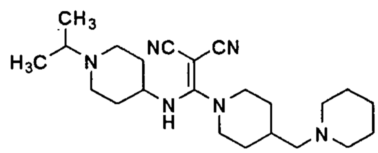

178

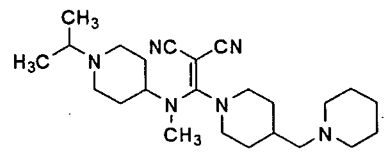

179

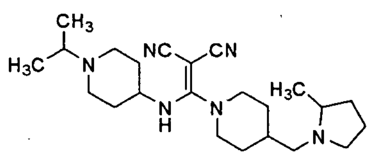

180

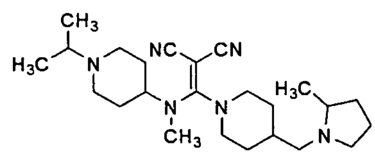

181

Further, among the compounds as illustrated in Table 10 to Table 15, preferred examples of the compound include compounds 202, 205, 213, 215, 219, 236 and the like. The chemical structures of these compounds are shown below. More preferred examples of the compound include compounds 193, 216, 226, 231, 233, 247 and the like.

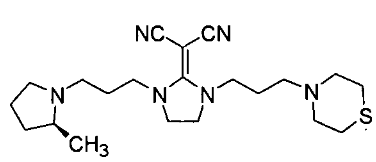

202

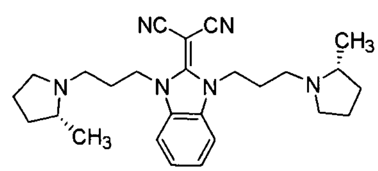

205

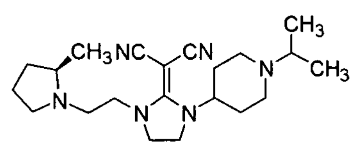

210

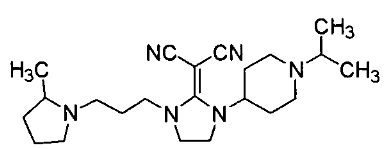

219

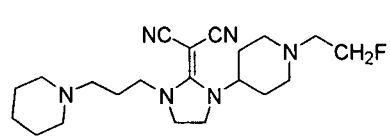

222

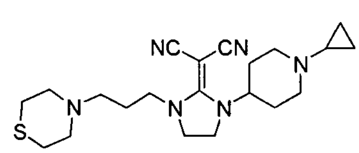

236

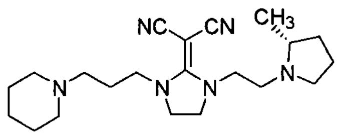

193

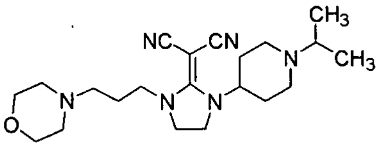

216

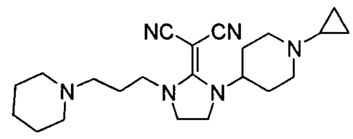

226

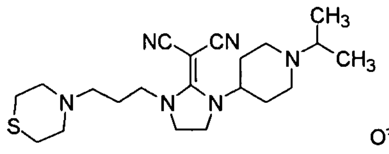

231

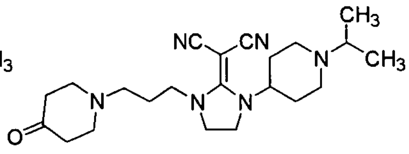

233

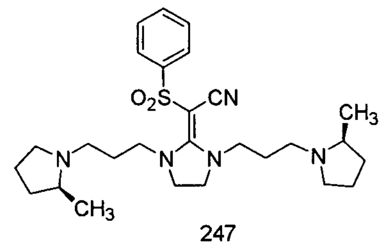

247

Hereinafter, a pharmacological action of Compound (I) will be described with reference to Test examples.

Test example 1: Histamine $H_3$ receptor binding assay

To the striata of Sprague-Dawley male rats (6 weeks of age), a 10-fold volume of lysis buffer {50 mmol/L [tris (hydroxyamino-methyl)ethane (Tris)]/HCl (pH 7.4), 5 mmol/L ethylenediaminetetraacetic acid (EDTA), a protease inhibitor mixture [1 mg/mL phenylmethylsulfonyl fluoride (PMSF), 1 μg/mL pepstatin A, 1 μg/mL leupeptin, 1 μg/mL chymostatin]} was added and the striata were homogenized. The resulting homogenate was centrifuged (30,000 x g, 4°C, 30 min), and the obtained precipitate was resuspended in a 10-fold volume of lysis buffer, followed by centrifugation under the same condition. To the obtained precipitate, a 3-fold volume of lysis buffer was added to suspend the precipitate, and the resulting tissue homogenate was used for a test.

A test compound was dissolved in an assay buffer [50 mmol/L Tris/HCl (pH 7.4), 5 mmol/L EDTA], and [$^3$H]-N$^\alpha$-methylhistamine (1 nmol/L) and the tissue homogenate (20 μg, in terms of protein amount) were added thereto, and then, the mixture was incubated at 25°C for 1 hour. Then, the reaction was terminated by rapid suction filtration on a glass filter [GF/B: Whatman, previously soaked in 0.3 w/v% polyethylenimine for about 30 min]. Then, the glass filter was washed three times with 3 mL of assay buffer. This glass filter was transferred into a scintillation vial and dried at 60°C for 30 minutes. Then, 0.5 mL of Ultimagold (Perkin Elmer) was added thereto, and the radioactivity (binding amount of [$^3$H]-N$^\alpha$- methylhistamine) was measured using a liquid scintillation counter. The obtained value was used as a binding amount in the presence of the test compound. The same procedure as described above was carried out in the absence of the test compound, and the binding amount of [$^3$H]-N$^\alpha$-methylhistamine obtained at this time was used as a binding amount in the absence of the test compound. Further, the binding amount of [$^3$H]-N$^\alpha$-methylhistamine obtained in the presence of 10 μmol/L thioperamide was used as a non-specific binding amount. An inhibitory action of [$^3$H]-N$^\alpha$-methylhistamine binding was represented by an inhibition ratio in terms of 1 μmol/L of the test compound calculated by the following Equation (1).

$$\text{Inhibition ratio (\%)} = \{1 - [(\text{Binding amount in the presence of test compound}) - (\text{Non-specific binding amount})] / [(\text{Binding amount in the absence of test compound}) - (\text{Non-specific binding amount})]\} \times 100 \quad \cdots (1)$$

Incidentally, with regard to [$^3$H]-N$^\alpha$-methylhistamine binding to the rat $H_3$ receptor, the dissociation constant Kd and the maximum number of binding sites Bmax were calculated to be 0.57 ± 0.04 nmol/L and 250 ± 20 fmol/mg protein, respectively.

Compounds 2, 4 to 11, 13, 17, 18, 20, 27, 33, 40, 42, 46 to 66, 68 to 71, 76, 78, 80, 82, 83, 85 to 120, 122 to 127, 129, 130, 133, 134, 136 to 141, 143, 144, 147 to 149, 151, 153, 155, 157 to 163, 165, 166, 168 to 170, 172 to 176, 178 to 181, 187 to 191, 193 to 203, 205, 207, 210, 213 to 216, 219, 222, 226 to 236, 238, 243, 244, 246, 247 and 250

showed an inhibition ratio of 80% or higher at 1 μmol/L.

That is, it was shown that Compound (I) has an excellent binding activity against a histamine $H_3$ receptor. Test example 2: Inhibitory effect on histamine release suppressive action of histamine $H_3$ receptor agonist

To the forebrains of Sprague-Dawley male rats (6 to 8 weeks of age), a 30-fold volume of a 0.32 mol/L aqueous sucrose solution was added and the forebrains were homogenized. The resulting homogenate was centrifuged (1,000 x g, 4˚C, 10 min), and to the obtained supernatant, an equivalent volume of Krebs-Ringer solution [120 mmol/L sodium chloride, 0.8 mmol/L potassium chloride, 2.6 mmol/L calcium chloride, 0.67 mmol/L magnesium sulfate, 1.2 mmol/L potassium dihydrogen phosphate, 27.5 mmol/L sodium bicarbonate, 10 mmol/L glucose (pH 7.3)] was added, and the resulting mixture was centrifuged (10,000 x g, 4˚C, 15 min). To the obtained precipitate, a 15-fold volume of Krebs-Ringer solution was added to suspend the precipitate. Then, the concentration of protein in the resulting tissue homogenate (synaptosomes) was measured and used for a test.

To the synaptosomes (300 μg in terms of protein amount) prepared by the above-described method, a [$^3$H]-histidine solution [final concentration: 0.4 mmol/L (740 kBq)/mL]: 6 μl of a 37 MBq/mL (specific activity: 1.85 TBq/mL) solution] was added, and the total volume was adjusted to 300 μL with Krebs-Ringer solution. Then, the resulting mixture was incubated at 37˚C for 30 minutes and centrifuged (10,000 x g, 25˚C, 3 min). To the resulting precipitate (synaptosomes), 300 μL of Krebs-Ringer solution was added, and the mixture was incubated at 37˚C for 5 minutes and centrifuged (10,000 x g, 25˚C, 3 min). This procedure was repeated 5 times, whereby washing of synaptosomes was carried out. The resulting synaptosomes were suspended in 100 μL of Krebs-Ringer solution, and the resulting suspension was used for the following procedure.

A test compound and $N^{\alpha}$-methylhistamine were separately dissolved in Krebs-Ringer solution to give a concentration of 20 μmol/L (the resulting mixtures were referred to as a test compound solution and a $N^{\alpha}$-methylhistamine solution, respectively). To 100 μL of the above-described suspended synaptosomes (300 μg in terms of protein amount), 140 μL of Krebs-Ringer solution was added. Then, 30 μL of the test compound solution and 30 μL of the $N^{\alpha}$-methylhistamine solution were added thereto, and the resulting mixture was incubated at 37˚C for 2 minutes. Then, an equivalent volume of a stimulating solution [64 mmol/L sodium chloride, 56.8 mmol/L potassium chloride, 2.6 mmol/L calcium chloride, 0.67 mmol/L magnesium sulfate, 1.2 mmol/L potassium dihydrogen phosphate, 27.5 mmol/L sodium bicarbonate, 10 mmol/L glucose (pH 7.3)] (final potassium concentration: 30 mmol/L) was added, and the resulting mixture was incubated at 37˚C for 2 minutes to release [$^3$H]-histamine. The reaction mixture was centrifuged (10,000 x g, 25˚C, 3 min), and the obtained supernatant was passed through Amberlite CG-50 column (250 μL), and the non-adsorbed fractions were discarded. Then, 2 mL of 50 mmol/L [tris(hydroxyamino- methyl)ethane] /hydrochloric acid (HCl) (pH 7.5) (Tris) was passed through the column 3 times, and 2 mL of distilled water, and 1.5 mL of a 0.2 mol/L aqueous sodium hydroxide solution were passed through the column in this order, and the eluted fractions were discarded. Then, 2 mL of a 0.2 mol/L aqueous sodium hydroxide solution was passed through the column, and the eluted fraction was recovered in a scintillation vial. Then, 8 mL of Ultimagold (Perkin Elmer) was added to the recovered eluted solution, and the radioactivity ([$^3$H]-histamine) was measured using a liquid scintillation counter. The inhibitory action of the test compound on the histamine release suppressive action of $N^{\alpha}$-methylhistamine was evaluated by obtaining the inhibition ratio to the histamine release suppressive action in the presence of 1 μmol/L $N^{\alpha}$-methylhistamine and 1 μmol/L of the test compound. The inhibition ratio of the [$^3$H]-histamine release suppressive action was calculated using the following Equation (2).

$$\text{Inhibition ratio (\%)} = [(\text{Release amount in the presence of test compound}) - (\text{Release amount in control})] / [(\text{Release amount in blank}) - (\text{Release amount in control})] \times 100 \quad \cdots (2)$$

Release amount in control: Release amount of [$^3$H]-histamine in the presence of 1 μmol/L $N^{\alpha}$-methylhistamine

Release amount in the presence of test compound: Release amount of [$^3$H]-histamine in the presence of 1 μmol/L $N^{\alpha}$-methylhistamine and test compound

Release amount in blank: Release amount of [$^3$H]-histamine in the absence of 1 μmol/L $N^{\alpha}$-methylhistamine and test compound.

Compounds 6, 7, 10, 11, 47, 51, 56, 57, 58, 64, 65, 71, 103, 111, 113, 115, 119, 122, 124, 126, 129, 130, 137, 138, 143, 144, 149, 159 to 161, 165, 166, 175, 176, 181, 188, 189, 193, 205, 213, 215, 216, 226, 231, 233 to 236 and 247 showed an inhibition ratio of 70% or higher at 1 μmol/L.

Thus, it was shown that Compound (I) has an inhibitory action against the action of a histamine $H_3$ receptor agonist ($N^{\alpha}$-methylhistamine).

It is known that a histamine $H_3$ receptor is an autoreceptor and the release of histamine from the synaptosomes is suppressed by an agonist for the receptor (Journal of Pharmacology and Experimental Therapeutics, vol. 263, pp. 304-310, 1992; Journal of Pharmacology and Experimental Therapeutics, vol. 305, pp. 887-896, 2003).

In view of this, it is considered that Compound (I) has a histamine $H_3$ receptor antagonism and/or inverse agonism and is useful as a therapeutic agent and/or a preventive agent for various diseases associated with a histamine $H_3$ receptor such as allergy, inflammation, a cardiovascular disease (for example, hypertension, hypotension and the like), gastrointestinal disorder (for example, inappropriate secretion of acid or gastrointestinal hormones, movement disorder and the like), central nervous system disorder including impaired attention and cognitive disability (for example, Alzheimer's disease, ADHD, memory function impairment due to aging and the like), central nervous system psychiatric disorder (for example, anxiety, schizophrenic disorder and the like), epilepsy, convulsion, obesity, sleep disorder (for example, sleep attack, sleep apnea, insomnia, disturbance of circadian rhythm and the like), acute pain, chronic pain, neuropathic pain and the like.

Although Compound (I) or a pharmaceutically acceptable salt thereof can be administered alone as such, it is generally prefered to offer them in the form of various pharmaceutical preparations. Further, such pharmaceutical preparations are to be used in animals or humans.

The pharmaceutical preparation of the present invention can comprise Compound (I) or a pharmaceutically acceptable salt thereof alone as an active ingredient or in a combination with any other active ingredient for the therapy for other diseases. Further, such a pharmaceutical preparation can be produced by mixing the active ingredient with one or more pharmaceutically acceptable carriers and then according to any method well known in the technical field of pharmaceutics.

As for the administration route, it is preferred to select the most effective administration route in the treatment. Examples thereof include oral administration and administration such as intravenous administration.

Examples of the dosage form include tablets, injections and the like.

A suitable dosage form for oral administration, for example, a tablet or the like can be prepared by using an excipient such as lactose, a disintegrator such as starch, a lubricant such as magnesium stearate, a binder such as hydroxypropyl cellulose or the like.

A suitable dosage form for parenteral administration, for example, an injection can be prepared by using a salt solution, glucose solution, a mixture of an aqueous salt solution and glucose solution or the like.

The dose and the frequency of administration of Compound (I) or a pharmaceutically acceptable salt thereof may vary depending on the dosage form, age and body weight of a patient, nature or seriousness of the symptom to be treated and the like. However, in the oral administration, in general, a dose of 0.01 to 1000 mg, preferably 0.05 to 500 mg is administered to an adult patient once to several times a day. In parenteral administration such as intravenous administration, in general, a dose of 0.001 to 1000 mg, preferably 0.01 to 300 mg is administered to an adult patient once to several times a day. However, these doses and frequencies of administration vary depending on the various conditions described above.

Hereinafter, the present invention will be described more specifically with reference to Examples, and Reference examples. However, the scope of the present invention is not limited to these Examples.

Unless otherwise stated, proton nuclear magnetic resonance spectrum ($^1$H-NMR) to be used in Examples was measured at 270 MHz or 300 MHz. Further, in proton nuclear magnetic resonance spectrum, exchangeable hydrogen may not be clearly observed depending on the compound and measurement conditions, and hydrogen atom on quaternary nitrogen atom may be observed in the case where the compound is in a hydrochloride salt form. Incidentally, a symbol "br" means a broad signal.

Example 1

[(2-Piperidinoethylamino)(3-piperidinopropylamino) methylene]malononitrile (Compound 1)

[Bis(methylthio)methylene]malononitrile (300 mg, 1.76 mmol) was dissolved in THF (4 mL), and a solution obtained by dissolving 3-piperidinopropylamine (251 mg, 1.76 mmol) in THF (1 mL) was added thereto. After stirring the mixture at room temperature for 1.5 hours, a solution obtained by dissolving 1-(2-aminoethyl) piperidine (226 mg, 1.76 mmol) in THF (1 mL) was added thereto, followed by stirring at room temperature for 1.5 hours, and further stirring at 30˚C for 5 hours. After cooling, the resulting mixture was added with water, and the pH of the mixture was adjusted to 4 with 2 mol/L hydrochloric acid. Then, the pH of the mixture was adjusted to 9 with a saturated aqueous sodium hydrogen carbonate solution, and the precipitated solid was obtained by filtration. The obtained solid was washed with water, triturated with diisopropyl ether and recrystallized from n-hexane/ethyl acetate, to obtain the titled compound (273 mg, 45%) as a white solid.

Example 2

[Bis(3-piperidinopropylamino)methylene]malononitrile (Compound 2)

[Bis(methylthio)methylene]malononitrile (300 mg, 1.76 mmol) was dissolved in THF (4 mL) and a solution of 3-piperidinopropylamine (251 mg, 1.76 mmol) in THF (1 mL) was added thereto. After stirring the mixture at room temperature for 2 hours, the mixture was added with a solution of 3-piperidinopropylamine (251 mg, 1.76 mmol) in THF (1 mL) and stirred at room temperature for 16 hours. Then, a solution of 3-piperidinopropylamine (75.2 mg, 0.529 mmol) in THF (0.5mL) was added thereto, followed by stirring at room temperature for 1.5 hours, and further stirring at 30˚C for 5 hours. After cooling, the mixture was added with water and the pH of the mixture was adjusted to 5 with 2 mol/L hydrochloric acid. Then, the pH of the mixture was adjusted to 9 with saturated aqueous sodium hydrogencarbonate solution and the precipitated solid was collected by filtration. The obtained solid was washed with water and triturated with diisopropylether, followed by recrystallizing from n-hexane/ethyl acetate to obtain the titled compound (266 mg, 42.0%) as a white solid.
$^1$H NMR ($CDCl_3$, δppm): 1.44-1.50 (m, 4H), 1.57-1.64 (m, 8H), 1.71-1.80 (m, 4H), 2.39-2.45 (m, 12H), 3.43 (dd, J = 5.7, 11.0 Hz, 4H), 7.41 (brs, 2H).
Melting point: 110-112˚C.

Example 3

[Bis(3-morpholinopropylamino)methylene]malononitrile (Compound 3)

In a similar manner to Example 2, Compound 3 was obtained by using [bis(methylthio)methylene]malononitrile (0.243 g, 1.42 mmol) and N-(3-aminopropyl)morpholine (327 mL, 2.24 mmol) (yield: 10.9%).
$^1$H NMR ($CDCl_3$, δppm): 1.76-1.85 (m, 4H), 2.48-2.54 (m, 12H), 3.44-3.50 (m, 4H), 3.75 (t, J = 4.7 Hz, 8H), 7.10 (br s, 2H).

Example 4

{1,3-Bis[2-(1-methylpyrrolidin-2-yl)ethyl] imidazolidin-2-ylidene}malononitrile (Compound 4) (Step 1)

[Bis(methylthio)methylene]malononitrile (3.00 g, 17.6 mmol) was dissolved in THF (20 mL) and the solution was added with ethylenediamine (1.20 mL, 18.0 mmol), followed by stirring at room temperature for 1.5 hours. Then, the mixture was added with diisopropylether (35 mL), stirred under ice-cooilng for 2.5 hours and the precipitated white solid was collected by filtration to obtain (imidazolidin-2-ylidene)malononitrile (2.02 g, 85.5%) .
$^1$H NMR (DMSO-$d_6$, δppm): 3.55 (s, 4H), 8.16 (br s, 2H).

(Step 2)

2-(2-Chloroethyl)-1-methylpyrrolidine hydrochloride (605 mg, 3.28 mmol) was dissolved in DMF (5 ml) and the solution was added with (imidazolidin-2-ylidene)malononitrile (200 mg, 1.49 mmol) obtained in Step 1, potassium carbonate (1.03 g, 7.45 mmol) and sodium iodide (227 mg, 1.49 mmol). The mixture was stirred at room temperature for 1 hour, followed by stirring at 50˚C for 3 hours and at 70˚C for 3 hours. Then, the mixture was added with 2-(2-chloroethyl)-1-methylpyrrolidine hydrochloride (330 mg, 1.79 mmol) and potassium carbonate (247 mg, 1.78 mmol), followed by stirring at 70˚C for 19 hours. After cooling, the mixture was added with water and extracted with ethyl acetate. The organic layer was dried over anhydrous magnesium sulfate and the solvent was evaporated under reduced pressure. The residue was triturated with diisopropylether and recrystallized from n-hexane/ethyl acetate (20:1) to obtain the titled compound (402 mg, 76%).
$^1$H NMR ($CDCl_3$, δppm):1.54-1.91 (m, 8H), 1.93-2.06 (m, 4H), 2.13-2.22 (m, 4H), 2.34 (s, 6H), 3.03-3.10 (m, 2H), 3.39-3.71 (m, 8H).
Melting point: 121-123˚C.
APCIMS m/z: $[M+H]^+$357.

Example 5

[1,3-Bis (2-piperidinoethyl)imidazolidin-2-ylidene] malononitrile (Compound 5)

(Step 1)

[Bis(methylthio)methylene]malononitrile (3.00 g, 17.6 mmol) was dissolved in THF (20 mL) and the solution was added with N,N-bis (2-hydroxyethyl)ethylenediamine (2.66 g, 18.0 mmol), followed by heating under reflux for 1 hour. After cooling, the mixture was added with diisopropylether (10 mL) and stirred under ice-cooling for 1.5 hours. The precipitated white solid was collected by filtration to obtain [1,3-bis (2-hydroxyethyl)imidazolidin -2-ylidene]malononitrile (3.73 g, 95.3%). $^1$H NMR (DMSO-$d_6$, δppm) : 3.47-3.55 (m, 4H), 3.57-3.87 (m, 4H), 3.73-3.87 (m, 4H).

(Step 2)

[1,3-Bis (2-hydroxyethyl)imidazolidin-2-ylidene] malononitrile (500 mg, 2.25 mmol) obtained in Step 1 was dissolved in dichloromethane (5 mL), and the solution was sequentially added with triethylamine (0.942 mL, 6.76 mmol) and methanesulfonylchloride (0.384 mL, 4.96 mmol). After stirring the mixture at room temperature for 1 hour, the mixture was added with saturated brine and extracted with ethyl acetate. The organic layer was washed with saturated brine, dried over anhydrous magnesium sufate and the solvent was evaporated under reduced pressure to obtain an oily substance (790mg).

The obtained oily substance was dissolved in 1,4-dioxane (10 mL) and the solution was added with piperidine (1.03 mL, 10.4 mmol), followed by heating under reflux for 16 hours. After cooling, the precipitated crystal was collected by filtration and the filtrate was concentrated. The residue was purified by silica gel column chromatography (ethyl acetate) and triturated with diisopropylether to obtain the titled compound (534 mg, 66.6%) as a white solid.
$^1$H NMR ($CDCl_3$, δppm) : 1.38-1.50 (m, 4H), 1.50-1.60 (m, 8H), 2.39-2.47 (m, 8H), 2.60 (t, J = 6.4 Hz, 4H), 3.63 (t, J = 6.4 Hz,4H), 3.70 (s,4H).
Melting point: 104-106˚C.

Example 6

{1,3-Bis[2-(2-methylpyrrolidin-1-yl)ethyl] imidazolidin-2-ylidene}malononitrile (Compound 6)

[1,3-Bis (2-hydroxyethyl)imidazolidin-2-ylidene] malononitrile (350 mg, 1.57 mmol) obtained in Step 1 of Example 5 was dissolved in dichloromethane (5mL) and the solution was sequentially added with triethylamine (0.527 mL, 3.78 mmol) and methanesulfonylchloride (0.267 mL, 3.45 mmol). After stirring the mixture at room temperature for 1.5 hours, the mixture was added with triethylamine (0.109 mL, 0.790 mmol) and methanesulfonylchloride (0.036 mL, 0.47 mmol), followed by stirring at room temperature for 3.5 hours. The mixture was added with saturated brine and extracted with ethyl acetate. The organic layer was washed with saturated brine, dried over anhydrous magnesium sulfate, and the solvent was evaporated under reduced pressure to obtain an oily substance (443 mg).

The obtained oily substance was dissolved in 1,4-dioxane (10 mL), and the solution was added with potassium carbonate (648 mg, 4.68 mmol), 2-methylpyrrolidine (0.431 mL, 4.22 mmol) and sodium iodide (0.176 g, 1.17 mmol). After stirring at 60˚C for 64 hours, the mixture was added with water and extracted with ethyl acetate. The organic layer was washed with saturated brine, dried over anhydrous magnesium sulfate and the solvent was evaporated under reduced pressure. The residue was purified by silica gel column chromatography (ethyl acetate) and the obtained soild was washed with diisopropylether to obtain the titled compound (0.112 g, 20.0%) as a white solid.
$^1$H NMR ($CDCl_3$,δppm) : 1.07 (d, J = 6.1 Hz, 6H), 1.28-1.45 (m, 2H), 1.65-1.80 (m, 4H), 1.85-1.99 (m, 2H), 2.22 (q, J = 8.6 Hz, 2H),2.32-2.46 6 (m, 4H), 3.03-3.20 (m, 4H), 3.40-3.70 (m, 4H), 3.76-3.89 (m, 4H) .
Melting point: 114-116˚C.

Example 7

[1-(4-Piperidinobutyl)-3-(2-piperidinoethyl) imidazolidin-2-ylidene]malononitrile (Compound 7)

(Step 1)

[Bis(methylthio)methylene]malononitrile (10.0 g, 58.7 mmol) was dissolved in THF (67 mL) and under ice-cooling, a solution of N-(2-hydroxyethyl)ethylenediamine (6.24 g, 59.9 mmol) was dissolved in THF (15 mL). After stirring at room temperature for 3 hours, the mixture was added with diisopropylether (100 mL), followed by stirring for 2 hours

under ice-cooling. The precipitated white solid was collected by filtration to obtain [1-(2-hydroxyethyl)imidazolidin-2-ylidene]malononitrile (9.96 g, 95.2%).
$^1$H NMR (DMSO-$d_6$, δppm) : 3.39-3.64 (m, 6H), 3.72-3.82 (m,2H).

(Step 2)

[1-(2-Hydroxyethyl)imidazolidin-2-ylidene] malononitrile (2.00 g, 11.2 mmol) obtained in the Step 1 was dissolved in DMF (20 mL) and the solution was sequentially added with potassium carbonate (3.10 g, 22.4 mmol) and 1-chloro-4-iodobutane (2.75 mL, 22.4 mmol). After stirring the mixture at room temperature for 71 hours, potassium carbonate (3.10 g, 22.4 mmol) and 1-chloro-4-iodobutane (2.75 mL, 22.4 mmol) were added, followed by stirring at 35˚C for 6 hours and at a room temperature for 14 hours. The mixture was added with water and extracted with ethyl acetate. The organic layer was washed with saturated brine, dried over anhydrous magnesium sulfate and the solvent was evaporated under reduced pressure. The residue was washed with diisopropylether to obtain [1-(4-chlorobutyl)-3-(2-hydroxyethyl)imidazolidin-2-ylidene]malononitrile (2.55 g, 84.6%) as a white solid.
$^1$H NMR ($CDCl_3$, δppm) : 1.87-1.99 (m, 2H), 2.12-2.24 (m, 2H), 3.61-3.76 (m, 12H).

(Step 3)

[1-(4-Chlorobutyl)-3-(2-hydroxyethyl)imidazolidin-2-ylidene]malononitrile (1.50 g, 5.58 mmol) obtained in the Step 2 was dissolved in dichloromethane (15 mL) and the solution was sequentially added with triethylamine (0.930 mL, 6.70 mmol) and methanesulfonylchloride (0.475 mL, 6.14 mmol). After stirring at room temperature for 3 hours, the mixture was added with saturated brine and extracted with ethyl acetate. The organic layer was washed with saturated brine and the solvent was evaporated under reduced pressure. The residue (1.87 g) was used to the next reaction without further purification.

The obtained residue (1.87 g) was dissolved in DMF (18 mL) and the solution was added with piperidine (2.67 mL, 27.0 mmol) and potassium iodide (1.79 g, 10.8 mmol), followed by stirring at 80˚C for 4 hours. After cooling, the mixture was added with saturated brine and extracted with ethyl acetate. The organic layer was washed with saturated brine, dried over anhydrous magnesium sulfate and the solvent was evaporated under reduced pressure. The residue was washed with diisopropylether to obtain the titled compound (1.28 g, 61.7%) as a white solid.
$^1$H NMR ($CDCl_3$, δppm) : 1.38-1.75 (m, 16H), 2.28-2.49 (m, 10H), 2.59 (t, J = 6.3 Hz, 2H), 3.51-3.76 (m, 8H).
Melting point: 78-79˚C.

Example 8

[1-(4-Piperidinobutyl)-3-(3-piperidinopropyl) imidazolidin-2-ylidene]malononitrile (Compound 8)

(Step 1)

[Bis(methylthio)methylene]malononitrile (10.2 g, 59.9 mmol) was dissolved in THF (68 mL) and under ice-cooling, a solution of N-(3-hydroxypropyl)ethylenediamine (7.24 g, 61.3 mmol) in THF (10 mL) was added thereto. After stirring at room temperature for 2 hours, the mixture was added with ethyl acetate/diisopropylether (1:1) (70 mL) and under ice-cooling, the mixture was stirred for 1 hour. The precipitated white solid was collected by filtration to obtain [1-(3-hydroxypropyl) imidazolidin-2-ylidene]malononitrile (9.48 g, 82.4%).
$^1$H NMR (DMSO-$d_6$, δppm) : 1.65-1.79 (m, 2H), 3.38-3.55 (m,6H), 3.63-3.75 (m, 2H), 4.54 (t, J = 4.6 Hz, 1H), 7. 87 (br s, 1H) .

(Step 2)

[1-(3-Hydroxypropyl)imidazolidin-2-ylidene] malononitrile (1.00 g, 5.21 mmol) obtained in the Step 1 was dissolved in DMF (10 mL) and the solution was sequentially added with potassium carbonate (1.44 g, 10.4 mmol) and 1-chloro-4-iodobutane (1.27 mL, 10.4 mmol). After stirring at room temperature for 18 hours, the mixture was added with saturated brine and extractd with ethyl acetate. The organic layer was washed with saturated brine, dried over anhydrous magnesium sulfate and the solvent was evaporated under reduced pressure. The residue was washed with diisopropylether to obtain [1-(4-chlorobutyl)-3-(3-hydroxypropyl)imidazolidin-2-ylidene]malononitrile (1.35 g, 91.8%) as a white solid.
$^1$H NMR ($CDCl_3$, δppm) : 1.80-2.00 (m, 6H), 3.53-3.78 (m, 12H) .

(Step 3)

[1-(4-Chlorobutyl)-3-(3-hydroxypropyl)imidazolidin-2-ylidene]malononitrile (0.500 g, 1.77 mmol) obtained in the Step 2 was dissolved in dichloromethane (5 mL) and the solution was sequentially added with triethylamine (0.296 mL, 2.12 mmol) and methanesulfonylchloride (0.150 mL, 1.95 mmol) . After stirring at room temperature for 1.5 hours, the mixture was added with triethylamine (0.148 mL, 1.06 mmol) and methanesulfonylchloride (0.068 mL, 0.89 mmol), followed by stirring at room temperature for 0.3 hours.

The mixture was added with saturated brine and extracted with ethyl acetate. The organic layer was washed with saturated brine and the solvent was evaporated under reduced pressure. The residue (0.657g) was used to the next reaction without further purification.

The obtained residue (0.657 g) was dissolved in DMF (10 mL) and the solution was added with piperidine (0.867 mL, 8.85 mmol) and sodium iodide (0.587 g, 3.54 mmol), followed by stirring at 80˚C for 0.4 hour. After cooling, the mixture was added with saturated brine and extractd with ethyl acetate. The organic layer was washed with saturated brine, dried over anhydrous magnesium sulfate and the solvent was evaporated under reduced pressure. The residue was dissolved in DMF (7 mL) and the solution was added with piperidine (0.525 mL, 5.31 mmol) and sodium iodide (0.294 g, 1.77 mmol), followed by stirring at 80˚C for 2.3 hours. After cooling, the mixture was added with saturated brine and extracted with ethyl acetate. The organic layer was washed with saturated brine, dried over anhydrous magnesium sulfate and the solvent was evaporated under reduced pressure. The residue was washed with diisopropylether to obtain the titled compound (0.546 g, 77.4%) as a white solid.
$^1$H NMR ($CDCl_3$, δppm) : 1.37-1.78 (m, 16H), 1.78-1.92 (m, 2H), 2.27-2.45 (m, 12H), 3.50-3.65 (m, 8H).
Melting point: 88-90˚C.

Example 9

[1,3-Bis(4-piperidinobutyl)imidazolidin-2-ylidene] malononitrile (Compound 9)

(Step 1)

(Imidazolidin-2-ylidene)malononitrile (0.50 g, 3.73 mmol) obtained in Step 1 of Example 4 was dissolved in DMF (5 mL) and the solution was sequentially added with potassium carbonate (1.54 g, 11.2 mmol) and 1-chloro-4-iodobutane (2.28 mL, 18.7 mmol). After stirring at room temperature for 16.5 hours, the mixture was added with potassium carbonate (1.54 g, 11.2 mmol) and 1-chloro-4-iodobutane (2.28 mL, 18.7 mmol), followed by stirring at room temperature for 5 hours. The mixture was added with saturated brine and extracted with ethyl acetate. The organic layer was washed with saturated brine, dried over anhydrous magnesium sulfate and the solvent was evaporated under reduced pressure. The residue was purified by silica gel column chromatography (n-hexane/ethyl acetate (1:1 to 3:7) to obtain [1,3-bis(4-chlorobutyl)imidazolidin -2-ylidene]malononitrile (1.09 g, 92.7%).
$^1$H NMR ($CDCl_3$, δppm) : 1.80-1.90 (m, 8H), 3.53-3.65 (m, 12H).

(Step 2)

[1,3-Bis(4-chlorobutyl)imidazolidin-2-ylidene] malononitrile (1.09 g, 3.46 mmol) obtained in the Step 1 was dissolved in DMF (10 mL) and the solution was added with piperidine (1.71 mL, 17.3 mmol) and potassium iodide (1.15 g, 6.92 mmol), followed by stirring at 80˚C for 4 hours. After cooling, the mixture was added with saturated brine and extracted with ethyl acetate. The organic layer was washed with saturated brine, dried over anhydrous magnesium sulfate and the solvent was evaporated under reduced pressure. The residue was washed with diisopropylether to obtain the titled compound (1.20 g, 84.1%) as a white solid.
$^1$H NMR ($CDCl_3$, δppm) : 1.37-1.77 (m, 20H), 2.27-2.42 (m, 12H), 3.49-3.62 (m, 8H).
Melting point: 96-98˚C.

Example 10

[1-(2-Piperidinoethyl)-3-(3-piperidinopropyl) imidazolidin-2-ylidene]malononitrile (Compound 10)

(Step 1)

[1-(2-Hydroxyethyl)imidazolidin-2-ylidene] malononitrile (2.00 g, 11.2 mmol) obtained in Step 1 of Example 7 was dissolved in DMF (20 mL) and the solution was sequentially added with potassium carbonate (3.10 g, 22.4 mmol) and 1,3-dibromopropane (5.70 mL, 56.1 mmol) After stirring at room temperature for 24.5 hours, the mixture was added

with potassium carbonate (3.85 g, 27.9 mmol) and 1,3-dibromopropane (5 mL, 49.3 mmol), followed by stirring at room temperature for 71 hours and then at 35˚C for 6 hours. The mixture was further added with potassium carbonate (3.10 g, 22.4 mmol) and 1,3-dibromopropane (5.70 mL, 56.1 mmol), stirred at room temperature for 13 hours, and added with water, followed by extracting with ethyl acetate. The organic layer was washed with saturated brine, dried over anhydrous magnesium sulfate and the solvent was evaporated under reduced pressure. The residue was washed with diisopropylether to obtain [1-(3-bromopropyl)-3-(2-hydroxyethyl)imidazolidin-2-ylidene]malononitrile (2.23 g, 66.7%) as a white solid.
$^1$H NMR ($CDCl_3$, δppm) : 2.20-2.34 (m, 2H), 3.48 (t, J=6.2Hz, 2H), 3.62-4.00 (m, 10H).

(Step 2)

[1-(3-Bromopropyl)-3-(2-hydroxyethyl)imidazolidin-2-ylidene]malononitrile (8.30 g, 27.8 mmol) obtained in the Step 1 was dissolved in dichloromethane (70 mL) and the solution was sequentially added with triethylamine (5.83 mL, 41.8 mmol) and methanesulfonylchloride (2.16 mL, 27.9 mmol). After stirring at room temperature for 0.5 hours, the mixture was added with saturated brine and extracted with ethyl acetate. The organic layer was washed with saturated brine and the solvent was evaporated under reduced pressue. The residue was purified by silica gel column chromatography (n-hexane/ethyl acetate (1:5) to ethyl acetate) to obtain [1-(3-bromopropyl)-3-(2-methanesulfonyloxyethyl) imidazolidin-2-ylidene] malononitrile (5.33 g, 50.9%) as a white solid.
$^1$H $NM_R$ ($CDCl_3$, δppm) : 2.21-2.34 (m, 2H), 3.07 (s, 3H), 3.49 (t, J = 6.3 Hz, 2H), 3.61-3.85 (m, 6H), 3.92 (t, J = 4.9 Hz, 2H), 4.51 (t, J =4.9 Hz, 2H).

(Step 3)

(1-(3-Bromopropyl)-3-(2-methanesulfonyloxyethyl) imidazolidin-2-ylidene]malononitrile (5.33 g) obtained in the Step 2 was dissolved in 1,4-dioxane (50 mL) and the solution was added with piperidine (7.01 mL, 70.9 mmol), followed by heating under reflux for 15 hours. After cooling, the precipitated white solid was collected by filtration and the filtrate was concentrated. After purifying the residue by silica gel column chromatography (ethyl acetate), the residue was triturated with n-hexane/diisopropylether (1:1) to obtain the titled compound (4.16 g, 79.5%) as a white powder.
$^1$H NMR ($CDCl_3$,δppm): 1.36-1.62 (m, 12H), 1.79-1.93 (m, 2H), 2.30-2.47 (m, 10H), 2.59 (t, J = 6.5 Hz, 2H), 3.53-3.76 (m, 8H).
Melting point: 88-89˚C.

Example 11

{1-[2-(2-Methylpyrrolidin-1-yl)ethyl]-3-[3-(2-methylpyrrolidin-1-yl)propyl]imidazolidin-2-ylidene} malononitrile (Compound 11)

[1-(3-Bromopropyl)-3-(2-methanesulfonyloxyethyl) imidazolidin-2-ylidene]malononitrile (0.15 g, 0.4 mmol) obtained in Step 2 of Example 10 was dissolved in 1,4-dioxane (2 mL) and the solution was added with 2-methylpyrrolidine (0.21 mL, 2.0 mmol), followed by stirring at 90˚C for 8 hours. After cooling, the mixture was concentrated. The residue was purified by silica gel column chromatography (ethyl acetate) and triturated with n-hexane/diisopropylether (1:1) to obtain the titled compound (0.10 g, 67.9%).
$^1$H NMR ($CDCl_3$, δppm) : 1.06 (d, J = 6.0 Hz, 3H), 1.07 (d,J=6.1 Hz, 3H), 1.33-1.45 (m, 2H), 1.65-1.78 (m, 4H), 1.81-1.97 (m, 4H), 2.07-2.17 (m, 2H), 2.21-2.31 (m, 2H), 2.34-2.42 (m, 2H), 2.79 (td, J =8.0,11.8 Hz, 1H), 3.05-3.18 (m, 3H), 3.43-3.70 (m, 6H), 3.77-3.87 (m, 2H).
APCIMS m/z: $[M+H]^+$371.
Melting point: 69-71˚C.

Example 12

{1-[2-(4-Carbamoylpiperidino)ethyl]-3-(3-piperidinopropyl)imidazolidin-2-ylidene}malononitrile (Compound 12)

[1-(3-Bromopropyl)-3-(2-hydroxyethyl)imidazolidin-2-ylidene]malononitrile (0.30 g, 0.10 mmol) obtained in Step 1 of Exmaple 10 was dissolved in DMF (0.2 mL) and the solution was added with piperidine (0.025 g, 0.30 mmol), potassium carbonate (0.07 g, 0.49 mmol) and potassium iodide (0.017 g, 0.10 mmol), followed by stirring at room temperature for 6.5 hours. After concentrating the mixture, the residue was dissolved in DMF (0.7 mL) and the solution was added with N-methyl isatoic acid resin (0.20 g, 2.0 mmol/g, 0.40 mmol), followed by stirring at room temperature overnight. After removing the resin, the solvent was evaporated under reduced pressue. The residue was purified by

BONDESIL-SCX (manufactured by VARIAN) (chloroform to 2 mol/L ammonia-methanol) to obtain a colorless oily substance (0.013 g, 44%).

The colorless oily substance obtained above was dissolved in DMF (0.3 mL) and the solution was added with carbon tetrachloride (0.30 ml) and triphenylphosphine resin (0.086 g, 3.0 mmol/g, 0.25 mmol), followed by stirring at room temperature for 15.5 hours. After removing the resin, the solvent was evaporated under reduced pressue. The residue was purified by silica gel column chromatography (chloroform/methanol (10:1)) to obtain a yellow oily substance.

The yellow oily substance obtained above was dissolved in 1,4-dioxane (0.2 ml) and the solution was added with piperidine-4-carboxylic amide (0.03 g, 0.25 mmol), potassium carbonate (0.07 g, 0.49 mmol) and potassium iodide (0.08 g, 0.05 mmol), followed by stirring at 90˚C for 25.5 hours. After cooling, the mixture was concentrated. The residue was dissolved in DMF (0.7 mL) and the solution was added with N-methyl isatoic acid resin (0.20 g, 2.0 mmol/g, 0.40 mmol), followed by stirring at room temperature overnight. After removing the resin, the solvent was evaporated under reduced pressure. The residue was purified by BONDESIL-SCX (manufactured by VARIAN) (chloroform to 2 mol/L ammonia-methanol) to obtain the titled compound (0.006 g, 14.5% (3 steps)) as a yellow oily substance.
APCIMS m/z: $[M+H]^+$414.

Example 13

{1-[2-(3,5-Dimethylpiperidino)ethyl]-3-(3-piperidinopropyl)imidazolidin-2-ylidene}malononitrile (Compound 13)

In a similar manner to Example 12, the titled compound (0.005 g, 12.6% (3 steps)) was obtained as a yellow oily substance by using [1-(3-bromopropyl)-3-(2-hydroxyethyl)imidazolidin-2-ylidene]malononitrile (0.30 g, 0.10 mmol) obtained in Step 1 of Example 10, piperidine (0.025 g, 0.30 mmol) and 3,5-dimethylpiperidine (0.028 g, 0.25 mmol) in place of piperidine-4-carboxylic amide.
APCIMS m/z: $[M+H]^+$399.

Example 14

[1-(2-Morpholinoethyl)-3-(3-piperidinopropyl) imidazolidin-2-ylidene]malononitrile (Compound 14)

In a similar manner to Example 12, the titled compound (0.005 g, 13.4% (3 steps)) was obtained as a yellow oily substance by using [1-(3-bromopropyl)-3-(2-hydroxyethyl)imidazolidin-2-ylidene]malononitrile (0.30 g, 0.10 mmol) obtained in Step 1 of Exmaple 10, piperidine (0.025 g, 0.30 mmol) and morpholine (0.022 g, 0.25 mmol) in place of piperidine-4-carboxylic amide.
APCIMS m/z: $[M+H]^+$373.

Example 15

{1-[2-(1,2,3,6-Tetrahydropyridin-1-yl)ethyl]-3-(3-piperidinopropyl)imidazolidin-2-ylidene}malononitrile (Compound 15)

In a similar manner to Example 12, the titled compound (0.006 g, 16.3% (3 steps)) was obtained as a yellow oily substance by using [1-(3-bromopropyl)-3-(2-hydroxyethyl)imidazolidin-2-ylidene]malononitrile (0.30 g, 0.10 mmol) obtained in Step 1 of Example 10, piperidine (0.025 g, 0.30 mmol) and 1,2,3,6-tetrahydropyridine (0.021 g,0 .25 mmol) in place of piperidine-4-carboxylic amide.
APCIMS m/z: $[M+H]^+$369.

Example 16

{1-[2-(4-Carbamoylpiperidino)ethyl]-3-[3-(3,5-dimethylpiperidino)propyl]imidazolidin-2-ylidene} malononitrile (Compound 16)

In a similar manner to Example 12, the titled compound (0.008 g, 18.1% (3 steps)) was obtained as a yellow oily substance by using [1-(3-bromopropyl)-3-(2-hydroxyethyl)imidazolidin-2-ylidene]malononitrile (0.30 g, 0.10 mmol) obtained in Step 1 of Example 10, 3,5-dimethylpiperidine (0.034 g, 0.30 mmol) in place of piperidine and piperidine-4-carboxylic amide (0.03 g, 0.25 mmol).
APCIMS m/z: $[M+H]^+$442.

Example 17

{1-[2-(3,5-Dimethylpiperidino)ethyl]-3-[3-(3,5-dimethylpiperidino)propyl]imidazolidin-2-ylidene} malononitrile (Compound 17)

In a similar manner to Example 12, the titled compound (0.008 g, 18.8% (3 steps)) was obtained as a yellow oily substance by using [1-(3-bromopropyl)-3-(2-hydroxyethyl)imidazolidin-2-ylidene]malononitrile (0.30 g, 0.10 mmol) obtained in Step 1 of Example 10, 3,5-dimethylpiperidine (0.034 g, 0.30 mmol) in place of piperidine and 3,5-dimethylpiperidine (0.028 g, 0.25 mmol) in place of piperidine-4-carboxylic amide.
APCIMS m/z: $[M+H]^+$427.

Example 18

{1-[2-(Cis-3,5-dimethylpiperidino)ethyl]-3-[3-(pyrrolidin-1-yl)propyl]imidazolidin-2-ylidene} malononitrile (Compound 18)

In a similar manner to Example 12, the titled compound (0.008 g, 20.8% (3 steps)) was obtained as a yellow oily substance by using [1-(3-bromopropyl)-3-(2-hydroxyethyl)imidazolidin-2-ylidene]malononitrile (0.30 g, 0.10 mmol) obtained in Step 1 of Example 10, pyrrolidine (0.021 g, 0.30 mmol) in place of piperidine and 3,5-dimethylpiperidine (0.028 g, 0.25 mmol) in place of piperidine-4-carboxylic amide.
APCIMS m/z: $[M+H]^+$385.

Example 19

{1-[2-(4-Methylpiperazin-1-yl)ethyl]-3-[3-(pyrrolidin-1-yl)propyl]imidazolidin-2-ylidene} malononitrile (Compound 19)

In a similar manner to Example 12, the titled compound (0.005 g, 13.5 % (3 steps)) was obtained as a yellow oily substance by using [1-(3-bromopropyl)-3-(2-hydroxyethyl)imidazolidin-2-ylidene]malononitrile (0.30 g, 0.10 mmol) obtained in Step 1 of Example 10, pyrrolidine (0.021 g, 0.30 mmol) in place of piperidine and 1-methylpiperazine (0.025 g, 0.25 mmol) in place of piperidine-4-carboxylic amide.
APCIMS m/z: $[M+H]^+$372.

Example 20

{1-[2-(Cis-3,5-dimethylpiperidino)ethyl]-3-[3-(2-methylpyrrolidin-1-yl)propyl]imidazolidin-2-ylidene} malononitrile (Compound 20)

In a similar manner to Example 12, the titled compound (0.006 g, 15.1% (3 steps)) was obtained as a yellow oily substance by using [1-(3-bromopropyl)-3-(2-hydroxyethyl)imidazolidin-2-ylidene]malononitrile (0.30 g, 0.10 mmol) obtained in Step 1 of Example 10, 2-methylpyrrolidine (0.026 g, 0.30 mmol) in place of piperidine and 3,5-dimethylpiperidine (0.028 g, 0.25 mmol) in place of piperidine-4-carboxylic amide.
APCIMS m/z: $[M+H]^+$399.

Example 21

{1-[3-(2-Methylpyrrolidin-1-yl)propyl]-3-(2-Morpholinoethyl)imidazolidin-2-ylidene}malononitrile (Compound 21)

In a similar manner to Example 12, the titled compound (0.005 g, 13.4% (3 steps)) was obtained as a yellow oily substance by using [1-(3-bromopropyl)-3-(2-hydroxyethyl)imidazolidin-2-ylidene]malononitrile (0.30 g, 0.10 mmol) obtained in Step 1 of Exampl 10, 2-methylpyrrolidine (0.026 g, 0.30 mmol) in place of piperidine and morpholine (0.022 g, 0.25 mmol) in place of piperidine-4-carboxylic amide.
APCIMS m/z: $[M+H]^+$373.

Example 22

{1-[2-(1,2,3,6-Tetrahydropyridin-1-yl)ethyl]-3-[3-(2-methylpyrrolidin-1-yl)propyl]imidazolidin-2-ylidene} malononitrile (Compound 22)

In a similar manner to Example 12, the titled compound (0.006 g, 16.3% (3 steps)) was obtained as a yellow

oily substance by using [1-(3-bromopropyl)-3-(2-hydroxyethyl)imidazolidin-2-ylidene]malononitrile (0.30 g, 0.10 mmol) obtained in Step 1 of Example 10, 2-methylpyrrolidine (0.026 g, 0.30 mmol) in place of piperidine and 1,2,3,6-tetrahydropyridine (0.021 g, 0.25 mmol) in place of piperidine-4-carboxylic amide.
APCIMS m/z: $[M+H]^+$369.

Example 23

{1-[2-(3,5-Dimethylpiperidino)ethyl]-3-[3-(4-methylpiperazin-1-yl)propyl]imidazolidin-2-ylidene} malononitrile (Compound 23)

In a similar manner to Example 12, the titled compound (0.002 g, 4.8% (3 steps)) was obtained as a yellow oily substance by using [1-(3-bromopropyl)-3-(2-hydroxyethyl)imidazolidin-2-ylidene]malononitrile (0.30 g, 0.10 mmol) obtained in Step 1 of Example 10, 1-methylpiperazine (0.030 g, 0.30 mmol) in place of piperidine and 3,5-dimethylpiperidine (0.028 g, 0.25 mmol) in place of piperidine-4-carboxylic amide.
APCIMS m/z: $[M+H]^+$414.

Example 24

{1-[2-(3,5-Dimethylpiperidino)ethyl]-3-(3-morpholinopropyl)imidazolidin-2-ylidene}malononitrile (Compound 24)

In a similar manner to Example 12, the titled compound (0.009 g, 22.5% (3 steps)) was obtained as a yellow oily substance by using [1-(3-bromopropyl)-3-(2-hydroxyethyl)imidazolidin-2-ylidene]malononitrile (0.30 g, 0.10 mmol) obtained in Step 1 of Example 10, morpholine (0.026 g, 0.30 mmol) in place of piperidine and 3,5-dimethylpiperidine (0.028 g, 0.25 mmol) in place of piperidine-4-carboxylic amide.
APCIMS m/z: $[M+H]^+$401.

Example 25

[1-(2-Morpholinoethyl)-3-(3-morpholinopropyl) imidazolidin-2-ylidene]malononitrile (Compound 25)

In a similar manner to Example 12, the titled compound (0.008 g, 21.4% (3 steps)) was obtained as a yellow oily substance by using [1-(3-bromopropyl)-3-(2-hydroxyethyl)imidazolidin-2-ylidene]malononitrile (0.30 g, 0.10 mmol) obtained in Step 1 of Example 10, morpholine (0.026 g, 0.30 mmol) in place of piperidine and morpholine (0.022 g, 0.25 mmol) in place of piperidine-4-carboxylic amide.
APCIMS m/z: $[M+H]^+$375.

Example 26

{1-[2-(4-Carbamoylpiperidino)ethyl]-3-[3-(1,2,3,6-tetrahydropyridin-1-yl)propyl]imidazolidin-2-ylidene} malononitrile (Compound 26)

In a similar manner to Example 12, the titled compound (0.009 g, 21.2% (3 steps)) was obtained as a yellow oily substance by using [1-(3-bromopropyl)-3-(2-hydroxyethyl)imidazolidin-2-ylidene]malononitrile (0.30 g, 0.10 mmol) obtained in Step 1 of Example 10, 1,2,3,6-tetrahydropyridine (0.025 g, 0.30 mmol) in place of piperidine and piperidine-4-carboxylic amide (0.03 g, 0.25 mmol).
APCIMS m/z: $[M+H]^+$412.

Example 27

{1-[3-(1,2,3,6-Tetrahydropyridin-1-yl)propyl]-3-[2-(cis-3,5-dimethylpiperidino)ethyl]imidazolidin-2-ylidene} malononitrile (Compound 27)

In a similar manner to Example 12, the titled compound (0.007 g, 17.7% (3 steps)) was obtained as a yellow oily substance by using [1-(3-bromopropyl)-3-(2-hydroxyethyl)imidazolidin-2-ylidene]malononitrile (0.30 g, 0.10 mmol) obtained in Step 1 of Example 10, 1,2,3,6-tetrahydropyridine (0.025 g, 0.30 mmol) in place of piperidine and 3,5-dimethylpiperidine (0.028 g, 0.25 mmol) in place of piperidine-4-carboxylic amide.
APCIMS m/z: $[M+H]^+$397.

Example 28

{1-[3-(1,2,3,6-Tetrahydropyridin-1-yl)propyl]-3-(2-morpholinoethyl)imidazolidin-2-ylidene}malononitrile (Compound 28)

In a similar manner to Example 12, the titled compound (0.008 g, 21.6% (3 steps)) was obtained as a yellow oily substance by using [1-(3-bromopropyl)-3-(2-hydroxyethyl)imidazolidin-2-ylidene]malononitrile (0.30 g, 0.10 mmol) obtained in Step 1 of Example 10, 1,2,3,6-tetrahydropyridine (0.026 g, 0.30 mmol) in place of piperidine and morpholine (0.022 g, 0.25 mmol) in place of piperidine-4-carboxylic amide.
APCIMS m/z: $[M+H]^+$371.

Example 29

{1-[2-(1,2,3,6-Tetrahydropyridin-1-yl)ethyl]-3-[3-(1,2,3,6-tetrahydropyridin-1-yl)propyl]imidazolidin-2-ylidene}malononitrile (Compound 29)

In a similar manner to Example 12, the titled compound (0.009 g, 24.6% (3 steps)) was obtained as a yellow oily substance by using [1-(3-bromopropyl)-3-(2-hydroxyethyl)imidazolidin-2-ylidene]malononitrile (0.30 g, 0.10 mmol) obtained in Step 1 of Example 10, 1,2,3,6-tetrahydropyridine (0.026 g, 0.30 mmol) in place of piperidine and 1,2,3,6-tetrahydropyridine (0.021 g, 0.25 mmol) in place of piperidine-4-carboxylic amide.
APCIMS m/z: $[M+H]^+$367.

Example 30

{1-[2-(4-Methylpiperazin-1-yl)ethyl]-3-(3-piperidinopropyl)imidazolidin-2-ylidene}malononitrile (Compound 30)

In a similar manner to Example 12, the titled compound (0.007 g, 18.2% (3 steps)) was obtained as a yellow oily substance by using [1-(3-bromopropyl)-3-(2-hydroxyethyl)imidazolidin-2-ylidene]malononitrile (0.30 g, 0.10 mmol) obtained in Step 1 of Example 10, piperidine (0.025 g, 0.30 mmol) and 1-methylpiperazine (0.025 g, 0.25 mmol) in place of piperidine-4-carboxylic amide.
APCIMS m/z: $[M+H]^+$385.

Example 31

{1-(2-Morpholinoethyl)-3-[3-(3,5-dimethylpiperidino)propyl]imidazolidin-2-ylidene} malononitrile (Compound 31)

In a similar manner to Example 12, the titled compound (0.007g, 17.5% (3 steps)) was obtained as a yellow oily substance by using [1-(3-bromopropyl)-3-(2-hydroxyethyl)imidazolidin-2-ylidene]malononitrile (0.30 g, 0.10 mmol) obtained in Step 1 of Exampl 10, 3,5-dimethylpiperidine (0.034g, 0.30 mmol) in place of piperidine and morpholine (0.022 g, 0.25 mmol) in place of piperidine-4-carboxylic amide.
APCIMS m/z: $[M+H]^+$400.

Example 32

{1-[2-(1,2,3,6-Tetrahydropyridin-1-yl)ethyl]-3-[3-(pyrrolidin-1-yl)propyl]imidazolidin-2-ylidene} malononitrile (Compound 32)

In a similar manner to Example 12, the titled compound (0.003 g, 8.5% (3 steps)) was obtained as a yellow oily substance by using [1-(3-bromopropyl)-3-(2-hydroxyethyl)imidazolidin-2-ylidene]malononitrile (0.30 g, 0.10 mmol) obtained in Step 1 of Example 10, pyrrolidine (0.021 g, 0.30 mmol) in place of piperidine and 1,2,3,6-tetrahydropyridine (0.021 g, 0.25 mmol) in place of piperidine-4-carboxylic amide.
APCIMS m/z: $[M+H]^+$355.

Example 33

{1-[3-(2-Methylpyrrolidin-1-yl)propyl]-3-(2-piperidinoethyl)imidazolidin-2-ylidene}malononitrile (Compound 33)

In a similar manner to Example 12, the titled compound (0.005 g, 13.5% (3 steps)) was obtained as a yellow oily substance by using [1-(3-bromopropyl)-3-(2-hydroxyethyl)imidazolidin-2-ylidene]malononitrile (0.30 g, 0.10 mmol)

obtained in Step 1 of Example 10, 2-methylpyrrolidine (0.026 g, 0.30 mmol) in place of piperidine and piperidine (0.021 g, 0.25 mmol) in place of piperidine-4-carboxylic amide.
APCIMS m/z: $[M+H]^+371$.

Example 34

{1-[2-(4-Carbamoylpiperidino)ethyl]-3-[3-(2-methylpyrrolidin-1-yl)propyl]imidazolidin-2-ylidene} malononitrile (Compound 34)

In a similar manner to Example 12, the titled compound (0.006 g, 14.5% (3 steps)) was obtained a yellow oily substance by using [1-(3-bromopropyl)-3-(2-hydroxyethyl)imidazolidin-2-ylidene]malononitrile (0.30 g, 0.10 mmol) obtained in Step 1 of Example 10, 2-methylpyrrolidine (0.026 g, 0.30 mmol) in place of piperidine and piperidine-4-carboxylic amide (0.03 g, 0.25 mmol).
APCIMS m/z: $[M+H]^+414$.

Example 35

{1-[2-(4-Methylpiperazin-1-yl)ethyl]-3-[3-(2-methylpyrrolidin-1-yl)propyl]imidazolidin-2-ylidenel malononitrile (Compound 35)

In a similar manner to Example 12, the titled compound (0.008 g, 20.8% (3 steps)) was obtained as a yellow oily substance by using [1-(3-bromopropyl)-3-(2-hydroxyethyl)imidazolidin-2-ylidene]malononitrile (0.30 g, 0.10 mmol) obtained in Step 1 of Example 10, 2-methylpyrrolidine (0.026 g, 0.30 mmol) in place of piperidine and 1-methylpiperazine (0.025 g, 0.25 mmol) in place of piperidine-4-carboxylic amide.
APCIMS m/z: $[M+H]^+386$.

Example 36

{1-[3-(4-Methylpiperazin-1-yl)propyl]-3-(2-piperidinoethyl)imidazolidin-2-ylidene}malononitrile (Compound 36)

In a similar manner to Example 12, the titled compound (0.004 g, 10.4% (3 steps)) was obtained as a yellow oily substance by using [1-(3-bromopropyl)-3-(2-hydroxyethyl)imidazolidin-2-ylidene]malononitrile (0.30 g, 0.10 mmol) obtained in Step 1 of Example 10, 1-methylpiperazine (0.030 g, 0.30 mmol) in place of piperidine and piperidine (0.021 g, 0.25 mmol) in place of piperidine-4-carboxylic amide.
APCIMS m/z: $[M+H]^+386$.

Example 37

{1-[3-(4-Methylpiperazin-1-yl)propyl]-3-[2-(pyrrolidin-1-yl)ethyl]imidazolidin-2-ylidene} malononitrile (Compound 37)

In a similar manner to Example 12, the titled compound (0.003 g, 8.1 % (3 steps)) was obtained as a yellow oily substance by using [1-(3-bromopropyl)-3-(2-hydroxyethyl)imidazolidin-2-ylidene]malononitrile (0.30 g, 0.10 mmol) obtained in Step 1 of Exampl 10, 1-methylpiperazine (0.030 g, 0.30 mmol) in place of piperidine and pyrrolidine (0.018 g, 0.25 mmol) in place of piperidine-4-carboxylic amide.
APCIMS m/z: $[M+H]^+372$.

Example 38

[1-(3-Morpholinopropyl)-3-(2-piperidinoethyl) imidazolidin-2-ylidene]malononitrile (Compound 38)

In a similar manner to Example 12, the titled compound (0.006 g, 16.1% (3 steps)) was obtained as a yellow oily substance by using [1-(3-bromopropyl)-3-(2-hydroxyethyl)imidazolidin-2-ylidene]malononitrile (0.30 g, 0.10 mmol) obtained in Step 1 of Example 10, morpholine (0.026 g, 0.30 mmol) in place of piperidine and piperidine (0.021 g, 0.25 mmol) in place of piperidine-4-carboxylic amide.
APCIMS m/z: $[M+H]^+373$.

Example 39

{1-[3-(1,2,3,6-Tetrahydropyridin-1-yl)propyl]-3-[2-(4-methylpiperazin-1-yl)ethyl]imidazolidin-2-ylidene} malononitrile (Compound 39)

In a similar manner to Example 12, the titled compound (0.009 g, 23.5% (3 steps)) was obtained as a yellow oily substance by using [1-(3-bromopropyl)-3-(2-hydroxyethyl)imidazolidin-2-ylidene]malononitrile (0.30 g, 0.10 mmol) obtained in Step 1 of Example 10, 1,2,3,6-tetrahydropyridine (0.025 g, 0.30 mmol) in place of piperidine and 1-methylpiperazine (0.025 g, 0.25 mmol) in place of piperidine-4-carboxylic amide.
APCIMS m/z: $[M+H]^+$384.

Example 40

{1-[3-(4-Cyanopiperidino)propyl]-3-(2-piperidinoethyl)imidazolidin-2-ylidene}malononitrile (Compound 40)

In a similar manner to Example 12, the titled compound (0.005 g, 12.7% (3 steps)) was obtained as a yellow oily substance by using [1-(3-bromopropyl)-3-(2-hydroxyethyl)imidazolidin-2-ylidene]malononitrile (0.30 g, 0.10 mmol) obtained in Step 1 of Exampl 10, 4-cyanopiperidine (0.033 g, 0.30 mmol) in place of piperidine and piperidine (0.021 g, 0.25 mmol) in place of piperidine-4-carboxylic amide.
APCIMS m/z: $[M+H]^+$396.

Example 41

2-{1-[2-(4-Carbamoylpiperidino)ethyl]-3-[3-(4-cyanopiperidino)propyl]imidazolidin-2-ylidene} malononitrile (Compound 41)

In a similar manner to Example 12, the titled compound (0.006 g, 13.7% (3 steps)) was obtained as a yellow oily substance by using [1-(3-bromopropyl)-3-(2-hydroxyethyl)imidazolidin-2-ylidene]malononitrile (0.30 g, 0.10 mmol) obtained in Step 1 of Example 10, 4-cyanopiperidine (0.033 g, 0.30 mmol) in place of piperidine and piperidine-4-carboxylic amide (0.03 g, 0.25 mmol).
APCIMS m/z: $[M+H]^+$439.

Example 42

{1-[3-(4-Cyanopiperidino)propyl]-3-[2-(3,5-dimethylpiperidino)ethyl]imidazolidin-2-ylidene} malononitrile (Compound 42)

In a similar manner to Example 12, the titled compound (0.005 g, 11.8% (3 steps)) was obtained as a yellow oily substance by using [1-(3-bromopropyl)-3-(2-hydroxyethyl)imidazolidin-2-ylidene]malononitrile (0.30 g, 0.10 mmol) obtained in Step 1 of Example 10, 4-cyanopiperidine (0.033 g, 0.30 mmol) in place of piperidine and 3,5-dimethylpiperidine (0.028 g, 0.25 mmol) in place of piperidine-4-carboxylic amide.
APCIMS m/z: $[M+H]^+$424.

Example 43

{1-[3-(4-Cyanopiperidino)propyl]-3-[2-(4-methylpiperazin-1-yl)ethyl]imidazolidin-2-ylidene} malononitrile (Compound 43)

In a similar manner to Example 12, the titled compound (0.003 g, 7.3% (3 steps)) was obtained as a yellow oily substance by using [1-(3-bromopropyl)-3-(2-hydroxyethyl)imidazolidin-2-ylidene]malononitrile (0.30 g, 0.10 mmol) obtained in Step 1 of Example 10, 4-cyanopiperidine (0.033 g, 0.30 mmol) in place of piperidine and 1-methylpiperazine (0.025 g, 0.25 mmol) in place of piperidine-4-carboxylic amide.
APCIMS m/z: $[M+H]^+$411.

Example 44

{1-[3-(4-Cyanopiperidino)propyl]-3-(2-morpholinoethyl)imidazolidin-2-ylidene}malononitrile (Compound 44)

In a similar manner to Example 12, the titled compound (0.005 g, 12.6% (3 steps)) was obtained as a yellow oily substance by using [1-(3-bromopropyl)-3-(2-hydroxyethyl)imidazolidin-2-ylidene]malononitrile (0.30 g, 0.10 mmol) obtained in Step 1 of Example 10, 4-cyanopiperidine (0.033 g, 0.30 mmol) in place of piperidine and morpholine (0.022 g, 0.25 mmol) in place of piperidine-4-carboxylic amide.
APCIMS m/z: $[M+H]^+$398.

Example 45

{1-[3-(4-Cyanopiperidino)propyl]-3-[2-(1,2,3,6-tetrahydropyridin-1-yl)ethyl]imidazolidin-2-ylidene} malononitrile (Compound 45)

In a similar manner to Example 12, the titled compound (0.006 g, 15.3% (3 steps)) was obtained as a yellow oily substance by using [1-(3-bromopropyl)-3-(2-hydroxyethyl)imidazolidin-2-ylidene]malononitrile (0.30 g, 0.10 mmol) obtained in Step 1 of Example 10, 4-cyanopiperidine (0.033 g, 0.30 mmol) in place of piperidine and 1,2,3,6-tetrahydropyridine (0.021 g, 0.25 mmol) in place of piperidine-4-carboxylic amide.
APCIMS m/z: $[M+H]^+$394.

Example 46

{1-[3-(Azepan-1-yl)propyl]-3-(2-piperidinoethyl) imidazolidin-2-ylidene}malononitrile (Compound 46)

(Step 1)

[1-(3-Hydroxypropyl)imidazolidin-2-ylidene] malononitrile (1.50 g, 7.81 mmol) obtained in Step 1 of Example 8 was dissolved in DMF (15 mL) and the solution was sequentially added with potassium carbonate (2.16 g, 15.6 mmol) and 1,2-dibromoethane (3.37 mL, 39.1 mmol), followed by stirring at room temperature for 71 hours and then, at 35˚C for 6 hours. After cooling to room temperature, the mixture was added with potassium carbonate (2.16 g, 15.6 mmol) and 1,2-dibromoethane (3.37 mL, 39.1 mmol), followed by stirring at room temperature for 36 hours. The mixture was added with saturated brine and extractd with ethyl acetate. The organic layer was washed with saturated brine, dried over anhydrous magnesium sulfate and the solvent was evaporated under reduced pressure. The residue was washed with a mixed solution of diisopropylether and ethyl acetate to obtain [1-(2-bromoethyl)-3-(3-hydroxypropyl)imidazolidin-2-ylidene]malononitrile (1.19 g, 51 %) as a white solid.
$^1$H NMR ($CDCl_3$, δppm) : 1.88-2.07 (m, 3H), 3.62-3.89 (m, 10H), 3.98 (t, J = 7.5 Hz, 2H).

(Step 2)

[1-(2-Bromoethyl)-3-(3-hydroxypropyl)imidazolidin-2-ylidene]malononitrile (1.00 g, 3.36 mmol) obtained in the Step 1 was dissolved in 1,4-dioxane (20 mL) and the solution was added with piperidine (0.996 mL, 10.1 mmol). After stirring at room temperature for 24 hours, the mixture was further added with piperidine (0.996 mL, 10.1 mmol) and stirred at room temperature for 19 hours and then, at 45˚C for 3 hours. After cooling, the mixture was added with saturated brine and extracted with ethyl acetate. The organic layer was dried over anhydrous magnesium sulfate and the solvent was evaporated under reduced pressue. The obtained white solid was washed with diisopropylether. Further, the solid was purified by BONDESIL-SCX (manufactured by VARIAN) (chloroform/ (2 mol/L ammonia-methanol) (9:1)) to obtain [1-(3-hydroxypropyl)-3-(2-piperidinoethyl)imidazolidin-2-ylidene]malononitrile (0.929 g, 91.4 %) as a white solid.
$^1$H NMR ($CDCl_3$, δppm) : 1.39-1.50 (m, 2H), 1.50-1.62 (m, 4H), 1.87-2.00 (m, 2H), 2.45-2.54 (m, 4H), 2.61 (t, J = 6.5 Hz, 2H), 3.58-3.80 (m, 10H).

(Step 3)

[1-(3-Hydroxypropyl)-3-(2-piperidinoethyl) imidazolidin-2-ylidene]malononitrile (0.300 g, 0.990 mmol) obtained in the Step 2 was dissolved in dichloromethane (3 mL) and the solution was sequentially added with triethylamine (0.276 mL, 1.98 mmol) and methanesulfonylchloride (0.092 mL, 1.19 mmol). After stirring at room temperature for 0.25 hour, the mixture was added with methanesulfonylchloride (0.008 mL, 0.10 mmol), followed by stirring at room temperature for 0.75 hour. The mixture was added with saturated brine and extracted with ethyl acetate. The organic layer was dried

over anhydrous magnesium sulfate and the solvent was evaporated under reduced pressure to obtain [1-(3-methanesulfonyloxypropyl)-3-(2-piperidinoethyl) imidazolidin-2-ylidene]malononitrile (0.373 g, 98.9 %) as a pale yellow solid.
$^1$H NMR ($CDCl_3$, δppm) : 1.39-1.50 (m, 2H), 1.50-1.65 (m, 4H), 2.12-2.23 (m, 2H), 2.39-2.48 (m, 4H), 2.60 (t, J = 6.5 Hz, 2H), 3.05 (s, 3H), 3.58-3.80 (m, 8H), 4.34 (t, J = 5.9 Hz, 2H).

(Step 4)

[1-(3-Methanesulfonyloxypropyl)-3-(2-piperidinoethyl)imidazolidin-2-ylidene]malononitrile (0.050 mg, 0.13 mmol) obtained in the Step 3 was dissolved in 1,4-dioxane (5 mL) and the solution was added with hexamethyleneimine (0.088 mL, 0.78 mmol) and potassium iodide (0.032g,0.20mmol). After stirring at 80˚C for 4.5 hours, the mixture was added with water and extracted with ethyl acetate. The organic layer was washed with saturated brine, dried over anhydrous magnesium sulfate and the solvent was evaporated under reduced pressure. The residue was washed with diisopropylether to obtain the titled compound (0.018 g, 36.6 %) as a white solid.
$^1$H NMR ($CDCl_3$, δppm) : 1.40-1.50 (m, 2H), 1.52-1.64 (m, 4H), 1.66-1.76 (m, 6H), 1.86-2.00 (m, 4H), 2.20-2.33 (m, 2H), 2.40-2.50 (m, 4H), 2.58-2.67 (m, 2H), 2.82-3.03 (m, 2H), 3.17-3.25 (m, 2H), 3.57-3.70 (m, 4H), 3.77 (s, 4H).
Melting point: 149-152˚C.

Example 47

{1-[2-(Azepan-1-yl)ethyl]-3-(3-piperidinopropyl) imidazolidin-2-ylidene}malononitrile (Compound 47)

(Step 1)

[1-(3-Bromopropyl)-3-(2-hydroxyethyl)imidazolidin-2-ylidene]malononitrile (1.50 g, 5.03 mmol) obtained in Step 1 of Example 10 was dissolved in 1,4-dioxane (35 mL) and the solution was added with piperidine (1.49 mL, 15.1 mmol). After stirring at room temperature for 24 hours, the mixture was added with piperidine (0.99 mL, 10.1 mmol), followed by stirring at room temperature for 19 hours and then at 45˚C for 3 hours. The mixture was added with saturated brine and extracted with ethyl acetate. The organic layer was dried over anhydrous magnesium sulfate and the solvent was evaporated under reduced pressure. The obtained solid was washed with diisopropylether to obtain [1-(2-hydroxyethyl)-3-(3-piperidinopropyl) imidazolidin-2-ylidene]malononitrile (1.33 g, 89.8 %) as a white solid.
$^1$H NMR ($CD_3OD$, δppm) : 1.42-1.53 (m, 2H), 1.55-1.67 (m, 4H), 1.81-1.95 (m, 2H), 2.34-2.49 (m, 6H), 3.50-3.84 (m, 10H). (Step 2)
[1-(2-Hydroxyethyl)-3-(3-piperidinopropyl) imidazolidin-2-ylidene]malononitrile (0.300 g, 0.990 mmol) obtained in the Step 1 was dissolved in dichloromethane (3 mL) and the solution was sequentially added with triethylamine (0.276 mL, 1.98 mmol) and methanesulfonylchloride (0.100 mL, 1.29 mmol). After stirring under ice-cooling for 0.3 hour, the mixture was added with saturated brine and extracted with ethyl acetate. The organic layer was washed with saturated brine and dried over dried over anhydrous magnesium sulfate. The solvent was evaporated under reduced pressure to obtain [1-(2-methanesulfonyloxyethyl)-3-(3-piperidinopropyl)imidazolidin-2-ylidene]malononitrile (0.333 g, 88:3 %) as a brown oily substance.
$^1$H NMR ($CDCl_3$, δppm) : 1.41-1.50 (m, 2H), 1.52-1.65 (m, 4H), 1.82-1.95 (m, 2H), 2.33-2.44 (m, 6H), 3.06 (s, 3H), 3.57-3.80 (m, 6H), 3.90 (t, J = 5.1 Hz, 2H), 4.51 (t, J = 5.1 Hz, 2H).

(Step 3)

[1-(2-Methanesulfonyloxyethyl)-3-(3-piperidinopropyl)imidazolidin-2-ylidene]malononitrile (0.050 mg, 0.13 mmol) obtained in the Step 2 was dissolved in 1,4-dioxane (5 mL) and the solution was added with hexamethyleneimine (0.087 mL, 0.79 mmol) and potassium iodide (0.032 g, 0.20 mmol). After stirring at 80˚C for 24 hours, the mixture was added with water and extracted with ethyl acetate. The organic layer was washed with saturated brine and dried over anhydrous magnesium sulfate and the solvent was evaporated under reduced pressure. The residue was washed with diisopropylether to obtain the titled compound (0.024 g, 48.2 %) as a white solid.
$^1$H NMR ($CDCl_3$, δppm) : 1.41-1.50 (m, 2H), 1.52-1.86 (m, 12H), 1.86-2.00 (m, 2H), 2.35-2.52 (m, 6H), 2.62-2.70 (m, 4H), 2.78 (t, J =6.1Hz,2H), 3.54-3.78 (m, 8H).
Melting point: 80-86˚C.

Example 48

[1-(3-Diethylaminopropyl)-3-(2-piperidinoethyl) imidazolidin-2-ylidene]malononitrile (Compound 48)

[1-(3-Methanesulfonyloxypropyl)-3-(2-piperidinoethyl)imidazolidin-2-ylidene]malononitrile (0.050 g, 0.13 mmol) obtained in Step 3 of Example 46 was dissolved in 1,4-dioxane(5 mL) and the solution was added with diethylamine (0.040 mL, 0.39 mmol) and sodium iodide (0.032 g, 0.20 mmol). After stirring at 80˚C for 17 hours, the mixture was further added with diethylamine (0.040 mL, 0.39 mmol) and stirred for 6 hours. The mixture was added with water and extracted with ethyl acetate. The organic layer was washed with saturated brine, dried over anhydrous magnesium sulfate and the solvent was evaporated under reduced pressure. The residue was purified by silica gel column chromatography (n-hexane/ethyl acetate (1:2) to ethyl acetate to chloroform/methanol (100:1)). The obtained crude crystal was washed with n-hexane to obtain the titled compound (0.009 g, 19.2 %) as a white solid.
$^1$H NMR ($CDCl_3$, δppm) : 1.02 (t, J = 7.1 Hz,6H), 1.39-1.50 (m, 2H), 1.50-1.61 (m, 4H), 1.77-1.90 (m, 2H), 2.38-2.64 (m, 12H), 3.57-3.77 (m, 8H).
Melting point: 58-61˚C.

Example 49

[1-(2-Diethylaminoethyl)-3-(3-piperidinopropyl) imidazolidin-2-ylidene]malononitrile (Compound 49)

[1-(2-Methanesulfonyloxyethyl)-3-(3-piperidinopropyl)imidazolidin-2-ylidene]malononitrile (0.300 g, 0.786 mmol) obtained in Step 2 of Example 47 was dissolved in 1,4-dioxane (30 mL) and the solution was added with diethylamine (0.817 mL, 7.90 mmol) and sodium iodide (0.177 g, 1.18 mmol). After stirring at 80˚C for 65 hours, the mixture was added with diethylamine (0.817 mL, 7.90 mmol), stirred at 80˚C for 4 hours, and further added with diethylamine (2.00 mL, 19.3 mmol) followed by stirring at 80˚C for 4 hours. The mixture was further added with diethylamine (2.00 mL, 19.3 mmol) and stirred at 80˚C for 16.5 hours. The mixture was added with water and extracted with ethyl acetate. The organic layer was washed with saturated brine, dried over anhydrous magnesium sulfate and the solvent was evaporated under reduced pressure. The residue was purified by silica gel column chromatography (chloroform/ (7mol/L ammonia-methanol) (10:1)). The obtained crude crystal was recrystallized from n-hexane/diisopropylether (1:1) to obtain the titled compound (0.044 g, 15.4 %) as a white solid.
$^1$H NMR ($CDCl_3$,δppm) : 1.01 (t, J = 7.1 Hz, 6H), 1.38-1.48 (m, 2H), 1.51-1.62 (m, 4H), 1.79-1.92 (m, 2H), 2.30-2.42 (m, 6H), 2.53 (q, J =7.1 Hz, 4H), 2.70 (t, J = 6.1 Hz, 2H), 3.54-3.65 (m, 6H), 3.67-3.78 (m,2H).
Melting point: 78-80˚C.

Example 50

{1-[3-(Cis-2,6-dimethylpiperidino)propyl]-3-(2-piperidinoethyl)imidazolidin-2-ylidene}malononitrile difumarate (Compound 50)

[1-(3-Methanesulfonyloxypropyl)-3-(2-piperidinoethyl)imidazolidin-2-ylidene]malononitrile (0.050 g, 0.131 mmol) obtained in Step 3 of Example 46 was dissolved in 1,4-dioxane (5 mL) and the solution was added with cis-2,6-dimethylpiperidine (0.106 mL, 3.14 mmol) and potassium iodide (0.032 g, 0.193 mmol). After stirring at 80˚C for 4.5 hours, the mixture was added with cis-2,6-dimethylpiperidine (0.106 mL, 3.14 mmol) and stirred at 80˚C for 24.5 hours. The mixture was added with water and extracted with ethyl acetate. The organic layer was washed with saturated brine, dried over anhydrous magnesium sulfate and the solvent was evaporated under reduced pressure. The residue was purified by silica gel column chromatography (n-hexane/ethyl acetate (1:2) to chloroform) and further purified by preparative thin-layer chromatography (TLC) (chloroform/(2 mol/L ammonia-methanol) (15:1)). The obtained oily substance (24 mg) was dissolved in methanol (2 mL) and the solution was added with 0.4 mol/L fumaric acid/methanol solution (0.300 mL, 0.120 mmol), followed by stirring at room temperature for 0.5 hour. The solvent was evaporated under reduced pressue and the obtained solid was washed with acetone to obtain the titled compound (0.027 g, 51.7 %) as a white solid.
$^1$H NMR ($CD_3OD$, δppm) : 1.40 (d, J = 6.4 Hz,6H), 1.55-1.68 (m, 5H), 1.75-1.88 (m, 5H), 1.88-2.15 (m, 4H), 3.03-3.47 (m, 10H), 3.56-3.65 (m, 2H), 3.70-3.78 (m, 4H), 3.84-3.93 (m, 2H), 6.69 (s, 4H).

Example 51

{1-[2-(Cis-2,6-dimethylpiperidino)ethyl]-3-(3-piperidinopropyl)imidazolidin-2-ylidene}malononitrile (Compound 51)

[1-(2-Methanesulfonyloxyethyl)-3-(3-piperidinopropyl)imidazolidin-2-ylidene]malononitrile (0.300 mg, 0.786

mmol) obtained in Step 2 of Example 47 was dissolved in 1,4-dioxane (30 mL) and the solution was added with cis-2,6-dimethylpiperidine (1.06 mL, 7.86 mmol) and potassium iodide (0.196 g, 1.19 mmol). After stirring at 80˚C for 25 hours, the mixture was added with cis-2,6-dimethylpiperidine (1.06 mL, 7.86 mmol), stirred at 80˚C for 6.5 hours and further added with cis-2,6-dimethylpiperidine (1.06 mL, 7.86 mmol), followed by stirring at 80˚C for 65 hours. The mixture was added with water and extracted with ethyl acetate. The organic layer was washed with saturated brine, dried over anhydrous magnesium sulfate and the solvent was evaporated under reduced pressure. After purified by silica gel column chromatography (chloroform/(2 mol/L ammonia-methanol) (9:1)), the residue was recrystallized from isopropyl alcohol to obtain the titled compound (0.045 g, 14.3 %) as a white solid.
$^1$H NMR ($CDCl_3$, δppm) : 1.12 (d, J = 6.1 Hz, 6H), 1.16-1.70 (m, 12H), 1.81-1.95 (m, 2H), 2.32-2.52 (m, 8H), 2.84-2.93 (m, 2H), 3.52-3.68 (m, 6H), 3.72-3.83 (m, 2H).
Melting point: 106-108˚C.

Example 52

{1-[3-(1,2,3,4-Tetrahydroisoquinolin-2-yl)propyl]-3-(2-piperidinoethyl)imidazolidin-2-ylidene}malononitrile (Compound 52)

[1-(3-Methanesulfonyloxypropyl)-3-(2-piperidinoethyl)imidazolidin-2-ylidene]malononitrile (0.100 g, 0.262 mmol) obtained in Step 3 of Example 46 was dissolved in 1,4-dioxane (3 mL) and the solution was added with 1,2,3,4-tetrahydroisoquinoline (0.394 mL, 3.14 mmol) and potassium iodide (0.065 g, 0.39 mmol). After stirring at 80˚C for 7.5 hours, the mixture was added with water and extracted with ethyl acetate. The organic layer was washed with saturated brine, dried over anhydrous magnesium sulfate and the solvent was evaporated under reduced pressure. The residue was purified by silica gel column chromatography (n-hexane/ethyl acetate (2:1 to 1:1) to ethyl acetate). The obtained crude crystal was washed with diisopropylether to obtain the titled compound (0.078 g, 71.4 %) as a white solid.
$^1$H NMR ($CDCl_3$, δppm) : 1.38-1.50 (m, 2H), 1.51-1.60 (m, 4H), 1.92-2.05 (m, 2H), 2.37-2.47 (m, 4H), 2.55-2.65 (m, 4H), 2.70-2.78 (m, 2H), 2.87-2.94 (m, 2H), 3.58-3.70 (m, 10H), 7.00-7.07 (m, 1H), 7.07-7.17 (m,3H).
Melting point: 79-81˚C.

Example 53

{1-[2-(1,2,3,4-Tetrahydroisoquinolin-2-yl)ethyl]-3-(3-piperidinopropyl)imidazolidin-2-ylidene}malononitrile (Compound 53)

[1-(2-Methanesulfonyloxyethyl)-3-(3-piperidinopropyl)imidazolidin-2-ylidene]malononitrile (0.100 g, 0.262 mmol) obtained in Step 2 of Example 47 was dissolved in 1,4-dioxane (3 mL) and the solution was added with 1,2,3,4-tetrahydroisoquinoline (0.394 mL, 3.14 mmol) and potassium iodide (0.065 g, 0.39 mmol). After stirring at 80˚C for 26 hours and further adding 1,2,3,4-tetrahydroisoquinoline (0.394 mL, 3.14 mmol), the mixture was stirred at 80˚C for 3 hours. The mixture was added with water and extracted with ethyl acetate. The organic layer was washed with saturated brine, dried over anhydrous magnesium sulfate and the solvent was evaporated under reduced pressure. The residue was purified by silica gel column chromatography (n-hexane/ethyl acetate (4:1 to 2:1) to ethyl acetate). The obtained crude crystal was washed with diisopropylether to obtain the titled compound (0.073 g, 66.8 %) as a white solid.
$^1$H NMR ($CDCl_3$,δppm) : 1.37-1.47 (m, 2H), 1.50-1.63 (m, 4H), 1.78-1.92 (m, 2H), 2.28-2.40 (m, 6H), 2.75-2.94 (m, 6H), 3.52-3.63 (m, 4H), 3.67-3.78 (m, 6H), 7.00-7.07 (m, 1H), 7.07-7.17 (m, 3H).
Melting point: 101-102˚C.

Example 54

{1-[2-(2-Methylpyrrolidin-1-yl)ethyl]-3-[3-(3-pyrrolidin-1-yl)propyl]imidazolidin-2-ylidene} malononitrile (Compound 54)

(Step 1)

[1-(2-Bromoethyl)-3-(3-hydroxypropyl)imidazolidin-2-ylidene]malononitrile (0.654 g) obtained in Step 1 of Example 46 was dissolved in 1,4-dioxane (10 mL) and the solution was added with 2-methylpyrrolidine (0.33 mL, 3.23 mmol), potassium carbonate (0.910 g, 6.58 mmol) and potassium iodide (0.364 g, 2.19 mmol). After stirring at room temperature for 35 hours, the mixture was further stirred at 40˚C for 17 hours. After cooling, the mixture was concentrated under reduced pressure. The residue was added with saturated aqueous sodium hydrogencarbonate solution and the extracted crystal was collected by filtration and washed with water. The obtained solid was triturated with diisopropylether to obtain {1-(3-hydroxypropyl)-3-[2-(2-methylpyrrolidin-1-yl)ethyl] imidazolidin-2-ylidene}malononitrile (0.269 g, 40 %)

as a white solid.
$^1$H NMR ($CDCl_3$, δppm) : 1.07 (d, J = 6.0 Hz,3H), 1.30-1.42 (m, 1H), 1.58-1.74 (m, 2H), 1.89-1.96 (m, 4H), 2.21-2.41 (m, 3H), 3.13-3.15 (m, 2H), 3.49-3.86 (m, 10H).

(Step 2)

{1-(3-Hydroxypropyl)-3-[2-(2-methylpyrrolidin-1-yl) ethyl]imidazolidin-2-ylidene}malononitrile (0.258 g, 0.850 mmol) obtained in the Step 1 was dissolved in dichloromethane (5 mL) and under ice-cooling, the solution was sequentially added with triethylamine (0.36 mL, 2.58 mmol) and methanesulfonylchloride (0.13 mL, 1.68 mmol). After stirring at room temperature for 24 hours, the mixture was added with saturated aqueous sodium hydrogencarbonate solution and extracted with ethyl acetate. The organic layer was washed with saturated brine and the solvent was evaporated under reduced pressure. The residue was triturated with diisopropylether/n-hexane to obtain a white solid (0.269 g). The obtained white solid (0.269 g) was dissolved in 1,4-dioxane (4 mL) and the solution was added with pyrrolid.ine (0.3 mL, 3.59 mmol), followed by stirring at 45˚C for 2 hours. After cooling, the mixture was added with saturated aqueous sodium hydrogencarbonate solution and extracted with ethyl acetate. The organic layer was washed with saturated brine, dried over anhydrous magnesium sulfate and the solvent was evaporated under reduced pressure. The residue was dissolved in diisopropylether under heating and after cotton-wool filtration, the filtrate was concentrated under reduced pressure. The obtained solid was recrystallized from diisopropylether to obtain the titled compound (0.166 g, 54.6 %) as a white solid.
$^1$H NMR ($CDCl_3$, δppm) : 1.07 (d, J = 6.0 Hz, 3H), 1.30-1.43 (m, 1H), 1.70-1.91 (m, 9H), 2.20-2.53 (m, 9H), 3.11-3.14 (m, 2H), 3.47-3.63 (m, 6H), 3.79-3.85 (m, 2H).
Melting point: 77-81˚C.

Example 55

{1-[2-((R)-2-methylpyrrolidin-1-yl)ethyl]-3-[3-((R)-2-methylpyrrolidin-1-yl)propyl]imidazolidin-2-ylidene}malononitrile (Compound 55)

(R)-2-methylpyrrolidinehydrobromate (0.20 g, 2.32 mmol) prepared in a similar manner to the method described in J. Org. Chem., Vol. 54, p.209 (1989) was dissolved in 1,4-dioxane (1 ml) and the solution was added with 2 mol/L aqueous sodium hydroxide solution (1.19 ml, 2.39 mmol), followed by stirring at room temperature for 30 minutes. Next, [1-(3-bromopropyl)-3-(2-methanesulfonyloxyethyl) imidazolidin-2-ylidene]malononitrile (0.24 g, 0.64 mmol) obtained in Step 2 of Example 10 was added with potassium carbonate (0.27 g, 1.93 mmol), sodium iodide (0.10 g, 0.64 mmol) and 1,4-dioxane (2 mL), followed by stirring at 90˚C for 7.5 hours. After cooling, the pH of the mixture was adjusted to 10 with saturated aqueous sodium hydrogencarbonate solution and the mixture was extracted with ethyl acetate. The organic layer was washed with saturated brine, dried over anhydrous magnesium sulfate and the solvent was evaporated under reduced pressure. After the residue was purified by silica gel column chromatography (ethyl acetate), the obtained solid was triturated with diisopropylether and the solid was recrystallized from n-hexane/diisopropylether (1:1) to obtain the titled compound (0.06, g, 25.1 %) .
$^1$H NMR ($CDCl_3$, δppm) : 1.06 (d, J = 6.2 Hz, 3H), 1.07 (d, J = 5.8 Hz, 3H), 1.32-1.45 (m, 2H), 1.65-1.79 (m, 4H), 1.81-1.97 (m, 4H), 2.05-2.18 (m, 2H), 2.21-2.30 (m, 2H), 2.32-2.42 (m, 2H), 2.79 (td, J = 8.1, 11.9Hz, 1H), 3.05-3.18 (m, 3H), 3.43-3.70 (m, 6H), 3.77-3.87 (m, 2H).
APCIMS m/z: $[M+H]^+$371.
Melting point: 74-75˚C.

Example 56

{1-[2-((S)-2-methylpyrrolidin-1-yl)ethyl]-3-[3-((S)-2-methylpyrrolidin-1-yl)propyl]imidazolidin-2-ylidene} malononitrile (Compound 56)

(S)-2-methylpyrrolidinehydrobromate (0.97 g, 5.87 mmol) prepared in a similar manner to the method described in J. Org. Chem., Vol. 54, p.209 (1989) was dissolved in 1,4-dioxane (3 ml) and the solution was added with 2 mol/L aqueous sodium hydroxide solution (3.0 ml, 6.03 mmol), followed by stirring at room temperature for 30 minutes. Next, [1-(3-bromopropyl)-3-(2-methanesulfonyloxyethyl) imidazolidin-2-ylidene]malononitrile (0.61 g, 1.63 mmol) obtained in Step 2 of Example 10 was added with potassium carbonate (0.68 g, 4.89 mmol), sodium iodide (0.24 g, 1.63 mmol) and 1,4-dioxane (5 mL), followed by strring at 90˚C for 16.5 hours. After cooling,the mixture was added with saturated brine and extracted with ethyl acetate. The organic layer was washed with saturated brine, dried over anhydrous magnesium sulfate and the solvent was evaporated under reduced pressure. After the residue was purified by silica gel column chromatography (chloroform/methanol (3:1)), the obtained solid was triturated with n-hexane/diisopropylether (10:1) to

obtain the titled compound (0.24 g, 40.2 %).
$^1$H NMR ($CDCl_3$, δppm) : 1.07 (m, 6H), 1.32-1.46 (m, 2H), 1.65-1.79 (m, 4H), 1.81-1.98 (m, 4H), 2.04-2.17 (m, 2H), 2.20-2.30 (m, 2H), 2.32-2.42 (m, 2H), 2.79 (td, J = 8.1, 11.7 Hz, 1H), 3.07-3.18 (m, 3H), 3.42-3.72 (m, 6H), 3.78-3.88 (m, 2H).
APCIMS m/z: $[M+H]^+$371.
Melting point: 70-72˚C.

Example 57

{1-[2-(2-Methylpyrrolidin-1-yl)ethyl]-3-(3-piperidinopropyl)imidazolidin-2-ylidene}malononitrile (Compound 57)

[1-(2-Hydroxyethyl)-3-(3-piperidinopropyl) imidazolidin-2-ylidene]malononitrile (0.30 g, 0.98 mmol) obtained in Step 1 of Example 47 was dissolved in chloroform/carbon tetrachloride (1:1) (10 mL) and the solution was added with triphenylphosphine resin (1.63 g, 3.0 mmol/g, 4.90 mmol), followed by stirring at room temperature for 3 hours. Then, the mixture was stirred at 60˚C for 2 hours. After cooling the mixture, an insoluble matter was collected by filtration and the filtrate was concentrated under reduced pressure. After the residue was purified by silica gel column chromatography (ethyl acetate/n-hexane (1:1)), the obtained solid was washed with diisopropylether to obtain a white solid (0.248 g).
A portion of the obtained white solid (0.14 g) was dissolved in 1,4-dioxane (2 mL) and the solution was added with 2-methylpyrrolidine (0.13 ml, 1.32 mmol), potassium carbonate (0.12 g, 0.88 mmol) and potassium iodide (0.07 g, 0.44 mmol), followed by stirring at 90˚C for 28 hours. After cooling, the mixture was added with saturated brine and extracted with ethyl acetate. The organic layer was washed with saturated brine, dried over anhydrous magnesium sulfate and the solvent was evaporated under reduced pressure. After the residue was purified by silica gel column chromatography (chloroform/methanol (3:1)), the obtained solid was washed with n-hexane/diisopropylether (10:1) to obtain the titled compound (0.06 g, 30.5 %) as a white solid.
$^1$H NMR ($CDCl_3$, δppm) : 1.06 (d, J = 6.2 Hz, 3H), 1.38-1.44 (m, 2H), 1.53-1.58 (m, 4H), 1.67-1.78 (m, 2H), 1.81-1.95 (m, 4H), 2.32-2.42 (m, 8H), 3.04-3.17 (m, 2H), 3.47 (ddd, J = 6.2, 8.6, 14.8 Hz, 1H), 3.55-3.66 (m, 6H), 3.79-3.87 (m, 2H).
APCIMS m/z: $[M+H]^+$371.
Melting point: 84-86˚C.

Example 58

{1-[2-((S)-2-methylpyrrolidin-1-yl)ethyl]-3-(3-piperidinopropyl)imidazolidin-2-ylidene}malononitrile (Compound 58)

(S)-2-methylpyrrolidinehydrobromate (0.48 g, 2.88 mmol) prepared in a similar manner to the method described in J. Org. Chem., Vol. 54, p.209 (1989) was dissolved in 1,4-dioxane (1 ml) and the solution was added with 2 mol/L aqueous sodium hydroxide solution (1.51 ml, 3.02 mmol), followed by stirring at room temperature for 30 minutes. Next, [1-(2-methanesulfonyloxyethyl)-3-(3-piperidinopropyl)imidazolidin-2-ylidene]malononitrile (0.55 g, 1.44 mmol) obtained in Step 2 of Example 47, potassium carbonate (0.40 g, 2.88 mmol) and 1,4-dioxane (4 mL) were added and stirred at 90˚C for 16 hours. After cooling, the mixture was added with saturated brine and extracted with ethyl acetate. The organic layer was washed with saturated brine, dried over anhydrous magnesium sulfate and the solvent was evaporated under reduced pressure. After the residue .was purified by silica gel column chromatography (chloroform/methanol (3:1)), the obtained solid was recrystallized from n-hexane/ethyl acetate (20:1) to obtain the titled compound (0.27 g, 50.7 %).
$^1$H NMR ($CDCl_3$, δppm) : 1.07 (d, J = 5.8 Hz, 3H), 1.35-1.45 (m, 2H), 1.53-1.59 (m, 4H), 1.68-1.78 (m, 2H), 1.82-1.95 (m, 4H), 2.34-2.40 (m, 8H), 3.04-3.18 (m, 2H), 3.47 (ddd, J = 6.2, 8.4, 14.6 Hz, 1H), 3.54-3.66 (m, 6H), 3.79-3.87 (m, 2H).
APCIMS m/z: $[M+H]^+$371.
Melting point: 95-96˚C.

Example 59

{1-(3-Piperidinopropyl)-3-[2-(pyrrolidin-1-yl) ethyl]imidazolidin-2-ylidene}malononitrile (Compound 59)

(Step 1)

[1-(2-Bromoethyl)-3-(3-hydroxypropyl)imidazolidin-2-ylidene]malononitrile (1.54 g, 5.15 mmol) obtained in Step 1 of Example 46 was dissolved in 1,4-dioxane (10 mL) and the solution was added with pyrrolidine (2.15 ml, 25.8 mmol), followed by stirring at room temperature for 22 hours. After cooling, the mixture was added with water and extracted with chloroform. The organic layer was washed with saturated brine, dried over anhydrous magnesium sulfate and the solvent was evaporated under reduced pressure. The residue was purified by BONDESIL-SCX (manufactured by VARIAN) (chloroform to 2 mol/L ammonia-methanol) to obtain {1-(3-hydroxypropyl)-3-[2-(pyrrolidin-1-yl)ethyl]imidazolidin-2-ylidene} malononitrile (0.75 g, 50.4 %) as a colorless oily substance.
$^1$H NMR ($CDCl_3$, δppm) : 1.72-1.78 (m, 4H), 1.89-1.97 (m, 2H), 2.52-2.59 (m, 4H), 2.78 (t, J = 6.5 Hz, 2H), 3.60-3.78 (m, 10H).

(Step 2)

{1-(3-Hydroxypropyl)-3-[2-(pyrrolidin-1-yl)ethyl] imidazolidin-2-ylidene}malononitrile (0.72 g, 2.51 mmol) obtained in the Step 1 was dissolved in dichloromethane (5 mL) and the solution was added with triethylamine (0.53 ml, 3.76 mmol) and methanesulfonylchloride (0.21 mL, 2.76 mmol)at 0˚C, followed by stirring at room temperature for 2.5 hours. After cooling to 0˚C, the mixture was added with saturated brine and extracted with ethyl acetate. The organic layer was washed with saturated brine, dried over anhydrous magnesium sulfate and the solvent was evaporated under reduced pressure. The residue was purified by silica gel column chromatography (ethyl acetate/n-hexane (2:1) to chloroform/methanol (10:1)) to obtain {1-(3-methanesulfonyloxypropyl)-3-[2-(pyrrolidin-1-yl)ethyl]imidazolidin-2-ylidene] malononitrile (0.510 g, 55.4 %) as a pale yellow oily substance.
$^1$H NMR ($CDCl_3$, δppm) : 1.75-1.80 (m, 4H), 2.13-2.22 (m, 2H), 2.54-2.58 (m, 4H), 2.78 (t, J = 6.5 Hz, 2H), 3.05 (s, 3H), 3.60-3.79 (m,8H),4.35 (t, J = 5. 9 Hz, 2H).

(Step 3)

{1-(3-Methanesulfonyloxypropyl)-3-[2-(pyrrolidin-1-yl)ethyl]imidazolidin-2-ylidene]malononitrile (0.17 g, 0.45 mmol) obtained in the Step 2 was dissolved in 1,4-dioxane (2 mL) and the solution was added with piperidine (0.13 ml, 1.35 mmol), potassium carbonate (0.12 g, 0.90 mmol) and potassium iodide (0.07 g, 0.45 mmol), followed by stirring at 50˚C for 21 hourss. After cooling, the mixture was added with saturated brine and extracted with ethyl acetate. The organic layer was washed with saturated brine, dried over anhydrous magnesium sulfate and the solvent was evaporated under reduced pressure. After the residue was purified by silica gel column chromatography (chloroform/methanol (3: 1)), the obtained solid was washed with n-hexane/diisopropylether (10:1) to obtain the titled compound (0.82 g, 51 %) as a white solid. $^1$H NMR ($CDCl_3$,δppm) : 1.42-1.46 (m, 2H), 1.53-1.59 (m, 4H), 1.76-1.79 (m, 4H), 1.81-1.91 (m, 2H), 2.31-2.36 (m, 6H), 2.54-2.58 (m, 4H), 2.77 (t, J = 6.6 Hz, 2H), 3.55-3.74 (m, 8H).
APCIMS m/z: $[M+H]^+$357.
Melting point: 63-64˚C.

Example 60

{1-[2-(Pyrrolidin-1-yl)ethyl]-3-[3-(pyrrolidin-1-yl)propyl]imidazolidin-2-ylidene}malononitrile (Compound 60)

{1-(3-Methanesulfonyloxypropyl)-3-[2-(pyrrolidin-1-yl)ethyl]imidazolidin-2-ylidene]malononitrile (0.17 g, 0.45 mmol) obtained in Step 2 of Example 59 was dissolved in 1,4-dioxane (2 mL) and the solution was added with pyrrolidine (0.11 ml, 1.35 mmol), potassium carbonate (0.12 g, 0.90 mmol) and potassium iodide (0.07 g, 0.45 mmol), followed by stirring at 50˚C for 18 hours. After cooling, the mixture was added with saturated brine and extracted with ethyl acetate. The organic layer was washed with saturated brine, dried over anhydrous magnesium sulfate and the solvent was evaporated under reduced pressure. After the residue was purified by silica gel column chromatography (chloroform/ methanol (3:1)), the obtained solid was washed with n-hexane/diisopropylether (10:1) to obtain the titled compound (0.07 g, 45.3 %) as a white solid.
$^1$H NMR ($CDCl_3$, δppm) : 1.60-1.79 (m, 8H), 1.83-1.93 (m, 2H), 2.48-2.58 (m, 10H), 2.77 (t, J = 6.4 Hz, 2H), 3.58-3.74 (m, 8H).
APCIMS m/z: $[M+H]^+$343.
Melting point: 70-71˚C.

Example 61

{1-[3-(2-Methylpyrrolidin-1-yl)propyl]-3-[2-(pyrrolidin-1-yl)ethyl]imidazolidin-2-ylidene} malononitrile (Compound 61)

{1-(3-Methanesulfonyloxypropyl)-3-[2-(pyrrolidin-1-yl)ethyl]imidazolidin-2-ylidene]malononitrile (0.17 g, 0.45 mmol) obtained in Step 2 of Example 59 was dissolved in 1,4-dioxane (2 mL) and the solution was added with 2-methylpyrrolidine (0.14 ml, 1.35 mmol), potassium carbonate (0.12 g, 0.90 mmol) and potassium iodide (0.07 g, 0.45 mmol), followed by stirring at 50˚C for 18 hours. After cooling, the mixture was added with saturated brine and extracted with ethyl acetate. The organic layer was washed with saturated brine, dried over anhydrous magnesium sulfate and

the solvent was evaporated under reduced pressure. After the residue was purified by silica gel column chromatography (chloroform/methanol (3:1)), the obtained solid was washed with n-hexane/diisopropylether (10:1) to obtain the titled compound (0.08 g, 50 %) as a white solid.
$^1$H NMR ($CDCl_3$, δppm) : 1.07 (d, J = 6.2 Hz, 3H), 1.32-1.45 (m, 1H), 1.65-1.79 (m, 5H), 1.81-1.97 (m, 4H), 2.05-2.17 (m, 2H), 2.25-2.32 (m, 1H), 2.54-2.58 (m, 4H), 2.75-2.84 (m, 3H), 3.11 (td, J = 8.4, 2.9Hz, 1H), 3.48-3.74 (m, 8H).
APCIMS m/z: $[M+H]^+$357.
Melting point: 95-96˚C.

Example 62

[1-(3-Cyclohexylaminopropyl)-3-(2-piperidinoethyl) imidazolidin-2-ylidene]malononitrile (Compound 62)

[1-(3-Methanesulfonyloxypropyl)-3-(2-piperidinoethyl)imidazolidin-2-ylidene]malononitrile (0.159 g, 0.417 mmol) obtained in Step 3 of Example 46 was dissolved in 1,4-dioxane (31 mL) and the solution was added with cyclohexylamine (0.586 mL, 5.13 mmol) and potassium iodide (0.211 g, 1.28 mmol). After stirring at 80˚C for 18.5 hours, the mixture was added with water and extracted with ethyl acetate. The organic layer was washed with saturated brine, dried over anhydrous magnesium sulfate and the solvent was evaporated under reduced pressure. The residue was purified by silica gel column chromatography (chloroform/(2 mol/L ammonia-methanol) (9:1)) and after the obtained solid was washed with diisopropylether, the solid was recrystallized from n-hexane/diisopropylether (1:1) to obtain the titled compound (0.125 g, 78.0 %) as a white solid.
$^1$H NMR ($CDCL_3$, δppm) : 1.00-1.35 (m, 5H), 1.38-1.97 (m, 13H), 2.38-2.52 (m, 5H), 2.60 (t, J = 6.2 Hz, 2H), 2.73 (t, J = 7.0 Hz, 2H), 3.57-3.77 (m, 8H).
Melting point: 78-80˚C.

Example 63

[1-(2-Cyclohexylaminoethyl)-3-(3-piperidinoprop yl) imidazolidin-2-ylidene]malononitrile difumarate (Compound 63)

[1-(2-Methanesulfonyloxyethyl)-3-(3-piperidinopropyl)imidazolidin-2-ylidene]malononitrile (0.050 g, 0.13 mmol) obtained in Step 2 of Example 47 was dissolved in 1,4-dioxane (5 mL) and the solution was added with cyclohexylamine (0.045 mL, 0.39 mmol) and potassium iodide (0.032 g, 0.20 mmol). After stirring at 80˚C for 3 hours, the mixture was added with cyclohexylamine (0.045 mL, 0.39 mmol), stirred at 80˚C for 5.5 hours, and further added with cyclohexylamine (0.090 mL, 0.78 mmol), followed by stirring at 80˚C for 20 hours. The mixture was further added with cyclohexylamine (0.180 mL, 1.56 mmol) and stirred at 80˚C for 70 hours. The mixture was added with water and extracted with ethyl acetate. The organic layer was washed with saturated brine, dried over anhydrous magnesium sulfate and the solvent was evaporated under reduced pressure. The residue was purified by silica gel column chromatography (chloroform/methanol (100:1)). The obtained oily substance (29 mg) was dissolved in methanol (2 mL) and the solution was added with 0.4 mol/L fumaric acid-methanol solution (0.40 mL, 0.16 mmol), followed by stirring at room temperature for 1 hour. The solvent was evaporated under reduced pressue and the obtained solid was washed with acetone to obtain the titled compound (0.024 g, 29.7 %) as a white solid.
$^1$H NMR ($CD_3OD$, δppm): 1 .25-1 . 45 (m, 5H), 1.60-1.74 (m, 3H), 1.78-1.94 (m, 6H), 1.93-2.17 (m, 4H), 3.00-3.40 (m, 9H), 3.55-3.85 (m, 8H), 6.68 (s, 4H).
Melting point: 109-112˚C.

Example 64

[1,3-Bis(3-piperidinopropyl)imidazolidin-2-ylidene] malononitrile (Compound 64)

(Step 1)

[1-(3-Hydroxypropyl)imidazolidin-2-ylidene] malononitrile (0.370 g, 1.92 mmol) obtained in Step 1 of Example 8 was dissolved in DMF (4 mL) and the solution was sequentially added with potassium carbonate (0.532 g, 3.85 mmol) and 1-chloro-3-iodopropane (0.247 mL, 2.30 mmol). After stirring at room temperature for 26 hours, the mixture was added with potassium carbonate (0.185 g, 1.34 mmol) and 1-chloro-3-iodopropane (0.103 mL, 0.960 mmol), followed by stirring at room temperature for 16 hours. The mixture was added with saturated brine and extracted with ethyl acetate. The organic layer was washed with saturated brine, dried over anhydrous magnesium sulfate and the solvent was evaporated under reduced pressure. The residue was purified by silica gel column chromatography (chloroform/methanol (98:2 to 90:10)) to obtain (1-(3-chloropropyl)-3-(3-hydroxypropyl)imidazolidin-2-ylidene]malononitrile (0.454 g, 88.0 %)

as a white solid.
$^1$H NMR ($CDCl_3$, δppm) : 1.87-1.99 (m, 2H), 2.12-2.24 (m, 2H), 3.61-3.76 (m, 12H).

(Step 2)

[1-(3-Chloropropyl)-3-(3-hydroxypropyl)imidazolidin-2-ylidene]malononitrile (0.454 g, 1.69 mmol) obtained in the Step 1 was dissolved in dichloromethane (5 mL) and the solution was sequentially added with triethylamine (0.282 mL, 2.03 mmol) and methanesulfonylchloride (0.144 mL, 1.86 mmol). After stirring at room temperature for 5 hours and further adding triethylamine (0.141 mL, 1.01 mmol) and methanesulfonylchloride (0.052 mL, 0.68 mmol), the mixture was stirred at room temperature for 1 hour. The mixture was added with saturated brine and extracted with ethyl acetate. The organic layer was washed with saturated brine and the solvent was evaporated under reduced pressure. The residue (0.576 g) was used to the next reaction without further purification.

The above obtained residue (0.576 g) was dissolved in DMF (6 mL) and the solution was added with potassium carbonate (0.700 g, 5.07 mmol) and piperidine (0.836 mL, 8.45 mmol). After stirring at 100˚C for 22 hours, the mixture was added with potassium carbonate (0.117 g, 0.850 mmol) and piperidine (0.050 mL, 0.51 mmol), followed by stirring at 100˚C for 2 hours. After cooling, the mixture was added with saturated brine and extracted with ethyl acetate. The organic layer was washed with saturated brine, dried over anhydrous magnesium sulfate and the solvent was evaporated under reduced pressure.. After the residue was purified by silica gel column chromatography (n-hexane/ethyl acetate (1:1) to ethyl acetate to chloroform/methanol (9:1)), the obtained solid was washed with diethylether to obtain the titled compound (0.347 g, 53.4 %) as a white solid.
$^1$H NMR ($CDCl_3$, δppm) : 1.37-1.48 (m, 4H), 1.50-1.63 (m, 8H), 1.78-1.93 (m, 4H), 2.28-2.44 (m, 12H), 3.52-3.64 (m, 8H).
Melting point: 99-101˚C.

Example 65

{1,3-Bis[3-(2-methylpyrrolidin-1-yl)propyl]imidazolidin-2-ylidene}malononitrile (Compound 65)

(Step 1)

[1-(3-Hydroxypropyl)imidazolidin-2-ylidene] malononitrile (2.00 g, 10.4 mmol) obtained in Step 1 of Example 8 was dissolved in DMF (20 mL) and the solution was sequentially added with potassium carbonate (2.88 g, 20.8 mmol), and 1,3-dibromopropane (5.29 mL, 52.1 mmol). After stirring at room temperature for 25 hours, the mixture was added with saturated brine and extracted with ethyl acetate. The organic layer was washed with saturated brine, dried over anhydrous magnesium sulfate and the solvent was evaporated under reduced pressure. The obtained white solid was washed with diisopropylether and the solid was purified by silica gel column chromatography (chloroform/methanol (100:0 to 98:2 to 97:3 to 96:4) to obtain [1-(3-bromopropyl)-3-(3-hydroxypropyl) imidazolidin-2-ylidene]malononitrile (2.35 g, 72.1 %) as a white solid.
$^1$H NMR ($CDCl_3$, δppm) : 1.80-2.00 (m, 3H), 2.21-2.23 (m, 2H), 3.48 (t, J = 6.2 Hz, 2H), 3.64-3.78 (m, 10H).

(Step 2)

[1-(3-Bromopropyl)-3-(3-hydroxypropyl)imidazolidin-2-ylidene]malononitrile (0.33 g, 1.05 mmol) obtained in the Step 1 was dissolved in DMF (5mL) and the solution was sequentially added with potassium carbonate (0.44 g, 3.16 mmol), 2-methylpyrrolidine (0.32 mL, 3.16 mmol) and sodium iodide (0.18 g, 1.05 mmol). After stirring at room temperature for 5 hours, the mixture was added with saturated brine and extracted with ethyl acetate. The organic layer was washed with saturated brine and the solvent was evaporated under reduced pressure. The residue was purified by BONDESIL-SCX (manufactured by VARIAN) (chloroform to 2 mol/L ammonia-methanol) to obtain {1-(3-hydroxypropyl)-3-[3-(2-methylpyrrolidin-1-yl)propyl] imidazolidin-2-ylidene}malononitrile (0.18 g, 53.9 %).
$^1$H NMR ($CDCl_3$, δppm) : 1.13 (d, J = 6.1 Hz, 3H), 1.41-1.51 (m, 1H), 1.71-1.83 (m, 2H), 1.84-2.01 (m, 5H), 2.15-2.26 (m, 2H), 2.35-2.44 (m, 1H), 2.85 (td, J = 8.0, 11.9 Hz, 1H), 3.18 (td, J = 8.0, 3.4Hz, 1H), 3.50-3.70 (m, 8H), 3.73 (t, J = 5.8 Hz, 2H).

(Step 3)

{1-(3-Hydroxypropyl)-3-[3-(2-methylpyrrolidin-1-yl) propyl]imidazolidin-2-ylidene}malononitrile (0.17 g, 0.55 mmol) obtained in the Step 2 was dissolved in carbon tetrachloride/chloroform (1:1) (5 mL) and the solution was added with triphenylphosphine (0.43 g, 1.63 mmol), followed by heating under reflux for 1.5 hours. After cooling, the mixture was concentrated under reduced pressure and the residue was purified by BONDESIL-SCX (manufactured by VARIAN)

(chloroform to 2 mol/L ammonia-methanol) to obtain {1-(3-chloropropyl)-3-[3-(2-methylpyrrolidin-1-yl)propyllimidazolidin-2-ylidene} malononitrile (0.16 g, 90.2 %).
$^1$H NMR ($CDCl_3$, δppm) : 1.08 (d, J = 6. 1 Hz, 3H), 1.37-1.47 (m, 1H), 1.68-1.76 (m, 2H), 1.80-1.95 (m, 3H), 2.06-2.23 (m, 4H), 2.27-2.34 (m, 1H), 2.81 (td, J = 8.1, 12.0 Hz, 1H), 3.12 (td, J = 8.1, 3.1 Hz,1H), 3.47-3.74 (m, 10H).

(Step 4)

{1-(3-Chloropropyl)-3-[3-(2-methylpyrrolidin-1-yl) propyl]imidazolidin-2-ylidene}malononitrile (0.16 g, 0.48 mmol) obtained in the Step 3 was dissolved in 1,4-dioxane (2 mL) and the solution was added with potassium carbonate (0.200 g, 1.45 mmol), 2-methylpyrrolidine (0.15 mL, 1.45 mmol) and potassium iodide (0.08 g, 0.48 mmol), followed by stirring at 90˚C for 16.5 hours while heating. After cooling, the mixture was added with saturated brine and extracted with ethyl acetate. The organic layer was washed with saturated brine, dried over anhydrous magnesium sulfate and the solvent was evaporated under reduced pressure. The residue was purified by silica gel column chromatography (ethyl acetate) and the obtained solid was washed with diisopropylether to obtain the titled compound (0.104 g, 55.9 %) as a white solid.
$^1$H NMR ($CDCl_3$, δppm) : 1.07 (d, J = 6.0 Hz, 6H), 1.33-1.45 (m, 2H), 1.65-1.79 (m, 4H), 1.81-1.97 (m, 6H), 2.04-2.16 (m, 4H), 2.23-2.32 (m, 2H), 2.79 (td, J = 8.1, 11.9 Hz, 2H), 3.11 (td, J = 2.9, 8.1Hz, 2H), 3.47-3.71 (m, 8H).
APCIMS m/z: $[M+H]^+$385.
Melting point: 89-93 ˚C.

Example 66

{1,3-Bis[3-(azepan-1-yl)propyl]imidazolidin-2-ylidene}malononitrile (Compound 66)

[1-(3-Bromopropyl)-3-(3-hydroxypropyl)imidazolidin-2-ylidene]malononitrile (0.031 g, 0.10 mmol) obtained in Step 1 of Example 65 was dissolved in DMF (0.2 mL) and the solution was added with hexamethyleneimine (a) (0.03 g, 0.30 mmol), potassium carbonate (0.07 g, 0.49 mmol) and potassium iodide (0.017 g, 0.10 mmol), followed by stirring at room temperature for 5.5 hours. After concentrating the mixture, the residue was dissolved in DMF (0.7 mL) and the solution was added with N-methyl isatoic acid resin (0.20 g, 2.0 mmol/g, 0.40 mmol), followed by stirring at room temperature overnight. After removing the resin, the solvent was evaporated under reduced pressure. The residue was purified by BONDESIL-SCX (manufactured by VARIAN) (chloroform to 2 mol/L ammonia-methanol) to obtain a colorless oily substance (0.014 g).
The obtained colorless oily substance was dissolved in DMF (0.3 mL) and the solution was added with carbon tetrachloride (0.30 ml) and triphenylphosphine resin (0.086 g, 3.0 mmol/g, 0.25 mmol), followed by stirring at room temperature for 10.5 hours. After removing the resin, the solvent was evaporated under reduced pressure. The residue was purified by silica gel column chromatography (chloroform/methanol (9:1)) to obtain a yellow oily substance.
The obtained yellow oily substance was dissolved in 1,4-dioxane (0.4 ml) and the solution was added with hexamethyleneimine (b) (0.025 g, 0.25 mmol), potassium carbonate (0.07 g, 0.49 mmol) and potassium iodide (0.08 g, 0.05 mmol), followed by stirring at 90˚C for 20 hours while heating. After cooling, the mixture was concentrated. The residue was dissolved in DMF (0.7 mL) and the solution was added with N-methyl isatoic acid resin (0.20 g, 2.0 mmol/g, 0.40 mmol), followed by stirring at room temperature overnight. After removing the resin, the solvent was evaporated under reduced pressure. The residue was purified by BONDESIL-SCX (manufactured by VARIAN) (chloroform to 2 mol/L ammonia-methanol) to obtain the titled compound (0.001 g, 2.4 % (3 steps)) as a yellow oily substance.
APCIMS m/z: $[M+H]^+$413.

Example 67

{1-[3-(Azepan-1-yl)propyl]-3-[3-(4-methylpiperazin-1-yl)propyl]imidazolidin-2-ylidene}malononitrile (Compound 67)

In a similar manner to Example 66, the titled compound (0.002 g, 4.8% (3 steps)) was obtained as a yellow oily substance by using [1-(3-Bromopropyl)-3-(3-hydroxypropyl)imidazolidin-2-ylidene]malononitrile (0.031 g, 0.10 mmol) obtained in Step 1 of Example 65, hexamethyleneimine (0.03 g, 0.30 mmol) and 1-methyl piperazine (0.025 g, 0.25 mmol) in place of hexamethyleneimine (b)
APCIMS m/z: $[M+H]^+$414.

Example 68

{1-[3-(Azepan-1-yl)propyl]-3-[3-(1,2,3,6-tetrahydropyridin-1-yl)propyl]imidazolidin-2-ylidene} malononitrile (Compound 68)

In a similar manner to Example 66, the titled compound (0.001 g, 2.5% (3 steps)) was obtained as a yellow oily substance by using [1-(3-bromopropyl)-3-(3-hydroxypropyl)imidazolidin-2-ylidene]malononitrile (0.031 g, 0.10 mmol) obtained in Step 1 of Example 65, hexamethyleneimine (0.03 g, 0.30 mmol) and 1,2,3,6-tetrahydropyridine (0.021 g, 0.25 mmol) in place of hexamethyleneimine (b).
APCIMS m/z: $[M+H]^+$397.

Example 69

{1-[3-(Azepan-1-yl)propyl]-3-[3-(pyrrolidin-1-yl) propyl]imidazolidin-2-ylidene}malononitrile (Compound 69)

In a similar manner to Example 66, the titled compound (0.001 g, 2.6 % (3 steps)) was obtained as a yellow oily substance by using [1-(3-bromopropyl)-3-(3-hydroxypropyl)imidazolidin-2-ylidene]malononitrile (0.031 g, 0.10 mmol) obtained in Step 1 of Example 65, hexamethyleneimine (0.03 g, 0.30 mmol) and pyrrolidine (0.018 g, 0.25 mmol) in place of hexamethyleneimine (b).
APCIMS m/z: $[M+H]^+$385.

Example 70

{1-[3-(Azepan-1-yl)propyl]-3-[3-(3,5-dimethylpiperidino)propyl]imidazolidin-2-ylidene} malononitrile (Compound 70)

In a similar manner to Example 66, [1-(3-bromopropyl)-3-(3-hydroxypropyl)imidazolidin-2-ylidene]malononitrile (0.031 g, 0.10 mmol) obtained in Step 1 of Example 65, hexamethyleneimine (0.03 g, 0.30 mmol) and 3,5-dimethylpiperidine (0.028 g, 0.25 mmol) in place of hexamethyleneimine (b) were used to obtain the titled compound (0.002 g, 4.7% (3 steps)) as a yellow oily substance.
APCIMS m/z: $[M+H]^+$427.

Example 71

{1-[3-(Azepan-1-yl)propyl]-3-[3-(2-methylpyrrolidin-1-yl)propyl]imidazolidin-2-ylidene}malononitrile (Compound 71)

In a similar manner to Example 66, the titled compound (0.001 g, 2.5 % (3 steps)) was obtained as a yellow oily substance by using [1-(3-bromopropyl)-3-(3-hydroxypropyl)imidazolidin-2-ylidene]malononitrile (0.031 g, 0.10 mmol) obtained in Step 1 of Example 65, hexamethyleneimine (0.03 g, 0.30 mmol) and 2-methylpyrrolidine (0.028 g, 0.21 mmol) in place of hexamethyleneimine (b).
APCIMS m/z: $[M+H]^+$399.

Example 72

{1-[3-(4-Methylpiperazin-1-yl)propyl]-3-[3-(2-methylpiperidino)propyl]imidazolidin-2-ylidene} malononitrile (Compound 72)

In a similar manner to Example 66, the titled compound (0.003 g, 7.3% (3 steps)) was obtained as a yellow oily substance by using [1-(3-bromopropyl)-3-(3-hydroxypropyl)imidazolidin-2-ylidene]malononitrile (0.031 g, 0.10 mmol) obtained in Step 1 of Example 65, 1-methylpiperazine (0.03 g, 0.30 mmol) in place of hexamethyleneimine (a) and 2-methylpiperidine (0.025 g, 0.25 mmol) in place of hexamethyleneimine (b).
APCIMS m/z: $[M+H]^+$414.

Example 73

{1,3-Bis[3-(4-methylpiperazin-1-yl)propyl] imidazolidin-2-ylidene}malononitrile (Compound 73)

In a similar manner to Example 66, the titled compound (0.005 g, 12.1% (3 steps)) was obtained as a yellow oily substance by using [1-(3-bromopropyl)-3-(3-hydroxypropyl)imidazolidin-2-ylidene]malononitrile (0.031 g, 0.10 mmol)

obtained in Step 1 of Example 65, 1-methylpiperazine (0.03 g, 0.30 mmol) in place of hexamethyleneimine (a) and 1-methylpiperazine (0.025 g, 0.25 mmol) in place of hexamethyleneimine (b).
APCIMS m/z: $[M+H]^+$415.

Example 74

{1-[3-(1,2,3,6-Tetrahydropyridin-1-yl)propyl]-3-[3-(4-methylpiperazin-1-yl)propyl]imidazolidin-2-ylidene} malononitrile (Compound 74)

In a similar manner to Example 66, the titled compound (0.002 g, 5.0% (3 steps)) was obtained as a yellow oily substance by using [1-(3-bromopropyl)-3-(3-hydroxypropyl)imidazolidin-2-ylidene]malononitrile (0.031 g, 0.10 mmol) obtained in Step 1 of Example 65, 1-methylpiperazine (0.03 g, 0.30 mmol) in place of hexamethyleneimine (a) and 1,2,3,6-tetrahydropyridine (0.021 g, 0.25 mmol) in place of hexamethyleneimine (b).
APCIMS m/z: $[M+H]^+$398.

Example 75

{1-[3-(4-Methylpiperazin-1-yl)propyl]-3-[3-(pyrrolidin-1-yl)propyl]imidazolidin-2-ylidene} malononitrile (Compound 75)

In a similar manner to Example 66, the titled compound (0.003 g, 7.8% (3 steps)) was obtained as a yellow oily substance by using [1-(3-bromopropyl)-3-(3-hydroxypropyl)imidazolidin-2-ylidene]malononitrile (0.031 g, 0.10 mmol) obtained in Step 1 of Example 65, 1-methylpiperazine (0.03 g, 0.30 mmol) in place of hexamethyleneimine (a) and pyrrolidine (0.018 g, 0.25 mmol) in place of hexamethyleneimine (b).
APCIMS m/z: $[M+H]^+$386.

Example 76

{1-[3-(4-Methylpiperazin-1-yl)propyl]-3-(3-piperidinopropyl)imidazolidin-2-ylidene}malononitrile (Compound 76)

In a similar manner to Example 66, the titled compound (0.005 g, 12.5% (3 steps)) was obtained as a yellow oily substance by using [1-(3-bromopropyl)-3-(3-hydroxypropyl)imidazolidin-2-ylidene]malononitrile (0.031 g, 0.10 mmol) obtained in Step 1 of Example 65, 1-methylpiperazine (0.03 g, 0.30 mmol) in place of hexamethyleneimine (a) and piperidine (0.021 g, 0.25 mmol) in place of hexamethyleneimine (b).
APCIMS m/z: $[M+H]^+$400.

Example 77

{1-[3-(3,5-Dimethylpiperidino)propyl]-3-[3-(4-methylpiperazin-1-yl)propyl]imidazolidin-2-ylidene} malononitrile (Compound 77)

In a similar manner to Example 66, the titled compound (0.004 g, 9.4% (3 steps)) was obtained as a yellow oily substance by using [1-(3-bromopropyl)-3-(3-hydroxypropyl)imidazolidin-2-ylidene]malononitrile (0.031 g, 0.10 mmol) obtained in Step 1 of Example 65, 1-methylpiperazine (0.03 g, 0.30 mmol) in place of hexamethyleneimine (a) and 3,5-dimethylpiperidine (0.028 g, 0.25 mmol) in place of hexamethyleneimine (b).
APCIMS m/z: $[M+H]^+$428.

Example 78

{1-[3-(4-Methylpiperazin-1-yl)propyl]-3-[3-(2-methylpyrrolidin-1-yl)propyl]imidazolidin-2-ylidene} malononitrile (Compound 78)

In a similar manner to Example 66, the titled compound (0.004 g, 10.0% (3 steps)) was obtained as a yellow oily substance by using [1-(3-bromopropyl)-3-(3-hydroxypropyl)imidazolidin-2-ylidene]malononitrile (0.031 g, 0.10 mmol) obtained in Step 1 of Example 65, 1-methylpiperazine (0.03 g, 0.30 mmol) in place of hexamethyleneimine (a) and 2-methylpyrrolidine (0.021 g, 0.25 mmol) in place of hexamethyleneimine (b).
APCIMS m/z: $[M+H]^+$400.

Example 79

{1-[3-(2-Methylpiperidino)propyl]-3-(3-morpholinopropyl)imidazolidin-2-ylidene}malononitrile (Compound 79)

In a similar manner to Example 66, the titled compound (0.007 g, 17.5% (3 steps)) was obtained as a yellow oily substance by using [1-(3-bromopropyl)-3-(3-hydroxypropyl)imidazolidin-2-ylidene]malononitrile (0.031 g, 0.10 mmol) obtained in Step 1 of Example 65, morpholine (0.026 g, 0.30 mmol) in place of hexamethyleneimine (a) and 2-methylpiperidine (0.025 g, 0.25 mmol) in place of hexamethyleneimine (b).
APCIMS m/z: $[M+H]^+$401.

Example 80

{1-[3-(3-Methylpiperidino)propyl]-3-(3-morpholinopropyl)imidazolidin-2-ylidene}malononitrile (Compound 80)

In a similar manner to Example 66, the titled compound (0.012 g, 30.0% (3 steps)) was obtained as a yellow oily substance by using [1-(3-bromopropyl).-3-(3-hydroxypropyl)imidazolidin-2-ylidene]malononitrile (0.031 g, 0.10 mmol) obtained in Step 1 of Example 65, morpholine (0.026 g, 0.30 mmol) in place of hexamethyleneimine (a) and 3-methylpiperidine (0.025 g, 0.25 mmol) in place of hexamethyleneimine (b).
APCIMS m/z: $[M+H]^+$401.

Example 81

{1-[3-(1,2,3,6-Tetrahydropyridin-1-yl)propyl]-3-(3-morpholinopropyl)imidazolidin-2-ylidene}malononitrile (Compound 81)

In a similar manner to Example 66, the titled compound (0.005 g, 13.0% (3 steps)) was obtained as a yellow oily substance by using [1-(3-bromopropyl)-3-(3-hydroxypropyl)imidazolidin-2-ylidene]malononitrile (0.031 g, 0.10 mmol) obtained in Step 1 of Example 65, morpholine (0.026 g, 0.30 mmol) in place of hexamethyleneimine (a) and 1,2,3,6-tetrahydropyridine (0.021 g, 0.25 mmol) in place of hexamethyleneimine (b).
APCIMS m/z: $[M+H]^+$385.

Example 82

{1-(3-Morpholinopropyl)-3-[3-(pyrrolidin-1-yl) propyl]imidazolidin-2-ylidene}malononitrile (Compound 82)

In a similar manner to Example 66, the titled compound (0.009 g, 24.2% (3 steps)) was obtained as a yellow oily substance by using [1-(3-bromopropyl)-3-(3-hydroxypropyl)imidazolidin-2-ylidene]malononitrile (0.031 g, 0.10 mmol) obtained in Step 1 of Example 65, morpholine (0.026 g, 0.30 mmol) in place of hexamethyleneimine (a) and pyrrolidine (0.018 g, 0.25 mmol) in place of hexamethyleneimine (b).
APCIMS m/z: $[M+H]^+$373.

Example 83

[1-(3-Morpholinopropyl)-3-(3-piperidinopropyl) imidazolidin-2-ylidene]malononitrile (Compound 83)

In a similar manner to Example 66, the titled compound (0.01 g, 25.9% (3 steps)) was obtained as a yellow oily substance by using [1-(3-bromopropyl)-3-(3-hydroxypropyl)imidazolidin-2-ylidene]malononitrile (0.031 g, 0.10 mmol) obtained in Step 1 of Example 65, morpholine (0.026 g, 0.30 mmol) in place of hexamethyleneimine (a) and piperidine (0.021 g, 0.25 mmol) in place of hexamethyleneimine (b).
APCIMS m/z: $[M+H]^+$387.

Example 84

{1-[3-(3,5-Dimethylpiperidino)propyl]-3-(3-morpholinopropyl)imidazolidin-2-ylidene}malononitrile (Compound 84)

In a similar manner to Example 66, the titled compound (0.012 g, 29.0% (3 steps)) was obtained as a yellow oily substance by using [1-(3-bromopropyl)-3-(3-hydroxypropyl)imidazolidin-2-ylidene]malononitrile (0.031 g, 0.10 mmol) obtained in Step 1 of Example 65, morpholine (0.026 g, 0.30 mmol) in place of hexamethyleneimine (a) and 3,5-dimethylpiperidine (0.028 g, 0.25 mmol) in place of hexamethyleneimine (b).
APCIMS m/z: $[M+H]^+$415.

Example 85

{1-[3-(2-Methylpyrrolidin-1-yl)propyl]-3-(3-morpholinopropyl)imidazolidin-2-ylidene}malononitrile (Compound 85)

In a similar manner to Example 66, the titled compound (0.009 g, 23.3% (3 steps)) was obtained as a yellow oily substance by using [1-(3-bromopropyl)-3-(3-hydroxypropyl)imidazolidin-2-ylidene]malononitrile (0.031 g, 0.10 mmol) obtained in Step 1 of Example 65, morpholine (0.026 g, 0.30 mmol) in place of hexamethyleneimine (a) and 2-methylpyrrolidine (0.021 g, 0.25 mmol) in place of hexamethyleneimine (b).
APCIMS m/z: $[M+H]^+$387.

Example 86

{1-[3-(1,2,3,6-Tetrahydropyridin-1-yl)propyl]-3-[3-(2-methylpiperidino)propyl]imidazolidin-2-ylidene} malononitrile (Compound 86)

In a similar manner to Example 66, the titled compound (0.003 g, 7.5% (3 steps)) was obtained as a yellow oily substance by using [1-(3-bromopropyl)-3-(3-hydroxypropyl)imidazolidin-2-ylidene]malononitrile (0.031 g, 0.10 mmol) obtained in Step 1 of Example 65, 1,2,3,6-tetrahydropyridine (0.025 g, 0.30 mmol) in place of hexamethyleneimine (a) and 2-methylpiperidine (0.025 g, 0.25 mmol) in place of hexamethyleneimine (b)
APCIMS m/z: $[M+H]^+$397.

Example 87

{1-[3-(1,2,3,6-Tetrahydropyridin-1-yl)propyl]-3-[3-(3-methylpiperidino)propyl]imidazolidin-2-ylidene} malononitrile (Compound 87)

In a similar manner to Example 66, the titled compound (0.003 g, 7.5% (3 steps)) as a yellow oily substance by using [1-(3-bromopropyl)-3-(3-hydroxypropyl) imidazolidin-2-ylidene]malononitrile (0.031 g, 0.10 mmol) obtained in Step 1 of Example 65, 1,2,3,6-tetrahydropyridine (0.025 g, 0.30 mmol) in place of hexamethyleneimine (a), 3-methylpiperidine (0.025 g, 0.25 mmol) in place of hexamethyleneimine (b).
APCIMS m/z: $[M+H]^+$397.

Example 88

{1,3-bis[3-(1,2,3,6-Tetrahydropyridin-1-yl)propyl] imidazolidin-2-ylidene}malononitrile (Compound 88)

In a similar manner to Example 66, the titled compound (0.002 g, 5.3% (3 steps)) was obtained as a yellow oily substance by using [1-(3-bromopropyl)-3-(3-hydroxypropyl)imidazolidin-2-ylidene]malononitrile (0.031 g, 0.10 mmol) obtained in Step 1 of Example 65, 1,2,3,6-tetrahydropyridine (0.025 g, 0.30 mmol) in place of hexamethyleneimine (a) and 1,2,3,6-tetrahydropyridine (0.021 g, 0.25 mmol) in place of hexamethyleneimine (b).
APCIMS m/z: $[M+H]^+$381.

Example 89

{1-[3-(1,2,3,6-Tetrahydropyridin-1-yl)propyl]-3-[3-(pyrrolidin-1-yl)propyl]imidazolidin-2-ylidene} malononitrile (Compound 89)

In a similar manner to Example 66, the titled compound (0.004 g, 10.9% (3 steps)) was obtained as a yellow oily substance by using [1-(3-bromopropyl)-3-(3-hydroxypropyl)imidazolidin-2-ylidene]malononitrile (0.031 g, 0.10 mmol) obtained in Step 1 of Example 65, 1,2,3,6-tetrahydropyridine (0.025 g, 0.30 mmol) in place of hexamethyleneimine (a) and pyrrolidine (0.018 g, 0.25 mmol) in place of hexamethyleneimine (b).
APCIMS m/z: $[M+H]^+$369.

Example 90

{1-[3-(1,2,3,6-Tetrahydropyridin-1-yl)propyl]-3-(3-piperidinopropyl)imidazolidin-2-ylidene}malononitrile (Compound 90)

In a similar manner to Example 66, the titled compound (0.004 g, 10.5% (3 steps)) was obtained as a yellow oily substance by using [1-(3-bromopropyl)-3-(3-hydroxypropyl)imidazolidin-2-ylidene]malononitrile (0.031 g, 0.10 mmol) obtained in Step 1 of Example 65, 1,2,3,6-tetrahydropyridine (0.025 g, 0.30 mmol) in place of hexamethyleneimine (a) and piperidine (0.021 g, 0.25 mmol) in place of hexamethyleneimine (b).
APCIMS m/z: $[M+H]^+$383.

Example 91

{1-[3-(1,2,3,6-Tetrahydropyridin-1-yl)propyl]-3-[3-(3,5-dimethylpiperidino)propyl]imidazolidin-2-ylidene} malononitrile (Compound 91)

In a similar manner to Example 66, the titled compound (0.004 g, 9.7% (3 steps)) was obtained as a yellow oily substance by using [1-(3-bromopropyl)-3-(3-hydroxypropyl)imidazolidin-2-ylidene]malononitrile (0.031 g, 0.10 mmol) obtained in Step 1 of Example 65, 1,2,3,6-tetrahydropyridine (0.025 g, 0.30 mmol) in place of hexamethyleneimine (a) and 3,5-dimethylpiperidine (0.028 g, 0.25 mmol) in place of hexamethyleneimine (b).
APCIMS m/z: $[M+H]^+$411.

Example 92

{1-[3-(1,2,3,6-Tetrahydropyridin-1-yl)propyl]-3-[3-(2-methylpyrrolidin-1-yl)propyl]imidazolidin-2-ylidene} malononitrile (Compound 92)

In a similar manner to Example 66, the titled compound (0.005 g, 13.1% (3 steps)) was obtained as a yellow oily substance by using [1-(3-bromopropyl)-3-(3-hydroxypropyl)imidazolidin-2-ylidene]malononitrile (0.031 g, 0.10 mmol) obtained in Step 1 of Example 65, 1,2,3,6-tetrahydropyridine (0.025 g, 0.30 mmol) in place of hexamethyleneimine (a) and 2-methylpyrrolidine (0.021 g, 0.25 mmol) in place of hexamethyleneimine (b).
APCIMS m/z: $[M+H]^+$383.

Example 93

{1-[3-(2-Methylpiperidino)propyl]-3-[3-(pyrrolidin-1-yl)propyl]imidazolidin-2-ylidene}malononitrile (Compound 93)

In a similar manner to Example 66, the titled compound (0.001 g, 2.6% (3 steps)) was obtained as a yellow oily substance by using [1-(3-bromopropyl)-3-(3-hydroxypropyl)imidazolidin-2-ylidene]malononitrile (0.031 g, 0.10 mmol) obtained in Step 1 of Example 65, pyrrolidine (0.021 g, 0.30 mmol) in place of hexamethyleneimine (a) and 2-methylpiperidine (0.025 g, 0.25 mmol) in place of hexamethyleneimine (b).
APCIMS m/z: $[M+H]^+$385.

Example 94

{1,3-Bis[3-(pyrrolidin-1-yl)propyl]imidazolidin-2-ylidene}malononitrile (Compound 94)

In a similar manner to Example 66, the titled compound (0.001 g, 2.8% (3 steps)) was obtained as a yellow oily substance by using [1-(3-bromopropyl)-3-(3-hydroxypropyl)imidazolidin-2-ylidene]malononitrile (0.031 g, 0.10 mmol) obtained in Step 1 of Example 65, pyrrolidine (0.021 g, 0.30 mmol) in place of hexamethyleneimine (a) and pyrrolidine (0.018 g, 0.25 mmol) in place of hexamethyleneimine (b).
APCIMS m/z: $[M+H]^+$357.

Example 95

{1-(3-Piperidinopropyl)-3-[3-(pyrrolidin-1-yl) propyl]imidazolidin-2-ylidene}malononitrile (Compound 95)

In a similar manner to Example 66, the titled compound (0.001 g, 2.7% (3 steps)) was obtained as a yellow oily substance by using [1-(3-bromopropyl)-3-(3-hydroxypropyl)imidazolidin-2-ylidene]malononitrile (0.031 g, 0.10 mmol)

obtained in Step 1 of Example 65, pyrrolidine (0.021 g, 0.30 mmol) in place of hexamethyleneimine (a) and piperidine (0.021 g, 0.25 mmol) in place of hexamethyleneimine (b).
APCIMS m/z: $[M+H]^+$371.

Example 96

{1-[3-(3,5-Dimethylpiperidino)propyl]-3-[3-(pyrrolidin-1-yl)propyl]imidazolidin-2-ylidene} malononitrile (Compound 96)

In a similar manner to Example 66, the titled compound (0.001 g, 2.5% (3 steps)) as a yellow oily substance by using [1-(3-bromopropyl)-3-(3-hydroxypropyl) imidazolidin-2-ylidene]malononitrile (0.031 g, 0.10 mmol) obtained in Step 1 of Example 65, pyrrolidine (0.021 g, 0.30 mmol) in place of hexamethyleneimine (a) and 3,5-dimethylpiperidine (0.028 g, 0.25 mmol) in place of hexamethyleneimine (b).
APCIMS m/z: $[M+H]^+$399.

Example 97

{1-[3-(2-Methylpyrrolidin-1-yl)propyl]-3-[3-(pyrrolidin-1-yl)propyl]imidazolidin-2-ylidene} malononitrile (Compound 97)

In a similar manner to Example 66, the titled compound (0.001 g, 2.7% (3 steps)) was obtained as a yellow oily substance by using [1-(3-bromopropyl)-3-(3-hydroxypropyl)imidazolidin-2-ylidene]malononitrile (0.031 g, 0.10 mmol) obtained in Step 1 of Example 65, pyrrolidine (0.021 g, 0.30 mmol) in place of hexamethyleneimine (a) and 2-methylpyrrolidine (0.021 g, 0.25 mmol) in place of hexamethyleneimine (b).
APCIMS m/z: $[M+H]^+$371.

Example 98

{1-[3-(2-Methylpiperidino)propyl]-3-(3-piperidinopropyl)imidazolidin-2-ylidene}malononitrile (Compound 98)

In a similar manner to Example 66, the titled compound (0.002 g, 5.0% (3 steps)) was obtained as a yellow oily substance by using [1-(3-bromopropyl)-3-(3-hydroxypropyl)imidazolidin-2-ylidene]malononitrile (0.031 g, 0.10 mmol) obtained in Step 1 of Example 65, piperidine (0.026 g, 0.30 mmol) in place of hexamethyleneimine (a) and 2-methylpiperidine (0.025 g, 0.25 mmol) in place of hexamethyleneimine (b).
APCIMS m/z: $[M+H]^+$399.

Example 99

{1-[3-(3-Methylpiperidino)propyl]-3-(3-piperidinopropyl)imidazolidin-2-ylidene}malononitrile (Compound 99)

In a similar manner to Example 66, the titled compound (0.002 g, 5.0% (3 steps)) was obtained as a yellow oily substance by using [1-(3-bromopropyl)-3-(3-hydroxypropyl)imidazolidin-2-ylidene]malononitrile (0.031 g, 0.10 mmol) obtained in Step 1 of Example 65, piperidine (0.026 g, 0.30 mmol) in place of hexamethyleneimine (a) and 3-methylpiperidine (0.025 g, 0.25 mmol) in place of hexamethyleneimine (b).
APCIMS m/z: $[M+H]^+$399.

Example 100

{1-[3-(4-Methylpiperidino)propyl]-3-(3-piperidinopropyl)imidazolidin-2-ylidene}malononitrile (Compound 100)

In a similar manner to Example 66, the titled compound (0.001 g, 2.5% (3 steps)) was obtained as a yellow oily substance by using [1-(3-bromopropyl)-3-(3-hydroxypropyl)imidazolidin-2-ylidene]malononitrile (0.031 g, 0.10 mmol) obtained in Step 1 of Example 65, piperidine (0.026 g, 0.30 mmol) in place of hexamethyleneimine (a) and 4-methylpiperidine (0.025 g, 0.25 mmol) in place of hexamethyleneimine (b).
APCIMS m/z: $[M+H]^+$399.

Example 101

{1-[3-(3-Hydroxypyrrolidin-1-yl)propyl]-3-(3-piperidinopropyl)imidazolidin-2-ylidene}malononitrile (Compound 101)

In a similar manner to Example 66, the titled compound (0.002 g, 5.2% (3 steps)) was obtained as a yellow oily substance by using [1-(3-bromopropyl)-3-(3-hydroxypropyl)imidazolidin-2-ylidene]malononitrile (0.031 g, 0.10 mmol) obtained in Step 1 of Example 65, piperidine (0.026 g, 0.30 mmol) in place of hexamethyleneimine (a) and 3-hydroxypyrrolidine (0.022 g, 0.25 mmol) in place of hexamethyleneimine (b).
APCIMS m/z: $[M+H]^+$387.

Example 102

{1-[3-(3,5-Dimethylpiperidino)propyl]-3-(3-piperidinopropyl)imidazolidin-2-ylidene}malononitrile (Compound 102)

In a similar manner to Example 66, the titled compound (0.003 g, 7.3% (3 steps)) was obtained as a yellow oily substance by using [1-(3-bromopropyl)-3-(3-hydroxypropyl)imidazolidin-2-ylidene]malononitrile (0.031 g, 0.10 mmol) obtained in Step 1 of Example 65, piperidine (0.026 g, 0.30 mmol) in place of hexamethyleneimine (a) and 3,5-dimethylpiperidine (0.028 g, 0.25 mmol) in place of hexamethyleneimine (b).
APCIMS m/z: $[M+H]^+$413.

Example 103

{1-[3-(2-Methylpyrrolidin-1-yl)propyl]-3-(3-piperidinopropyl)imidazolidin-2-ylidene}malononitrile (Compound 103)

In a similar manner to Example 66, the titled compound (0.002 g, 5.2% (3 steps)) was obtained as a yellow oily substance by using [1-(3-bromopropyl)-3-(3-hydroxypropyl)imidazolidin-2-ylidene]malononitrile (0.031 g, 0.10 mmol) obtained in Step 1 of Example 65, piperidine (0.026 g, 0.30 mmol) in place of hexamethyleneimine (a) and 2-methylpyrrolidine (0.021 g, 0.25 mmol) in place of hexamethyleneimine (b).
APCIMS m/z: $[M+H]^+$385.

Example 104

{1-[3-(3,5-Dimethylpiperidino)propyl]-3-[3-(2-methylpiperidino)propyl]imidazolidin-2-ylidene} malononitrile (Compound 104)

In a similar manner to Example 66, the titled compound (0.003 g, 7.0% (3 steps)) was obtained as a yellow oily substance by using [1-(3-bromopropyl)-3-(3-hydroxypropyl)imidazolidin-2-ylidene]malononitrile (0.031 g, 0.10 mmol) obtained in Step 1 of Example 65, 3,5-dimethylpiperidine (0.034 g, 0.30 mmol) in place of hexamethyleneimine (a) and 2-methylpiperidine (0.025 g, 0.25 mmol) in place of hexamethyleneimine (b).
APCIMS m/z: $[M+H]^+$427.

Example 105

{1-[3-(3,5-Dimethylpiperidino)propyl]-3-[3-(3-methylpiperidino)propyl]imidazolidin-2-ylidene} malononitrile (Compound 105)

In a similar manner to Example 66, the titled compound (0.004 g, 9.4% (3 steps)) was obtained as a yellow oily substance by using [1-(3-bromopropyl)-3-(3-hydroxypropyl)imidazolidin-2-ylidene]malononitrile (0.031 g, 0.10 mmol) obtained in Step 1 of Example 65, 3,5-dimethylpiperidine (0.034 g, 0.30 mmol) in place of hexamethyleneimine (a) and 3-methylpiperidine (0.025 g, 0.25 mmol) in place of hexamethyleneimine (b).
APCIMS m/z: $[M+H]^+$427.

Example 106

{1-[3-(3,5-Dimethylpiperidino)propyl]-3-[3-(4-methylpiperidino)propyl]imidazolidin-2-ylidene} malononitrile (Compound 106)

In a similar manner to Example 66, the titled compound (0.002 g, 4.7% (3 steps)) was obtained as a yellow

oily substance by using [1-(3-bromopropyl)-3-(3-hydroxypropyl) imidazolidin-2-ylidene] malononitrile (0.031 g, 0.10 mmol) obtained in Step 1 of Example 65, 3,5-dimethylpiperidine (0.034 g, 0.30 mmol) in place of hexamethyleneimine (a) and 4-methylpiperidine (0.025 g, 0.25 mmol) in place of hexamethyleneimine (b).
APCIMS m/z: $[M+H]^+$427.

Example 107

{1-[3-(3,5-Dimethylpiperidino)propyl]-3-[3-(3-hydroxypyrrolidin-1-yl)propyl]imidazolidin-2-ylidene} malononitrile (Compound 107)

In a similar manner to Example 66, the titled compound (0.004 g, 9.7% (3 steps)) was obtained as a yellow oily substance by using [1-(3-bromopropyl)-3-(3-hydroxypropyl)imidazolidin-2-ylidene]malononitrile (0.031 g, 0.10 mmol) obtained in Step 1 of Example 65, 3,5-dimethylpiperidine (0.034 g, 0.30 mmol) in place of hexamethyleneimine (a) and 3-hydroxypyrrolidine (0.022 g, 0.25 mmol) in place of hexamethyleneimine (b).
APCIMS m/z: $[M+H]^+$415.

Example 108

{1-[3-(2,6-Dimethylpiperidino)propyl]-3-[3-(3,5-dimethylpiperidino)propyl]imidazolidin-2-ylidene} malononitrile (Compound 108)

In a similar manner to Example 66, the titled compound (0.002 g, 4.5% (3 steps)) was obtained as a yellow oily substance by using [1-(3-bromopropyl)-3-(3-hydroxypropyl)imidazolidin-2-ylidene]malononitrile (0.031 g, 0.10 mmol) obtained in Step 1 of Example 65, 3,5-dimethylpiperidine (0.034 g, 0.30 mmol) in place of hexamethyleneimine (a) and 2,6-dimethylpiperidine (0.028 g, 0.25 mmol) in place of hexamethyleneimine (b).
APCIMS m/z: $[M+H]^+$441.

Example 109

{1,3-Bis[3-(3,5-dimethylpiperidino)propyl] imidazolidin-2-ylidene}malononitrile (Compound 109)

In a similar manner to Example 66, the titled compound (0.004 g, 9.1% (3 steps)) was obtained as a yellow oily substance by using [1-(3-bromopropyl)-3-(3-hydroxypropyl)imidazolidin-2-ylidene]malononitrile (0.031 g, 0.10 mmol) obtained in Step 1 of Example 65, 3,5-dimethylpiperidine (0.034 g, 0.30 mmol), in place of hexamethyleneimine (a) and 3,5-dimethylpiperidine (0.028 g, 0.25 mmol) in place of hexamethyleneimine (b).
APCIMS m/z: $[M+H]^+$441.

Example 110

{1-[3-(3,5-Dimethylpiperidino)propyl]-3-[3-(2-methylpyrrolidin-1-yl)propyl]imidazolidin-2-ylidene} malononitrile (Compound 110)

In a similar manner to Example 66, the titled compound (0.003 g, 7.3% (3 steps)) was obtained as a yellow oily substance by using [1-(3-bromopropyl)-3-(3-hydroxypropyl)imidazolidin-2-ylidene]malononitrile (0.031 g, 0.10 mmol) obtained in Step 1 of Example 65, 3,5-dimethylpiperidine (0.034 g, 0.30 mmol) in place of hexamethyleneimine (a) and 2-methylpyrrolidine (0.021 g, 0.25 mmol) in place of hexamethyleneimine (b).
APCIMS m/z: $[M+H]^+$413.

Example 111

{1-[3-(2-Methylpiperidino)propyl]-3-[3-(2-methylpyrrolidin-1-yl)propyl]imidazolidin-2-ylidene} malononitrile (Compound 111)

In a similar manner to Example 66, the titled compound (0.003 g, 7.5% (3 steps)) was obtained as a yellow oily substance by using [1-(3-bromopropyl)-3-(3-hydroxypropyl)imidazolidin-2-ylidene]malononitrile (0.031 g, 0.10 mmol) obtained in Step 1 of Example 65, 2-methylpyrrolidine (0.026 g, 0.30 mmol) in place of hexamethyleneimine (a) and 2-methylpiperidine (0.025 g, 0.25 mmol) in place of hexamethyleneimine (b).
APCIMS m/z: $[M+H]^+$399.

Example 112

{1-[3-(3-Methylpiperidino)propyl]-3-[3-(2-methylpyrrolidin-1-yl)propyl]imidazolidin-2-ylidene} malononitrile (Compound 112)

In a similar manner to Example 66, the titled compound (0.002 g, 5.0% (3 steps)) was obtained as a yellow oily substance by using [1-(3-bromopropyl)-3-(3-hydroxypropyl)imidazolidin-2-ylidene]malononitrile (0.031 g, 0.10 mmol) obtained in Step 1 of Example 65, 2-methylpyrrolidine (0.026 g, 0.30 mmol) in place of hexamethyleneimine (a) and 3-methylpiperidine (0.025 g, 0.25 mmol) in place of hexamethyleneimine (b).
APCIMS m/z: $[M+H]^+$399.

Example 113

{1-[3-(4-methylpiperidinopropyl)-3-[3-(2-methylpyrrolidin-1-yl)propyl]imidazolidin-2-ylidene] malononitrile (Compound 113)

In a similar manner to Example 66, the titled compound (0.002 g, 5.0% (3 steps)) was obtained as a yellow oily substance by using [1-(3-bromopropyl)-3-(3-hydroxypropyl)imidazolidin-2-ylidene]malononitrile (0.031 g, 0.10 mmol) obtained in Step 1 of Example 65, 2-methylpyrrolidine (0.026 g, 0.30 mmol) in place of hexamethyleneimine (a) and 4-methylpiperidine (0.025 g, 0.25 mmol) in place of hexamethyleneimine (b).
APCIMS m/z: $[M+H]^+$399.

Example 114

{1-[3-(3-Hydroxypyrrolidin-1-yl)propyl]-3-[3-(2-methylpyrrolidin-1-yl)propyl]imidazolidin-2-ylidene} malononitrile (Compound 114)

In a similar manner to Example 66, the titled compound (0.003 g, 7.8% (3 steps)) was obtained as a yellow oily substance by using [1-(3-bromopropyl)-3-(3-hydroxypropyl)imidazolidin-2-ylidene]malononitrile (0.031 g, 0.10 mmol) obtained in Step 1 of Example 65, 2-methylpyrrolidine (0.026 g, 0.30 mmol) in place of hexamethyleneimine (a) and 3-hydroxypyrrolidine (0.022 g, 0.25 mmol) in place of hexamethyleneimine (b).
APCIMS m/z: $[M+H]^+$387.

Example 115

{1,3-Bis[3-((S)-2-methylpyrrolidin-1-yl)propyl] imidazolidin-2-ylidene}malononitrile (Compound 115)

(Step 1)

[1-(3-Bromopropyl)-3-(3-hydroxypropyl)imidazolidin-2-ylidene]malononitrile (0.47 g, 1.50 mmol) obtained in Step 1 of Example 65 was dissolved in dichloromethane (5 mL) and the solution was added with triethylamine (0.31 ml, 2.25 mmol) and methanesulfonylchloride (0.13 mL, 1.65 mmol) at 0˚C, followed by stirring at room temperature for 3 hours. After cooling to 0˚C, the mixture was added with saturated brine and extracted with ethyl acetate. The organic layer was washed with saturated brine, dried over anhydrous magnesium sulfate and the solvent was evaporated under reduced pressure. The residue was purified by silica gel column chromatography (chloroform/methanol (95:5)) and the obtained solid was washed with diisopropylether to obtain [1-(3-bromopropyl)-3-(3-methanesulfonyloxypropyl)imidazolidin-2-ylidene] malononitrile (0.643 g, 100 %) as a pale yellow solid.
$^1$H NMR ($CDCl_3$, δppm): 2.13-2.21 1 (m, 2H), 2.24-2.31 (m, 2H), 3.06 (s, 3H), 3.48 (t, J = 6.2 Hz, 2H), 3.63-3.75 (m, 8H), 4.35 (t, J=5.8 8 Hz, 2H).

(Step 2)

(S)-2-methylpyrrolidinehydrobromate (0.93 g, 5.60 mmol) prepared in a similar manner to the method described in J. Org. Chem., Vol. 54, p.209 (1989) was dissolved in 1,4-dioxane (3 ml) and the solution was added with 2 mol/L aqueous sodium hydroxide solution (2.88 ml, 5.75 mmol). After stirring at room temperature for 30 minutes, the mixture was added with [1-(3-bromopropyl)-3-(3-methanesulfonyloxypropyl)imidazolidin-2-ylidene] malononitrile (0.61 g, 1.55 mmol) obtained in the Step 1, potassium carbonate (0.64 g, 4.66 mmol), sodium iodide (0.23 g, 1.55 mmol) and 1,4-dioxane (5 mL), followed by stirring at 90˚C for 20.5 hours. After cooling, the mixture was added with saturated brine

and extracted with ethyl acetate. The organic layer was washed with saturated brine, dried over anhydrous magnesium sulfate and the solvent was evaporated under reduced pressure. The residue was purified by silica gel column chromatography (chloroform/methanol (3:1)) and the obtained solid was triturated with diisopropylether to obtain the titled compound (0.30 g, 50.6 %).
$^1$H NMR ($CDCl_3$, δppm) : 1.07 (d, J = 6.2 Hz,6H), 1.33-1.45 (m, 2H), 1.65-1.78 (m, 4H), 1.81-1.96 (m, 6H), 2.04-2.16 (m, 4H), 2.24-2.34 (m, 2H), 2.79 (td, J = 8.1, 11.8 Hz, 2H), 3.12 (td, J = 3.3, 8.1Hz,2H), 3.47-3.71 (m, 8H).
APCIMS m/z: $[M+H]^+$385.
Melting point: 79-80˚C.

Example 116

{1,3-Bis[3-((R)-2-methylpyrrolidin-1-yl)propyl] imidazolidin-2-ylidene}malononitrile difumarate (Compound 116)

(Step 1)

[1-(3-Bromopropyl)-3-(3-hydroxypropyl)imidazolidin-2-ylidene]malononitrile (2.00 g, 6.34 mmol) obtained in Step 1 of Example 65 was dissolved in 1,4-dioxane (10 mL) and the solution was added with a solution of (R)-2-methylpyrrolidinehydrobromate (1.85 g, 11.1 mmol) prepared in a similar manner to the method described in J. Org. Chem., Vol. 54, p.209 (1989), dissolved in a mixed solvent of 1,4-dioxane (10 mL) and 2 mol/L aqueous sodium hydroxide solution (5.57 mL, 11.1 mmol), sodium iodide (1.17 g, 7.80 mmol) and potassium carbonate (1.77 g, 12.6 mmol). The mixture was stirred at room temperature for 14 hours and at 50˚C for 6.5 hours. Next, the mixture was acidified by 1 mol/L hydrochloric acid and the aqueous layer was washed with chloroform. The obtained aqueous layer was turned basic with 2 mol/L aqueous sodium hydroxide solution and extracted with chloroform. The organic layer was washed with saturated brine, dried over anhydrous magnesium sulfate and the solvent was evaporated under reduced pressure. The residue was washed with diisopropylether to obtain {1-(3-hydroxypropyl)-3-[3-((R)-2-methylpyrrolidin-1-yl)propyl] imidazolidin-2-ylidene} malononitrile (1.63 g, 80.5 %) as a white solid.
$^1$H NMR ($CDCl_3$, δppm) : 1.08 (d, J = 6.0 Hz, 3H), 1.35-1.48 (m, 1H), 1.85-2.20 (m, 9H), 2.22-2.38 (m, 1H), 2.73-2.86 (m, 1H), 3.08-3.17 (m, 1H), 3.48-3.78 (m, 10H).

(Step 2)

{1-(3-Hydroxypropyl)-3-[3-((R)-2-methylpyrrolidin-1-yl)propyl]imidazolidin-2-ylidene}malononitrile (1.60 g, 5.05 mmol) obtained in the Step 1 was dissolved in dichloromethane (32 mL) and the solution was sequentially added with triethylamine (0.469 mL, 6.06 mmol) and methanesulfonylchloride (0.469 mL, 6.06 mmol) followed by stirring under ice-cooling for 2 hours and then at room temperature for 1 hour. The mixture was added with methanesulfonylchloride (0.039 mL, 0.50 mmol) and stirred at room temperature for 2 hours. The mixture was further added with methanesulfonylchloride (0.039 mL, 0.50 mmol), stirred at room temperature for 1.5 hours and added with triethylamine (0.141 mL, 1.01 mmol) and methanesulfonylchloride (0.039 mL, 0.50 mmol), followed by stirring at room temperature for 1 hour. The mixture was added with saturated brine and extracted with ethyl acetate. The organic layer was washed with saturated brine and the solvent was evaporated under reduced pressure. The residue was purified by silica gel column chromatography (chloroform) and recrystallized from n-hexane/ethyl acetate to obtain {1-(3-methanesulfonyloxypropyl)-3-[3-((R)-2-methylpyrrolidin-1-yl)propyl]imidazolidin-2-ylidene}malononitrile (0.983 g, 49.3 %) as a white solid.
$^1$H NMR ($CDCl_3$, δppm) : 1.08 (d, J = 5.9 Hz, 3H), 1.32-1.47 (m, 1H), 1.65-2.00 (m, 5H), 2.03-2.37 (m, 5H), 2.73-2.86 (m, 1H), 3.04-3.17 (m, 4H), 3.48-3.76 (8H), 4.35 (t, J = 5.9 Hz, 2H).

(Step 3)

{1-(3-Methanesulfonyloxypropyl)-3-[3-((R)-2-methylpyrrolidin-1-yl)propyl]imidazolidin-2-ylidene} malononitrile (0.530 g, 1.34 mmol) obtained in the Step 2 was dissolved in 1,4-dioxane (3 mL) and the solution was added with a solution of (R)-2-methylpyrrolidinehydrobromate (0.446 g, 2.68 mmol) prepared in a similar manner to the method described in J. Org. Chem., Vol. 54, p.209 (1989), dissolved in a mixed solvent of 1,4-dioxane(1 mL) and 2 mol/L aqueous sodium hydroxide solution (1.34 mL, 2.68 mmol) and potassium carbonate (0.371 g, 2.68 mmol). After stirring at room temperature for 0.5 hour, the mixture was stirred at 40˚C for 18.5 hours, added with saturated brine and extracted with ethyl acetate. The organic layer was washed with saturated brine, dried over anhydrous magnesium sulfate and the solvent was evaporated under reduced pressure. The residue was recrystallized from n-hexane/ethyl acetate to obtain a white solid (0.230 g). The obtained white solid was dissolved in methanol (4 mL) and a solution in which fumaric acid (0.139 g, 1.20 mmol) was dissolved in methanol (7 mL) was added thereto. After stirring at room temperature for 1.5 hours, the mixture was concentrated under reduced pressure. The residue was recrystallized from methanol/acetone

to obtain the titled compound (0.291 g, 35.2 %) as a white solid.
$^1$H NMR ($CD_3OD$, δppm) : 1.42 (d, J = 5.9 Hz, 6H), 1.66-1.85 (m, 2H), 2.00-2.20 (m, 8H), 2.22-2.40 (m, 2H), 2.92-3.08 (m, 2H), 3.10-3.27 (m, 2H), 3.28-3.78 (m, 14H), 6.69 (s, 4H).
Melting point: 173-174˚C.

Example 117

{1-[3-((R)-2-methylpiperidino)propyl]-3-[3-((S)-2-methylpyrrolidin-1-yl)propyl]imidazolidin-2-ylidene} malononitrile (Compound 117)

In a similar manner to Step 3 of Example 116, the titled compound (0.111 g, 46.6 %) was obtained by using {1-(3-methanesulfonyloxypropyl)-3-[3-((R)-2-methylpyrrolidin-1-yl)propyllimidazolidin-2-ylidene} malononitrile (0.245 g, 0.62 mmol) obtained in the Step 2 of Example 116 and (S)-2-methylpyrrolidine hydrobromate (0.21 g, 1.24 mmol) prepared in a similar manner to the method described in J. Org. Chem., Vol. 54, p.209 (1989).
$^1$H NMR ($CDCl_3$, δppm): 1.07 (d, J = 6.0 Hz, 6H), 1.35-1.45 (m, 2H), 1.65-1.79 (m, 4H), 1.81-1.97 (m, 6H), 2.04-2.17 (m, 4H), 2.24-2.31 (m, 2H), 2.79 (td, J = 7.9, 12.0 Hz, 2H), 3.12 (td, J = 3.1, 7.9Hz, 2H), 3.48-3.70 (m, 8H).
APCIMS m/z: $[M+H]^+$385.
Melting point: 87-89˚C.

Example 118

{1,3-Bis[3-((R)-2-hydroxymethylpyrrolidin-1-yl) propyl]imidazolidin-2-ylidene}malononitrile (Compound 118)

In a similar manner to Step 2 of Example 115, the titled compound (0.17 g, 26.3 %) was obtained as a white solid by using [1-(3-bromopropyl)-3-(3-methanesulfonyloxypropyl)imidazolidin-2-ylidene] malononitrile (0.595 g, 1.52 mmol) obtained in Step 1 of Example 115 and (R)-2-hydroxymethylpyrrolidine (0.60 ml, 6.08 mmol).
$^1$H NMR ($CDCl_3$, δppm) : 1.72-1.82 (m, 6H), 1.86-1.94 (m, 6H), 2.24-2.33 (m, 2H), 2.41 (td, J = 5.9, 12.1 Hz, 2H), 2.59-2.66 (m, 2H), 2.79 (td, J = 8.0, 12.2 Hz, 2H), 3.18-3.24 (m, 2H), 3.42 (dd, J = 2.8, 10.9Hz, 2H), 3.50-3.69 (m, 10H).
APCIMS m/z: $[M+H]^+$417.
Melting point: 79-80˚C.

Example 119

{1-[3-((R)-2-hydroxymethylpyrrolidin-1-yl)propyl]-3-[3-(2-methylpyrrolidin-1-yl)propyl]imidazolidin-2-ylidene.}malononitrile (Compound 119)

(Step 1)

{1-(3-Hydroxypropyl)-3-[3-(2-methylpyrrolidin-1-yl) propyl]imidazolidin-2-ylidene}malononitrile (7.12 g, 22.46 mmol) obtained in Step 2 of Example 65 was dissolved in dichloromethane (100 mL) and the solution was sequentially added with triethylamine (9.39 mL, 67.38 mmol)and methanesulfonylchloride (2.09 mL, 26.95 mmol) under ice-cooling. After stirring at room temperature for 1.5 hours, the mixture was added with saturated brine and extracted with ethyl acetate. The organic layer was washed with saturated brine, dried over anhydrous magnesium sulfate and the solvent was evaporated under reduced pressure. The residue was recrystallized from ethyl acetate/n-hexane (1:1) to obtain {1-(3-methanesulfonyloxypropyl)-3-[3-(2-methylpyrrolidin-1-yl)propyl]imidazolidin-2-ylidene} malononitrile (5.81 g, 65.5 %) as a white solid.
$^1$H NMR ($CDCl_3$, δppm) : 1.09 (d, J = 5.8 Hz,3H), 1.36-1.48 (m, 1H), 1.67-1.76 (m, 2H), 1.81-1.99 (m, 3H), 2.09-2.21 (m, 4H), 2.30-2.37 (m, 1H), 2.77-2.87 (m, 1H), 3.05 (s, 3H), 3.15 (dt, J = 3.3, 9.2Hz, 1H), 3.50-3.73 (m, 8H), 4.35 (t, J = 5.7 Hz, 2H).

(Step 2)

In a similar manner to Step 3 of Example 116, the titled compound (0.22 g, 55.9 %) was obtained as a white solid by using {1-(3-methanesulfonyloxypropyl)-3-[3-(2-methylpyrrolidin-1-yl)propyllimidazolidin-2-ylidene} malononitrile (0.40 g, 1.0 mmol) obtained in the Step 1 and (R)-2-hydroxymethylpyrrolidine (0.20 ml, 2.0 mmol).
$^1$H NMR ($CDCl_3$, δppm) : 1.08 (d, J = 5.9 Hz, 3H), 1.37-1.42 (m, 1H), 1.72-1.78 (m, 4H), 1.82-1.93 (m, 5H), 2.05-2.17 (m, 2H), 2.22-2.30 (m, 2H), 2.32-2.43 (m, 1H), 2.60-2.72 (m, 2H), 2.73-2.84 (m, 3H), 3.10-3.19 (m, 2H), 3.39-3.42 (m, 1H), 3.58-3.63 (m, 9H).

APCIMS m/z: $[M+H]^+$401.
Melting point: 57-59°C.

Example 120

{1-[3-(3,3-Difluoropyrrolidin-1-yl)propyl]-3-[3-(2-methylpyrrolidin-1-yl)propyl]imidazolidin-2-ylidene} malononitrile (Compound 120)

3,3-Difluoropyrrolidinehydrochloride (0.063 g, 0.43 mmol) was dissolved in 1,4-dioxane (2 ml) and the solution was added with 2 mol/L aqueous sodium hydroxide solution (0.215 mL). After stirring at room temperature for 3 minutes, the mixture was added with {1-(3-methanesulfonyloxypropyl)-3-[3-(2-methylpyrrolidin-1-yl) propyl]imidazolidin-2-ylidene}malononitrile (0.100 g, 0.25 mmol) obtained in Step 1 of Example 119 and potassium carbonate (0.070 g, 0.510 mmol), followed by stirring at 40°C for 3 days. Next, the mixture was added with a solution obtained by dissolving 3,3-difluoropyrrolidine hydrochloride (55.6 mg, 0.38 mmol) in 1,4-dioxane (0.2 mL) and then adding 2 mol/L aqueous sodium hydroxide solution (0.190 mL), followed by stirring at room temperature for 3 minutes. After stirring at 50°C for 2.5 days, the mixture was added with water and extracted with ethyl acetate. The organic layer was dried over anhydrous magnesium sulfate, and the solvent was evaporated under reduced pressure to obtain the titled compound (0.087 g, 85.0%).
$^1$H NMR ($CDCl_3$, δppm) : 0.73 (d, J = 5.9 Hz, 3H), 1.33-1.46 (m, 1H), 1.66-1.93 (m, 10H), 2,05-2.34 (m, 5H), 2.54 (t, J = 7.0 Hz, 2H), 2.74 (t, J= 7.0 Hz, 2H), 2.77-2.81 (m, 1H), 2.88 (t, J = 12.8 Hz, 2H), 3.12 (dt, J= 1.8, 9.2Hz, 1H), 3.48-3.70 (m, 8H).
APCIMS m/z: $[M+H]^+$407.
Melting point: 99-102°C.

Example 121

{1,3-Bis[3-(3,3-difluoropyrrolidin-1-yl)propyl] imidazolidin-2-ylidene}malononitrile (Compound 121)

3,3-Difluoropyrrolidine hydrochloride (0.197 g, 1.33 mmol) was dissolved in 1,4-dioxane (3 mL) and the solution was added with 2 mol/L aqueous sodium hydroxide solution (0.190 mL). After stirring the mixture at room temperature for 3 minutes, the mixture was added with [1-(3-bromopropyl)-3-(3-methanesulfonyloxypropyl)imidazclidin -2-ylidene] malononitrile (150 mg, 0.38 mmol) obtained in Step 1 of Example 115 and potassium carbonate (105 mg, 0.76 mmol), followed by stirring at 50°C for 7 hours. The mixture was added with water and the precipitated solid was collected by filtration. The obtained solid was washed with water to obtain the titled compound (73 mg, 45%) as a white solid.
$^1$H NMR ($CDCl_3$, δppm) : 1.87 (tt, J = 6.8, 14.7 Hz, 4H), 2.28 (tt, J = 7.0, 14.9 Hz, 4H), 2.55 (t, J = 6.8 Hz, 4H), 2.75 (t, J = 7.0 Hz, 4H), 2.89 (t, J = 13.0 Hz, 4H), 3.59-3.64 (m, 8H).
APCIMS m/z: $[M+H]^+$429.
Melting point: 122-124°C.

Example 122

[1,3-Bis(3-piperidinopropyl)hexahydropyrimidin-2-ylidene]malononitrile (Compound 122)

(Step 1)

A mixture of 3-hydroxypropylamine (10.0 g, 133 mmol) and acrylonitrile (6.00 mL, 90.5 mmol) was stirred at room temperature for 5 hours and then at 100°C for 30 minutes. The mixture was purified by silica gel column chromatography (chloroform/methanol (85:15)) to obtain 3-(3-hydroxypropylamino)propionitrile (13.0 g, 76 %).
$^1$H NMR ($CDCl_3$, δppm) : 1.69-1.78 (m, 2H), 2.54 (t, J=6.7Hz, 2H), 2.89 (t, J = 5.9 Hz, 2H), 2.95 (t, J = 6.7 Hz, 2H), 3.80 (t, J=5.4Hz, 2H).

(Step 2)

Lithium aluminum hydride (2.97 g, 78.1 mmol) was suspended in THF (135 mL) and a solution of 3-(3-hydroxypropylamino)propionitrile (5.00 g, 39.1 mmol) obtained in the Step 1. in THF (20 mL) was added dropwise at room temperature. The mixture was stirred at room temperature for 30 minutes and heated under reflux for 2 hours. After cooling the mixture to room temperature, the mixture was added with sodium sulfate decahydrate and the precipitate was collected by filtration. The obtained precipitate was suspended in THF and stirred under reflux for 1 hour. The mixture was filtered and the filtrate was combined with the filtrate previously obtained and the solvent was evaporated

under reduced pressure to obtain 3-(3-aminopropylamino)-1-propanol (3.99 g, 77 %).
$^1$H NMR ($CDCl_3$, δppm) : 1.43-1.74 (m, 4H), 2.68 (t, J=7.0Hz, 2H), 2.76 (t, J = 6.8 Hz, 2H), 2.86 (t, J = 5.7 Hz, 2H), 3.80 (t, J=5.3Hz, 2H).

(Step 3)

[Bis(methylthio)methylene]malononitrile (4.02 g, 23.6 mmol) was dissolved in THF (25 mL) and the solution was added with a solution of 3-(3-aminopropylamino)-1-propanol (3.18 g, 24.1 mmol) obtained in the Step 2 in THF (7 mL). After stirring at room temperature for 2 hours, the mixture was added with diisopropylether (64 mL) and ice-cooled. The precipitated solid was collected by filtration to obtain [1-(3-hydroxypropyl) hexahydropyrimidin-2-ylidene]malononitrile (3.54 g, 73 %).
$^1$H NMR ($CD_3OD$, δppm) : 1.91-2.00 (m, 4H), 3.24-3.41 (m, 4H), 3.60-3.66 (m, 4H).

(Step 4)

[1-(3-Hydroxypropyl)hexahydropyrimidin-2-ylidene] malononitrile (2.00 g, 9.71 mmol) obtained in the Step 3 was dissolved in DMF (20mL) and the solution was added with potassium carbonate (2.68 g, 19.4 mmol) and 1,3-dibromopropane (9.80 g, 48.6 mmol). After stirring at room temperature for 22 hours, the mixture was added with potassium carbonate (2.68 g, 19.4 mmol) and 1,3-dibromopropane (9.80 g, 48.6 mmol), followed by further stirring at room temperature for 27 hours. The mixture was added with water, extracted with ethyl acetate and then with chloroform. The organic layer was collected, washed with saturated brine, dried over anhydrous sodium sulfate and the solvent was evaporated under reduced pressure. The residue was purified by silica gel column chromatography (methanol/ethyl acetate (1:9)) to obtain an oily substance (1.75g).

A mixture of the obtained oily substance (0.720 g), 1,4-dioxane (11 mL), sodium iodide (0.33 g, 2.2 mmol), potassium carbonate (0.46 g, 3.3 mmol) and piperidine (0.38 g, 4.4 mmol) was stirred at 60˚C for 7 hours. Piperidine (0.38 g, 4.4 mmol) was added to the mixture and the mixture was stirred at 60˚C for 3 hours. The mixture was cooled to room temperature, added with chloroform and extracted with 1 mol/L hydrochloric acid. After washing with chloroform, the aqueous layer was added with aqueous sodium hydroxide solution to turn it basic (a pH of 12 or less), the mixture was extracted with chloroform. The organic layer was dried over anhydrous sodium sulfate and the solvent was evaporated under reduced pressure to obtain [1-(3-hydroxypropyl)-3-(3-piperidinopropyl) hexahydropyrimidin-2-ylidene] malononitrile (0.386 g, 29 %). $^1$H NMR ($CDCl_3$, δppm) : 1.40-1.47 (m, 2H), 1.53-1.61 (m, 6H), 1.87-2.10 (m, 4H), 2.31-2.38 (m, 6H), 3.24-3.28 (m, 4H), 3.59 (t, J =7.7Hz, 2H), 3.66-3.76 (m, 4H).

(Step 5)

[1-(3-Hydroxypropyl)-3-(3-piperidinopropyl) hexahydropyrimidin-2-ylidene]malononitrile (0.384 g, 1.16 mmol) obtained in the Step 4 was dissolved in dichloromethane (12 mL) and the solution was sequentially added with triethylamine (0.32 mL, 2.3 mmol) and methanesulfonylchloride (0.11 mL, 1.4 mmol) under ice-cooling. After stirring at room temperature for 1 hour, the mixture was added with saturated aqueous sodium hydrogencarbonate solution and extracted with chloroform. The organic layer was washed with saturated brine, dried over anhydrous sodium sulfate and the solvent was evaporated under reduced pressure. The residue was purified by silica gel column chromatography (ethyl acetate) to obtain an oily substance (0.381 g).

The obtained oily substance (0.373 g) was dissolved in 1,4-dioxane (4 mL) and the solution was added with potassium carbonate (0.33 g, 2.4 mmol), potassium iodide (0.12 g) and piperidine (0.14 g, 1.6 mmol). After stirring at room temperature for 13 hours, the mixture was added with piperidine (0.14 g, 1.6 mmol), followed by stirring at 50˚C for 6 hours. After cooling, the mixture was added with water and extracted with ethyl acetate. The organic layer was washed with saturated brine, dried over anhydrous sodium sulfate and the solvent was evaporated under reduced pressure. The residue was triturated with n-hexane/diisopropylether to obtain the titled compound (0.204 g, 44.2 %) as a white solid.
$^1$H NMR ($CDCl_3$, δppm) : 1.40-1.47 (m, 4H), 1.53-1.61 (m, 8H), 1.87-2.08 (m, 6H), 2.31-2.38 (m, 12H), 3.24 (t, J = 7.7 Hz, 4H), 3.57 (t, J =6.3 Hz., 4H).
Melting point: 88-90˚C.

Example 123

{1,3-Bis[3-(2-methylpyrrolidin-1-yl)propyl] hexahydropyrimidin-2-ylidene}malononitrile (Compound 123) (Step 1)

[1-(3-Hydroxypropyl)hexahydropyrimidin-2-ylidene] malononitrile (2.00 g, 9.71 mmol) obtained in Step 3 of

Example 122 was dissolved in DMF (20 mL) and the solution was added with potassium carbonate (2.68 g, 19.4 mmol) and 1,3-dibromopropane (9.80 g, 48.6 mmol). After stirring at room temperature for 22 hours, the mixture was added with potassium carbonate (2.68 g, 19.4 mmol) and 1,3-dibromopropane (9.80 g, 48.6 mmol), followed by stirring at room temperature for 27 hours. The mixture was added with water, extracted with ethyl acetate and then with chloroform. The organic layer was washed with saturated brine, dried over anhydrous sodium sulfate and the solvent was evaporated under reduced pressure. The residue was purified by silica gel column chromatography (methanol/ethyl acetate (1:9)) to obtain an oily substance (1.75 g).

The obtained oily substance (0.906 g) was dissolved in 1,4-dioxane (8 mL) and the solution was added with 2-methylpyrrolidine (0.32 mL, 3.13 mmol), potassium carbonate (656 mg, 4.74mmol) and potassium iodide (262 mg, 1.58 mmol), followed by stirring at 50˚C for 5 hours. After cooling, the mixture was concentrated under reduced pressure. The residue was added with 2 mol/L hydrochloric acid and the aqueous layer was washed with ethyl acetate. The aqueous layer was added with 2 mol/L aqueous sodium hydroxide solution to adjust the pH of the mixture to 10.1 and the mixture was extracted with ethyl acetate. The organic layer was washed with saturated brine, dried over anhydrous magnesium sulfate and the solvent was evaporated under reduced pressure.

The residue (335 mg) was dissolved in dichloromethane (5.2 mL) and the solution was sequentially added with triethylamine (0.282 mL, 2.02 mmol) and methanesulfonylchloride (751 μL, 0.970 mmol) under ice-cooling. After stirring at room temperature for 0.5 hour, the mixture was added with saturated aqueous sodium hydrogencarbonate solution and extracted with ethyl acetate. The organic layer was washed with saturated brine and the solvent was evaporated under reduced pressure. The residue was purified by NH silica gel column chromatography (ethyl acetate) to obtain {1-(3-methanesulfonyloxypropyl)-3-[3-(2-methylpyrrolidin-1-yl) propyl]hexahydropyrimidin-2-ylidene}malononitrile (251.0 mg).

$^1$H NMR ($CDCl_3$, δppm) : 1.07 (d, J = 5.9 Hz,3H), 1.32-2.30 (m, 12H), 2.72-2.82 (m, 1H), 3.05 (s, 3H), 3.06-3.32 (m, 6H), 3.49-3.73 (m, 4H), 4.35 (t, J = 5.9 Hz, 2H).

(Step 2)

{1-(3-Methanesulfonyloxypropyl)-3-[3-(2-methylpyrrolidin-1-yl)propyl]hexahydropyrimidin-2-ylidene}malononitrile (0.251 g, 0.612 mmol) obtained in the Step 1 was dissolved in 1,4-dioxane (4 mL) and the solution was added with 2-methylpyrrolidine (0.13 mL, 1.27 mmol) and potassium carbonate (0.254 g, 1.83 mmol), followed by stirring at room temperature for 13 hours and then, at 45˚C for 7 hours. After cooling, the mixture was added with saturated aqueous sodium hydrogencarbonate solution and extracted with ethyl acetate. The organic layer was washed with saturated brine, dried over anhydrous magnesium sulfate and the solvent was evaporated under reduced pressure. The obtained oily substance was purified by NH silica gel column chromatography (ethyl acetate) to obtain the titled compound (171 mg, 70.2 %) as an oily substance.

$^1$H NMR ($CDCl_3$, δppm) : 1.12 (d, J = 6.0 Hz, 6H), 1.35-1.49 (m, 4H), 1.60-2.45 (m, 16H), 2.76-2.86 (m, 2H), 3.12-3.30 (m, 6H), 3.49-3.73 (m, 4H).

Example 124

{1,3-Bis[3-(2-methylpyrrolidin-1-yl)propyl] benzimidazolidin-2-ylidene}malononitrile (Compound 124)

(Step 1)

[Bis(methylthio)methylene]malononitrile (6.00 g, 35.2 mmol) was dissolved in ethanol (50 mL) and a solution of phenylenediamine (3.80 g, 35.6 mmol) in ethanol (20 mL) was added dropwise, followed by stirring at 70˚C for 4 hours. The mixture was cooled and the precipitated white solid was collected by filtration to obtain (benzimidazolidin-2-ylidene)malononitrile (4.97 g, 77.6 %). $^1$H NMR (DMSO-$d_6$, δppm) : 7.19 (dd, J = 3.1, 6.1 Hz, 2H), 7.30 (dd, J = 3.1, 6.1 Hz, 2H), 12.82 (br s, 2H).

(Step 2)

(Benzimidazolidin-2-ylidene)malononitrile (0.890 g, 4.89 mmol) obtained in the Step 1 was dissolved in DMF (10 mL) and the solution was sequentially added with potassium carbonate (3.40 g, 24.5 mmol) and 1,3-dibromopropane (5.00 mL, 48.9 mmol). After stirring at room temperature for 1 hour and then at 60˚C for 30 minutes, the mixture was added with saturated brine and extracted with ethyl acetate. The organic layer was washed with saturated brine, dried over anhydrous magnesium sulfate and the solvent was evaporated under reduced pressure. The obtained white solid was washed with chloroform to obtain [1-(3-bromopropyl)benzimidazolidin-2-ylidene]malononitrile (0.497 g, 33.6 %) as a white solid.

$^1H$ NMR ($CDCl_3$, δppm) : 2.47-2.56 (m, 2H), 2.81 (t, J=5.9Hz, 2H), 4.24 (t, J = 6.1 Hz, 2H), 7.34-7.41 (m, 3H), 7.85-7.89 (m, 1H).

(Step 3)

[1-(3-Bromopropyl)benzimidazolidin-2-ylidene] malononitrile (0.461 g, 1.52 mmol) obtained in the Step 2 was dissolved in DMF (5 mL) and the solution was added with 1,3-dibromopropane (0.310 mL, 3.04 mmol). The mixture was added with sodium hydride (0.182 g, 4.56 mmol) under ice-cooling and stirred at room temperature for 3 hours. The mixture was added with water and extracted with ethyl acetate. The organic layer was washed with saturated brine, dried over anhydrous magnesium sulfate and the solvent was evaporated under reduced pressure. The residue was purified by silica gel column chromatography (n-hexane/ethyl acetate (3:1)) to obtain [1,3-bis(3-bromopropyl)benzimidazolidin-2-ylidene] malononitrile (0.209 g, 32.6 %) as a white solid.
$^1H$ NMR ($CDCl_3$, δppm) : 2.44-2.54 (m, 4H), 3.54 (t, J=5.4Hz, 4H), 4.55 (t, J = 7.7 Hz, 4H), 7.37-7.47 (m, 4H).

(Step 4)

[1,3-Bis(3-bromopropyl)benzimidazolidin-2-ylidene] malononitrile (209 mg, 0.495 mmol) obtained in the Step 3 was dissolved in 1,4-dioxane (3 mL) and the solution was added with potassium carbonate (207 mg, 1.49 mmol) and 2-methylpyrrolidine (0.152 mL, 1.49 mmol). After stirring at 60˚C for 2.5 hours, the mixture was added with water and extracted with ethyl acetate. The organic layer was washed with saturated brine, dried over anhydrous magnesium sulfate and the solvent was evaporated under reduced pressure. After the residue was purified by silica gel column chromatography (chloroform/(7 mol/L ammonia-methanol) (10:1)), the obtained solid was washed with n-hexane to obtain the titled compound (0.042 g, 19.5 %) as a white solid.
$^1H$ NMR ($CDCl_3$,δppm) : 1.02 (d, J = 5.9 Hz, 6H), 1.32-1.46 (m, 2H), 1.65-1.80 (m, 4H), 1.85-1.98 (m, 2H), 2.02-2.34 (m, 10H), 2.85 (dt, J= 7.8, 12.4 Hz, 2H), 3.12 (dt, J = 3.4, 8.3 Hz, 2H), 4.42 (t, J=7.6 Hz, 4H), 7.29-7.39 (m, 4H).
Melting point: 115-116˚C.

Example 125

{1,3-Bis[3-((S)-2-methylpyrrolidin-1-yl)propyl] benzimidazolidin-2-ylidene}malononitrile (Compound 125)

(Step 1)

(Benzimidazolidin-2-ylidene)malononitrile (0.500 g, 2.75 mmol) obtained in Step 1 of Example 124 was dissolved in DMF (5 mL) and the solution was sequentially added with potassium carbonate (1.10 g, 8.25 mmol) and 1-bromo-3-(tert-butyldimethylsiloxy)propane (1.30 mL, 5.50 mmol). After stirring at 80˚C for 15 hours, the mixture was added with saturated brine and extracted with chloroform. The organic layer was washed with saturated brine, dried over anhydrous magnesium sulfate and the solvent was evaporated under reduced pressure. The residue was purified by silica gel column chromatography (n-hexane/ethyl acetate (4:1)) to obtain {1,3-bis[3-(tert-butyldimethylsiloxy) propyl] benzimidazolidin-2-ylidene}malononitrile (0.343 g, 23.7 %) as a white solid.
$^1H$ NMR ($CDCl_3$, δppm) : 0.08 (s, 12H), 0.93 (s,18H),2.08-2.17 (m, 4H), 3.71 (t, J = 5.4 Hz, 4H), 4.47 (t, J = 7.3 Hz, 4H), 7.31 (dd,J = 3.1, 6.0 Hz, 2H), 7.44 (dd, J = 3.1, 6.0 Hz, 2H).

(Step 2)

{1,3-Bis[3-(tert-butyldimethylsiloxy)propyl] benzimidazolidin-2-ylidene}malononitrile (0.342 g, 0.650 mmol) obtained in the Step 1 was dissolved in ethyl acetate (4 mL) and the solution was added with 4 mol/L hydrochloric acid-ethyl acetate (2.00 mL, 8.00 mmol), followed by stirring at room temperature for 30 minutes. The mixture was sequentially added with 2 mol/L aqueous sodium hydroxide solution and water and the mixture was turned basic. The precipitated white solid was collected by filtration to obtain [1,3-bis (3-hydroxypropyl) benzimidazolidin-2-ylidene]malononitrile (0.167 g, 85.9 %).
$^1H$ NMR (DMSO-$d_6$, δppm) : 1.88-1.99 (m, 4H), 3.43-3.49 (m,4H), 4.37 (t, J = 7.4 Hz, 4H), 4.70 (t, J = 5.0 Hz, 2H), 7.37 (dd, J = 3.0, 5.9Hz, 2H), 7.62 (dd, J = 3.0, 5.9 Hz, 2H).

(Step 3)

[1,3-Bis(3-hydroxypropyl)benzimidazolidin-2-ylidene] malononitrile (160 mg, 0.540 mmol) obtained in the Step 2 was dissolved in dichloromethane (3 mL) and the solution was sequentially added with triethylamine (0.190 mL, 1.35

mmol) and methanesulfonylchloride (0.150 mL, 1.89 mmol) under ice-cooilng. After the mixture was stirred at room temperature for 1 hour, triethylamine (0.190 mL, 1.35 mmol) and methanesulfonylchloride (0.150 mL, 1.89 mmol) were sequentially added, followed by stirring at room temperature for 1 hour. The mixture was added with saturated aqueous sodium hydrogencarbonate solution and extracted with chloroform. The organic layer was washed with saturated brine, dried over anhydrous magnesium sulfate and the solvent was evaporated under reduced pressure to obtain clear crystal (245 mg).

The obtained crystal was dissoved in 1,4-dioxane (3 mL) and the solution was added with a solution of (S)-2-methylpyrrolidinehydrobromate (0.323 g, 1.94 mmol) prepared in a similar manner to the method described in J. Org. Chem., Vol. 54, p.209 (1989) dissolved in a mixed solvent of 1,4-dioxane (3 mL) and 2 mol/L aqueous sodium hydroxide solution (1.00 mL, 2.00 mmol), followed by adding potassium carbonate (0.220 g, 1.62 mmol). After stirring at 60˚C for 15 hours, the mixture was added with water and extracted with chloroform. The organic layer was washed with saturated brine, dried over anhydrous magnesium sulfate and the solvent was evaporated under reduced pressure. The residue was purified by silica gel column chromatography (chloroform/(7 mol/L ammonia-methanol) (10:1)). The obtained crude crystal was washed with n-hexane to obtain the titled compound (0.073 g, 31.1 %) as a white solid.
$^1$H NMR ($CDCl_3$, δppm) : 1.02 (d, J = 5.9 Hz, 6H),1.32-1.46 (m, 2H), 1.65-1.80 (m, 4H), 1.85-1.98 (m, 2H), 2.02-2.34 (m, 10H), 2.85 (dt, J=7.8, 12.4 Hz, 2H), 3.12 (dt, J = 3.4, 8.3 Hz, 2H), 4.42 (t, J = 7.6Hz,4H),7.29-7.39 (m, 4H).
Melting point: 83-85˚C.

Example 126

[1-(1-Benzylpiperidin-4-yl)-3-(3-piperidinopropyl) imidazolidin-2-ylidene]malononitrile (Compound 126)

(Step 1)

[Bis(methylthio)methylene]malononitrile (3.00 g, 17.6 mmol) was dissolved in THF (30 mL) and the solution was added with 4-amino-1-benzylpiperidine (3.59 mL, 17.6 mmol), followed by stirring at room temperature for 19 hours. Next, the mixture was added with 2-aminoethanol (1.06 mL, 17.6 mmol) and heated under reflux for 5 hours. The mixture was further added with 2-aminoethanol (0.30 mL, 5.0 mmol), heated under reflux for 3 hours and further added with 2-aminoethanol (0.40 mL, 6.6 mmol), followed by heating under reflux for 2 hours. After cooling the mixure to room temperathre, the solvent was evaporated under reduced' pressure. The obtained brown solid was washed with ethyl acetate/diisopropylether (1:1) to obtain [(1-benzylpiperidin-4-ylamino)(2-hydroxyethylamino) methylene]malononitrile (3.18 g, 57.7 %) as a brown solid.
$^1$H NMR ($CD_3OD$, δppm) : 1.52-1.68 (m, 2H), 1.92-2.04 (m, 2H), 2.10-2.22 (m, 2H), 2.82-2.93 (m, 2H), 3.31 (t, J = 4.5 Hz, 2H), 3.52 (s, 2H), 3.62-3.80 (m, 3H), 7.20-7.35 (m, 5H).

(Step 2)

[(1-Benzylpiperidin-4-ylamino)(2-hydroxyethylamino) methylene]malononitrile (1.88 g, 5.78 mmol) obtained in the Step 1 was dissolved in dichloromethane (20 mL) and the solution was sequentially added with triethylamine (1.37 mL, 9.82 mmol) and methanesulfonylchloride (0.671 mL, 8.67 mmol). After stirring at room temperature for 1.5 hours, the mixture was added with triethylamine (0.403 mL, 2.89 mmol) and methanesulfonylchloride (0.134 mL, 1.73 mmol), followed by stirring at room temperature for 0.5 hour. The mixture was further added with triethylamine (1.61 mL, 11.6 mmol) and stirred at room temperature for 67 hours. The mixture was added with saturated brine and extracted with chloroform. The organic layer was washed with saturated brine and evaporated under reduced pressure to obtain [1-(1-benzylpiperidin-4-yl)imidazolidin-2-ylidene]malononitrile (0.705 g, 39.7 %) as a white solid.
$^1$H NMR ($CDCl_3$, δppm) : 1.68-1.90 (m, 4H), 2.08-2.22 (m, 2H), 2.92-3.02 (m, 2H), 3.50 (s, 2H), 3.55-3.76 (m, 4H), 4.18-4.34 (m, 1H), 7.22-7.36 (m, 5H).

(Step 3)

[1-(1-Benzylpiperidin-4-yl)imidazolidin-2-ylidene] malononitrile (0.705 g, 2.29 mmol) obtained in the Step 2 was dissolved in DMF (7 mL) and the solution was sequentially-added with potassium carbonate (0.475 g, 3.44 mmol) and 1,3-dibromopropane (1.39 mL, 13.7 mmol). After stirring at room temperature for 26 hours, the mixture was added with saturated brine and extracted with ethyl acetate. The organic layer was washed with saturated brine, dried over anhydrous magnesium sulfate and the solvent was evaporated under reduced pressure. The obtained crude crystal was washed with n-hexane to obtain [1-(1-benzylpiperidin-4-yl)-3-(3-bromopropyl)imidazolidin-2-ylidene]malononitrile (0.694 g, 70.7 %) as a white solid.
$^1$H NMR ($CDCl_3$, δppm): 1.72-1.88 (m, 4H), 2.08-2.32 (m, 4H), 2.93-3.03 (m, 2H), 3.45-3.75 (m, 10H), 4.12-4.29 (m,

1H), 7.23-7.36 (m, 5H).

(Step 4)

[1-(1-Benzylpiperidin-4-yl)-3-(3-bromopropyl) imidazolidin-2-ylidene]malononitrile (0.694 g, 1.62 mmol) obtained in the Step 3 was dissolved in 1,4-dioxane (70 mL) and the solution was added with potassium iodide (0.269 g, 1.62 mmol), potassium carbonate (0.448 g, 3.24 mmol) and piperidine (0.481 mL, 4.86 mmol), followed by stirring at 80˚C for 7 hours. After cooling, the mixture was added with saturated brine and extracted with ethyl acetate. The organic layer was washed with saturated brine, dried over anhydrous magnesium sulfate and the solvent was evaporated under reduced pressure. The residue was recrystallized from acetone/diisopropylether to obtain the titled compound (0.360 g, 51.4 %) as a white solid.
$^1$H NMR ($CDCl_3$, δppm) : 1.37-1.93 (m, 12H), 2.08-2.22 (m, 2H), 2.29-2.43 (m, 6H), 2.90-3.02 (m, 2H), 3.47-3.65 (m, 8H), 4.14-4.28 (m, 1H), 7.23-7.36 (m, 5H).
Melting point: 156-158 ˚C.

Example 127

{1-(1-Benzylpiperidin-4-yl)-3-[3-(2-methylpyrrolidin-1-yl)propyl]imidazolidin-2-ylidene} malononitrile (Compound 127)

[1-(1-Benzylpiperidin-4-yl)-3-(3-bromopropyl) imidazolidin-2-ylidene]malononitrile (0.890 g, 2.08 mmol) obtained in Step 3 of Example 126 was dissolved in 1,4-dioxane (80 mL) and the solution was added with potassium iodide (0.340 g, 2.08 mmol), potassium carbonate (0.575 g, 4.16 mmol) and 2-methylpyrrolidine (0.637 mL, 6.24 mmol). After stirring at 80˚C for 4 hours, the mixture was added with 2-methylpyrrolidine (0.637 mL, 6.24 mmol) and stirred at 80˚C for 2.5 hours. The mixture was further added with potassium iodide (0.17 g, 1.04 mmol) and potassium carbonate (0.287 g, 2.08 mmol), followed by stirring at 80˚C for 18 hours. After cooling, the mixture was added with saturated brine and extracted with ethyl acetate. The organic layer was washed with saturated brine, dried over anhydrous magnesium sulfate and the solvent was evaporated under reduced pressure. The residue was purified by silica gel column chromatography (chloroform/(2 mol/L ammonia-methanol) (9:1)) and recrystallized from isopropylalcohol to obtain the titled compound (0.425 g, 47.2 %) as a white solid.
$^1$H NMR ($CDCl_3$, δppm) : 1.06 (d, J = 6.2 Hz,3H),1.30-1.45 (m, 1H), 1.63-1.98 (m, 9H), 2.00-2.32 (m, 5H), 2.72-2.84 (m, 1H), 2.91-3.02 (m,2H), 3.06-3.16 (m, 1H), 3.47-3.72 (m, 8H), 4.16-4.27 (m,1H), 7.23-7.36 (m, 5H) .
Melting point: 130-131˚C.

Example 128

[1-(Piperidin-4-yl)-3-(3-piperidinopropyl) imidazolidin-2-ylidene]malononitrile (Compound 128)

[1-(1-Benzylpiperidin-4-yl)-3-(3-piperidinopropyl) imidazolidin-2-ylidene]malononitrile (0.200 g, 0.463 mmol) obtained in Example 126 was dissolved in 1,2-dichloroethane (3 mL) and the solution was added with 1-chloroethyl chloroformate (0.180 mL, 1.67 mmol). After heating under reflux for 4.5 hours, the mixture was concentrated under reduced pressure and the residue was added with methanol (3 mL), followed by heating under reflux for 1.3 hours. The mixture was concentrated under reduced pressure and the residue was crystallized from methanol/ethyl acetate to obtain dihydrochloride of [1-(piperidin-4-yl)-3-(3-piperidinopropyl)imidazolidin-2-ylidene]malononitrile as a brown solid.
The obtained solid was added with 1 mol/L aqueous sodium hydroxide solution and extracted with ethyl acetate. The organic layer was dried over anhydrous magnesium sulfate and the solvent was evaporated under reduced pressure to obtain the titled compound (0.134 g, 85 %) as a brown oily substance.
$^1$H NMR ($CD_3OD$, δppm) : 1.46-1.54 (m, 2H), 1.58-1.96 (m, 10H), 2.08-2.20 (m, 2H), 2.64-2.78 (m, 6H), 2.65-2.77 (m, 2H), 3.11-3.22 (m, 2H), 3.60-3.70 (m, 6H), 4.13-4.30 (m, 1H).

Example 129

[1-(1-Methylpiperidin-4-yl)-3-(3-piperidinopropyl) imidazolidin-2-ylidene]malononitrile (Compound 129)

A dihydrochloride (0.060 g, 0.14 mmol) of [1-(piperidin-4-yl)-3-(3-piperidinopropyl)imidazolidin-2-ylidene] malononitrile obtained in Example 128 was dissolved in 1,2-dichloroethane (5 mL) and the solution was added with 36% aqueous formalin solution (0.044 g, 0.54 mmol) and triacetoxy sodium borohydride (0.093 g, 0.45 mmol). After stirring at room temperature for 4 hours, the mixture was added with 36% aqueous formalin solution (0.022 g, 0.27 mmol) and triacetoxy sodium borohydride (0.047 g, 0.23 mmol), followed by stirring at room temperature for 4 hours. The mixture

was further added with 36% aqueous formalin solution (0.022 g, 0.27 mmol) and triacetoxy sodium borohydride (0.047 g, 0.23 mmol), followed by stirring at room temperature for 22.5 hours. The mixture was added with aqueous sodium carbonate solution and extracted with ethyl acetate. The organic layer was dried over anhydrous magnesium sulfate and the solvent was evaporated under reduced pressure. The residue was purified by preparative TLC (chloroform/(2 mol/L ammonia-methanol)) to obtain the titled compound (0.021 g, 40.8 %).
$^1$H NMR ($CDCl_3$,δppm) : 1.46-1.60 (m, 6H), 1.72-1.92 (m, 4H), 2.07-2.21 (m, 2H), 2.29 (s, 3H), 2.32-2.46 (m, 6H), 2.86-2.98 (m, 2H), 3.52-3.65 (m, 6H), 4.10-4.28 (m, 1H).

Example 130

[1-(1-Isopropylpiperidin-4-yl)-3-(3-piperidinopropyl)imidazolidin-2-ylidene]malononitrile (Compound 130)

A dihydrochloride (0.238 g, 0.573 mmol) of [1-(piperidin-4-yl)-3-(3-piperidinopropyl)imidazolidin-2-ylidene] malononitrile obtained in Example 128 was dissolved in THF (10 mL) and the solution was added with acetone (0.418 mL, 5.73 mmol) and triacetoxy sodium borohydride (0.604 g, 2.85 mmol). After stirring at room temperature for 43.5 hours, the mixture was added with aqueous sodium carbonate solution and extracted with ethyl acetate. The organic layer was washed with saturated brine, dried over anhydrous magnesium sulfate and the solvent was evaporated under reduced pressure. The residue was recrystallized from diisopropylether to obtain the titled compound (0.081 g, 36.8 %) as a white solid.
$^1$H NMR ($CDCl_3$, δppm) : 1.03 (d, J = 6.4 Hz, 6H), 1.37-1.50 (m, 2H), 1.50-1.93 (m, 10H), 2.22-2.42 (m, 8H), 2.68-2.81 (m, 1H), 2.89-3.01 (m, 2H), 3.50-3.65 (m, 6H), 4.09-4.25 (m, 1H).
Melting point: 199-201˚C.

Example 131

[1-(1-Cyclopropanecarbonylpiperidin-4-yl)-3-(3-piperidinopropyl)imidazolidin-2-ylidene]malononitrile (Compound 131)

A dihydrochloride (0.060 g, 0.14 mmol) of [1-(piperidin-4-yl)-3-(3-piperidinopropyl)imidazolidin-2-ylidene] malononitrile obtained in Example 128 was dissolved in 1,2-dichloroethane (3 mL) and the solution was sequentially added with 1-(3-dimethylaminopropyl)-3-ethylcarbodiimide hydrochloride (WSC HCl) (0.037 g, 0.190 mmol), 1-hydroxy-benzotriazole monohydrate (HOBt $H_2O$) (0.026 g, 0.190 mmol), cyclopropanecarboxylic acid (0.015 mL, 0.188 mmol) and diisopropylethylamine (0.083 mL, 0.592 mmol). After stirring at room temperature for 4.5 hours, the mixture was added with aqueous sodium carbonate solution and extracted with ethyl acetate. The organic layer was dried over anhydrous magnesium sulfate and the solvent was evaporated under reduced pressure. The residue was purified by preparative TLC (chloroform/(8 mol/L ammonia-methanol) (9:1)) to obtain the titled compound (0.010 g, 16.9 %) as a white solid.
$^1$H NMR ($CDCl_3$, δppm) : 0.73-0.83 (m, 2H), 0.92-1.03 (m, 2H), 1.38-2.05 (m, 13H), 2.32-2.45 (m, 6H), 2.58-2.75 (m, 1H), 3.17-3.32 (m, 1H), 3.45-3.70 (m, 6H), 4.28-4.54 (m, 2H), 4.70-4.82 (m, 1H).
Melting point: 131-133˚C.

Example 132

{1-[1-(Tert-butyloxycarbonyl)piperidin-4-yl]-3-(3-piperidinopropyl)imidazolidin-2-ylidene}malononitrile (Compound 132)

A dihydrochloride (0.159 g, 0.383 mmol) of [1-(piperidin-4-yl)-3-(3-piperidinopropyl)imidazolidin-2-ylidene] malononitrile obtained in Example 128 was dissolved in acetonitrile (2 mL) and the solution was added with triethylamine (0.267 mL, 1.91 mmol) and di-tert-butyl dicarbonate (0.124 g, 0.568 mmol). After stirring at room temperature for 1 hour, the mixture was added with water and extracted with ethyl acetate. The organic layer was washed with saturated brine, dried over anhydrous magnesium sulfate and the solvent was evaporated under reduced pressure. The residue was purified by preparative TLC (chloroform/(2 mol/L ammonia-methanol) (10:1)) and the obtained solid was washed with diisopropylether to obtain the titled compound (0.062 g, 36.6 %) as a white solid.
$^1$H NMR ($CDCl_3$, δppm) : 1.40-1.48 (m, 11H), 1.53-1.68 (m, 6H), 1.80-1.95 (m, 4H), 2.35-2.47 (m, 6H), 2.75-2.90 (m, 2H), 3.48-3.69 (m, 6H), 4.06-4.43 (m, 3H).
Melting point: 141-143˚C.

Example 133

{1-[(1-Isopropylpiperidin-4-yl)methyl]-3-(3-piperidinopropyl).imidazolidin-2-ylidene}malononitrile (Compound 133)

(Step 1)

[1-(3-Hydroxypropyl)imidazolidin-2-ylidene] malononitrile (1.19 g, 6.19 mmol) obtained in step 1 of Example 8 was dissolved in DMF (12 mL) and the solution was sequentially added with potassium carbonate (1.71 g, 12.4 mmol) and 1-(tert-butoxycarbonyl)-4-methanesulfonyloxymethylpiperidine (2.94 g, 9.29 mmol) obtained in a similar manner to the method described in Bioorg. Med. Chem. Lett., Vol. 8, p.1595 (1998). After stirring at 80˚C for 21 hours and then at 100˚C for 25 hours, the mixture was added with water and was extracted with ethyl acetate. The organic layer was washed with saturated brine, dried over anhydrous magnesium sulfate and the solvent was evaporated under reduced pressure. The residue was purified by silica gel column chromatography (chloroform/methanol (97:3 to 92:8) to obtain {1-[1-(tert-butoxycarbonyl)piperidin-4-ylmethyl]-3-(3-hydroxypropyl)imidazolidin-2-ylidene}malononitrile (1.21 g, 50.2 %).
$^1$H NMR ($CDCl_3$, δppm) : 1.20-1.40 (m, 2H), 1.46 (s,9H), 1.65-1.75 (m, 2H), 1.88-2.04 (m, 3H), 2.62-2.80 (m, 2H), 3.30-3.50 (m, 2H), 3.60-3.78 (m, 8H), 4.05-4.24 (m, 2H).

(Step 2)

{1-[1-(Tert- butoxycarbonyl) piperidin- 4- ylmethyl]- 3-(3- hydroxypropyl) imidazolidin- 2- ylidene} malononitrile (0.600 g, 1.60 mmol) obtained in the Step 1 was dissolved in dichloromethane (6 mL) and the solution was sequentially added with triethylamine (0.334 mL, 2.40 mmol) and methanesulfonylchloride (0.149 mL, 1.92 mmol). After stirring at room temperature for 4.5 hours, the mixture was added with triethylamine (0.111 mL, 0.800 mmol) and methanesulfonylchloride (0.037 mL, 0.480 mmol), followed by stirring at room temperature for 0.5 hour. The mixture was added with water and extracted with ethyl acetate. The organic layer was washed with saturated brine and the solvent was evaporated under reduced pressure to obtain {1-[1-(tert-butoxycarbonyl)piperidin-4-ylmethyl]-3-(3-methanesulfonyloxypropyl)imidazolidin-2-ylidene}malononitrile (0.700 g, 97.2 %).
$^1$H NMR ($CDCl_3$, δppm) : 1.12-1.28 (m, 2H), 1.46 (s, 9H), 1.65-1.75 (m, 2H), 1.90-2.05 (m, 1H), 2.12-2.45 (m, 2H), 2.63-2.78 (m, 2H), 3.06 (s, 3H), 3.30-3.50 (m, 2H), 3.62-3.78 (m, 6H), 4.05-4.23 (m, 2H), 4.35 (t,J =6.0 Hz, 2H).

(Step 3)

{1-[1-(Tert- butoxycarbonyl) piperidin- 4- ylmethyl]- 3-(3- methanesulfonyloxypropyl) imidazolidin- 2- ylidene} malononitrile (0.780 g, 1.67 mmol) obtained in the Step 2 was dissolved in 1,4-dioxane (80 mL) and the solution was added with potassium iodide (0.415 g, 2.50 mmol) and piperidine (0.496 mL, 5.01 mmol). After stirring at 80˚C for 2.5 hours, the mixture was added with water and extracted with ethyl acetate. The organic layer was washed with saturated brine, dried over anhydrous magnesium sulfate and the solvent was evaporated under reduced pressure. The residue was purified by silica gel column chromatography (chloroform/methanol (100:1 to 100:5 to 100:10 to 100:15) to obtain {1-[1-(tert-butoxycarbonyl)piperidin-4-ylmethyl]-3-(3-piperidinopropyl)imidazolidin-2-ylidene}malononitrile (0.490 g, 64.3 %).
$^1$H NMR ($CDCl_3$, δppm) : 1.12-1.29 (m, 2H), 1.43-1.52 (m, 11H), 1.58-1.75 (m, 6H), 1.87-2.05 (m, 3H), 2.40-2.55 (m, 6H), 2.62-2.79 (m, 2H), 3.28-3.50 (m, 2H), 3.55-3.70 (m, 6H), 4.08-4.24 (m, 2H).

(Step 4)

{1-[1-(Tert- butoxycarbonyl) piperidin- 4- ylmethyl]- 3-(3- piperidinopropyl) imidazolidin- 2- ylidene} malononitrile (0.072 g, 0.16 mmol) obtained in the Step 3 was dissolved in dichloromethane (2 mL) and the solution was added with trifluoroacetic acid (1 mL), followed by stirring at room temperature for 0.5 hour. The mixture was concentrated under reduced pressure and the residue was used to the next reaction without further purification.

The residue obtained above was dissolved in DMF (2 mL) and the solution was added with potassium carbonate (0.110 g, 0.800 mmol) and isopropyl iodide (0.031 mL, 0.32 mmol). After stirring at 50˚C for 5 hours, the mixture was added with water and extracted with ethyl acetate. The organic layer was washed with saturated brine, dried over anhydrous magnesium sulfate and the solvent was evaporated under reduced pressure. The residue was purified by silica gel column chromatography (chloroform/(2 mol/L ammonia-methanol) (100:20)) to obtain the titled compound (0.013 g, 19.6 %) as a white solid.
$^1$H NMR ($CDCl_3$, δppm) : 1.03 (d, J = 6.6 Hz,6H), 1.23-1.50 (m, 4H), 1.51-1.63 (m, 4H), 1.68-1.94 (m, 5H.), 2.10-2.23 (m, 2H), 2.25-2.47 (m, 6H), 2.67-2.79 (m, 1H), 2.84-2.95 (m, 2H), 3.38 (d, J = 7.3Hz,2H), 3.53-3.67 (m, 6H).

Melting point: 115-117°C.

Example 134

[1,3-Bis(1-isopropylpiperidin-4-ylmethyl) imidazolidin-2-ylidene]malononitrile (Compound 134)

(Step 1)

To a solution of 60% sodium hydride (0.224 g, 5.59 mmol) suspended in DMF (30 mL), a solution of (imidazolidin-2-ylidene)malononitrile (0.300 g, 2.24 mmol) obtained in Step 1 of Example 4 dissolved in DMF (6 mL) was added dropwise under ice-cooling. After stirring at room temperature for 0.5 hour, the mixture was added with a solution of 1-(tert-butoxycarbonyl)-4-methanesulfonyloxymethylpiperidine (1.97 g, 6.72 mmol) obtained in a similar manner to the method described in Bioorg. Med. Chem. Lett., Vol. 8, p.1595 (1998) dissolved in DMF (6 mL) and stirred at 90°C for 48 hours. Further, a solution of 1-(tert-butoxycarbonyl)-4-methanesulfonyloxymethylpiperidine (1.97 g, 6.72 mmol) dissolved in DMF (6 mL) was added to the mixture and stirred at 110°C for 72 hours. The mixture was added with aqueous ammonium chloride solution and extracted with ethyl acetate. The organic layer was washed with saturated brine, dried over anhydrous magnesium sulfate and the solvent was evaporated under reduced pressure. The residue was purified by silica gel column chromatography (chloroform/methanol (97:5)) to obtain {1,3-bis[1-(tert-butoxycarbonyl)piperidin-4-ylmethyl] imidazolidin-2-ylidene}malononitrile (0.324 g, 27.0 %) as a white solid.
$^1$H NMR ($CDCl_3$, δppm) : 1.10-1.28 (m, 4H), 1.46 (s, 18H), 1.68-1.76 (m, 4H), 1.90-2.10 (m, 2H), 2.62-2.78 (m, 4H), 3.30-3.54 (m, 4H), 3.64 (s, 4H), 4.04-4.24 (m, 4H).

(Step 2)

{1,3-Bis[1-(tert-butoxycarbonyl)piperidin-4-ylmethyl]imidazolidin-2-ylidene}malononitrile (0.175 g, 0.330 mmol) obtained in the Step 1 was dissolved in dichloromethane (2 mL) and the solution was added with trifluoroacetic acid (1 mL), followed by stirring at room temperature for 1 hour. The mixture was concentrated under reduced pressure and the residue was used to the next reaction without further purification.

The residue obtained above was dissolved in DMF (5 mL) and the solution was added with potassium carbonate (0.456 g, 3.30 mmol) and isopropyl iodide (0.097 mL, 0.99 mmol). After stirring at room temperature for 7.5 hours, the mixture was added with water and extracted with ethyl acetate. The organic layer was washed with saturated brine, dried over anhydrous magnesium sulfate and the solvent was evaporated under reduced pressure. The obtained solid was washed with diethylether to obtain the titled compound (0.027 g, 18.2%) as a white solid.
$^1$H NMR ($CDCl_3$,δppm) : 1.05 (d, J = 6.2 Hz, 12H), 1.28-1.44 (m, 4H), 1.70-1.95 (m, 6H), 2.23-2.25 (m, 4H), 2.70-2.83 (m, 2H), 2.85-2.97 (m, 4H), 3.39 (d, J = 7.3 Hz, 4H), 3.62 (s, 4H).
Melting point: 172-174°C.

Example 135

1,3-Bis(3-piperidinopropyl)-2-nitromethylene imidazolidine (Compound 135)

(Step 1)

Ethylenediamine (2.0 ml, 29.9 mmol) was dissolved in dichloromethane (100 ml) and the solution was added with pyridine (5.34 ml, 66 mmol), o-nitrobenzene sulfonylchloride (12.6 g, 56.9 mmol) and 4-dimethylaminopyridine (1.83 g, 15 mmol). After stirring at room temperature for 2.5 hours, the mixture was added with 1 mol/L hydrochloric acid. The precipitated solid was collected by filtration and the obtained solid was washed with dichloromethane to obtain N,N'-bis (2-nitrobenzenesulfonyl)ethylenediamine (7.81 g, 60.5 %) as a white solid.
$^1$H NMR (DMSO-$d_6$, δppm) : 2.97-2.99 (m, 4H),7.84-7.89 (m,4H), 7.94-7.99 (m, 4H), 8.13-8.18 (m, 2H).

(Step 2)

N,N'-bis(2-nitrobenzenesulfonyl)ethylenediamine (1.07 g, 2.5 mmol) obtained in the Step 1 was dissolved in DMF (10 ml) and the solution was added with potassium carbonate (1.73 g, 12.5 mmol), 3-bromopropanol (0.68 ml, 7.5 mmol) and sodium iodide (0.38 g, 2.5 mmol), followed by stirring at 90°C for 16 hours. After cooling, the mixture was added with water and extracted with ethyl acetate. The organic layer was washed with saturated brine and the solvent was evaporated under reduced pressure to obtain N,N'-bis(3-hydroxypropyl)-N,N'-bis(2-nitrobenzenesulfonyl)ethylenediamine (1.32 g, 96.4 %).

$^1H$ NMR ($CDCl_3$, δppm): 2.05-2.15 (m, 4H), 3.38-3.56 (m, 8H), 4.29 (t, J = 5.8 Hz, 4H), 7.62-7.67 (m, 2H), 7.70-7.78 (m, 4H), 7.98-8.03 (m, 2H).

(Step 3)

N,N'-bis(3-hydroxypropyl)-N,N'-bis(2-nitrobenzenesulfonyl)ethylenediamine (0.11 g, 0.20 mmol) obtained in the Step 2 was dissolved in DMF (1.5 ml) and the solution was added with carbon tetrabromide (0.20 g, 0.60 mmol) and triphenylphosphine (0.16 g, 0.60mmol). After stirring at room temperature for 3.5 hours, the mixture was added with water and extracted with ethyl acetate. The organic layer was washed with saturated brine and the solvent was evaporated under reduced pressure. The residue was purified by silica gel chromatography (n-hexane/ethyl acetate (1:1)) to obtain N,N'-bis(3-bromopropyl)-N,N'-bis(2-nitrobenzenesulfonyl) ethylenediamine (0.14g, quantitative).
$^1H$ NMR ($CDCl_3$, δppm) : 2.19-2.27 (m, 4H), 3.40 (t, J =6.3Hz, 4H), 3.44-3.58 (m, 8H), 7.62-7.67 (m, 2H), 7.68-7.79 (m, 4H), 8.00-8.09 (m,2H).

(Step 4)

N,N'-bis(3-bromopropyl)-N,N'-bis(2-nitrobenzenesulfonyl)ethylenediamine (0.14 g, 0.21 mmol) obtained in the Step 3 was dissolved in 1,4-dioxane (2 mL) and the solution was added with potassium carbonate (0.14 g, 1.05 mmol), piperidine (0.10 ml, 1.05 mmol) and sodium iodide (0.06 g, 0.21 mmol), followed by stirring at 40˚C for 16 hours. After cooling, the mixture was added with saturated brine and extracted with ethyl acetate. The organic layer was washed with saturated brine, dried over anhydrous magnesium sulfate and the solvent was evaporated under reduced pressure to obtain N,N'-bis(2-nitrobenzenesulfonyl)-N,N'-bis(3-piperidinopropyl) ethylenediamine (0.091 g, 63.7 %).
$^1H$ NMR ($CDCl_3$, δppm) : 1.36-1.48 (m, 4H), 1.51-1.59 (m, 8H), 1.69-1.81 (m, 4H), 2.23-2.49 (m, 12H), 3.34-3.48 (m, 8H), 7.60-7.66 (m, 2H), 7.67-7.72 (m, 4H), 8.00-8.07 (m, 2H).

(Step 5)

N,N'-bis(2-nitrobenzenesulfonyl)-N,N'-bis(3-piperidinopropyl)ethylenediamine (0.87 g, 1.28 mmol) obtained in the Step 4 was dissolved in DMF (6mL) and the solution was added with potassium carbonate (1.77 g, 12.8 mmol) and 2-melcaptoethanol (0.45 ml, 6.4 mmol), followed by stirring at 50˚C for 16 hours. After cooling, the precipitated solid was collected by filtration and the obtained filtrate was concentrated under reduced pressure. The residue was purified by BONDESIL-SCX (manufactured by VARIAN) (chloroform) to obtain N,N'-bis(3-piperidinopropyl)ethylenediamine (0.35 g, 89.2 %).
$^1H$ NMR ($CDCl_3$, δppm) : 1.38-1.44 (m, 4H), 1.53-1.57 (m, 8H), 1.79-1.96 (m, 4H), 2.30-2.37 (m, 12H), 3.48-3.66 (m, 8H).
APCIMS m/z: $[M+H]^+$311.

(Step 6)

1,1-Bis(methylsulfanyl)-2-nitroethane (0.06 g, 0.39 mmol) was dissolved in THF (1 mL) and the solution was added with N,N'-bis(3-piperidinopropyl)ethylenediamine (0.12 g, 0.39 mmol) obtained in the Step 5, followed by stirring at 60˚C for 10.5 hours. After cooling, the mixture was concentrated under reduced pressure. The residue was purified by silica gel chromatography (chloroform/(2 mol/L ammonia-methanol) (9:1)) to obtain the titled compound (0.007 g, 4.7 %).
$^1H$ NMR ($CDCl_3$, δppm) : 1.38-1.46 (m, 4H), 1.52-1.61 (m, 8H), 1.75-1.85 (m, 4H), 2.25-2.36 (m, 12H), 3.31-3.46 (m, 8H), 6.51 (s, 1H).

Example 136

[1,3-Bis(3-piperidinopropyl)imidazolidin-2-ylidene] methanesulfonylacetonitrile (Compound 136)

(Step 1)

Potassium hydroxide (0.56 g, 9.91 mmol) and methanesulfonylacetonitrile (0.50 g, 4.20 mmol) were dissolved in ethanol (2.5 ml) and carbon disulfide (0.35 ml, 5.88 mmol) was added thereto dropwise, followed by stirring at 50˚C for 3.5 hours. After cooling, the precipitated solid was collected by filtration and washed with ethanol to obtain white solid (0.69 g).
The solid obtained above (0.69 g) was dissolved in DMF (1.5 ml) and the solution was added with methyl iodide (0.64 ml, 10.2 mmol), followed by stirring at 50˚C for 2 hours. After cooling, the mixture was concentrated and the residue

was filtered. The filtrate was added with water and extracted with ethyl acetate. The organic layer was washed with saturated brine, dried over anhydrous magnesium sulfate, and the solvent was evaporated under reduced pressure to obtain 2-methanesulfonyl-3,3-bis(methylsulfanyl)acrylonitrile (0.49 g, 52.2 %) as a yellow oily substance.
$^1$H NMR ($CDCl_3$, δppm) : 2.67 (s, 3H), 2.79 (s, 3H), 3.23 (s,3H).

(Step 2)

2-Methanesulfonyl-3,3-bis(methylsulfanyl) acrylonitrile (0.49 g, 2.18 mmol) obtained in the Step 1 was dissolved in THF (1.5 ml) and the solution was added with N-(3-hydroxypropyl)ethylenediamine (0.27 g, 2.29 mmol). After stirring at room temperature for 2 hours, the mixture was added with diisopropylether and the precipitated solid was collected by filtration. The obtained solid was washed with diisopropylether to obtain [1-(3-hydroxypropyl)imidazolidin-2-ylidene] methanesulfonylacetonitrile (0.37 g, 68.6 %) as a white solid.
$^1$H NMR ($CDCl_3$, δppm) : 1.89-1.98 (m, 2H), 3.08 (s, 3H), 3.59-3.65 (m, 2H), 3.69-3.78 (m, 6H).

(Step 3)

[1-(3-Hydroxypropyl)imidazolidin-2-ylidene] methanesulfonylacetonitrile (0.36 g, 1.45 mmol) obtained in the Step 2 was dissolved in DMF (3 mL) and the solution was sequentially added with potassium carbonate (0.40 g, 2.90 mmol) and 1,3-dibromopropane (0.74 mL, 7.25 mmol), followed by stirring at room temperature for 22 hours and then at 50˚C for 1 hour. After cooling, the mixture was added with saturated brine and extracted with ethyl acetate. The organic layer was washed with saturated brine and the solvent was evaporated under reduced pressure. The residue was purified by silica gel chromatography (chloroform/methanol (10:1)) to obtain [1-(3-bromopropyl)-3-(3-hydroxypropyl) imidazolidin-2-ylidene] methanesulfonylacetonitrile (0.59 g, 100 %).
$^1$H NMR ($CDCl_3$, δppm) : 1.82-1.96 (m, 2H), 2.13-2.15 (m, 2H), 2.27-2.34 (m, 2H), 3.19 (s, 3H), 3.47 (t, J = 6.3 Hz, 2H), 3.65-3.79 (m, 8H).

(Step 4)

[1-(3-Bromopropyl)-3-(3-hydroxypropyl)imidazolidin-2-ylidene]methanesulfonylacetonitrile (0.59 g, 1.61 mmol) obtained in the Step 3 was dissolved in dichloromethane (3 mL) and the solution was sequentially added with triethylamine (0.61 ml, 4.35 mmol) and methanesulfonylchloride (0.17 mL, 2.18 mmol). After stirring at room temperature for 1.5 hours, the mixture was added with saturated brine and extracted with ethyl acetate. The organic layer was washed with saturated brine and the solvent was evaporated under reduced pressure. The residue was purified by silica gel chromatography (chloroform/methanol (10:1)) to obtain [1-(3-bromopropyl)-3-(3-methanesulfonyloxypropyl)imidazolidin -2-ylidene]methanesulfonylacetonitrile (0.31 g, 43.4 %).
$^1$H NMR ($CDCl_3$, δppm): 2.15-2.32 (m, 4H), 3.06 (s, 3H), 3.18 (s, 3H), 3.48 (t, J = 6.1 Hz, 2H), 3.64-3.75 (m, 8H), 4.35 (t, J = 5.8Hz, 2H).

(Step 5)

[1-(3-Bromopropyl)-3-(3-methanesulfonyloxypropyl) imidazolidin-2-ylidene]methanesulfonylacetonitrile (0.15 g, 0.34 mmol) obtained in the Step 4 was dissolved in 1,4-dioxane (2 mL) and the solution was added with potassium carbonate (0.24 g, 1.72 mmol), piperidine (0.17 ml, 1.72 mmol) and sodium iodide (0.05g, 0.34 mmol), followd by stirring at 40˚C for 17 hours. After cooling, the mixture was added with saturated brine and extracted with ethyl acetate. The organic layer was washed with saturated brine, dried over anhydrous magnesium sulfate and the solvent was evaporated under reduced pressure. The residue was purified by silica gel column chromatography (chloroform/methanol (3:1)) and the obtained solid was triturated with n-hexane/diisopropylether (10:1) to obtain the titled compound (0.055 g, 36.6 %).
$^1$H NMR ($CDCl_3$, δppm) : 1.41-1.52 (m, 4H), 1.54-1.60 (m, 8H), 1.87 (tt, J = 14.9, 7.6 Hz, 4H), 2.30-2.38 (m, 12H), 3.17 (s, 3H), 3.53 (t, J =7.6 Hz, 4H), 3.66-3.70 (m, 4H).
APCIMS m/z: $[M+H]^+$438.
Melting point: 105-107 ˚C.

Example 137

{1,3-Bis[3-(2-methylpyrrolidin-1-yl)propyl] imidazolidin-2-ylidene}methanesulfonylacetonitrile (Compound 137)

In a similar manner to Step 5 of Example 136, the titled compound (0.048 g, 31.9 %) was obtained as a white solid by using [1-(3-bromopropyl)-3-(3-methanesulfonyloxypropyl)imidazolidin-2-ylidene] methanesulfonylacetonitrile

(0.15 g, 0.344 mmol) obtained in Step 4 of Example 136 and 2-methylpyrrolidine (0.18 ml, 1.72 mmol) .
$^1$H NMR ($CDCl_3$, δppm) : 1.07 (d, J = 6.0 Hz, 6H), 1.31-1.45 (m, 2H), 1.63-1.78 (m, 4H), 1.82-1.97 (m, 6H), 2.03-2.17 (m, 4H), 2.22-2.32 (m, 2H), 2.78 (td, J = 7.9, 12.2 Hz, 2H), 3.11-3.16 (m, 2H), 3.17 (s,3H), 3.55 (t,J = 7.6 Hz, 4H), 3.68-3.70 (m, 4H).
APCIMS m/z: $[M+H]^+$438.
Melting point: 106-110˚C.

Example 138

{1,3-Bis[3-((S)-2-methylpyrrolidin-1-yl)propyl] imidazolidin-2-ylidene}methanesulfonylacetonitrile (Compound 138)

In a similar manner to Step 5 of Example 136, the titled compound (0.066 g, 20.0%) was obtained as a white solid by using [1-(3-bromopropyl)-3-(3-methanesulfonyloxypropyl)imidazolidin-2-ylidene] methanesulfonylacetonitrile (0.37 g, 0.76 mmol) obtained in Step 4 of Example 136 and (S)-2-methylpyrrolidinehydrobromate (0.50 g, 3.02 mmol) obtained in a similar manner to the method described in J. Org. Chem., Vol. 54, p.209 (1989).
$^1$H NMR ($CDCl_3$, δppm) : 1.08 (d, J = 5.9 Hz, 6H), 1.33-1.45 (m, 2H), 1.68-1.78 (m, 4H), 1.81-1.98 (m, 6H), 2.05-2.18 (m, 4H), 2.27-2.36 (m, 2H), 2.78 (td, J = 8.1, 11.8 Hz, 2H), 3.12-3.16 (m, 2H), 3.17 (s, 3H), 3.55 (t,J = 7.7 Hz, 4H), 3.65-3.72 (m, 4H).
APCIMS m/z: $[M+H]^+$438.
Melting point: 91-93˚C.

Example 139

1,3-Bis(3-piperidinopropyl)imidazolidin-2-ylidene cyanoamide difumarate (Compound 139)

(Step 1)

Dimethyl cyanodithioiminocarbonate (3.00 g, 20.5 mmol) was dissolved in THF (15 mL) and a solution of N-(3-hydroxypropyl)ethylenediamine (2.55 g, 20.5 mmol) in THF (15 mL) was added thereto dropwise under ice-cooling. After stirring under ice-cooling for 1 hour and at room temperature for 21 hours, the mixture was added with diisopropylether and the supernatant was removed to obtain an oily substance. The obtained oily substance was dissolved in acetone and the solution was added with diisopropylether. Then, the supernatant was removed in a similar manner. The obtained oily substance was concentrated under reduced pressure to obtain 1-(3-hydroxypropyl)imidazolidin-2-ylidenecyanoamide (2.83 g, 82.1 %) as a brown oily substance.
$^1$H NMR ($CDCl_3$, δppm) : 1.67-1.77 (m, 2H), 3.36-3.45 (m, 2H), 3.57-3.68 (m, 6H).

(Step 2)

1-(3-Hydroxypropyl)imidazolidin-2-ylidenecyanoamide (1.00 g, 5.95 mmol) obtained in the Step 1 was dissolved in DMF (10 mL) and the solution was sequentially added with potassium carbonate (1.65 g, 11.9 mmol) and 1-chloro-3-iodopropane (6.08 mL, 29.8 mmol). After stirring at room temperature for 24 hours, the mixture was added with saturated brine and the aqueous layer was washed with ethyl acetate. The obtained aqueous layer was concentrated under reduced pressure and the residue was extracted with chloroform. The organic layer was concentrated under reduced pressure and the residue was purified by silica gel column chromatography (ethyl acetate) to obtain 1-(3-chloropropyl)-3-(3-hydroxypropyl) imidazolidin-2-ylidenecyanoamide (0.680 g, 46.8 %) as a white clear oily substance.
$^1$H NMR ($CDCl_3$, δppm) : 1.77-1.87 (m, 2H), 2.05-2.18 (m, 2H), 3.53-3.75 (m, 12H).

(Step 3)

1-(3-Chloropropyl)-3-(3-hydroxypropyl)imidazolidin-2-ylidenecyanoamide (0.369 g, 1.51 mmol) obtained in the Step 2 was dissolved in 1,4-dioxane (3 mL) and the solution was added with piperidine (0.449 mL,4.54 mmol), followed by stirring at 80˚C for 21 hours. After the mixture was concentrated under reduced pressure, the residue was added with water and purified by BONDESIL-SCX (manufactured by VARIAN) (water to methanol to 7 mol/L ammonia-methanol) to obtain 1-(3-hydroxypropyl)-3-(3-piperidinopropyl)imidazolidin-2-ylidenecyanoamide (0.348 g, 78.5 %) as a brown oily substance.
$^1$H NMR ($CDCl_3$, δppm) : 1.38-1.50 (m, 2H), 1.50-1.64 (m, 4H), 1.72-1.87 (m, 4H), 2.29-2.44 (m, 6H), 3.45-3.70 (m, 10H).

(Step 4)

1-(3-Hydroxypropyl)-3-(3-piperidinopropyl) imidazolidin-2-ylidenecyanoamide (0.157 g, 0.536 mmol) obtained in the Step 3 was dissolved in dichloromethane (3 mL) and the solution was sequentially added with triethylamine (0.112 mL, 0.804 mmol) and methanesulfonylchloride (0.050 mL, 0.643 mmol), followed by stirring at room temperature for 2.5 hours. The mixture was added with methanesulfonylchloride (0.010 mL, 0.107 mmol), stirred at room temperature for 1.5 hours and further added with triethylamine (0.041 mL, 0.268 mmol) and methanesulfonylchloride (0.013 mL, 0.161 mmol), followed by stirring at room temperature for 3 hours. The mixture was concentrated under reduced pressure and the residue was purified by silica gel column chromatography (chloroform/methanol (9:1)) to obtain an orange oily substance.

The oily substance obtained above was dissolved in 1,4-dioxane (3 mL) and the solution was added with piperidine (0.159 mL, 1.61 mmol), followed by stirring at room temperature for 2 hours, then at 50˚C for 2 hours, and then at 70˚C for 3 hours. After cooling, the mixture was added with saturated brine and extracted with ethyl acetate. The organic layer was dried over anhydrous magnesium sulfate and the solvent was evaporated under reduced pressure. The residue was dissolved in methanol (2 mL) and the solution was added with a solution of fumaric acid (0.029 g, 0.25 mmol) dissolved in methanol (1 mL), followed by stirring at room temperature for 0.5 hours. The solvent was evaporated under reduced pressure and the residue was recrystallized from acetone/diisopropylether to obtain the titled compound (0.050 g, 15.7 %) as a white solid.
$^1$H NMR ($CD_3OD$, δppm) : 1.60-1.73 (m, 4H), 1.81-1.92 (m, 8H), 1.97-2.02 (m, 4H), 3.05-3.35 (m, 12H), 3.52 (t, J = 7.1 Hz, 4H), 3.58 (s, 4H), 6.68 (s, 4H).
Melting point: 174-175˚C.

Example 140

1,3-Bis[3-(2-methylpyrrolidin-1-yl)propyl] imidazolidin-2-ylidenecyanoamide difumarate (Compound 140)

(Step 1)

1-(3-Chloropropyl)-3-(3-hydroxypropyl)imidazolidin-2-ylidenecyanoamide (0.720 g, 2.94 mmol) obtained in Step 2 of Example 139 was dissolved in 1,4-dioxane (7 mL) and the solution was added with 2-methylpyrrolidine (0.898 mL, 8.80 mmol), followed by stirring at 60˚C for 24 hours. After concentrating under reduced pressure, the mixture was purified by BONDESIL-SCX (manufactured by VARIAN) (chloroform to 2 mol/L ammonia-methanol) to obtain 1-(3-hydroxypropyl)-3-[3-(2-methylpyrrolidin-1-yl)propyl] imidazolidin-2-ylidenecyanoamide (0.569 g, 65.9 %) as a brown oily substance.
$^1$H NMR (DMSO-$d_6$, δppm) : 0.98 (d, J = 6.2 Hz,3H),1.18-1.33 (m, 1H), 1.52-1.74 (m, 6H), 1.77-2.05 (m, 3H), 2.12-2.25 (m, 1H), 2.65-2.78 (m, 1H), 3.00-3.10 (m, 1H), 3.28-3.50 (m, 10H).

(Step 2)

1-(3-Hydroxypropyl)-3-[3-(2-methylpyrrolidin-1-yl) propyl]imidazolidin-2-ylidenecyanoamide (0.569 g, 1.94 mmol) obtained in the Step 1 was dissolved in dichloromethane (6 mL) and the solution was sequentially added with triethylamine (0.427 mL, 3.06 mmol) and methanesulfonylchloride (0.190 mL, 2.45 mmol), followed by stirring at room temperature for 1 hour. After cooling, the mixture was added with water and extracted with ethyl acetate. The organic layer was washed with saturated brine, dried over anhydrous magnesium sulfate and the solvent was evaporated under reduced pressure.

The residue was dissolved in 1,4-dioxane (2 mL) and the solution was added with 2-methylpyrrolidine (0.104 mL, 1.46 mmol), followed by stirring at 40˚C for 24 hours. After cooling, the mixture was added with saturated brine and extracted with ethyl acetate. The organic layer was dried over anhydrous magnesium sulfate and the solvent was evaporated under reduced pressure. The residue was dissolved in methanol (2 mL) and the solution was added with a solution of fumaric acid (0.030 g, 0.26 mmol) in methanol (1 mL). After stirring the mixture at room temperature for 0.5 hour, the solvent was evaporated under reduced pressure. The residue was purified by silica gel column chromatography (chloroform/(2 mol/L ammonia-methanol) (97:3 to 95:5 to 85:15) and the residue was recrystallized from methanol/acetone to obtain the titled compound (0.010 g, 1.4%) as a white solid.
$^1$H NMR ($CD_3OD$, δppm) : 1.11 (d, J = 6.3 Hz, 6H), 1.32-1.50 (m, 2H), 1.65-1.85 (m, 8H), 1.88-2.04 (m, 2H), 2.30-2.50 (m, 4H), 2.62-2.77 (m, 2H), 2.85-2.98 (m, 2H), 3.20-3.31 (m, 2H), 3.40 (br t, J = 7.1Hz, 4H), 3.48 (s, 4H), 6.51 (s, 4H).
Melting point: 159-163˚C.

Example 141

1,3-Bis[3-(2-methylpyrrolidin-1-yl)propyl] imidazolidine-2-one (Compound 141)

Ethyleneurea (0.200 g, 2.32 mmol) was dissolved in DMF (5 mL) and the solution was added with 1,3-dibromopropane (2.40 mL, 23.2 mmol). The mixture was added with sodium hydride (0.304 g, 6.96 mmol) under ice-cooling and stirred at room temperature for 4 hours. Next, the mixture was added with aqueous ammonium chloride solution and extracted with ethyl acetate. The organic layer was washed with saturated brine, dried over anhydrous magnesium sulfate and the solvent was evaporated under reduced pressure. The residue was purified by silica gel column chromatography (n-hexane/ethyl acetate (3:1)) to obtain an oily substance (0.453 g).

The oily substance obtained above was dissolved in 1,4-dioxane (4 mL) and the solution was added with potassium carbonate (0.290 mg, 2.10 mmol) and 2-methylpyrrolidine (0.223 mL, 2.10 mmol). After stirring at 50°C for 2 hours, the mixture was added with 1 mol/L hydrochloric acid and the aqueous layer was washed with ethyl acetate. The aqueous layer was turned basic with 2 mol/L aqueous sodium hydroxide solution and extracted with chloroform. The organic layer was washed with saturated brine, dried over anhydrous magnesium sulfate and the solvent was evaporated under reduced pressure. The residue was purified by silica gel column chromatography (n-hexane/ethyl acetate (1:1) to ethyl acetate to chloroform to chloroform/methanol (6:1)) to obtain the titled compound (0.021 g, 2.7 %) as an oily substance.
$^1$H NMR ($CDCl_3$, δppm) : 1.08 (d, J = 5.9 Hz,6H), 1.33-1.48 (m, 2H), 1.59-1.82 (m, 8H), 1.84-1.97 (m, 2H), 1.99-2.12 (m, 4H), 2.20-2.33 (m,2H), 2.74-2.85 (m, 2H), 3.11-3.25 (m, 6H), 3.30 (s, 4H).

Example 142

1,3-Bis[3-(2-methylpyrrolidin-1-yl)propyl] imidazolidin-2-ylidenesulfamoylamide (Compound 142)

(Step 1)

Bis(methyl-N-sulfamoyl)dithioimidecarbonate (0.920 g, 4.59 mmol) prepared in a similar manner to the method described in EP159533 was suspended in THF (10 mL) and a solution of N-(3-hydroxypropyl)ethylenediamine (0.544 g, 4.59 mmol) in ethanol (5 mL) was added thereto dropwise under ice-cooling. After the mixture was stirred at room temperature for 16 hours, an insoluble matter was filtered and the filtrate was concentrated under reduced pressure. The residue was added with acetone and the precipitated crystal was collected by filtration to obtain 1-(3-hydroxypropyl) imidazolidin-2-ylidenesulfamoylamide (0.522 g, 51.1 %).
$^1$H NMR ($CD_3OD$, δppm) : 1.68-1.82 (m, 2H), 3.36 (t, J =6.7Hz, 2H), 3.51-3.64 (m, 6H).

(Step 2)

1-(3-Hydroxypropyl)imidazolidin-2-ylidene sulfamoylamide (0.400 g, 1.80 mmol) obtained in the Step 1 was dissolved in DMF (4 mL) and the solution was sequentially added with potassium carbonate (0.500 g, 3.62 mmol) and 1,3-dibromopropane (0.916 mL, 9.02 mmol). After stirring at room temperature for 22.5 hours, the mixture was added with water and extracted with ethyl acetate. The organic layer was dried over magnesium sulfate and the solvent was evaporated under reduced pressure. The residue was washed with diisopropylether and dried to obtain 1-(3-bromopropyl)-3-(3-hydroxypropyl)imidazolidin-2-ylidenesulfamoylamide (0.249 g, 40.3 %) as a brown oily substance.
$^1$H NMR ($CDCl_3$, δppm) : 1.68-1.78 (m, 2H), 2.07-2.18 (m, 2H), 3.15-3.28 (m, 2H), 3.40-3.70 (m, 10H).

(Step 3)

1-(3-Bromopropyl)-3-(3-hydroxypropyl)imidazolidin-2-ylidenesulfamoylamide (0.240 g, 0.700 mmol) obtained in the Step 2 was dissolved in 1,4-dioxane (5 mL) and the solution was added with 2-methylpyrrolidine (0.215 mL, 2.11 mmol), followed by stirring at 50°C for 14 hours. After concentrating under reduced pressure, the mixture was purified by BONDESIL-SCX (manufactured by VARIAN) (chloroform to 2 mol/L ammonia-methanol) to obtain 1-(3-hydroxypropyl)-3-[3-(2-methylpyrrolidin-1-yl)propyl] imidazolidin-2-ylidenesulfamoylamide (0.213 g, 87.5 %) as a brown oily substance.
$^1$H NMR ($CDCl_3$, δppm) : 1.13 (d, J = 8.9 Hz, 3H), 1.35-1.50 (m, 1H), 1.60-2.35 (m, 10H), 2.82-2.94 (m, 1H), 3.10-3.28 (m, 1H), 3.40-3.68 (m, 10H).

(Step 4)

1-(3-Hydroxypropyl)-3-[3-(2-methylpyrrolidin-1-yl) propyl]imidazolidin-2-ylidenesulfamoylamide (0.123 g, 0.354 mmol) obtained in the Step 3 was dissolved in dichloromethane (3 mL) and the solution was sequentially added with triethylamine (0.010 mL, 0.724 mmol) and methanesulfonylchloride (0.033 mL, 0.425 mmol). After stirring at room temperature for 4.5 hours, the mixture was added with methanesulfonylchloride (0.006 mL, 0.072 mmol) and stirred at room temperature for 48 hours. The residue (0.254 g) obtained by concentrating the mixture was dissolved in 1,4-dioxane (3 mL) and the solution was added with 2-methylpyrrolidine (0.108 mL, 1.06 mmol). After stirring at room temperature for 29 hours, the mixture was added with 2-methylpyrrolidine (0.108 mL, 1.06 mmol), stirred for 34.5 hours and added with 2-methylpyrrolidine (0.108 mL, 1.06 mmol), followed by stirring for 72 hours. The mixture was concentrated under reduced pressure and the residue was purified by silica gel column chromatography (chloroform: (2 mol/L ammonia-methanol) (100:0 to 90:10 to 80:21). The obtained crude product was purified by preparative TLC (chloroform/(2 mol/L ammonia-methanol) (9:1)) to obtain the titled compound (0.007 g, 2.8 %) as a clear white oily substance. $^1$H NMR (DMSO-$d_6$, δppm) : 0.99 (d, J = 6.0 Hz, 6H), 1.21-1.32 (m, 2H), 1.52-1.70 (m, 8H), 1.80-2.25 (m, 6H), 2.12-2.25 (m, 2H), 2.75-2.88 (m, 2H), 2.95-3.07 (m, 2H), 3.15-3.55 (m, 8H).

Example 143

[(2-Piperidinoethylamino)(4-piperidinopiperidino) methylene]malononitrile (Compound 143)

[Bis(methylthio)methylene]malononitrile (242 mg, 1.42 mmol) was dissolved in ethanol (2 mL) and the solution was added with a solution of 4-piperidinopiperidine (251 mg, 1.49 mmol) in ethanol (3 mL), followed by stirring at room temperature for 0.5 hour. The mixture was added with 1-(2-aminoethyl)piperidine (0.319 mL, 2.24 mmol) and stirred at 70˚C for 6 hours. Further, the mixture was added with 1-(2-aminoethyl) piperidine (0.213 mL, 1.49 mmol) and stirred at 70˚C for 2 hours. After cooling, the reaction mixture was added with saturated brine and extracted with ethyl acetate. The organic layer was washed with saturated brine and dried over anhydrous magnesium sulfate and the solvent was evaporated under reduced pressure. The residue was purified by NH silica gel column chromatography (n-hexane/ethyl acetate (1:1)) to obtain the titled compound (465 mg, 88%) as a yellow oily substance.
$^1$H NMR ($CDCl_3$, δppm) : 1.38-1.69 (m, 13H), 1.94 (d, J=11.2Hz, 2H), 2.34-2.57 (m, 12H), 3.08 (t, J = 12.9 Hz, 2H), 3.31-3.38 (m, 2H), 3.88 (d, J = 12.9 Hz, 2H), 5.97 (br s, 1H).
APCIMS m/z: $[M+H]^+$371.
Melting point: 198-200˚C.

Example 144

{[Methyl(2-piperidinoethyl)amino](4-piperidinopiperidino)methylene}malononitrile (Compound 144)

[(2-Piperidinoethylamino) (4-piperidinopiperidino) methylene]malononitrile obtained in Example 143 (Compound 143) (150 mg, 0.405 mmol) was dissolved in THF (3 mL) and the solution was added with sodium hydride (24.0 mg, 0.608 mmol) and methyl iodide (0.0504 mL, 0.810 mmol) under ice bath, followed by stirring at room temperature for 1 hour. The reaction mixture was added with saturated brine and extracted with ethyl acetate. After washing with saturated brine, the organic layer was dried over anhydrous magnesium sulfate and the solvent was evaporated under reduced pressure. The residue was purified by NH silica gel column chromatography (n-hexane/ethyl acetate (1:1)). The obtained solid was washed with diisopropylether and the titled compound (60.8 mg, 39 %) was obtained as a white solid.
$^1$H NMR ($CDCl_3$, δppm) : 1.37-1.80 (m, 14H), 1.87-2.02 (m, 2H), 2.28-2.39 (m, 4H), 2.39-2.58 (m, 7H), 2.85 (s, 1.5H), 2.96 (s, 1.5H), 2.99-3.17 (m, 2H), 3.38-3.50 (m, 2H), 3.72-3.94 (m, 2H).
APCIMS m/z: $[M+H]^+$385.
Melting point: 109-111˚C.

Example 145

{[4-(N-methyl-N-phenylamino)piperidino](2-piperidinoethylamino)methylene}malononitrile (Compound 145)

(Step 1)

[Bis(methylthio)methylene]malononitrile (177 mg, 1.04 mmol) was dissolved in THF (3 mL) and the solution was added with a solution of 4-(N-methyl-N-phenylamino)piperidine dihydrochloride (300 mg, 1.14 mmol) obtained in

Reference Example 1 in a mixed solvent of THF (2 mL) and 2 mol/L aqueous sodium hydroxide solution (1.71 mL, 3.42 mmol) under ice bath, followed by stirring at room temperature for 0.25 hour. The reaction mixture was added with saturated brine and extracted with ethyl acetate. After washing with saturated brine, the organic layer was dried over anhydrous magnesium sulfate and the solvent was evaporated under reduced pressure. The residue was purified by preparative thin-layer chromatography (n-hexane/ethyl acetate (2:1)) to obtain {[4-(N-methyl-N-phenylamino)piperidino](methylthio) methylene}malononitrile (315 mg, 97 %) as a red crystal.
$^1$H NMR ($CDCl_3$, δppm) : 1.75-2.02 (m, 4H), 2.60 (s, 3H), 2.77 (s, 3H), 3.38 (td, J = 2.8, 13.0 Hz, 2H), 3.89 (m, 1H), 4.38 (dt, J =2.0Hz, 13.0 Hz, 2H), 6.75-6.87 (m, 3H), 7.21-7.30 (m, 2H).

(Step 2)

{[4-(N-methyl-N-phenylamino)piperidino](methylthio) methylene}malononitrile (268 mg, 0.857 mmol) obtained in the Step 1 was dissolved in ethanol (5 mL) and the solution was added with 1-(2-aminoethyl)piperidine (0.184 mL, 1.29 mmol), followed by stirring at 70˚C for 6 hours. Then, the mixture was added with 1-(2-aminoethyl)piperidine (0.0612 mL, 0.429 mmol) and stirred at 70˚C for 2 hours. After cooling, the reaction mixture was added with saturated brine and extracted with ethyl acetate. After washing with saturated brine, the organic layer was dried over anhydrous magnesium sulfate and the solvent was evaporated under reduced pressure. The residue was purified by NH silica gel column chromatography (n-hexane/ethyl acetate (1:1)) to obtain the titled compound (312 mg, 93%) as a red oily substance.
$^1$H NMR ($CDCl_3$, δppm) : 1.41-1.68 (m, 6H), 1.74-1.96 (m, 4H), 2.37-2.50 (m, 4H), 2.50-2.60 (m, 2H), 2.78 (s, 3H), 3.18 (td, J =3.0, 12.7 Hz, 2H), 3.33-3.44 (m, 2H), 3.76-3.89 (m, 1H), 3.97 (d, J = 12.7 Hz, 2H),6.03 (brs, 1H), 6.74-6.86 (m, 3H), 7.21-7.30 (m, 2H).
APCIMS m/z: $[M+H]^+$393.

Example 146

{[4-(N-methyl-N-phenylamino)piperidino][N-methyl-N-(2-piperidinoethyl)amino]methylene}malononitrile (Compound 146)

In a similar manner to Example 144, the titled compound (77.0 mg, 33 %) was obtained as a red solid by using {[4-(N-methyl-N-phenylamino)piperidino](2-piperidinoethylamino)methylene}malononitrile (Compound 145) (226 mg, 0.575 mmol) obtained in Example 145 and methyl iodide (0.0500 mL, 0.803 mmol).
$^1$H NMR ($CDCl_3$, δppm) : 1.38-1.60 (m, 6H), 1.82-2.00 (m, 4H), 2.30-2.41 (m, 4H), 2.45-2.54 (m, 2H), 2.79 (s, 3H), 2.84 (s, 1.5H), 3.02 (s, 1.5H), 3.10-3.25 (m, 2H), 3.38-3.55 (m, 2H), 3.78-4.03 (m, 3H), 6.74-6.87 (m, 3H), 7.22-7.31 (m, 2H).
APCIMS m/z: $[M+H]^+$407.
Melting point: 156-158˚C.

Example 147

[(2-Piperidinoethylamino)(4-piperidinomethylpiperidino)methylene]malononitrile (Compound 147)

(Step 1)

[Bis(methylthio)methylene]malononitrile (266 mg, 1.33 mmol) was dissolved in THF (4 mL) and the solution was added with a solution of 4-piperidinomethylpiperidine dihydrochloride (416 mg, 1.63 mmol) obtained in Reference Example 2 in a mixed solvent of THF (4 mL) and 2 mol/L aqueous sodium hydroxide solution (2.10 mL, 4.20 mmol) under ice bath, followed by stirring at room temperature for 0.25 hour. The reaction mixture was added with saturated brine and extracted with ethyl acetate. After washing with saturated brine, the organic layer was dried over anhydrous magnesium sulfate and the solvent was evaporated under reduced pressure. The residue was purified by NH silica gel column chromatography (n-hexane/ethyl acetate (1:1)) to obtain [(methylthio)(4-piperidinomethylpiperidino)methylene]malononitrile (268 mg, 64 %) as a yellow oily substance.
$^1$H NMR ($CDCl_3$, δppm) : 1.20-1.36 (m, 2H), 1.36-1.48 (m, 2H); 1.48-1.64 (m, 4H), 1.79-1.91 (m, 1H), 1.91-2.03 (m, 2H), 2.16 (d, J =7.6Hz, 2H), 2.26-2.40 (m, 4H), 2.57 (s, 3H), 3.30 (td, J = 2.4, 12.9 Hz, 2H), 4.26 (d,J = 12.9 Hz, 2H).

(Step 2)

[(Methylthio)(4-piperidinomethylpiperidino) methylene]malononitrile (145 mg, 0.476 mmol) obtained in the Step 1 was dissolved in ethanol (3 mL) and the solution was added with 1-(2-aminoethyl)piperidine (0.103 mL, 0.722 mmol), followed by stirring at 70˚C for 4 hours. After cooling, the reaction mixture was added with saturated brine and extracted

with ethyl acetate. After washing with saturated brine, the organic layer was dried over anhydrous magnesium sulfate and the solvent was evaporated under reduced pressure. The residue was purified by preparative thin-layer chromatography (chloroform/methanol (17:3)). The obtained solid was washed with diisopropylether and the titled compound (25.5 mg, 14%) was obtained as a white solid.
$^1$H NMR ($CDCl_3$, δppm) : 1.16-1.35 (m, 2H), 1.37-1.84 (m, 15H), 1.84-1.99 (m, 2H), 2.13-2.26 (m, 2H), 2.28-2.64 (m, 8H), 3.09 (t, J =13.0Hz, 2H), 3.32-3.45 (m, 2H), 3.84 (d, J = 13.0 Hz, 2H), 6.05 (br s, 1H).
APCIMS m/z: $[M+H]^+$385.
Melting point: 78-80˚C.

Example 148

{[4-(2-Methylpyrrolidin-1-ylmethyl)piperidino](2-piperidinoethylamino)methylene}malononitrile (Compound 148)

(Step 1)

[Bis(methylthio)methylene]malononitrile (582 mg, 3.42 mmol) was dissolved in THF (6 mL) and the solution was added with a solution of 4-(2-methylpyrrolidin-1-ylmethyl)piperidine dihydrochloride (873 mg, 3.42 mmol) obtained in Reference Example 3 in a mixed solvent of THF (4 mL) and 2 mol/L aqueous sodium hydroxide solution (5.20 mL, 10.4 mmol) under ice bath, followed by stirring at room temperature for 0.25 hour. The reaction mixture was added with saturated brine and extracted with ethyl acetate. After washing with saturated brine, the obtained organic layer was dried over anhydrous magnesium sulfate and the solvent was evaporated under reduced pressure. The residue was purified by NH silica gel column chromatography (n-hexane/ethyl acetate (1:1)) and {[4-(2-methylpyrrolidin-1-ylmethyl) piperidino] (methylthio) methylene}malononitrile (1.02 g, 98 %) was obtained as a yellow oily substance.
$^1$H NMR ($CDCl_3$, δppm) : 1.03 (d, J = 5.9 Hz, 3H), 1.17-1.45 (m, 3H), 1.63-1.97 (m, 5H), 2.00-2.20 (m, 3H), 2.20-2.33 (m, 1H), 2.48-2.57 (m, 1H), 2.58 (s, 3H), 3.05-3.12 (m, 1H), 3.24-3.38 (m, 2H), 4.22-4.31 (m, 2H).

(Step 2)

{[4-(2-Methylpyrrolidin-1-ylmethyl)piperidino] (methylthio)methylene}malononitrile (420 mg, 1.38 mmol) obtained in the Step 1 was dissolved in ethanol (8 mL) and the solution was added with 1-(2-aminoethyl)piperidine (0.300 mL, 2.07 mmol), followed by stirring at 70˚C for 9 hours. After cooling, the reaction mixture was added with saturated brine and extracted with ethyl acetate. After washing with saturated brine, the organic layer was dried over anhydrous magnesium sulfate and the solvent was evaporated under reduced pressure. The residue was purified by NH silica gel column chromatography (n-hexane/ethyl acetate (9:1) to ethyl acetate)and further purified by preparative thin-layer chromatography (chloroform/methanol (9:1)) to obtain the titled compound (23.4 mg, 4.4 %) as a yellow oily substance.
$^1$H NMR ($CDCl_3$, δppm) : 1.04 (d, J = 6.1 Hz, 3H), 1.12-1.63 (m, 8H), 1.63-1.98 (m, 6H), 1.98-2.14 (m, 3H), 2.21-2.32 (m, 1H), 2.34-2.49 (m, 4H), 2.49-2.61 (m, 3H), 3.01-3.18 (m, 3H), 3.30-3.43 (m, 2H), 3.85 (t, J = 14.9Hz, 2H), 5.99 (br s, 1H).
APCIMS m/z: $[M+H]^+$385.

Example 149

{[N-methyl-N-(2-piperidinoethyl)amino][4-(2-methylpyrrolidin-1-ylmethyl)piperidino]methylene} malononitrile (Compound 149)

{[4-(2-Methylpyrrolidin-1-ylmethyl)piperidino](2-piperidinoethylamino)methylene}malononitrile (Compound 148) (224 mg, 0.582 mmol) obtained in Example 148 was dissolved in THF (5 mL) and the solution was added with sodium hydride (42.0 mg, 0.875 mmol) and methyl iodide (0.0720 mL, 1.15 mmol) under ice-bath, followed by stirring at room temperature for 2 hours. The reaction mixture was added with saturated brine and extracted with ethyl acetate. After washing with saturated brine, the organic layer was dried over anhydrous magnesium sulfate and the solvent was evaporated under reduced pressure The residue was purified by preparative thin-layer chromatography (chloroform/hexane (9:1)) and the titled compound (114 mg, 49 %) was obtained as a yellow oily substance.
$^1$H NMR ($CDCl_3$, δppm) : 1.04 (d, J = 5.6 Hz, 3H), 1.16-1.97 (m, 15H), 1.97-2.12 (m, 3H), 2.19-2.37 (m, 4H), 2.42-2.62 (m, 3H), 2.84 (s, 1.5H), 2.95 (s, 1.5H), 2.99-3.20 (m, 3H), 3.34-3.55 (m, 2H), 3.66-3.90 (m,2H).
APCIMS m/z: $[M+H]^+$399.

Example 150

({[2-((R)-2-methylpyrrolidin-1-yl)ethyl]amino}(4-piperidinomethylpiperidino)methylene)malononitrile (Compound 150)

[(Methylthio)(4-piperidinomethylpiperidino) methylene]malononitrile (180 mg, 0.591 mmol) obtained in Step 1 of Example 147 was dissolved in ethanol (2 mL) and the solution was added with a solution of (R)-1-(2-aminoethyl)-2-methylpyrrolidine (150 mg, 1.17 mmol) obtained in Reference Example 4 in ethanol (2 mL), followed by stirring at 70˚C for 11 hours. After cooling, the reaction mixture was added with saturated brine and extracted with ethyl acetate. After washing with saturated brine, the organic layer was dried over anhydrous magnesium sulfate and the solvent was evaporated under reduced pressure. The residue was purified by NH silica gel column chromatography (n-hexane/ethyl acetate (1:1) to ethyl acetate) and further purified by preparative thin-layer chromatography (chloroform/hexane (9:1)) to obtain the titled compound (152 mg, 67%) as a yellow oily substance.
$^1$H NMR ($CDCl_3$, δppm) : 1.08 (d, J = 6.1 Hz, 3H), 1.15-1.35 (m, 2H), 1.35-1.48 (m, 3H), 1.48-1.62 (m, 5H), 1.68-1.83 (m, 2H), 1.83-2.01 (m, 3H), 2.09-2.22 (m, 3H), 2.25-2.53 (m, 5H), 2.85-3.17 (m, 5H), 3.31-3.45 (m, 2H), 3.75-3.91 (m, 2H), 5.81 (br s, 1H).

Example 151

({N-methyl-N-[2-((R)-2-methylpyrrolidin-1-yl)ethyl] amino}(4-piperidinomethylpiperidino)methylene) malononitrile (Compound 151)

In a similar manner to Example 149, the titled compound (72.7 mg, 47 %) was obtained as a yellow oily substance by using 2-({[2-((R)-2-methylpyrrolidin-1-yl)ethyl]amino}(4-piperidinomethylpiperidino)methylene) malononitrile (Compound 150) (148 mg, 0.385 mmol) obtained in Example 150 and methyl iodide (0.0470 mL, 0.755 mmol).
$^1$H NMR ($CDCl_3$, δppm): 1.05 (d, J = 6.1 Hz, 3H), 1.08-1.48 (m, 4H), 1.48-1.61 (m, 2H), 1.61-1.79 (m, 2H), 1.79-1.98 (m, 3H), 2.01-2.21 (m, 5H), 2.24-2.40 (m, 6H), 2.83 (s, 1.5H), 2.84-2.93 (m, 2H), 2.94 (s, 1.5H), 3.01-3.25 (m, 3H), 3.33-3.43 (m, 1H), 3.50-3.68 (m, 2H), 3.76-3.91 (m, 2H).
APCIMS m/z: $[M+H]^+$399.

Example 152

({[2-((S)-2-methylpyrrolidin-1-yl)ethyl]amino}(4-piperidinomethylpiperidino)methylene)malononitrile (Compound 152)

[(Methylthio)(4-piperidinomethylpiperidino) methylene]malononitrile (180 mg, 0.591 mmol) obtained in Step 1 of Example 147 was dissolved in ethanol (4 mL) and the solution was added with (S)-1-(2-aminoethyl)-2-methylpyrrolidine (150 mg, 1.17 mmol) obtained in Reference Example 5, followed by stirring at 70˚C for 8 hours. After cooling, the reaction mixture was added with saturated brine and extracted with ethyl acetate. After washing with saturated brine, the organic layer was dried over anhydrous magnesium sulfate and the solvent was evaporated under reduced pressure. The residue was purified by NH silica gel column chromatography (n-hexane/ethyl acetate (1:1)) to obtain the titled compound (122 mg, 54%) as a yellow oily substance.
$^1$H NMR ($CDCl_3$, δppm) : 1.08 (d, J = 6.1 Hz, 3H), 1.15-1.35 (m, 2H), 1.35-1.48 (m, 3H), 1.48-1.62 (m, 5H), 1.68-1.83 (m, 2H), 1.83-2.01 (m, 3H), 2.09-2.22 (m, 3H), 2.25-2.53 (m, 5H), 2.85-3.17 (m, 5H), 3.31-3.45 (m, 2H), 3.75-3.91 (m, 2H), 5.81 (br s, 1H).

Example 153

({N-methyl-N-[2-((S)-2-methylpyrrolidin-1-yl)ethyl] amino}(4-piperidinomethylpiperidino)methylene) malononitrile (Compound 153)

In a similar manner to Example 149, the titled compound (42.2 mg, 33 %) was obtained as a yellow oily substance by using ({[2-((S)-2-methylpyrrolidin-1-yl) ethyl]amino}(4-piperidinomethylpiperidino)methylene) malononitrile (Compound 152) (122 mg, 0.317 mmol) obtained in Example 152 and methyl iodide (0.0300 mL, 0.482 mmol).
$^1$H NMR ($CDCl_3$, δppm) : 1.05 (d, J = 6.1 1 Hz, 3H), 1.08-1.48 (m, 4H), 1.48-1.61 (m, 2H), 1.61-1.79 (m, 2H), 1.79-1.98 (m, 3H), 2.01-2.21 (m, 5H), 2.24-2.40 (m, 6H), 2.83 (s, 1.5H), 2.84-2.93 (m, 2H), 2.94 (s, 1.5H), 3.01-3.25 (m, 3H), 3.33-3.43 (m, 1H), 3.50-3.68 (m, 2H), 3.76-3.91 (m, 2H).
APCIMS m/z: $[M+H]^+$399.

Example 154

({[2-((R)-2-methylpyrrolidin-1-yl)ethyl]amino}[4-(2-methylpyrrolidin-1-ylmethyl)piperidino]methylene) malononitrile (Compound 154)

{[4-(2-Methylpyrrolidin-1-ylmethyl)piperidino] (methylthio)methylene}malononitrile (180 mg, 0.591 mmol) obtained in Step 1 of Example 148 was dissolved in ethanol (2 mL) and the solution was added with a solution of (R)-1-(2-aminoethyl)-2-methylpyrrolidine (150 mg, 1.17 mmol) obtained in Reference Example 4 in ethanol (2 mL), followed by stirring at 70˚C for 10 hours. After cooling, the reaction mixture was added with saturated brine and extracted with ethyl acetate. After washing with saturated brine, the organic layer was dried over anhydrous magnesium sulfate and the solvent was evaporated under reduced pressure. The residue was purified by NH silica gel column chromatography (n-hexane/ethyl acetate (1:1)) to obtain the titled compound (166 mg, 73%) as a yellow oily substance.
$^1$H NMR ($CDCl_3$, δppm) : 1.03 (d, J = 5.8 Hz, 3H), 1.07 (d, J=5.9 Hz, 3H), 1.12-1.47 (m, 4H), 1.50-1.63 (m, 4H), 1.63-1.84 (m, 4H), 1.84-2.12 (m, 4H), 2.12-2.31 (m, 1H), 2.31-2.60 (m, 3H), 2.84-3.19 (m, 5H), 3.26-3.51 (m, 2H), 3.71-3.96 (m, 2H), 5.82 (br s, 1H).

Example 155

({N-methyl-N-[2-((R)-2-methylpyrrolidin-1-yl)ethyl) amino}[4-(2-methylpyrrolidin-1-ylmethyl)piperidino] methylene) malononitrile (Compound 155)

In a similar manner to Example 149, the titled compound (65.0 mg, 38 %) was obtained as a yellow oily substance by using {[2-((R)-2-methylpyrrolidin-1-yl)ethyl] amino}[4-(2-methylpyrrolidin-1-ylmethyl) piperidino]methylene)malononitrile (Compound 154) (166 mg, 0.432 mmol) obtained in Example 154 and methyl iodide (0.0520 mL, 0.835 mmol).
$^1$H NMR ($CDCl_3$, δppm) : 1.05 (d, J = 5.9 Hz, 6H), 1.15-1.47 (m, 4H), 1.60-1.81 (m, 7H), 1.81-1.96 (m, 2H), 1.96-2.19 (m, 4H), 2.19-2.40 (m, 2H), 2.48-2.61 (m, 1H), 2.81 (s, 0.75H), 2.83 (s, 0.75H), 2.85-2.92 (m, 1H), 2.94 (s, 0.75H), 2.96 (s, 0.75H), 3.01-3.29 (m, 3H), 3.32-3.46 (m, 1H), 3.47-3.72 (m, 2H), 3.77-3.95 (m, 2H).
APCIMS m/z: $[M+H]^+$399.

Example 156

({[2-((S)-2-methylpyrrolidin-1-yl)ethyl]amino}[4-(2-methylpyrrolidin-1-ylmethyl)piperidino]methylene) malononitrile (Compound 156)

{[4-(2-Methylpyrrolidin-1-ylmethyl)piperidino] (methylthio)methylene}malononitrile (180 mg, 0.591 mmol) obtained in Step 1 of Example 148 was dissolved in ethanol (2 mL) and the solution was added with a solution of (S)-1-(2-aminoethyl)-2-methylpyrrolidine (150 mg, 1.17 mmol) obtained in Reference Example 5 in ethanol (2 mL), followed by stirring at 70˚C for 9 hours. After cooling, the reaction mixture was added with saturated brine and extracted with ethyl acetate. After washing with saturated brine, the organic layer was dried over anhydrous magnesium sulfate and the solvent was evaporated under reduced pressure. The residue was purified by NH silica gel column chromatography (n-hexane/ethyl acetate (1:1)) to obtain the titled compound (169 mg, 74 %) as a yellow oily substance.
$^1$H NMR ($CDC1_3$, δppm) : 1.03 (d, J = 5.8 Hz, 3H), 1.07 (d, J=5.9 Hz, 3H), 1.12-1.47 (m, 4H), 1.50-1.63 (m, 4H), 1.63-1.84 (m, 4H),1.84-2.12 (m, 4H), 2.12-2.31 (m, 1H), 2.31-2.60 (m, 3H), 2.84-3.19 (m, 5H), 3.26-3.51 (m, 2H), 3.71-3.96 (m, 2H), 5.82 (br s, 1H) .

Example 157

({N-methyl-N-[2-((S)-2-methylpyrrolidin-1-yl)ethyl] amino}[4-(2-methylpyrrolidin-1-ylmethyl)piperidino] methylene) malononitrile (Compound 157)

In a similar manner to Example 149, the titled compound (66.3 mg, 38 %) was obtained as a yellow oily substance by using ({[2-((S)-2-methylpyrrolidin-1-yl)ethyl]amino}[4-(2-methylpyrrolidin-1-ylmethyl) piperidino]methylene)malononitrile (Compound 156) (169 mg, 0.439mmol) obtained in Example 156 and methyl iodide (0.0550 mL, 0.883 mmol).
$^1$H NMR ($CDCl_3$, δppm) : 1.05 (d, J = 5.9 Hz, 6H), 1.15-1.47 (m, 4H), 1.60-1.81 (m, 7H), 1.81-1.96 (m, 2H), 1.96-2.19 (m, 4H), 2.19-2.40 (m, 2H), 2.48-2.61 (m, 1H), 2.81 (s, 0.75H), 2.83 (s, 0.75H), 2.85-2.92 (m, 1H), 2.94 (s, 0.75H), 2.96 (s, 0.75H), 3.01-3.29 (m, 3H), 3.32-3.46 (m, 1H), 3.47-3.72 (m, 2H), 3.77-3.95 (m, 2H).

APCIMS m/z: $[M+H]^+$399.

Example 158

{(3-Piperidinopropylamino)[4-(pyrrolidin-1-yl) piperidino]methylene}malononitrile (Compound 158)

In a similar manner to Example 143, the titled compound (249 mg, 47 %) was obtained as a white solid by using [bis(methylthio)methylene]malononitrile (242 mg, 1.42 mmol), 4-(pyrrolidin-1-yl)piperidine (277 mg, 1.80 mmol) in place of 4-piperidinopiperidine and 1-(3-aminopropyl)piperidine (384 mg, 2.70 mmol) in place of 1-(2-aminoethyl) piperidine.
$^1$H NMR ($CDCl_3$, δppm) : 1.42-1.28 (m, 9H), 1.68-1.84 (m, 5H), 1.97 (dd, J = 3.1, 13.4 Hz, 2H), 2.25-2.34 (m, 1H), 2.38-2.62 (m, 10H), 3.15 (td, J = 2.8, 12.3Hz, 2H), 3.49 (dd, J = 4.8, 10.3 Hz, 2H), 3.80 (d, J=12.3Hz, 2H), 7.63 (br s, 1H) .
APCIMS m/z: $[M+H]^+$371.
Melting point: 114-116˚C.

Example 159

{[N-methyl-N-(3-piperidinopropyl)amino][4-(pyrrolidin-1-yl)piperidino]methylene}malononitrile (Compound 159)

In a similar manner to Example 144, the titled compound (49.9 mg, 48 %) was obtained as a white solid by using {(3-piperidinopropylamino)[4-(pyrrolidin-1-yl) piperidino]methylene}malononitrile (Compound 158) (100 mg, 0.270 mmol) obtained in Example 158 and methyl iodide (0.0340 mL, 0.543 mL).
$^1$H NMR ($CDCl_3$, δppm) : 1.38-1.49 (m, 2H), 1.51-1.72 (m, 6H), 1.72-1.86 (m, 6H), 1.95-2.05 (m, 2H), 2.21-2.40 (m, 7H), 2.54-2.62 (m, 4H), 2.98 (s, 1.5H), 3.02 (s, 1.5H), 3.11-3.24 (m, 2H), 3.31 (dd, J = 7.2, 14.9Hz, 2H),3.53-3.65 (m, 2H).
APCIMS m/z: $[M+H]^+$385.
Melting point: 116-117˚C.

Example 160

[(4-Piperidinopiperidino)(3-piperidinopropylamino) methylene]malononitrile (Compound 160)

In a similar manner to Example 143, the titled compound (280 mg, 43 %) was obtained as a white solid by using [bis(methylthio)methylene]malononitrile (291 mg, 1.71 mmol), 4-piperidinopiperidine (303 mg, 1.80 mmol) and 1-(3-aminopropyl)piperidine (384 mg, 2.70mmol) in place of 1-(2-aminoethyl)piperidine.
$^1$H NMR ($CDCl_3$, δppm) : 1.44-1.63 (m, 15H), 1.70-1.79 (m, 2H), 1.91 (d, J = 11.5 Hz, 2H), 2.44-2.56 (m, 10H), 3.03 (t, J = 11.9 Hz, 2H), 3. 51 (dd, J = 4.8, 10. 0 Hz, 2H), 3. 90 (d, J = 11.9 Hz, 2H), 7.71 (br s, 1H).
APCIMS m/z: $[M+H]^+$385.
Melting point: 117-118˚C.

Example 161

{[N-methyl-N-(3-piperidinopropyl)amino](4-piperidinopiperidino)methylene}malononitrile (Compound 161)

In a similar manner to Example 144, the titled compound (56.9 mg, 27 %) was obtained as a white solid by using [(4-piperidinopiperidino)(3-piperidinopropylamino) methylene]malononitrile (Compound 160) (200 mg, 0.520 mmol) obtained in Example 160 and methyl iodide (0.0600 mL, 0.964 mmol).
$^1$H NMR ($CDCl_3$,δppm) : 1.38-1.20 (m, 4H), 1.50-1.83 (m, 12H), 1.93 (d, J = 13.0 Hz, 2H), 2.27 (t, J = 6.9 Hz, 2H), 2.30-2.41 (m, 4H), 2.43-2.56 (m, 5H), 2.97 (s, 1.5H), 3.01 (s, 1.5H), 3.05-3.16 (m, 2H), 3.25 (t, J = 6.8 Hz, 1H), 3.34 (t, J = 6.8 Hz, 1H), 3.60-3.68 (m, 2H).
APCIMS m/z: $[M+H]^+$399.
Melting point: 104-105˚C.

Example 162

{[N-ethyl-N-(3-piperidinopropyl)amino](4-piperidinopiperidino)methylene}malononitrile (Compound 162)

In a similar manner to Example 144, the titled compound (39.2 mg, 15 %) was obtained as a white solid by using [(4-piperidinopiperidino)(3-piperidinopropylamino) methylene]malononitrile (Compound 160) (248 mg, 0.645

mmol) obtained in Example 160 and iodoethane (0.150 mL, 1.88 mmol) .
$^1$H NMR ($CDCl_3$, δppm) : 1.17 (t, J = 7. 1 Hz, 1.5H), 1.18 (t,J= 7.1 Hz, 1.5 H), 1.38-1.50 (m, 4H), 1.50-1.80 (m, 12H), 1.87-1.99 (m, 2H), 2.22-2.40 (m, 6H), 2.43-2.57 (m, 5H), 3.01-3.16 (m, 2H), 3.22 (t, J = 6.9Hz, 1H), 3.30-3.45 (m, 3H), 3.61-3.73 (m, 2H).
APCIMS m/z: $[M+H]^+$413.
Melting point: 94-96˚C.

Example 163

{[4-(2-Methylpyrrolidin-1-yl)piperidino](3-piperidinopropylamino)methylene}malononitrile (Compound 163)

In a similar manner to Example 143, the titled compound (106 mg, 19 %) was obtained as a white solid by using [bis(methylthio)methylene]malononitrile (250 mg, 1.47 mmol), 4-(2-methylpyrrolidin-1-yl)piperidine (334 mg, 1.99 mmol) obtained in Reference Example 6 in place of 4-piperidinopiperidine and 1-(3-aminopropyl)piperidine (347 mg, 2.44 mmol) in place of 1-(2-aminoethyl)piperidine.
$^1$H NMR ($CDCl_3$, δppm): 1.05 (d, J = 6.3 Hz, 3H), 1.39-2.00 (m, 16H), 2.39-2.62 (m, 7H), 2.72-2.96 (m, 3H), 3.02-3.18 (m, 2H), 3.47-3.58 (m, 2H), 3.89 (t, J = 11.9 Hz, 2H), 7.69 (br s, 1H).
APCIMS m/z: $[M+H]^+$385.
Melting point: 83-84˚C.

Example 164

[(3-Piperidinopropylamino) (4-morpholinopiperidino) methylene]malononitrile (Compound 164)

In a similar manner to Example 143, the titled compound (93.5 mg, 23 %) was obtained as a white solid by using [bis(methylthio)methylene]malononitrile (178 mg, 1.05 mmol), 4-morpholinopiperidine (200 mg, 1.17 mmol) in place of 4-piperidinopiperidine and 1-(3-aminopropyl) piperidine (416 mg, 2.92 mmol) in place of 1-(2-aminoethyl)piperidine.
$^1$H NMR ($CDCl_3$, δppm) : 1.42-1.72 (m, 9H), 1.73-1.81 (m, 2H), 1.94 (d, J = 10.8 Hz, 2H), 2.39-2.58 (m, 10H), 3.06 (t, J = 12.0 Hz, 2H), 3.51 (dd, J = 5.1, 9.7 Hz, 2H), 3.71 (t, J = 4.6 Hz, 4H), 3.89 (d, J = 12.0Hz, 2H), 7.74 (br s, 1H).
APCIMS m/z: $[M+H]^+$387.
Melting point: 122-124˚C.

Example 165

[(4-Piperidinopiperidino)(3-morpholinopropylamino) methylene]malononitrile (Compound 165)

In a similar manner to Example 143, the titled compound (161 mg, 29 %) was obtained as a white solid by using [bis(methylthio)methylene]malononitrile (242 mg, 1.42 mmol), 4-piperidinopiperidine (251 mg, 1.49 mmol) and 4-(3-aminopropyl)morpholine (0.327 mL, 2.24 mmol) in place of 1-(2-aminoethyl)piperidine.
$^1$H NMR ($CDCl_3$, δppm) : 1.40-1.50 (m, 2H), 1.52-1.72 (m, 6H), 1.73-1.83 (m, 2H), 1.95 (d, J = 11.6 Hz, 2H), 2.46-2.60 (m, 11H), 3.08 (t, J =12.3 Hz, 2H), 3.44 (dd, J = 5.3, 10.6 6 Hz, 2H), 3.72 (t, J = 4.6 Hz, 4H), 3.85 (d, J = 12.3 Hz, 2H), 7.03 (br s, 1H).
APCIMS m/z: $[M+H]^+$387.
Melting point: 77-80˚C.

Example 166

{N-methyl-N-(3-morpholinopropyl)amino}(4-piperidinopiperidino)methylene}malononitrile (Compound 166)

In a similar manner to Example 144, the titled compound (75.7 mg, 61 %) was obtained as a white solid by using [(4-piperidinopiperidino)(3-morpholinopropylamino) methylene]malononitrile (Compound 165) (120 mg, 0.310 mmol) obtained in Example 165 and methyl iodide (0.0400 mL, 0.643 mmol).
$^1$H NMR ($CDCl_3$,δppm) : 1.40-1.52 (m, 2H), 1.52-1.82 (m, 8H), 1.89-2.01 (m, 2H), 2.32 (t, J = 6.8 Hz, 2H), 2.36-2.46 (m, 4H), 2.46-2.58 (m, 5H), 2.99 (s, 1.5H), 3.02 (s, 1.5H), 3.04-3.19 (m, 2H), 3.27 (t, J= 6.7Hz, 1H), 3.36 (t, J = 6.7 Hz, 1H), 3.57-3.74 (m, 6H).
APCIMS m/z: $[M+H]^+$401.
Melting point: 104-106˚C.

Example 167

{(4-Piperidinopiperidino)(3-(thiazolidin-3-yl) propylamino]methylene}malononitrile (Compound 167)

(Step 1)

[Bis(methylthio)methylene]malononitrile (500 mg, 2.94 mmol) was dissolved in ethanol (5 mL) and the solution was added with a solution of 4-piperidinopiperidine (520 mg, 3.09 mmol) in ethanol (5 mL), followed by stirring at room temperature for 0.5 hour. The reaction mixture was added with 3-aminopropanol (0.470 mL, 6.14 mmol) and the mixture was stirred under heating at 70˚C for 4 hours. After cooling, the mixture was added with saturated brine and extracted with ethyl acetate. After washing with saturated brine, the organic layer was dried over anhydrous magnesium sulfate and the solvent was evaporated under reduced pressure. The residue was purified by NH silica gel column chromatography (chloroform/methanol (9:1)). The obtained solid was washed with diisopropylether and [(3-hydroxypropylamino)(4-piperidinopiperidino)methylene]malononitrile (330 mg, 35 %) was obtained as a white solid.
$^1$H NMR ($CDCl_3$, δppm) : 1.36-1.50 (m, 2H), 1.50-1.77 (m, 5H), 1.80-1.98 (m, 4H), 2.42-2.57 (m, 6H), 3.06 (t, J = 11.7 Hz, 2H), 3.48-3.54 (m, 2H), 3.84-3.97 (m, 4H), 6.19 (br s, 1H).

(Step 2)

[(3-Hydroxypropylamino)(4-piperidinopiperidino) methylene]malononitrile (330 mg, 1.04 mmol) obtained in the Step 1 was dissolved in dichloromethane (3mL) and the solution was added with triethylamine (0.430 mL, 3.09 mmol) and methanesulfonylchloride (0.120 mL, 1.55 mmol) under ice bath, followed by stirring at 0˚C for 1 hour. The reaction mixture was added with saturated aqueous sodium hydrogencarbonate solution and extracted with chloroform. After washing with saturated brine, the organic layer was dried over anhydrous magnesium sulfate and the solvent was evaporated under reduced pressure to obtain [(3-methanesulfonyloxypropylamino)(4-piperidinopiperidino) methylene] malononitrile (411 mg, 100 %) as a clear oily substance.
$^1$H NMR ($CDCl_3$, δppm) : 1.41-1.55 (m, 2H), 1.59-1.80 (m, 2H), 1.86-2.06 (m, 6H), 2.06-2.36 (m, 2H), 2.52-2.72 (m, 5H), 3.08 (s, 3H),3.13 (t, J = 12.8 Hz, 2H), 3.46-3.60 (m, 2H), 3.89 (d, J = 12.8 Hz, 2H), 4.38 (t, J = 5.6Hz, 2H), 5.43 (br s, 1H).

(Step 3)

[(3-Methanesulfonyloxypropylamino)(4-piperidinopiperidino)methylene]malononitrile (166 mg, 0.420 mmol) obtained in the Step 2 was dissolved in 1,4-dioxane (5 mL) and the solution was added with a solution of thiazolidine (75.0 mg, 0.841 mmol) in 1,4-dioxane (5 mL) and potassium carbonate (174 mg, 1.26 mmol), followed by stirring at 80˚C for 12 hours. After cooling, the reaction mixture was added with saturated brine and extracted with ethyl acetate. After washing with saturated brine, the organic layer was dried over anhydrous magnesium sulfate and the solvent was evaporated under reduced pressure. The residue was purified by preparative thin-layer chromatography (chloroform/(2 mol/L ammonia-methanol) (9:1)) to obtain the titled compound (12.0 mg, 7.3 %) as a yellow oily substance.
$^1$H NMR ($CDCl_3$, δppm) : 1.38-1.64 (m, 3H), 1.70-1.85 (m, 6H), 1.92 (d, J = 11.6 Hz, 2H), 2.41-2.55 (m, 6H), 2.65 (t, J = 5.4 Hz, 2H), 2.93 (t, J = 6.3 Hz, 2H), 3.00-3.14 (m, 4H), 3.51 (dd, J = 5.0, 10.7 Hz, 2H), 3.83 (d, J = 13.4 Hz, 2H), 4.03 (s, 2H), 7.27 (br s, 1H).
APCIMS m/z: $[M+H]^+$389.

Example 168

{[3-(3,3-Difluoropyrrolidin-1-yl)propylamino](4-piperidinopiperidino)methylene}malononitrile (Compound 168)

3,3-Difluoropyrrolidine hydrochloride (457 mg, 3.18 mmol) was dissolved in a mixed solvent of 1,4-dioxane (10 mL) and 2 mol/L aqueous sodium hydroxide solution (1.70 mL, 3.40 mmol) and the solution was stirred for 0.25 hour. The mixture was added with a solution of [(3-methanesulfonyloxypropylamino)(4-piperidinopiperidino) methylene] malononitrile (699 mg, 1.77 mmol) obtained in Step 2 of Example 167 in 1,4-dioxane (20 mL) and potassium carbonate (730 mg, 5.28 mmol), followed by stirring at 60˚C for 12 hours. Further, sodium iodide (265 mg, 1.77 mmol) was added to the mixture and stirred at 60˚C for 12 hours. After cooling, the reaction mixture was added with saturated brine and extracted with ethyl acetate. After washing with saturated brine, the organic layer was dried over anhydrous magnesium sulfate and the solvent was evaporated under reduced pressure. The residue was purified by NH silica gel column chromatography (chloroform/methanol (9:1)) to obtain the titled compound (509 mg, 71 %) as a yellow oily substance.
$^1$H NMR ($CDCl_3$, δppm) : 1.37-1.50 (m, 2H), 1.50-1.85 (m, 8H), 1.85-1.98 (m, 2H), 2.31 (tt, J = 7.2, 14.5 Hz, 2H), 2.42-2.57 (m, 5H), 2.72 (t,J = 5.3 Hz, 2H), 2.82 (t, J = 7.2 Hz, 2H), 2.94 (t, J = 12.7 Hz, 2H), 3.03 (t, J= 12.1 Hz, 2H), 3.49 (dd, J

= 5.3, 10.8 Hz, 2H), 3.83 (d, J = 12.1Hz, 2H), 6.90 (br s, 1H).
APCIMS m/z: $[M+H]^+$407.

Example 169

({N-[3-(3,3-difluoropyrrolidin-1-yl)propyl]-N-methylamino}(4-piperidinopiperidino)methylene) malononitrile (Compound 169)

In a similar manner to Example 149, the titled compound (45.4 mg, 29 %) was obtained as a yellow oily substance by using {[3-(3,3-difluoropyrrolidin-1-yl)propylamino](4-piperidinopiperidino)methylene} malononitrile (Compound 168) (150 mg, 0.369 mmol) obtained in Example 168 and methyl iodide (0.0300 mL, 0.482 mmol).
$^1$H NMR ($CDCl_3$, δppm) : 1.36-1.80 (m, 10H), 1.86-1.98 (m, 2H), 2.15-2.34 (m, 2H), 2.41-2.56 (m, 7H), 2.66-2.74 (m, 2H), 2.75-2.90 (m, 2H), 3.00 (s, 1.5H), 3.02 (s, 1.5 H), 3.03-3.19 (m, 2H), 3.25 (t, J = 6.7Hz, 1H), 3.36 (t, J = 6.7 Hz, 1H), 3.55-3.70 (m, 2H).
APCIMS m/z: $[M+H]^+$421.

Example 170

{[3-(2-Methylpyrrolidin-1-yl)propylamino](4-piperidinopiperidino)methylene}malononitrile (Compound 170)

In a similar manner to Example 143, the titled compound (226 mg, 41 %) was obtained as a yellow oily substance by using [bis(methylthio)methylene]malononitrile (242 mg, 1.42 mmol), 4-piperidinopiperidine (251 mg, 1.49 mmol) and 1-(3-aminopropyl)-2-methylpyrrolidine (319 mg, 2.24 mmol) obtained in Reference Example 7 in place of 1-(2-aminoethyl) piperidine.
$^1$H NMR ($CDCl_3$, δppm) : 1.10 (d, J = 5.9 Hz,3H), 1.31-1.50 (m, 3H), 1.50-1.64 (m, 8H), 1.64-2.03 (m, 6H), 2.07-2.19 (m, 1H), 2.25-2.56 (m, 6H), 2.88-3.05 (m, 3H), 3.22-3.32 (m, 1H), 3.48-3.58 (m, 2H), 3.74-3.96 (m, 2H), 8.20 (br s, 1H).
APCIMS m/z: $[M+H]^+$385.

Example 171

{(3-Piperidinopropylamino)[(S)-2-(pyrrolidin-1-ylmethyl)pyrrolidin-1-yl]methylene}malononitrile (Compound 171)

In a similar manner to Example 143, the titled compound (147 mg, 28 %) was obtained as a yellow oily substance by using [bis(methylthio)methylene]malononitrile (242 mg, 1.42 mmol), (S)-2-(pyrrolidin-1-ylmethyl)pyrrolidine (0.253 mL, 1.49 mmol) in place of 4-piperidinopiperidine and 1-(3-aminopropyl)piperidine (319 mg, 2.24 mmol) in place of 1-(2-aminoethyl)piperidine.
$^1$H NMR ($CDCl_3$, δppm) : 1.39-1.51 (m, 2H), 1.51-2.18 (m, 14H), 2.29-2.57 (m, 9H), 2.68-2.79 (m, 2H), 2.88 (dd, J = 9.1, 13.4 Hz, 1H), 3.37-3.50 (m, 3H), 3.88 (t, J = 8.9 Hz, 1H), 4.03-4.15 (m, 1H), 10.04 (brs,1H).
APCIMS m/z: $[M+H]^+$371.

Example 172

[(4-Piperidinomethylpiperidino)(3-piperidinopropylamino)methylene]malononitrile (Compound 172)

[(Methylthio)(4-piperidinomethylpiperidino)methylene ]malononitrile (117 mg, 0.384 mmol) obtained in Step 1 of Example 147 was dissolved in ethanol (1.5 mL) and the solution was added with a solution of 1-(3-aminopropyl) piperidine (81.0 mg, 0.569 mmol) in ethanol (1.5 mL), followed by stirring at 70˚C for 11 hours. After cooling, the mixture was added with saturated brine and extracted with ethyl acetate. After washing with saturated brine, the organic layer was dried over anhydrous magnesium sulfate and the solvent was evaporated under reduced pressure. The residue was purified by preparative thin-layer chromatography (chloroform/methanol (9:1)) to obtain the titled compound (23.9 mg, 16 %) as a yellow oily substance.
$^1$H NMR ($CDCl_3$, δppm) : 1.18-1.35 (m, 3H), 1.36-2.69 (m, 12H), 1.70-1.94 (m, 5H), 2.15 (d, J = 7.0 Hz, 2H), 2.27-2.57 (m, 9H), 3.03 (t,J = 12.8Hz, 2H), 3.51 (dd, J = 4.8, 9.9 Hz, 2H), 3.85 (d, J = 12.8 Hz, 2H), 7.64 (br s, 1H).
APCIMS m/z: $[M+H]^+$399.

Example 173

{[3-(2-Methylpyrrolidin-1-yl)propylamino](4-piperidinomethylpiperidino)methylene}malononitrile (Compound 173)

[(Methylthio)(4-piperidinomethylpiperidino) methylene]malononitrile (432 mg, 1.42 mmol) obtained in Step 1 of Example 147 was dissolved in ethanol (5 mL) and the solution was added with a solution of 1-(3-aminopropyl)-2-methylpyrrolidine (272 mg, 1.91 mmol) obtained in Reference Example 7 in ethanol (3 mL), followed by stirring at 70˚C for 4 hours. After cooling, the reaction mixture was added with saturated brine and extracted with ethyl acetate. After washing with saturated brine, the organic layer was dried over anhydrous magnesium sulfate and the solvent was evaporated under reduced pressure. The residue was purified by preparative thin-layer chromatography (chloroform/methanol (9:1)) to obtain the titled compound (28.1 mg, 4.9 %) as a yellow oily substance.
$^1$H NMR ($CDCl_3$, δppm) : 1.12 (d, J = 6.05 Hz, 3H), 1.18-1.47 (m, 3H), 1.52-1.59 (m, 4H), 1.65-1.91 (m, 11H), 1.94-2.05 (m, 1H), 2.11-2.21 (m, 2H), 2.26-2.46 (m, 5H), 2.90-3.05 (m, 3H), 3.24-3.31 (m, 1H), 3.49-3.57 (m, 2H), 3.70-3.90 (m, 2H), 8.08 (br s, 1H).
APCIMS m/z: $[M+H]^+$399.

Example 174

{[4-(2-Methylpyrrolidin-1-ylmethyl)piperidino][3-(2-methylpyrrolidin-1-yl)propylamino]methylene} malononitrile (Compound 174)

{[4-(2-Methylpyrrolidin-1-ylmethyl)piperidino] (methylthio)methylene}malononitrile (106 mg, 0.348 mmol) obtained in Step 1 of Example 148 was dissolved in ethanol (3 mL) and the solution was added with 1-(3-aminopropyl)-2-methylpyrrolidine (100 mg, 0.703 mmol) obtained in Reference Example 7, followed by stirring at 70˚C for 12 hours. After cooling, the mixture was added with saturated brine and extracted with ethyl acetate. After washing with saturated brine, the organic layer was dried over anhydrous magnesium sulfate and the solvent was evaporated under reduced pressure. The residue was purified by preparative thin-layer chromatography (chloroform/(2 mol/L ammonia-methanol) (9:1)) to obtain the titled compound (40.7 mg, 29 %) as a yellow oily substance.
$^1$H NMR ($CDCl_3$, δppm) : 1.03 (d, J = 6.2 Hz, 1.5H), 1.04 (d, J= 5.9 Hz, 1.5H), 1.11 (d, J = 5.9 Hz, 3H), 1.15-1.30 (m, 1H), 1.30-1.47 (m, 2H), 1.50-1.65 (m, 6H), 1.65-1.95 (m, 5H), 1.95-2.17 (m, 4H), 2.21-2.46 (m, 3H), 2.48-2.59 (m, 1H), 2.90-3.15 (m, 4H), 3.23-3.32 (m, 1H), 3.47-3.62 (m, 2H), 3.68-3.96 (m, 2H), 8.11 (br s, 0.5H), 8.18 (br s, 0.5H).
APCIMS m/z: $[M+H]^+$399.

Example 175

[(1-Isopropylpiperidin-4-ylamino)(4-piperidinopiperidino)methylene]malononitrile (Compound 175)

(Step 1)

[Bis(methylthio)methylene]malononitrile (1.94 g, 11.3 mmol) was dissolved in THF (10 mL) and the solution was added with a solution of 4-piperidinopiperidine (2.30 g, 13.7 mmol) in THF (10 mL) under ice bath. After stirring at room temperature for 0.25 hour, the reaction mixture was concentrated under reduced pressure. The residue was purified by silica gel column chromatography (chloroform/methanol (9:1)) to obtain [(methylthio)(4-piperidinopiperidino)methylene]malononitrile (3.31 g, 100 %) as a yellow crystal.
$^1$H NMR ($CDCl_3$, δppm) : 1.39-1.50 (m, 2H), 1.52-1.74 (m, 6H), 1.94-2.05 (m, 2H), 2.44-2.56 (m, 5H), 2.58 (s, 3H), 3.32 (td, J =2.6, 12.7 Hz, 2H), 4.29 (d, J = 12.7 Hz, 2H).

(Step 2)

[(Methylthio)(4-piperidinopiperidino)methylene] malononitrile (613 mg, 2.11 mmol) obtained in the Step 1 and 4-amino-1-isopropylpiperidine (1.20 g, 8.44 mmol) are mixed and stirred at 60˚C for 2 hours. After cooling, the mixture was added with diisopropylether (5 mL) and hexane (10 mL), followed by stirring at room temperature for 0.25 hour. After the mixture was let stand for a while, the supernatant was removed and the residue was purified by NH silica gel column chromatography (hexane/chloroform (1:9) to chloroform) to obtain the titled compound (499 mg, 61 %) as a yellow oily substance.
$^1$H NMR ($CDCl_3$,δppm) : 1.03 (d, J = 6.6 6 Hz, 6H), 1.44-1.75 (m, 10H), 1.93-2.02 (m, 4H), 2.27 (td, J = 2.3, 11.7 Hz, 2H), 2.48-2.51 (m, 5H), 2.75 (sept, J = 6.6 Hz, 1H), 2.84 (d, J = 11.7 Hz, 2H), 3.10 (td, J =2.1, 12.7Hz, 2H), 3.40-3.52 (m, 1H), 3.85 (d, J = 12.7 Hz, 2H), 4.39 (d, J =9.2 Hz, 1H). APCIMS m/z: $[M+H]^+$385.

Example 176

{[N-(1-isopropylpiperidin-4-yl)-N-methylamino](4-piperidinopiperidino)methylene}malononitrile (Compound 176)

In a similar manner to Example 144, the titled compound (32.7 mg, 30%) was obtained as a white solid by using [(1-Isopropylpiperidin-4-ylamino)(4-piperidinopiperidino)methylene]malononitrile (Compound 175) (104 mg, 0.270 mmol) obtained in Example 175 and methyl iodide (0.0340 mL, 0.546 mmol).
$^1$H NMR ($CDCl_3$, δppm) : 1.04 (d, J = 6.5 Hz,6H), 1.39-1.51 (m, 2H), 1.51-2.00 (m, 12H), 2.25 (t, J = 11.2 Hz, 2H), 2.41-2.55 (m, 5H), 2.75 (sept., J = 6.5 Hz, 1H), 2.80 (s, 1.5H), 2.87 (s, 1.5H), 2.97 (d, J=11.2 Hz,2H), 3.02-3.17 (m, 2H), 3.44-3.69 (m, 3H).
APCIMS m/z: $[M+H]^+$399.
Melting point: 129-130˚C.

Example 177

[(1-Benzylpiperidin-4-ylamino)(4-piperidinopiperidino)methylene]malononitrile (Compound 177)

[(Methylthio)(4-piperidinopiperidino)methylene] malononitrile (4.50 g, 15.5 mmol) obtained in Step 1 of Example 175 and 4-amino-1-benzylpiperidine (15.8 mL, 77.5 mmol) were mixed and the mixture was stirred at 60˚C for 2 hours. After cooling, the mixture was added with diisopropylether (20 mL) and hexane (20 mL), followed by stirring at room temperature for 0.25 hour. After the mixture was let stand for a while, the supernatant was removed and the residue was purified by silica gel column chromatography (chloroform/methanol (9:1)). The obtained solid was recrystallized from ethyl acetate and the titled compound (1.96 g, 29 %) was obtained as a white solid.
$^1$H NMR ($CDCl_3$, δppm): 1.39-1.76 (m, 10H), 1.88-2.00 (m, 4H), 2.13 (td, J = 2.2, 11.8 Hz, 2H), 2.43-2.55 (m, 5H), 2.84 (d, J = 11.8Hz, 2H), 3.09 (td, J = 2.0, 12.9 Hz, 2H), 3.38-3.49 (m, 1H), 3.50 (s, 2H), 3.84 (d, J =12.9 Hz, 2H), 4.43 (d, J = 9.0 Hz, 1H), 7.22-7.36 (m, 5H).
APCIMS m/z: $[M+H]^+$433.

Example 178

[(1-Isopropylpiperidin-4-ylamino)(4-piperidinomethylpiperidino)methylene]malononitrile (Compound 178)

[(Methylthio)(4-piperidinomethylpiperidino) methylene]malononitrile (535 mg, 1.76 mmol) obtained in Step 1 of Example 147 and 4-amino-1-isopropylpiperidine (1.00 g, 7.03 mmol) were mixed and the mixture was stirred at 60˚C for 6 hours. After cooling, the reaction mixture was purified by NH silica gel column chromatography (chloroform) to obtain the titled compound (222 mg, 32 %) as a yellow oily substance.
$^1$H NMR ($CDCl_3$, δppm) : 1.04 (d, J = 6.5 Hz, 6H), 1.10-1.34 (m, 4H), 1.34-1.67 (m, 7H), 1.67-2.09 (m, 6H), 2.09-2.20 (m, 1H), 2.20-2.43 (m, 5H), 2.77 (sept, J = 6.5 Hz, 1H), 2.81-2.92 (m, 2H), 3.08 (t, J =12.8 Hz, 2H), 3.42-3.56 (m, 1H), 3.80 (d, J = 12.8 Hz, 2H), 4.45 (d, J = 8.9Hz,1H).

Example 179

{[N-(1-isopropylpiperidin-4-yl)-N-methylamino](4-piperidinomethylpiperidino)methylene}malononitrile (Compound 179)

In a similar manner to Example 144, the titled compound (87.7 mg, 43 %) was obtained as a white solid by using [(1-isopropylpiperidin-4-ylamino) (4-piperidinomethylpiperidino)methylene]malononitrile (Compound 178) (201 mg, 0.504 mmol) obtained in Example 178 and methyl iodide (0.100 mL, 1.61 mmol).
$^1$H NMR ($CDCl_3$, δppm) : 1.05 (d, J = 6.6 Hz, 6H), 1.15-1.48 (m, 3H), 1.48-1.67 (m, 5H), 1.69-2.02 (m, 7H), 2.10-2.41 (m, 8H), 2.79 (s, 1.5H), 2.87 (s, 1.5H), 2.93-3.16 (m, 5H), 3.46-3.68 (m, 3H) .
APCIMS m/z: $[M+H]^+$413.
Melting point: 197-198˚C.

Example 180

{(1-Isopropylpiperidin-4-ylamino)[4-(2-methylpyrrolidin-1-ylmethyl)piperidino]methylenel malononitrile (Compound 180)

{[4-(2-Methylpyrrolidin-1-ylmethyl)piperidino] (methylthio)methylene}malononitrile (535 mg, 1.76 mmol) obtained in Step 1 of Example 148 and 4-amino-1-isopropylpiperidine (1.00 g, 7.03 mmol) were mixed and the mixture

was stirred at 60˚C for 8 hours. After cooling, the solvent was evaporated under reduced pressure and the residue was purified by NH silica gel column chromatography (chloroform). The obtained solid was washed with diisopropylether and the titled compound (194 mg, 28 %) was obtained as a white solid.
$^1$H NMR ($CDCl_3$, δppm) : 1.04 (d, J = 5.9 Hz, 3H), 1.05 (d,J=6.6 Hz, 6H), 1.16-1.45 (m, 3H), 1.52-1.94 (m, 7H), 1.97-2.12 (m, 5H), 2.23-2.36 (m, 3H), 2.55 (dd, J = 9.6, 12.1 Hz, 1H), 2.79 (sept, J = 6.6Hz, 1H), 2.83-2.92 (m, 2H), 3.01-3.18 (m, 3H), 3.44-3.58 (m, 1H), 3.74-3.88 (m, 2H), 4.46 (d, J =8. 9 Hz, 1H).

Example 181

{[N-(1-isopropylpiperidin-4-yl)-N-methylamino][4-(2-methylpyrrolidin-1-ylmethyl)piperidino]methylene} malononitrile (Compound 181)

In a similar manner to Example 144, the titled compound (82.2 mg, 46 %) was obtained as a white solid by using {(1-isopropylpiperidin-4-ylamino)[4-(2-methylpyrrolidin-1-ylmethyl)piperidino]methylene} malononitrile (Compound 180) (170 mg, 0.426 mmol) obtained in Example 180 and methyl iodide (0.0800 mL, 1.29 mmol).
$^1$H NMR ($CDCl_3$, δppm) : 1.05 (d, J = 6.6 Hz, 9H), 1.16-1.47 (m, 2H),1.47-1.98 (m, 10H), 1.98-2.13 (m, 3H), 2.19-2.34 (m, 3H), 2.48-2.63 (m, 1H), 2.78 (s, 1.5H), 2.88 (s, 1.5H), 2.92-3.21 (m, 5H), 3.43-3.70 (m, 4H).
APCIMS m/z: $[M+H]^+$413.
Melting point: 138-140˚C.

Example 182

{(1-Benzylpiperidin-4-ylamino)[4-(2-methylpyrrolidin-1-ylmethyl)piperidinolmethylenel malononitrile (Compound 182)

{[4-(2-Methylpyrrolidin-1-ylmethyl)piperidino] (methylthio)methylene}malononitrile (1.00 g, 3.28 mmol) obtained in Step 1 of Example 148 and 4-amino-1-benzylpiperidine (3.34 mL, 16.4 mmol) were mixed and the mixture was stirred at 60˚C for 12 hours. After cooling, the mixture was added with diisopropylether (10 mL) and hexane (20 mL), followed by stirring at room temperature for 0.25 hour. After the mixture was let stand for a while, the supernatant was removed and the residue was purified by silica gel column chromatography (chloroform/methanol (9:1)) to obtain the titled compound (423 mg, 29 %) as a yellow oily substance.
$^1$H NMR ($CDCl_3$, δppm): 1.04 (d, J = 6.1 Hz, 3H), 1.11- 1.48 (m, 4H), 1.48-1.81 (m, 6H), 1.81-2.21 (m, 7H), 2.21-2.35 (m, 1H), 2.55 (dd, J =9.5, 12.1 Hz, 1H), 2.84 (d, J = 11.7 Hz, 2H), 2.99-3.24 (m, 3H), 3.39-3.64 (m, 3H), 3.75-3.85 (m, 2H), 4.44 (d, J = 9.2 Hz, 1H), 7.21-7.39 (m,5H).

Example 183

[(4-Cyclohexylpiperazin-1-yl)(3-piperidinopropylamino)methylene]malononitrile (Compound 183)

In a similar manner to Example 143, the titled compound (247 mg, 45 %) was obtained as a white solid by using [bis(methylthio)methylene]malononitrile (242 mg, 1.42 mmol), 1-cyclohexylpiperazine (251 mg, 1.49 mmol) in place of 4-piperidinopiperidine and 1-(3-aminopropyl) piperidine (319 mg, 2.70 mmol) in place of 1-(2-aminoethyl)piperidine.
$^1$H NMR ($CDCl_3$,δppm) : 1.05-1.29 (m, 5H), 1.43-1.89 (m, 13H), 2.26-2.37 (m, 1H), 2.39-2.59 (m, 6H), 2.66 (t, J = 4.9 Hz, 4H), 3.48 (t, J = 4.9Hz, 4H), 3.53 (dd, J = 4.4, 9.7 Hz, 2H), 7.82 (br s, 1H).
APCIMS m/z: $[M+H]^+$385.
Melting point: 136-137˚C.

Example 184

{1-(1-Benzylpiperidin-4-yl)-3-[2-(1-methylpyrrolidin-2-yl)ethyl]imidazolidin-2-ylidene} malononitrile (Compound 184)

A Compound obtained in Step 2 of Example 126, [1-(1-benzylpiperidin-4-yl)imidazolidin-2-ylidene]malononitrile (0.300 g, 0.976 mmol) was dissolved in dichloromethane (9.0 mL) and the solution was added with tetrabutylammoniumbromide (0.0158 g, 0.049 mmol), 2-(2-chloroethyl)-1-methylpyrrolidine hydrochloride (0.270 g, 1.47 mmol) and an aqueous potassium hydroxide solution (50 % w/w, 3.0 mL), followed by stirring at room temperature for one day. The mixture was added with saturated aqueous ammonium chloride solution and extracted with ethyl acetate. The organic layer was washed with saturated brine thrice, dried over anhydrous magnesium sulfate and the solvent was evaporated under reduced pressure to obtain the titled compound (0.358 mg, 85.5 %).

$^1$H NMR ($CDCl_3$, δppm): 1.72-1.78 (m, 7H), 1.97-2.01 (m, 2H), 2.11-2.20 (m, 4H), 2.35 (s, 3H), 2.95-2.99 (m, 2H), 3.04-3.10 (m, 1H), 3.42-3.68 (m, 9H), 4.18-4.26 (m, 1H), 7.28-7.31 (m, 5H).

Example 185

{1-[2-(1-Methylpyrrolidin-2-yl)ethyl]-3-(piperidin-4-yl)imidazolidin-2-ylidene}malononitrile dihydrochloride (Compound 185)

{1-(1-Benzylpiperidin-4-yl)-3-[2-(1-methylpyrrolidin -2-yl)ethyl]imidazolidin-2-ylidene}malononitrile (Compound 184) (0.300 g, 0.717 mmol) obtained in Example 184 was dissolved in 1,2-dichloroethane (3.0 mL) and the solution was added with 1-chloroethyl chloroformate (0.387 mL, 3.58 mmol), followed by stirring under reflux for 6 hours. The mixture was further added with 1-chloroethyl chloroformate (0.232 mL, 2.16 mmol), followed by stirring under reflux overnight. The solvent was evaporated under reduced pressure and the residue was added with diethylether and stirred. After repeating the procedure to remove the supernatant solution thrice, the remainig solvent was evaporated under reduced pressure. Further, the residue was added with methanol (3.0 mL) and stirred for 3 hours under reflux. The solvent was evaporated under reduced pressure and dried to obtain the titled compound, Compound 185.

Example 186

{[1-(Tert-butyloxycarbonyl)piperidin-4-yl]-3-[2-(1-methylpyrrolidin-2-yl)ethyl]imidazolidin-2-ylidene} malononitrile (Compound 186)

{1-[2-(1-Methylpyrrolidin-2-yl)ethyl]-3-(piperidin-4-yl)imidazolidin-2-ylidene}malononitrile dihydrochloride (Compound 185) obtained in Example 185 was dissolved in dichloromethane (5.0 mL) and the solution was added with dicarbonate di-tert-butyl (0.288 g, 1.32 mmol), followed by stirring at room temperature overnight. The mixture was added with water and extracted with ethyl acetate. The organic layer was washed with water thrice, dried over anhydrous magnesium sulfate and the solvent was evaporated under reduced pressure. The residue was purified by silica gel column chromatography (ethyl acetate to chloroform/ (2 mol/L ammonia-methanol) (10:1)) to obtain the titled compound (0.147 g, 39.1 %).
$^1$H NMR ($CDCl_3$, δppm) : 1.46 (s, 9H), 1.51-1.65 (m, 3H), 1.68-1.86 (m, 4H), 1.90-2.04 (m, 2H), 2.12-2.21 (m, 2H), 2.33 (s, 3H), 2.79-2.87 (m, 2H), 2.95-2.98 (m, 1H), 3.02-3.09 (m, 1H), 3.40-3.73 (m, 6H), 4.20-4.25 (m, 2H), 4.31-4.41 (m, 1H).
APCIMS m/z: $[M+H]^+$ 429.

Example 187

{1-(1-Isopropylpiperidin-4-yl)-3-[2-(1-methylpyrrolidin-2-yl)ethyl]imidazolidin-2-ylidene} malononitrile (Compound 187)

{[1-(Tert-butyloxycarbonyl)piperidin-4-yl]-3-[2-(1-methylpyrrolidin-2-yl)ethyl]imidazolidin-2-ylidene} malononitrile (Compound 186) (0.147 g, 0.343 mmol) obtained in Example 186 was dissolved in ethyl acetate (1.0 mL) and the solution was added with 4 mol/L hydrochloric acid-ethyl acetate (2.0 mL), followed by stirring at room temperature for 30 minutes. The mixture was added with diethylether and stirred. The procedure to remove the supernatant solution was repeated thrice. A solvent of the residue was evaporated under reduced pressure, and 1,4-dioxane (2.0 mL) was added thereto. The mixture was added with 2 mol/L aqueous sodium hydroxide solution (0.170 mL, 0.340 mmol) and stirred at room temperature for 1 minute. The mixture was added with isopropyl iodide (0.071 mL, 0.711 mmol) and potassium carbonate (0.064 mg, 0.463 mmol), stirred at 70˚C for 5 hours and then at 90˚C overnight. The mixture was added with 1 mol/L hydrochloric acid and extracted with ethyl acetate. The organic layer was dried over anhydrous magnesium sulfate and the solvent was evaporated under reduced pressure to obtain the titled compound (0.052 mg, 14.0 %).
$^1$H NMR ($CDCl_3$, δppm) : 1.05 (d, J = 5.4 Hz, 6H), 1.49-1.91 (m, 6H), 1.93-2.11 (m, 3H), 2.15-2.33 (m, 4H), 2.35 (s, 3H), 2.72-2.82 (m, 1H), 2.94-2.99 (m, 2H), 3.05-3.12 (m, 1H), 3.40-3.73 (m, 7H), 4.12-4.24 (m,1H).
APCIMS m/z: $[M+H]^+$ 371.

Example 188

{1,3-Bis[2-((S)-2-methylpyrrolidin-1-yl)ethyl] imidazolidin-2-ylidene}malononitrile (Compound 188)

(Step 1)

A Compound obtained in Step 1 of Example 5, [1,3-bis (2-hydroxyethyl)imidazolidin-2-ylidene]malononitrile (2.00 g, 9.00 mmol) was dissolved in dichloromethane (20 mL) and the solution was added with methanesulfonylchloride (1.81 mL, 23.4 mmol) and triethylamine (3.26 mL, 23.4 mmol).
After stirring at room temperature for 4.5 hours, the mixture was added with water and extracted with chloroform. The organic layer was dried over anhydrous magnesium sulfate and the solvent was evaporated under reduced pressure. The residue was recrystallized from methanolethyl acetate to obtain [1,3-bis(2-methanesulfonyloxyethyl)imidazolidin-2-ylidene] malononitrile (1.65 g, 48.4 %).
$^1$H NMR ($CDCl_3$, δppm) : 3.08 (s, 6H), 3.81 (s, 4H), 3.94 (t, J = 5.0 Hz, 4H), 4.51 (t, J = 5.0 Hz, 4H).

(Step 2)

(S)-2-methylpyrrolidine hydrobromate (0.211 g, 1.27 mmol) prepared in a similar manner to the method described in J. Org. Chem., Vol. 54, p.209 (1989) was dissolved in 1,4-dioxane (1.0 mL) and the solution was added with aqueous sodium hydroxide solution (2.0 mol/L, 0.699 mL). After stirring at room temperature for 1 minute, the mixture was added dropwise to a solution of a compound obtained in the Step 1, [1,3-bis(2-methanesulfonyloxyethyl)imidazolidin-2-ylidene] malononitrile (0.200 g, 0.530 mmol), in 1,4-dioxane (1.0 mL). The mixture was added with potassium carbonate (0.175 g, 1.27 mmol) stirred at room temperature for 2 hours, then at 50˚C for 22 hours and then at 70˚C for 14.5 hours. The mixture was added with water and the precipitated solid was collected by filtration to obtain the titled compound (0.052 g, 27.5 %).
$^1$H NMR ($CDCl_3$, δppm) : 1.07 (d, J = 6. 0 Hz, 6H), 1.30-1.42 (m, 2H), 1.68-1.77 (m, 4H), 1.86-1.98 (m, 2H), 2.18-2.27 (m, 2H), 2.35-2.43 (m, 4H), 3.06-3.16 (m, 4H), 3.44-3.67 (m, 4H), 3.78-3.87 (m, 4H).
APCIMS m/z: $[M+H]^+$357.

Example 189

{1,3-Bis[2-((R)-2-methylpyrrolidin-1-yl)ethyl] imidazolidin-2-ylidene}malononitrile (Compound 189)

(R)-2-methylpyrrolidine hydrochloride (0.637 g, 5.22 mmol) prepared in a similar manner to the method described in US 2004/0171845 was dissolved in dioxane (1.0 mL) and the solution was added with 10 mol/L aqueous sodium hydroxide solution (0.522 mL, 5.22 mmol), followed by stirring at room temperature for 3 minutes. The mixture was added dropwise to a suspension of a compound obtained in Step 1 of Example 188, [1,3-bis (2-methanesulfonyloxyethyl)imidazolidin-2-ylidene] malononitrile (0.550 g, 1.45 mmol), and potassium carbonate (0.802 g, 5.80 mmol) in 1,4-dioxane (4.0 mL). Further, the mixture was added with sodium iodide (0.217 g,1.45 mmol) and stirred at 60˚C for a day, followed by stirring at 70 ˚C for 8 hours. The mixture was added with 1 mol/L hydrochloric acid and washed with ethyl acetate. To the aqueous layer, 10 mol/L aqueous sodium hydroxide solution was added dropwise and the precipitated solid was collected by filtration to obtain the titled compound (0.111 g, 21.5 %).
$^1$H NMR ($CDCl_3$, δppm) : 1.07 (d, J = 6.2 Hz, 6H), 1.29-1.43 (m, 2H), 1.67-1.77 (m, 4H), 1.84-1.98 (m, 2H), 2.18-2.24 (m, 2H), 2.35-2.43 (m, 4H), 3.04-3.19 (m, 4H), 3.43-3.64 (m, 4H), 3.77-3.87 (m, 4H).
APCIMS m/z: $[M+H]^+$357.
Melting point: 139-141˚C.

Example 190

{1-[4-((S)-2-methylpyrrolidin-1-yl)butyl]-3-[2-((S) -2-methylpyrrolidin-1-yl)ethyl]imidazolidin-2-ylidene} malononitrile (Compound 190)

In a similar manner to Step 3 of Example 7, the titled compound (0.0281 g, 16.9 %) was obtained by using [1-(4-chlorobutyl)-3-(2-hydroxyethyl)imidazolidin-2-ylidene]malononitrile (0.120 g, 0.45 mmol) and (S)-2-methylpyrrolidine hydrobromate (0.320 g, 1.93 mmol) prepared in a similar manner to the method described in J. Org. Chem., Vol. 54, p.209 (1989) in place of piperidine.
$^1$H NMR ($CDC1_3$, δppm): 1.04-1.12 (m, 6H), 1.28-1.50 (m, 2H), 1.50-1.85 (m, 8H), 1.85-2.00 (m, 2H), 2.00-2.15 (m, 2H), 2.15-2.32 (m,2H),2.32-2.45 (m, 2H), 2.73-2.85 (m, 1H), 3.03-3.20 (m, 3H), 3.40-3.70 (m, 6H), 3.75-3.87 (m, 2H).
Melting point: 99-102˚C.

Example 191

{1-[4-((R)-2-methylpyrrolidin-1-yl)butyl]-3-[2-((R) -2-methylpyrrolidin-1-yl)ethyl]imidazolidin-2-ylidene} malononitrile (Compound 191)

In a similar manner to Step 3 of Example 7, the titled compound (0.0200 g, 12.0%) was obtained by using [1-(4-chlorobutyl)-3-(2-hydroxyethyl)imidazolidin-2-ylidene]malononitrile (0.120 g, 0.45 mmol) and (R)-2-methylpyrrolidine hydrobromate (0.320 g, 1.93 mmol) prepared in a similar manner to the method described in J.Org. Chem., Vol. 54, p.209 (1989) in place of piperidine.
$^1$H NMR ($CDCl_3$, δppm) : 1.04-1.12 (m, 6H), 1.28-1.50 (m, 2H), 1.50-1.85 (m, 8H), 1.85-2.00 (m, 2H), 2.00-2.15 (m, 2H), 2.15-2.32 (m, 2H), 2.32-2.45 (m, 2H), 2.73-2.85 (m, 1H), 3.03-3.20 (m, 3H), 3.40-3.70 (m, 6H), 3.75-3.87 (m, 2H).
Melting point: 99-102°C.

Example 192

[1,3-Bis-(2-piperidinoethyl)benzimidazolidin-2-ylidene]malononitrile (Compound 192)

(Benzimidazolidin-2-ylidene)malononitrile (100 mg, 0.55 mmol) obtained in Step 1 of Example 124, triphenylphosphine (433 mg, 1.65mmol) and piperidinoethanol (0.219 mL, 1.65 mmol) were dissolved in THF (2 mL) and the solution was added with 40% toluene solution of diethylazodicarboxylate (0.718 mL, 1.65 mmol) under ice-bath, followed by stirring at room temperature for 1 hour, and then at 60°C for 1 hour. The mixture was added with water and extracted with ethyl acetate. The organic layer was washed with saturated brine, dried over anhydrous magnesium sulfate and the solvent was evaporated under reduced pressure. The residue was purified by preparative TLC (n-hexane/ethyl acetate (3:1)) and then recrystallized from ethyl acetate-hexane to obtain the titled compound (52.0 mg, 23.4%) as a white solid.
$^1$H NMR ($CDCl_3$, δppm) : 1.35-1.57 (m, 12H), 2.50 (brs, 8H), 2.80 (t, J = 6.9 Hz, 4H), 4.46 (t, J = 6.9 Hz, 4H), 7.31-7.41 (m, 4H).
Melting point: 127-128°C.
APCIMS m/z: $[M+H]^+$405.

Example 193

{1-[2-((R)-2-methylpyrrolidin-1-yl)ethyl]-3-(3-piperidinopropyl)imidazolidin-2-ylidene}malononitrile (Compound 193)

In a similar manner to Step 3 of Example 47, the titled compound (0.076 g, 12.1 %) was obtained as a white solid by using [1-(2-methanesulfonyloxyethyl)-3-(3-piperidinopropyl)imidazolidin-2-ylidene]malononitrile (0.62 g, 1.69 mmol) and (R)-2-methylpyrrolidine hydrobromate (0.51 g, 3.04 mmol) in place of hexamethyleneimine.
$^1$H NMR ($CDCl_3$, δppm) : 1.15 (d, J = 6.0 Hz, 3H), 1.43-1.50 (m, 1H), 1.83-1.86 (m, 2H), 1.91-1.97 (m, 4H), 2.20-2.38 (m, 3H), 2.54-2.60 (m, 2H), 2.86-3.00 (m, 7H), 3.10-3.27 (m, 2H), 3.58-3.92 (m, 10H).
APCIMS m/z: $[M+H]^+$371.
Melting point: 89-90°C.

Example 194

{1-[3-((R)-2-methylpyrrolidin-1-yl)propyl]-3-(2-piperidinoethyl)imidazolidin-2-ylidene}malononitrile (Compound 194)

(Step 1)

[1-(3-Bromopropyl)-3-(2-hydroxyethyl)imidazolidin-2-ylidene]malononitrile (0.50 g, 1.68 mmol) obtained in Step 1 of Example 10 was dissolved in 1,4-dioxane (10 mL) and the solution was added with a solution of (R)-2-methylpyrrolidine hydrobromate (0.50 g, 3.02 mmol) in a mixed solvent of 1,4-dioxane (5 mL) and 2 mol/L aqueous sodium hydroxide solution (1.6 6 mL, 3.12 mmol). Further, the mixture was added with potassium carbonate (0.70 g, 5.04 mmol) and stirred at 70°C overnight: After cooling the mixture, the solvent was evaporated under reduced pressure and the residue was dissolved in chloroform. The solution was dried over anhydrous magnesium sulfate and the solvent was evaporated under reduced pressure. The residue was purified by silica gel column chromatography (chloroform/methanol (100:1 to 10:1)) to obtain {3-(2-hydroxyethyl)-1-[3-((R)-2-methylpyrrolidin-1-yl)propyl] imidazolidin-2-ylidene}malononitrile (0.50 g, 99 %).
$^1$H NMR ($CDCl_3$, δppm): 1.15 (d, J = 6.1 Hz, 3H), 1.42-1.54 (m, 1H), 1.71-1.84 (m, 2H), 1.90-1.98 (m, 2H), 2.20-2.29

(m, 2H), 2.41-2.49 (m, 2H), 2.88 (td, J = 8.1, 11.9 Hz, 1H), 3.21 (ddd, J = 3.5, 8.1, 9.2Hz, 1H), 3.53-3.82 (m, 8H), 3.90 (t, J = 5.3 Hz, 2H).

(Step 2)

{3-(2-Hydroxyethyl)-1-[3-((R)-2-methylpyrrolidin-1-yl)propyl]imidazolidin-2-ylidene}malononitrile (0.51 g, 1.69 mmol) obtained in the Step 1 was dissolved in dichloromethane (5 mL) and the solution was added with triethylamine (0.71 mL, 5.07 mmol) and methanesulfonylchloride (0.20 mL, 2.54 mmol), followed by stirring at room temperature for 0.5 hour. The mixture was added with saturated aqueous sodium hydrogencarbonate solution and extracted with chloroform. The organic layer was washed with saturated brine and the solvent was evaporated under reduced pressure. The obtained solid was washed with n-hexane/ethyl acetate (20/1) to obtain {3-(2-methanesulfonyloxyethyl)-1-[3-((R)-2-methylpyrrolidin-1-yl)propyl]imidazolidin-2-ylidene}malononitrile (0.51 g, 79.0 %) .
$^1$H NMR ($CDCl_3$,δppm) : 1.08 (d, J = 6.1 Hz,3H),1.37-1.46 (m, 1H), 1.66-1.78 (m, 3H), 1.84-1.96 (m, 2H), 2.09-2.19 (m, 1H),2.27-2.34 (m,1H), 2.58 (td, J = 12.1, 6.8 Hz, 1H), 2.80 (dt, J = 12.1, 8.1Hz,1H), 3.06 (s, 3H), 3.07-3.16 (m, 1H), 3.54-3.81 (m, 6H), 3.90 (dt, J = 1.3,4.6Hz, 2H), 4.51 (t, J = 4.9 Hz, 2H).

(Step 3)

{3-(2-Methanesulfonyloxyethyl)-1-[3-((R)-2-methylpyrrolidin-1-yl)propyl]imidazolidin-2-ylidene} malononitrile (0.64 g, 1.68 mmol) obtained in the Step 2 was dissolved in 1,4-dioxane (10 mL) and the solution was added with piperidine (0.50 ml, 5.04 mmol), followed by stirring at 60˚C overnight. After cooling the mixture, the solvent was evaporated under reduced pressure. The residue was dissolved in chloroform and the solution was dried over anhydrous magnesium sulfate and the solvent was evaporated under reduced pressure. The residue was purified by silica gel column chromatography (chloroform/methanol (100:1 to 10:1)), recrystallized from diisopropylether to obtain the titled compound (0.19 g, 31.0 %).
$^1$H NMR ($CDCl_3$, δppm): 1.09 (d, J = 6.0 Hz, 3H), 1.40-1.55 (m, 2H), 1.58-1.70 (m, 4H), 1.71-1.75 (m, 4H), 1.86-1.95 (m, 2H), 2.08-2.20 (m, 2H), 2.30-2.36 (m, 1H), 2.41-2.45 (m, 4H), 2.59 (t, J = 6.3Hz, 2H), 2.81 (td, J = 8.2, 11.9 Hz, 1H), 3.14 (td, J = 8.3, 3.1 Hz, 1H), 3.48-3.76 (m, 8H). APCIMS m/z: $[M+H]^+$371.
Melting point: 74-76˚C.

Example 195

{1-[3-((S)-2-methylpyrrolidin-1-yl)propyl]-3-(2-piperidinoethyl)imidazolidin-2-ylidene}malononitrile (Compound 195)

(Step 1)

[1-(3-Bromopropyl)-3-(2-hydroxyethyl)imidazolidin-2-ylidene]malononitrile (5.0 g, 16.7 mmol) obtained in Step 1 of Example 10 was dissolved in 1,4-dioxane (30 mL) and the solution was added with a solution of (S)-2-methylpyrrolidine hydrobromate (4.16 g, 25.1 mmol) in a mixed solvent of 1,4-dioxane (10 mL) and 2 mol/L aqueous sodium hydroxide solution (13.4 mL, 26.7 mmol), potassium carbonate (4.62 g, 33.4 mmol) and sodium iodide (2.5 g, 16.7 mmol), followed by stirring at 40˚C overnight. After cooling the mixture, the solvent was evaporated under reduced pressure and the residue was dissolved in chloroform followed by adding 2 mol/L hydroxy hydrochloric acid solution. The aqueous layer was washed with chloroform and the mixture was added with 2 mol/L aqueous sodium hydroxide solution to adjust the pH value to 10. Then, the mixture was extracted with chloroform. The organic layer was washed with saturated brine, dried over anhydrous magnesium sulfate and the solvent was evaporated under reduced pressure to obtain {3-(2-hydroxyethyl)-1-[3-((S)-2-methylpyrrolidin-1-yl)propyl]imidazolidin-2-ylidene}malononitrile (4.51 g, 89.0 %).
$^1$H NMR ($CDCl_3$, δppm) : 1.09 (d, J = 6.2 Hz, 3H), 1.39-1.48 (m, 1H), 1.74-1.81 (m, 3H), 1.85-1.98 (m, 3H), 2.04-2.21 (m, 1H), 2.29-2.39 (m, 1H), 2.82 (dt, J = 12.1, 8.1 Hz, 1H), 3.14 (td, J = 8.3, 3.1 Hz, 1H), 3.15 (td, J = 8.1, 3.0 Hz, 1H), 3.50-3.80 (m, 8H)

(Step 2)

{3-(2-Hydroxyethyl)-1-[3-((S)-2-methylpyrrolidin-1-yl)propyl]imidazolidin-2-ylidene}malononitrile (4.50 g, 14.83 mmol) obtained in the Step 1 was dissolved in dichloromethane (50 mL) and the solution was added with triethylamine (8.27 mL, 59.32 mmol) and methanesulfonylchloride (1.72 mL, 22.24 mmol) at 0˚C, followed by stirring at room temperature for 2 hours. The mixture was added with saturated brine and extracted with ethyl acetate. The organic layer was washed with saturated brine and the solvent was evaporated under reduced pressure to obtain {3-(2-methanesulfonyloxyethyl)-1-[3-((S)-2-methylpyrrolidin-1-yl)propyl]imidazolidin-2-ylidene}malononitrile (4.78 g, 84.5 %) as a pale yellow

solid.
$^1H$ NMR ($CDCl_3$, δppm): 1.08 (d, J = 6.3 Hz, 3H), 1.38-1.47 (m, 1H), 1.65-1.77 (m, 3H), 1.84-1.98 (m, 3H), 2.09-2.18 (m, 1H), 2.26-2.38 (m,1H), 2.81 (dt, J = 11.8, 8.1 Hz, 1H), 3.06 (s, 3H), 3.11-3.14 (m, 1H), 3.52-3.81 (m, 6H), 3.91 (td, J = 1.5, 4.8 Hz, 2H), 4.51 (t, J = 4.8Hz,2H).

(Step 3)

{3-(2-Methanesulfonyloxyethyl)-1-[3-((S)-2-methylpyrrolidin-1-yl)propyl]imidazolidin-2-ylidene} malononitrile (0.39 g, 1.03 mmol) obtained in the Step 2 was dissolved in 1,4-dioxane (5 mL) and the solution was added with piperidine (0.31 mL, 3.09 mmol) and potassium carbonate (0.29 g, 2.06 mmol), followec by stirring at 90˚C overnight. After cooling, the mixture was added with saturated brine and extracted with ethyl acetate. The organic layer was washed with saturated brine, dried over anhydrous magnesium sulfate and the solvent was evaporated under reduced pressure. The residue was purified by silica gel column chromatography (chloroform/methanol (3:1)) to obtain the titled compound (0.27 g, 71.1 %).
$^1H$ NMR ($CDCl_3$, δppm) : 1.08 (d, J = 6.0 Hz, 3H), 1.40-1.46 (m, 2H), 1.53-1.77 (m, 8H), 1.80-1.98 (m, 2H), 2.07-2.19 (m, 2H), 2.34-2.34 (m, 1H), 2.38-2.46 (m, 4H), 2.60 (t, J = 6.4 Hz, 2H), 2.80 (td, J =8.1, 12.1 Hz, 1H), 3.14 (td, J = 8.3, 3.3 Hz, 1H), 3.48-3.75 (m, 8H).
APCIMS m/z: $[M+H]^+$371.
Melting point: 70-71˚C.

Example 196

{3-[2-((R)-2-methylpyrrolidin-1-yl)ethyl]-1-[3-((S) -2-methylpyrrolidin-1-yl)propyl]imidazolidin-2-ylidene} malononitrile (Compound 196)

In a similar manner to Step 3 of Example 195, the titled compound (0.13 g, 22.2 %) was obtained as a white solid by using {3-(2-methanesulfonyloxyethyl)-1-[3-((S)-2-methylpyrrolidin-1-yl)propyl]imidazolidin-2-ylidene} malononitrile (0.58 g, 1.52 mmol) and (R)-2-methylpyrrolidine hydrobromate (0.38 g, 2.28 mmol) in place of piperidine.
$^1H$ NMR ($CDCl_3$, δppm) : 1.07 (d, J = 5.9 Hz, 3H), 1.08 (d, J=5.9 Hz, 3H), 1.25-1.46 (m, 2H), 1.66-1.79 (m, 4H), 1.82-1.97 (m, 4H), 2.10-2.17 (m, 3H), 2.20-2.43 (m, 3H), 2.80 (td, J = 8.1, 12.1 Hz, 1H), 3.05-3.18 (m, 3H), 3.42-3.70 (m, 6H), 3.77-3.87 (m, 2H).
APCIMS m/z: $[M+H]^+$371.
Melting point: 94-95˚C.

Example 197

{3-[2-((S)-2-methylpyrrolidin-1-yl)ethyl]-1-[3-((R)-2-methylpyrrolidin-1-yl)propyl]imidazolidin-2-ylidene}malononitrile (Compound 197)

In a similar manner to Step 3 of Example 194, the titled compound (0.20 g, 40.5 %) was obtained as a white solid by using {3-(2-methanesulfonyloxyethyl)-1-[3-((R)-2-methylpyrrolidin-1-yl)propyl]imidazolidin-2-ylidene} malononitrile (0.50 g, 1.32 mmol) and (S)-2-methylpyrrolidine hydrobromate (0.33 g, 1.98 mmol) in place of piperidine.
$^1H$ NMR ($CDCl_3$, δppm) : 1.07 (d, J = 6.3 Hz, 3H), 1.08 (d,J= 5.9 Hz, 3H), 1.32-1.46 (m, 2H), 1.66-1.79 (m, 4H), 1.82-1.97 (m, 4H), 2.06-2.43 (m, 6H), 2.80 (td, J = 8.1, 12.1 Hz, 1H), 3.05-3.18 (m, 3H), 3.42-3.70 (m, 6H), 3.77-3.87 (m, 2H).
APCIMS m/z: $[M+H]^+$371.
Melting point: 93-94˚C.

Example 198

{3-[2-(2-Methylpiperidino)ethyl]-1-[3-((S)-2-methylpyrrolidin-1-yl)propyl]imidazolidin-2-ylidene} malononitrile (Compound 198)

In a similar manner to Step 3 of Example 195, the titled compound (0.39 g, 9.6%) was obtained as a white solid by using {3-(2-methanesulfonyloxyethyl)-1-[3-((S)-2-methylpyrrolidin-l-yl)propyl]imidazolidin-2-ylidene} malononitrile (0.40 g, 1.05 mmol) and 2-methylpiperidine (0.21 g, 2.10 mmol) in place of piperidine.
$^1H$ NMR ($CDCl_3$, δppm) : 1.05 (d, J = 6.3 Hz, 3H), 1.08 (d, J= 6.1 Hz, 3H), 1.22-1.49 (m, 4H), 1.61-1.77 (m, 8H), 1.80-1.98 (m, 3H), 2.12-2.35 (m, 3H), 2.38-2.48 (m, 2H), 2.75-2.87 (m, 1H), 2.98-3.11 (m, 1H), 3.47-3.85 (m, 8H).
APCIMS m/z: $[M+H]^+$385.

Melting point: 88-89˚C.

Example 199

{3-[2-(3-Methylpiperidino)ethyl]-1-[3-((S)-2-methylpyrrolidin-1-yl)propyl]imidazolidin-2-ylidene} malononitrile (Compound 199)

In a similar manner to Step 3 of Example 195, the titled compound (0.009 g, 9.8 %) was obtained as a white solid by using {3-(2-methanesulfonyloxyethyl)-1-[3-((S)-2-methylpyrrolidin-1-yl)propyl]imidazolidin-2-ylidene} malononitrile (0.09 g, 0.24 mmol) and 3-methylpiperidine (0.06 mL, 0.48 mmol) in place of piperidine.
$^1$H NMR ($CDCl_3$, δppm) : 0.88 (d, J = 6.2 Hz, 3H), 1.08 (d, J=5.9 Hz, 3H), 1.35-1.51 (m, 2H), 1.53-1.77 (m, 6H), 1.84-1.99 (m, 5H), 2.00-2.18 (m, 2H), 2.23-2.33 (m, 1H), 2.60 (t, J = 6.3 Hz, 2H), 2.75-2.85 (m, 3H), 3.13 (dt, J = 3.0, 8.3 Hz, 1H), 3.50-3.76 (m, 8H).
APCIMS m/z: $[M+H]^+$385.

Example 200

{1-[3-(2-Methylpyrrolidin-1-yl)propyl]-3-(2-piperidinoethyl)benzimidazolidin-2-ylidene}malononitrile (Compound 200)

(Step 1)

(Benzimidazolidin-2-ylidene)malononitrile (0.690 g, 3.79 mmol) obtained in Step 1 of Example 124 was dissolved in DMF (10 mL) and the solution was added with (3-bromopropoxy)-tert-butyldimethylsilane (0.878 mL, 3.79 mmol) and potassium carbonate (1.05 g, 7.58 mmol), followed by stirring at 60˚C for 1 hour. Further, the mixture was added with (3-bromopropoxy)-tert-butyldimethylsilane (0.440 mL, 1.90 mmol) and stirred at room temperature for 12 hours. The solvent was evaporated under reduced pressure and the residue was added with water. Then, the precipitated solid was collected by filtration. The obtained solid was dissolved in chloroform and the insoluble matter was filtered. Then, the mixture was purified by silica gel column chromatography (n-hexane/ethyl acetate (1:1)) to obtain {1-[3-(tert-butyldimethylsilyloxy)propyl]benzimidazolidin-2-ylidene}malononitrile (0.298 g, 22.2 %) as a white solid.
$^1$H NMR ($CDCl_3$, δppm) : 0.07 (s, 6H), 0.93 (s, 9H), 2.05-2.14 (m, 2H), 3.71 (t, J = 5.4 Hz, 2H), 4.46 (t, J = 7.2 Hz, 2H), 7.23-7.38 (m, 4H),10.49 (br s, 1H).

(Step 2)

{1-[3-(Tert-butyldimethylsilyloxy)propyl] benzimidazolidin-2-ylidene}malononitrile (100 mg, 0.282mmol) obtained in the Step 1, triphenylphosphine (110 mg, 0.419 mmol) and 2-piperidinoethanol (0.0560 mL, 0.422 mmol) were dissolved in THF (2 mL) and the solution was added with 40% toluene solution of diethylazodicarboxylate (0.183 mL, 0.420 mmol) under ice bath, followed by stirring at room temperature for 10 minutes. The mixture was purified by preparative TLC (n-hexane/ethyl acetate (2:1)) to obtain {1-[3-(tert-butyldimethylsilyloxy)propyl]-3-(2-piperidinoethyl) benzimidazolidin-2-ylidene}malononitrile (122 mg, 92.8 %) as a white solid.
$^1$H NMR ($CDCl_3$, δppm) : 0.08 (s, 6H), 0.93 (s, 9H), 1.38-1.46 (m, 2H), 1.47-1.57 (m, 4H), 2.09-2.17 (m, 2H), 2.50 (br s, 4H), 2.80 (t, J = 6.8Hz, 2H), 3.71 (t, J = 5.5 Hz, 2H), 4.47 (t, J = 7.3 Hz, 4H), 7.30-7.46 (m, 4H).

(Step 3)

{1-[3-(Tert-butyldimethylsilyloxy)propyl]-3-(2-piperidinoethyl)benzimidazolidin-2-ylidene}malononitrile (0.176 g, 0.377 mmol) obtained in the Step 2 was dissolved in ethyl acetate (2 mL) and the solution was added with 4 mol/L hydrochloric acid-ethyl acetate (0.238 mL, 0.952 mmol), followed by stirring at room temperature for 1 hour. The precipitated solid was collected by filtration and washed with ethyl acetate. The obtained solid was added with saturated aqueous sodium hydrogencarbonate solution and the mixture was extracted with chloroform. The organic layer was dried over anhydrous magnesium sulfate and the solvent was evaporated under reduced pressure to obtain [1-(3-hydroxypropyl)-3-(2-piperidinoethyl)benzimidazolidin -2-ylidene]malononitrile (109 mg, 82.0 %) as a white solid.
$^1$H NMR ($CDCl_3$, δppm): 1.36-1.62 (m, 6H), 2.15-2.24 (m,2H),2.50 (br s, 4H), 2.80 (br s, 2H), 3.80 (t, J = 5.5 Hz, 2H), 4.43-4.54 (m, 4H), 7.31-7.45 (m, 4H).

(Step 4)

[1-(3-Hydroxypropyl)-3-(2-piperidinoethyl) benzimidazolidin-2-ylidene]malononitrile (108 mg, 0.307mmol) obtained in the Step 3 was dissolved in dichloromethane (2 mL) and the solution was added with triethylamine (0.0700 mL, 0.502 mmol) and methanesulfonylchloride (0.150 mL, 1.89 mmol) under ice-cooling, followed by stirring at room temperature for 1 hour. Further, the mixtrure was added with triethylamine (0.190 mL, 1.35 mmol) and methanesulfonylchloride (0.0290 mL, 0.375 mmol) and stirred at room temperature for 20 minutes. The mixture was added with saturated brine and extracted with ethyl acetate. The organic layer was washed with saturated brine, dried over anhydrous magnesium sulfate and the solvent was evaporated under reduced pressure to obtain clear crystal (59.9 mg).

The obtained crystal was dissoved in 1,4-dioxane (1.5 mL) and the solution was added with 2-methylpyrrolidine (0.0300 mL, 0.282 mmol) and potassium carbonate (0.0390 g, 0.282 mmol), followed by stirring at 60°C for 1 hour. Further, the mixture was added with 2-methylpyrrolidine (0.0300 mL, 0.282 mmol) and potassium carbonate (0.0390 g, 0.282 mmol), followed by stirring at 60°C for 6 hours. The mixture was added with saturated brine and extracted with ethyl acetate. The organic layer was washed with saturated brine, dried over anhydrous magnesium sulfate and the solvent was evaporated under reduced pressure. The residue was washed with diisopropylether to obtain the titled compound (0.0303 g, 23.6 %) as a white solid.
$^1$H NMR ($CDCl_3$, δppm) : 1.06 (d, J = 6.2 Hz, 3H), 1.36-1.60 (m, 6H), 1.60-1.85 (m, 3H), 1.87-2.00 (m, 1H), 2.06-2.41 (m, 5H), 2.50 (t, J =5.0 Hz, 4H), 2.79 (t, J = 7.0 Hz, 2H), 2.84-2.95 (m, 1H), 3.10-3.22 (m, 1H), 4.41 (t, J = 8.2 Hz, 2H), 4.47 (t, J = 7.0 Hz, 2H), 7.30-7.43 (m, 4H).
Melting point: 85-86°C.
APCIMS m/z: $[M+H]^+$ 419.

Example 201

{3-(3-Morpholinopropyl)-1-[3-((S)-2-methylpyrrolidin-1-yl)propyl]imidazolidin-2-ylidene} malononitrile (Compound 201)

In a similar manner to Example 116, the titled compound (0.10 g, 37.4 %) was obtained as a white solid by using [1-(3-bromopropyl)-3-(3-hydroxypropyl) imidazolidin-2-ylidene]malononitrile (0.50 g, 1.60 mmol) of Step 1 of Example 65, (S)-2-methylpyrrolidine hydrobromate (0.51 g, 3.06 mmol) in place of (R)-2-methylpyrrolidine hydrobromate of Step 1 and morpholine (0.12 mL, 1.42 mmol) in place of (R)-2-methylpyrrolidine hydrobromate of Step 3.
$^1$H NMR ($CDCl_3$, δppm) : 1.07 (d, J = 6.0 Hz, 3H), 1.33-1.46 (m, 1H), 1.67-1.88 (m, 3H), 1.80-1.98 (m, 4H), 2.05-2.19 (m, 2H), 2.25-2.33 (m, 1H), 2.37-2.46 (m, 6H), 2.79 (td, J = 7.9, 11.9 Hz, 1H), 3.12 (td, J = 3.0, 6.3Hz, 1H), 3.48-3.72 (m, 12H).
APCIMS m/z: $[M+H]^+$387.
Melting point: 72-73°C.

Example 202

{1-[3-((S)-2-methylpyrrolidin-1-yl)propyl]-3-[3-(thiomorpholin-1-yl)propyl]imidazolidin-2-ylidene} malononitrile (Compound 202)

In a similar manner to Example 116, the titled compound (0.008 g, 6.9 %) was obtained as a clear and colorless oily substance by using [1-(3-bromopropyl)-3-(3-hydroxypropyl)imidazolidin-2-ylidene]malononitrile (0.25 g, 0.80 mmol) of Step 1 of Example 65, (S)-2-methylpyrrolidine hydrobromate in place of (R)-2-methylpyrrolidine hydrobromate of Step 1 and thiomorpholine (0.06 mL, 0.60 mmol) in place of (R)-2-methylpyrrolidine hydrobromate of Step 3.
$^1$H NMR ($CDCl_3$, δppm): 1.08 (d, J = 6.1 Hz, 3H), 1.40-1.47 (m, 1H), 1.66-1.73 (m, 2H), 1.81-1.97 (m, 5H), 2.10-2.17 (m, 2H), 2.33-2.46 (m, 7H), 2.79 (td, J = 7.9, 11.9 Hz, 1H), 3.11-3.16 (m, 1H), 3.51-3.72 (m, 12H).
APCIMS m/z: $[M+H]^+$ 403.

Example 203

{1-[3-(2-Methylpyrrolidin-1-yl)propyl]-3-[3-(4-oxopiperidino)propyl]imidazolidin-2-ylidene}malononitrile (Compound 203)

4-Piperidone hydrochloride monohydrate (0.0759 g, 0.494 mmol) was dissolved in 1,4-dioxane (2.0 mL) and the solution was added with 2 mol/L aqueous sodium hydroxide solution (0.304 mL, 0.608 mmol), followed by stirring for 1 minute. The mixture was added with {1-(3-methanesulfonyloxypropyl)-3-[3-(2-methylpyrrolidin-1-yl) propyl]imidazolidin-2-ylidene}malononitrile (0.150 g, 0.379 mmol) obtained in Step 1 of Example 119 and potassium carbonate (0.105 g, 0.760 mmol), followed by stirring at 70°C for 17 hours. The mixture was added with water and extracted with ethyl acetate. The organic layer was washed with saturated brine and dried over anhydrous magnesium sulfate. Then, the solvent was evaporated under reduced pressure and purified by preparative TLC (chloroform/(2 mol/L ammonia-methanol) (10/1)) to obtain the titled compound (0.0711 g, 46.9 %).

$^1$H NMR ($CDCl_3$, δppm) : 1.10 (d, J = 6.0 Hz, 3H), 1.68-1.74 (m, 8H), 1.87-1.97 (m, 4H), 2.14-2.23 (m, 2H), 2.44-2.55 (m, 4H), 2.73-2.78 (m, 4H), 3.12-3.19 (m, 1H), 3.49-3.70 (m, 8H).
APCIMS m/z: $[M+H]^+$399.

Example 204

{1-[3-(2-Methylpyrrolidin-1-yl)propyl]-3-(3-thiazolidin-3-ylpropyl)imidazolidin-2-ylidene} malononitrile (Compound 204)

{1-(3-Methanesulfonyloxypropyl)-3-[3-(2-methylpyrrolidin-1-yl)propyllimidazolidin-2-ylidenel malononitrile (0.150 g, 0.379 mmol) obtained in Step 1 of Example 119 was dissolved in 1,4-dioxane (2.0 mL) and the solution was added with thiazolidine (0.0449 mL, 0.570 mmol) and potassium carbonate (0.105 g, 0.760 mmol), followed by stirring at room temperature for 2 hours, then at 50˚C for 3.5 hours, further at 70˚C for 4 hours. The mixture was added with DMF (1.5 mL) and stirred at 70˚C overnight. After 1,4-dioxane in the mixture was evaporated under reduced pressure, thiazolidine (0.0449 mL, 0.570 mmol) and potassium carbonate (0.0788 g, 0.570 mmol) were added thereto and stirred for one day. The mixture was added with water and extracted with ethyl acetate. The organic layer was washed with saturated brine and dried over anhydrous magnesium sulfate. Then, the solvent was evaporated under reduced pressure and purified by preparative thin-layer chromatography (chloroform/(2 mol/L ammonia-methanol) (10:1)) to obtain the titled compound (0.0252 g, 17.1 %).
$^1$H NMR ($CDCl_3$, δppm) : 1.07 (d, J = 6.3 Hz, 3H), 1.33-1.46 (m, 1H), 1.62-1.77 (m, 2H), 1.81-1.98 (m, 4H), 2.05-2.17 (m, 4H), 2.24-2.35 (m, 1H), 2.44-2.48 (m, 2H), 2.75-2.90 (m, 2H), 3.04-3.16 (m, 2H), 3.49-3.77 (m, 9H), 4.04 (s, 2H).
APCIMS m/z: $[M+H]^+$389.

Example 205

{1,3-Bis[3-((R)-2-methylpyrrolidin-1-yl)propyl] benzimidazolidin-2-ylidene}malononitrile (Compound 205)

In a similar manner to Step 3 of Example 12.5, the titled compound (0.141 g, 9.3 %) was obtained as a white solid by using [1,3-bis(3-hydroxypropyl)benzimidazolidin-2-ylidene]malononitrile (1.04 g, 3.49 mmol) and (R)-2-methylpyrrolidine hydrochloride (0.323 g, 1.94 mmol) in place of (S)-2-methylpyrrolidine hydrobromate.
$^1$H NMR ($CDCl_3$, δppm) : 1.02 (d, J = 5.9 Hz, 6H), 1.32-1.46 (m, 2H), 1.65-1.80 (m, 4H), 1.85-1.98 (m, 2H), 2.02-2.34 (m, 10H), 2.85 (dt, J= 7.8, 12.4 Hz, 2H), 3.12 (dt, J = 3.4, 8.3 Hz, 2H), 4.42 (t, J =7.6 Hz, 4H), 7.29-7.39 (m, 4H).
Melting point: 96-97˚C.
APCIMS m/z: $[M+H]^+$433.

Example 206

[1-(1-Benzylpiperidin-4-yl)-3-(2-piperidinoethyl) imidazolidin-2-ylidene]malononitrile (Compound 206)

(Step 1)

1-(1-Benzylpiperidin-4-yl)imidazolidin-2-ylidene] malononitrile (0.700 g, 2.3 mmol) obtained in Step 2 of Example 126 was dissolved in DMF (7 mL) and the solution was added with potassium carbonate (0.627 g, 4.5 mmol) and 1,2-dibromoethane (0.991 mL, 11.5 mmol). After stirring at room temperature for 90 hours, the mixture was further added with potassium carbonate (0.627 g, 4.5 mmol) and 1,2-dibromoethane (0.991 mL, 11.5 mmol), followed by stirring at room temperature for 24 hours. The mixture was added with water and extracted with ethyl acetate. The organic layer was washed with saturated brine, dried over anhydrous magnesium sulfate and the solvent was evaporated under reduced pressure. The obtained crude crystal was recrystallized from acetone-diisopropylether to obtain [1-(1-benzylpiperidin-4-yl)-3-(2-bromoethyl) imidazolidin-2-ylidene]malononitrile (0.556 g, 58.9 %) as a white solid.
$^1$H NMR ($CDCl_3$, δppm): 1.77-1.90 (m, 4H), 2.18-2.28 (m, 2H), 2.94-3.08 (m, 2H), 3.54 (s, 2H), 3.57-3.70 (m, 4H), 3.71-3.84 (m, 2H), 3.98 (t, J = 5.87 Hz, 2H), 4.17-4.32 (m, 1H), 7.23-7.37 (m, 5H).

(Step 2)

[1-(1-Benzylpiperidin-4-yl)-3-(2-bromoethyl) imidazolidin-2-ylidene]malononitrile (0.270 g, 0.652 mmol) obtained in the Step 1 was dissolved in 1,4-dioxane (10 mL) and the mixture was added with piperidine (0.323 mL, 3.26 mmol), followed by stirring at 80˚C for 5.5 hours. Further, the mixture was added with piperidine (0.129 mL, 1.30 mmol) and stirred at 80˚C for 1.5 hours. The mixture was added with water and extracted with ethyl acetate. The organic layer was washed with saturated brine, dried over anhydrous magnesium sulfate and the solvent was evaporated under

reduced pressure. The residue was purified by silica gel column chromatography (chloroform/(2 mol/L ammonia-methanol) (100:10)), recrystallized from acetone-diisopropylether to obtain the titled compound (0.195 g, 71.5 %) .
$^1$H NMR ($CDCl_3$, δppm) : 1.38-1.65 (m, 6H), 1.70-1.86 (m, 4H), 2.08-2.22 (m, 2H), 2.30-2.50 (m, 4H), 2.60 (t, J = 6.2 Hz, 2H), 2.90-3.02 (m, 2H), 3.43-3.71 (m, 8H), 4.10-4.26 (m, 1H), 7.18-7.36 (m, 5H).

Example 207

[1-(1-Isopropylpiperidin-4-yl)-3-(2-piperidinoethyl) imidazolidin-2-ylidene]malononitrile (Compound 207)

[1-(1-Benzylpiperidin-4-yl)-3-(2-piperidinoethyl) imidazolidin-2-ylidene]malononitrile (Compound 206) (0.190 g, 0.454 mmol) obtained in Example 206 was dissolved in 1,2-dichloroethane (20 mL) and the solution was added with 1-chloroethyl chloroformate (0.0215 mL, 0.068 mmol). After heating under reflux for 2 hours, the mixture was further added with 1-chloroethyl chloroformate (0.0215 mL, 0.068 mmol) and heated under reflux for 1 hour. The mixture was further added with 1-chloroethyl chloroformate (0.0100 mL, 0.032 mmol), heated under reflux for 0.5 hour and concentrated under reduced pressure. The residue was added with methanol (20 mL) and heated under reflux for 1.5 hours. The mixture was concentrated under reduced pressure and the residue was crystallized from 2-propanol to obtain a white solid (0.115 g).

The obtained white solid (0.110 g) was dissolved in DMF (3 mL) and the solution was added with potassium carbonate (0.227 g, 1.64 mmol) and isopropyl iodide (0.0790 mL, 0.81 mmol). After stirring at room temperature for 66 hours, the mixture was further added with isopropyl iodide (0.0790 mL, 0.81 mmol) and stirred at room temperature for 47 hours. The mixture was added with water and extracted with ethyl acetate. The organic layer was washed with saturated brine, dried over anhydrous magnesium sulfate and the solvent was evaporated under reduced pressure. After purifying by preparative TLC (chloroform/(2 mol/L ammonia-methanol) (9:1) and then by chloroform/ (2 mol/L ammonia-methanol) (20:1) 2 times), the residue was purified by silica gel column chromatography (chloroform/(2 mol/L ammonia-methanol) (99.9:0.1 to 90:10)) and further purified by preparative TLC (chloroform/methanol (4:1)). The obtained solid was washed with diisopropylether to obtain the titled compound (0.0140 g, 13.8 %).
$^1$H NMR ($CDCl_3$, δppm) : 1.04 (d, J = 6.6 Hz, 6H), 1.38-1.95 (m, 10H), 2.24-2.50 (m, 6H), 2.53-2.65 (m, 2H), 2.70-2.82 (m, 1H), 2.90-3.00 (m, 2H), 3.50-3.72 (m, 6H), 4.10-4.22 (m, 1H).
Melting point: 93-95˚C.

Example 208

{1-(1-Benzylpiperidin-4-yl)-3-[((S)-2-methylpyrrolidin-1-yl)ethyl]imidazolidin-2-ylidene} malononitrile (Compound 208)

(Step 1)

[1-(1-Benzylpiperidin-4-yl)-3-(2-bromoethyl) imidazolidin-2-ylidene]malononitrile (0.140 g, 0.34 mmol) obtained in Step 1 of Example 206 was dissolved in 1,4-dioxane (2 mL) and the mixture was added with potassium carbonate (0.141 g, 1.02 mmol). The mixture was further added with a solution of (S)-2-methylpyrrolidine hydrobromate (0.101 g, 0.61 mmol) in a mixed solvent of 2 mol/L aqueous sodium hydroxide solution (0.320 ml, 0.64 mmol) and 1,4-dioxane (2 mL). The mixture was stirred at 60˚C for 16 hours and then at 80˚C for 24 hours. Next, (S)-2-methylpyrrolidine hydrobromate (0.0170 g, 0.10 mmol) was added with a solution obtained by mixing 2 mol/L aqueous sodium hydroxide solution (0.0550 ml, 0.11 mmol) and 1,4-dioxane (1 mL) and stirred at 90˚C for 7 hours. After evaporating the solvent of the mixture, water was added and the mixture was extracted with ethyl acetate. The organic layer was washed with saturated brine, dried over anhydrous magnesium sulfate and the solvent was evaporated under reduced pressure. The residue was purified by silica gel column chromatography (chloroform/ (2 mol/L ammonia-methanol) (100:10)) to obtain the titled compound (0.119 g, 84.1 %).
$^1$H NMR ($CDCl_3$, δppm) : 1.06 (d, J = 6.1 Hz, 3H), 1.28-1.44 (m, 1H), 1.65-2.00 (m, 7H), 2.09-2.29 (m, 3H), 2.32-2.46 (m, 2H), 2.91-3.02 (m, 2H), 3.02-3.20 (m, 2H), 3.42-3.67 (m, 6H), 3.71-3.90 (m, 2H), 4.12-4.28 (m, 1H), 7.23-7.36 (m, 5H).

Example 209

(3-[((S)-2-methylpyrrolidin-1-yl)ethyl]-1-(piperidin-4-yl)imidazolidin-2-ylidene}malononitrile (Compound 209)

(Step 2)

{1-(1-Benzylpiperidin-4-yl)-3-[((S)-2-methylpyrrolidin-1-yl)ethyl]imidazolidin-2-ylidene} malononitrile (Compound 208) (0.119 g, 0.284 mmol) obtained in Example 208 was dissolved in 1,2-dichloroethane (10 mL) and the solution

was added with 1-chloroethyl chloroformate (0.0450 mL, 0.417 mmol). After heating under reflux for 1 hour, the mixture was concentrated under reduced pressure and the residue was added with methanol (20 mL), followed by heating under reflux for 1.5 hours. The mixture was concentrated under reduced pressure and the residue alkalified by adding methanol (3 mL) and 1 mol/L aqueous sodium hydroxide solution. Then, the solvent was evaporated under reduced pressure. The residue was extracted with chloroform and the organic layer was dried over anhydrous magnesium sulfate. Then, the solvent was evaporated under reduced pressure and the mixture was purified by silica gel column chromatography (chloroform/(2 mol/L ammonia-methanol) (100:10)) and then by preparative TLC (chloroform/(2 mol/L ammonia-methanol) (10:1)) to obtain the titled compound (0.0610 g, 65.3 %).
$^1$H NMR ($CDCl_3$, δppm) : 1.06 (d, J = 6.0 Hz, 3H), 1.31-1.45 (m, 1H), 1.60-2.00 (m, 7H), 2.16-2.29 (m, 1H), 2.33-2.46 (m, 2H), 2.70-2.83 (m, 2H), 3.03-3.22 (m, 4H), 3.42-3.70 (m, 4H), 3.73-3.90 (m, 2H), 4.20-4.35 (m,1H).

Example 210

{1-(1-Isopropylpiperidin-4-yl)-3-[2-((S)-2-methylpyrrolidin-1-yl)ethyl]imidazolidin-2-ylidene} malononitrile (Compound 210)

{3-[((S)-2-methylpyrrolidin-1-yl)ethyl]-1-(piperidin -4-yl)imidazolidin-2-ylidene}malononitrile (Compound 209) (0.0610 g, 0.186 mmol) obtained in Example 209 was dissolved in DMF (2 mL) and the solution was added with potassium carbonate (0.154 g, 1.11 mmol) and isopropyl iodide (0.0560 mL, 0.571 mmol). After stirring at room temperature for 20 hours and then at 60˚C for 5.5 hours, the mixture was further added with isopropyl iodide (0.0200 mL, 0.204 mmol) and potassium carbonate (0.0400 g, 0.289 mmol) followed by stirring for 1.5 hour. The mixture was added with water and extracted with ethyl acetate. The organic layer was washed with saturated brine, dried over anhydrous magnesium sulfate and the solvent was evaporated under reduced pressure. The residue was purified by preparative TLC (chloroform/(2 mol/L ammonia-methanol) (6:1)) to obtain the titled compound (0.0106 g, 15.4%).
$^1$H NMR ($CDCl_3$, δppm) : 1.04-1.17 (m, 9H), 1.31-1.47 (m, 1H), 1.68-1.83 (m, 2H), 1.83-2.10 (m, 5H), 2.20-2.32 (m, 1H), 2.35-2.52 (m, 4H), 2.83-2.96 (m, 1H), 3.00-3.22 (m, 4H), 3.46-3.90 (m, 6H), 4.15-4.30 (m, 1H).

Example 211

{1-(1-Benzylpiperidin-4-yl)-3-[((R)-2-methylpyrrolidin-1-yl)ethyl]imidazolidin-2-ylidene} malononitrile (Compound 211)

(Step 1)

[1-(1-Benzylpiperidin-4-yl)-3-(2-bromoethyl) imidazolidin-2-ylidene]malononitrile (0.140 g, 0.34 mmol) obtained in Step 1 of Example 206 was added with 1,4-dioxane (2 mL). The mixture was further added with potassium carbonate (0.141 g, 1.02 mmol) and a solution of (R)-2-methylpyrrolidine hydrobromate (0.101 g, 0.61 mmol) in a mixed solvent of 2 mol/L aqueous sodium hydroxide solution (0.320 mL, 0.64 mmol) and 1,4-dioxane (1 mL), followed by stirring at 80˚C for 2 hours and then at 90˚C for 1 hour. Next, a solution of (R)-2-methylpyrrolidine hydrobromate (0.0170 g, 0.10 mmol) in a mixed solvent of 2 mol/L aqueous sodium hydroxide solution (0.068 mL, 0.14 mmol) and 1,4-dioxane (1 mL) was added to the mixture and stirred at 90˚C for 20 hours. The solvent of the mixture was evaporated and extracted with ethyl acetate. The organic layer was dried over anhydrous magnesium sulfate and the solvent was evaporated under reduced pressure. The residue was purified by silica gel column chromatography (chloroform/(2 mol/L ammonia-methanol) (100:10)) to obtain the titled compound (0.140 g, 98.4 %).
$^1$H NMR ($CDCl_3$, δppm) : 1.06 (d, J = 6.1 Hz, 3H), 1.28-1.44 (m, 1H), 1.65-2.00 (m, 7H), 2.09-2.29 (m, 3H), 2.32-2.46 (m, 2H), 2.91-3.02 (m, 2H), 3.02-3.20 (m, 2H), 3.42-3.67 (m, 6H), 3.71-3.90 (m, 2H), 4.12-4.28 (m, 1H), 7.23-7.36 (m, 5H).

Example 212

{3-[((R)-2-methylpyrrolidin-1-yl)ethyl]-1-(piperidin-4-yl)imidazolidin-2-ylidene}malononitrile (Compound 212)

{1-(1-Benzylpiperidin-4-yl)-3-[((R)-2-methylpyrrolidin-1-yl)ethyl]imidazolidin-2-ylidene} malononitrile (Compound 211) (0.140 g, 0.334 mmol) obtained in Example 211 was dissolved in 1,2-dichloroethane (10 mL) and the solution was added with 1-chloroethyl chloroformate (0.0540 mL, 0.500 mmol). After refluxed for 1 hour, the mixture was concentrated under reduced pressure and the residue was added with methanol (10 mL) and refluxed for 0.5 hour. The mixture was concentrated under reduced pressure and the residue was purified by NH silica gel column chromatography (chloroform/methanol (100:10)) and further by preparative TLC (chloroform/(2 mol/L ammonia-methanol) (10:1)) to obtain the titled compound (0.0690 g, 62.8 %).
$^1$H NMR ($CD_3OD$, δppm) : 1.06 (d, J = 6.0 Hz, 3H), 1.31-1.45 (m, 1H), 1.60-2.00 (m, 7H), 2.22-2.50 (m, 3H), 2.66-2.80

(m, 2H), 3.08-3.17 (m, 4H), 3.53-3.80 (m, 6H), 4.17-4.30 (m, 1H).

Example 213

{1-(1-Isopropylpiperidin-4-yl)-3-[2-((R)-2-methylpyrrolidin-1-yl)ethyl]imidazolidin-2-ylidene} malononitrile (Compound 213)

{3-[((R)-2-methylpyrrolidin-1-yl)ethyl]-1-(piperidin -4-yl)imidazolidin-2-ylidene}malononitrile (Compound 212) (0.0690 g, 0.21 mmol) obtained in Example 212 was dissolved in DMF (2 mL) and the solution was added with potassium carbonate (0.174 g, 1.26 mmol) and isopropyl iodide (0.0620 mL, 0.63 mmol), followed by stirring at 50˚C for 5 hours. The mixture was added with water and extracted with ethyl acetate. The organic layer was washed with saturated brine, dried over anhydrous magnesium sulfate and the solvent was evaporated under reduced pressure. The residue was purified by preparative TLC (chloroform/methanol (10:1) and then by chloroform/(2 mol/L ammonia-methanol) (15:1), and further by chloroform/methanol (6:1)) to obtain the titled compound (0.0150 g, 19.2 %).
$^1$H NMR ($CDCl_3$, δppm) : 1.00-1.10 (m, 9H), 1.30-1.42 (m, 1H), 1.60-2.00 (m, 7H), 2.15-2.45 (m, 5H), 2.74-2.81 (m, 1H), 2.90-3.00 (m, 2H),3.02-3.20 (m, 2H), 3.42-3.70 (m, 4H), 3.72-3.88 (m, 2H); 3.98-4.24 (m,1H).

Example 214

[1-(1-Cyanomethylpiperidin-4-yl)-3-(3-piperidinopropyl)imidazolidin-2-ylidene]malononitrile (Compound 214)

A dihydrochloride of [1-(piperidin-4-yl)-3-(3-piperidinopropyl)imidazolidin-2-ylidene]malononitrile (0.097 g, 0.234 mmol) obtained in Example 128 was dissolved in DMF (2 mL) and the solution was added with iodoacetonitrile (0.0500 mL, 0.691 mmol). After stirring at room temperature for 64 hours, the mixture was added with water and extracted with ethyl acetate. The organic layer was washed with saturated brine, dried over anhydrous magnesium sulfate and the solvent was evaporated under reduced pressure. The residue was purified by silica gel column chromatography (chloroform/(2 mol/L ammonia-methanol) (100:20)) and by preparative TLC (chloroform/(2 mol/L ammonia-methanol) (15:1)). Then, the residue was recrystallized from isopropanol to obtain the titled compound (0.0155 g, 17.4 %).
$^1$H NMR ($CDCl_3$, δppm) : 1.40-1.50 (m, 2H), 1.50-2.00 (m, 10H), 2.32-2.56 (m, 8H), 2.90-3.00 (m, 2H), 3.46 (s, 2H), 3.50-3.70 (m, 6H), 4.15-4.32 (m, 1H).
Melting point: 135-136˚C.

Example 215

{1-[1-(2-Fluoroethyl)piperidin-4-yl]-3-(3-piperidinopropyl)imidazolidin-2-ylidene}malononitrile (Compound 215)

A dihydrochloride of 1-(Piperidin-4-yl)-3-(3-piperidinopropyl)imidazolidin-2-ylidene]malononitrile (0.097 g, 0.23 mmol) obtained in Example 128 was dissolved in DMF (2 mL) and the solution was added with potassium carbonate (0.190 g, 1.38 mmol) and 1-bromo-2-fluoroethane (0.051 mL, 0.69 mmol). After stirring at room temperature for 64 hours, the mixture was added with water and extracted with ethyl acetate. The organic layer was washed with saturated brine, dried over anhydrous magnesium sulfate and the solvent was evaporated under reduced pressure. The residue was purified by silica gel column chromatography (chloroform/(2 mol/L ammonia-methanol) (100:10)) and recrystallized from diisopropylether to obtain the titled compound (0.017 g, 19 %).
$^1$H NMR ($CDCl_3$, δppm) : 1.38-1.50 (m, 2H), 1.50-1.93 (m, 10H), 2.20-2.45 (m, 8H), 2.70 (dt, J = 28.4, 5. 0 Hz, 2H), 3.00-3.10 (m, 2H), 3.50-3.67 (m, 6H), 4.13-4.28 (m, 1H), 4.54 (dt, J = 47.5, 5.0 Hz, 2H).
Melting point: 56-57˚C.

Example 216

[1-(1-Isopropylpiperidin-4-yl)-3-(3-morpholinopropyl)imidazolidin-2-ylidene]malononitrile (Compound 216)

(Step 1)

[1-(1-Benzylpiperidin-4-yl)imidazolidin-2-ylidene] malononitrile (9.00 g, 29.3 mmol) obtained in Step 2 of Example 126 was dissolved in DMF (30mL) and the solution was added with potassium carbonate (8.10 g, 58.6 mmol) and (3-bromopropoxy)-tert-butyldimethylsilane (8.15 mL, 35.2 mmol), followed by stirring at room temperature for 2 hours and further at 50˚C for 17.5 hours. The mixture was added with saturated brine and extracted with ethyl acetate. The organic layer was washed with saturated brine, dried over anhydrous magnesium sulfate and the solvent was

evaporated under reduced pressure. The residue was washed with diisopropylether to obtain {1- (1-benzylpiperidin-4-yl)-3-[3-(tert-butyldimethylsilyloxy) propyl]-2-ylidene}malononitrile (12.3 g, 90.3 %) as a white solid.
$^1$H NMR ($CDCl_3$, δppm) : 0.05 (s, 6H), 0.89 (s, 9H), 1.70-1.93 (m, 6H), 2.10-2.23 (m, 2H), 2.92-3.02 (m, 2H), 3.56 (s, 2H), 3.53-3.73 (m, 8H), 4.13-4.28 (m, 1H), 7.22-7.36 (m, 5H).

(Step 2)

{1-(1-Benzylpiperidin-4-yl)-3-[3-(tert-butyldimethylsilyloxy)propyl]-2-ylidene}malononitrile (0.500 g, 1.02 mmol) obtained in the Step 1 was dissolved in 1,2-dichloroethane (5 mL) and the solution was added with 1-chloroethyl chloroformate (0.275 mL, 2.55 mmol). After refluxed for 1 hour, the mixture was concentrated under reduced pressure. The residue was added with methanol (5 mL) and refluxed for 0.7 hour. The mixture was concentrated under reduced pressure and the residue was washed with ethyl acetate to obtain [1-(3-hydroxypropyl)-3-(piperidin-4-yl)imidazolidin-2-ylidene] malononitrile hydrochloride (0.295 g, 92.7 %) as a white solid.
$^1$H NMR ($CD_3OD$, δppm) : 1.84-2.15 (m, 6H), 3.04-3.15 (m, 2H), 3.45-3.58 (m, 2H), 3.61-3.80 (m, 8H), 4.33-4.45 (m, 1H).

(Step 3)

[1-(3-Hydroxypropyl)-3-(piperidin-4-yl)imidazolidin-2-ylidene]malononitrile hydrochloride (6.36 g, 20.4 mmol) obtained in the Step 2 was dissolved in DMF (50 ml) and the solution was added with potassium carbonate (14.1 g, 102 mmol) and propyl iodide (6.1 ml, 61.2 mmol), followed by stirring at 60˚C for 23 hours. The mixture was added with saturated brine and extracted with ethyl acetate. The organic layer was washed with saturated brine, dried over anhydrous magnesium sulfate and the solvent was evaporated under reduced pressure. The residue was purified by NH silica gel column chromatography (chloroform/methanol (9:1)) to obtain [1-(3-hydroxypropyl) -3-(1-isopropylpiperidin-4-yl)imidazolidin-2-ylidene] malononitrile (1.96 g, 30.3 %) as a pale yellow solid.
$^1$H NMR ($CDCl_3$, δppm) : 1.05 (d, J = 6.6 Hz, 6H), 1.72-1.98 (m, 6H), 2.28-2.35 (m, 2H), 2.73-2.81 (m, 1H), 2.97 (brd, J = 11.9 Hz, 2H), 3.61 (s, 4H), 3.66-3.71 (m, 2H), 3.74 (t, J = 6.8 Hz, 2H), 4.13-4.23 (m,1H).

(Step 4)

[1-(3-Hydroxypropyl)-3-(1-isopropylpiperidin-4-yl) imidazolidin-2-ylidene]malononitrile (1.96 g, 6.19 mmol) obtained in the Step 3 was dissolved in dichloromethane (20 mL) and the solution was added with triethylamine (3.45 mL, 24.8 mmol) and methanesulfonylchloride (0.72 mL, 9.28 mmol), followed by stirring at room temperature for 1.5 hours. The mixture was added with saturated brine and extracted with ethyl acetate. The organic layer was washed with saturated brine, dried over anhydrous magnesium sulfate and the solvent was evaporated under reduced pressure. The obtained solid was washed with diisopropylether to obtain [1-(1-isopropylpiperidin-4-yl)-3-(3-methanesulfonyloxypropyl) imidazolidin-2-ylidene] malononitrile (1.34 g, 54.7 %) as a white solid.
$^1$H NMR ($CDCl_3$, δppm) : 1.06 (d, J = 6.6 Hz, 6H), 1.75-1.80 (m, 4H), 2.13-2.21 (m, 2H), 2.30-2.37 (m, 2H), 2.75-2.84 (m, 1H), 2.99 (brd, J= 11.3 Hz, 2H), 3.05 (s, 3H), 3.62 (s, 4H), 3.72 (t, J = 7.2 Hz, 2H), 4.14-4.25 (m, 1H), 4.35 (t, J = 5.9 Hz, 2H).

(Step 5)

[1-(1-Isopropylpiperidin-4-yl)-3-(3-methanesulfonyloxypropyl)imidazolidin-2-ylidene] malononitrile (1.77 g, 4.47 mmol) obtained in the Step 4 was dissolved in 1,4-dioxane (20 ml) and the solution was added with potassium carbonate (1.23 g, 8.94 mmol) and morpholine (0.78 ml, 8.94mmol), followed by stirring at 80˚C for 13 hours. The mixture was added with saturated brine and extracted with ethyl acetate. The organic layer was washed with saturated brine, dried over anhydrous magnesium sulfate and the solvent was evaporated under reduced pressure. The residue was recrystallized from n-hexane-ethyl acetate (20/1) to obtain the titled compound (1.32 g, 76.5 %) as a white solid.
$^1$H NMR ($CDCl_3$, δppm) : 1.04 (d, J = 6.6 Hz, 6H), 1.70-1.93 (m, 6H), 2.24-2.50 (m, 8H), 2.67-2.84 (m, 1H), 2.90-3.01 (m, 2H), 3.55-3.77 (m, 10H), 4.10-4.25 (m, 1H).
Melting point: 109-110˚C.

Example 217

{1-(1-Benzylpiperidin-4-yl)-3-[3-(2-methylpyrrolidin-1-yl)propyl]imidazolidin-2-ylidene} malononitrile (Compound 217)

[1-(1-Benzylpiperidin-4-yl)-3-(3-bromopropyl) imidazolidin-2-ylidene]malononitrile (1.00 g, 2.33 mmol) obtained in Step 3 of Example 126 was added with 1,4-dioxane (40 mL). The mixture was added with 2-methylpyrrolidine (1.19

mL, 11.7 mmol) and stirred at 50˚C for 19 hours and then at 80˚C for 6 hours. Further, the mixture was added with 2-methylpyrrolidine (1.19 mL, 11.7 mmol) and stirred at 80˚C for 18 hours. The mixture was added with water and extracted with ethyl acetate. The organic layer was washed with saturated brine, dried over anhydrous magnesium sulfate and the solvent was evaporated under reduced pressure. The residue was purified by silica gel column chromatography (chloroform/methanol (100:20)) to obtain the titled compound (0.830 g, 82.3 %).
$^1$H NMR ($CDCl_3$, δppm) : 1.10 (d, J = 5.9 Hz, 3H), 1.34-1.50 (m, 1H), 1.65-2.00 (m, 9H), 2.09-2.24 (m, 4H), 2.28-2.42 (m, 1H), 2.75-2.87 (m, 1H), 2.91-3.02 (m, 2H), 3.10-3.20 (m, 1H), 3.47-3.72 (m, 8H),4.15-4.27 (m, 1H), 7.22-7.35 (m, 5H).

Example 218

{3-[3-(2-Methylpyrrolidin-l-yl)propyl]-l-(piperidin -4-yl)imidazolidin-2-ylidene}malononitrile (Compound 218)

(Step 2)

{1-(1-Benzylpiperidin-4-yl)-3-[3-(2-methylpyrrolidin-1-yl)propyl]imidazolidin-2-ylidene} malononitrile (Compound 217) (0.830 g, 1.92 mmol) obtained in Example 217 was dissolved in 1,2-dichloroethane (60 mL) and the solution was added with 1-chloroethyl chloroformate (0.310 mL, 2.88 mmol). After refluxed for 3.5 hours, the mixture was further added with 1-chloroethyl chloroformate (0.207 mL, 1.92 mmol) and refluxed for 19.5 hours. The mixture was concentrated under reduced pressure and the residue was added with methanol (60 mL) and then refluxed for 1 hour. The mixture was concentrated under reduced pressure, and the residue was added with methanol. The mixture was alkalified by adding 1 mol/L aqueous sodium hydroxide solution and then the mixture was concentrated under reduced pressure. The residue was purified by silica gel column chromatography (chloroform/(2 mol/L ammonia-methanol) (4:1)) to obtain the titled compound (0.442 g, 67.2 %).
$^1$H NMR ($CDCl_3$, δppm) : 1.09 (d, J = 6.1 Hz, 3H), 1.55-2.00 (m, 10H), 2.07-2.22 (m, 2H), 2.26-2.40 (m, 1H), 2.70-2.87 (m, 3H), 3.10-3.20 (m, 3H), 3.48-3.74 (m, 6H), 4.22-4.35 (m, 1H) .

Example 219

{1-(1-Isopropylpiperidin-4-yl)-3-[3-(2-methylpyrrolidin-1-yl)propyl]imidazolidin-2-ylidene} malononitrile (Compound 219)

{3-[3-(2-Methylpyrrolidin-1-yl)propyl]-1-(piperidin-4-yl)imidazolidin-2-ylidene}malononitrile (Compound 218) (0.440 g, 1.28 mmol) obtained in Example 218 was dissolved in DMF (10 mL) and the solution was added with potassium carbonate (1.06 g, 7.68 mmol) and isopropyl iodide (0.376 mL, 3.85 mmol), followed by stirring at 50˚C for 25 hours. The mixture was added with water and extracted with ethyl acetate. The organic layer was washed with saturated brine, dried over anhydrous magnesium sulfate and the solvent was evaporated under reduced pressure. The residue was purified by silica gel column chromatography (chloroform/(2 mol/L ammonia-methanol) (5:1)) and recrystallized from acetone-diisopropylether to obtain the titled compound (0.076 g, 15.4 %).
$^1$H NMR ($CDCl_3$, δppm) : 1.00-1.10 (m, 9H), 1.32-1.48 (m, 1H), 1.62-2.00 (m, 9H), 2.06-2.20 (m, 2H), 2.20-2.40 (m, 3H), 2.69-2.85 (m, 2H), 2.90-3.00 (m, 2H), 3.05-3.15 (m, 1H), 3.45-3.73 (m, 6H), 4.10-4.24 (m, 1H).

Example 220

[1-(1-Benzylpiperidin-4-yl)-3-(3-morpholinopropyl) imidazolidin-2-ylidene]malononitrile (Compound 220)

[1-(1-Benzylpiperidin-4-yl)-3-(3-bromopropyl) imidazolidin-2-ylidene]malononitrile (1.00 g, 2.33 mmol) obtained in Step 3 of Example 126 was added with 1,4-dioxane (40 mL). The mixture was added with morpholine (1.01 mL, 11.7 mmol) and stirred at room temperature for 17.5 hours, and then at 80˚C for 5 hours. The mixture was added with water and extracted with ethyl acetate. The organic layer was washed with saturated brine, dried over anhydrous magnesium sulfate and the solvent was evaporated under reduced pressure. The residue was purified by silica gel column chromatography (chloroform to chloroform/methanol (95:5 to 90:10)) to obtain the titled compound (0.819 g, 80.7 %).
$^1$H NMR ($CDCl_3$, δppm) : 1.70-1.93 (m, 6H), 2.09-2.22 (m, 2H), 2.35-2.49 (m, 6H), 2.92-3.02 (m, 2H), 3.50 (s, 2H), 3.53-3.74 (m, 10H), 4.13-4.29 (m, 1H), 7.22-7.36 (m, 5H).

Example 221

[3-(3-Morpholinopropyl)-l-(piperidin-4-yl) imidazolidin-2-ylidene]malononitrile (Compound 221)

[1-(1-Benzylpiperidin-4-yl)-3-(3-morpholinopropyl) imidazolidin-2-ylidene]malononitrile (Compound 220)

(0.315 g, 0.725 mmol) obtained in Example 220 was dissolved in 1,2-dichloroethane (30 mL) and the solution was added with 1-chloroethyl chloroformate (0.117 mL, 1.09 mmol). After heating under reflux for 2 hours, the mixture was concentrated under reduced pressure and the residue was added with methanol (30 mL) and heated under reflux for 1 hour. The residue was alkalified by adding 1 mol/L aqueous sodium hydroxide solution and the mixture was concentrated under reduced pressure. The residue was purified by NH silica gel column chromatography (chloroform to chloroform/ methanol (95:5)) to obtain the titled compound (0.216 g, 86.5 %).
$^1$H NMR ($CDCl_3$, δppm) : 1.56-1.75 (m, 2H), 1.78-1.94 (m, 4H), 2.36-2.50 (m, 6H), 2.70-2.83 (m, 2H), 3.11-3.22 (m, 2H), 3.55-3.76 (m,10H), 4.21-4.37 (m, 1H).

Example 222

{1-[1-(2-Fluoroethyl)piperidin-4-yl]-3-(3-morpholinopropyl)imidazolidin-2-ylidene}malononitrile (Compound 222)

[3-(3-Morpholinopropyl)-1-(piperidin-4-yl) imidazolidin-2-ylidene]malononitrile (Compound 221) (0.216 g, 0.63 mmol) obtained in Example 221 was dissolved in DMF (4 mL) and the solution was added with potassium carbonate (0.520 g, 3.76 mmol) and 1-bromo-2-fluoroethane (0.140 mL, 1.89 mmol). After stirring at room temperature for 70 hours, the mixture was further stirred at 50°C for 1 hour. The mixture was added with water and extracted with ethyl acetate. The organic layer was washed with saturated brine and the aqueous layer was extracted with chloroform. The organic layer was combined and dried over anhydrous magnesium sulfate. The the solvent was evaporated under reduced pressure and the residue was purified by silica gel column chromatography (chloroform/(2 mol/L ammonia-methanol) (100:20)) to obtain the titled compound (0.0575 g, 23.5 %) .
$^1$H NMR ($CDCl_3$, δppm) : 1.73-1.94 (m, 6H), 2.20-2.32 (m, 2H), 2.37-2.50 (m, 6H), 2.70 (dt, J = 28.8, 4.8 Hz, 2H), 3.01-3.11 (m, 2H), 3.54-3.75 (m, 10H), 4.16-4.29 (m, 1H), 4.54 (dt, J = 47.2, 4.8 Hz, 2H).

Example 223

{1-[1-(Tert-butyloxycarbonyl)piperidin-4-yl]-3-(3-piperidinopropyl)imidazolidin-2-ylidene}malononitrile (Compound 223)

A dihydrochloride of [1-(piperidin-4-yl)-3-(3-piperidinopropyl)imidazolidin-2-ylidene]malononitrile (0.835 g, 2.01 mmol) obtained in Example 128 was dissolved in dichloromethane (8.0 mL) and the solution was added with triethylamine (0.841 mL, 6.03 mmol) and dicarbonate di-tert-butyl (0.658 g, 3.02 mmol), followed by stirring at room temperature for 2.5 hours. The mixture was added with water and extracted with ethyl acetate. The organic layer was washed with water thrice, dried over anhydrous magnesium sulfate and the solvent was evaporated under reduced pressure. The residue was purified by silica gel column chromatography (ethyl acetate to chloroform/ (2 mol/L ammonia-methanol) (10:1)) to obtain the titled compound (0.226 g, 25.4 %).
$^1$H NMR ($CDCl_3$, δppm) : 1.46 (s, 9H), 1.55-1.59 (m, 6H), 1.63-1.69 (m, 2H), 1.82-1.92 (m, 4H), 2.31-2.37 (m, 6H), 2.79-2.88 (m, 2H), 3.48-3.66 (m, 6H), 4.20-4.25 (m, 2H), 4.30-4.42 (m, 1H).
APCIMS m/z: $[M+H]^+$ 443.

Example 224

[1-(Piperidin-4-yl)-3-(3-piperidinopropyl) imidazolidin-2-ylidene]malononitrile dihydrochloride (Compound 224)

{1-[1-(Tert-butyloxycarbonyl)piperidin-4-yl]-3-(3-piperidinopropyl)imidazolidin-2-ylidene}malononitrile (0.200 g, 0.45 mmol) obtained in Example 223 was dissolved in 1,4-dioxane (1.0 mL) and the solution was added with 4 mol/L hydrochloric acid-dioxane (1.0 mL), followed by stirring at room temperature. The mixture was added with diethylether and stirred. After repeating the procedure to remove the supernatant solution thrice, the solvent of the residue was evaporated under reduced pressure to obtain the titled compound (0.248 g) as a solid.

Example 225

{1-(3-Piperidinopropyl)-3-[1-(2,2,2-trifluoroethyl) piperidin-4-yl]imidazolidin-2-ylidene}malononitrile (Compound 225)
[1-(Piperidin-4-yl)-3-(3-piperidinopropyl) imidazolidin-2-ylidene]malononitrile dihydrochloride (Compound 224) (0.163 g) obtained in Example 224 was dissolved in DMF (2.0 mL) and the solution was added with 2, 2, 2-trifluoroethyl trifluoromethanesulfonate (0.318 g, 1.26 mmol) prepared in a similar manner to the method described in Tetrahedoron., Vol. 21, p.1 (1965) and potassium carbonate (0.348 g, 2.52 mmol), followed by stirring at room temperature for 6 hours. The mixture was added with water and extracted with ethyl acetate. The organic layer was washed with saturated brine

twice, dried over anhydrous magnesium sulfate and the solvent was evaporated under reduced pressure. The residue was recrystallized from ethyl acetate-hexane to obtain the titled compound (0.089 g, 50.1 %).
$^1$H NMR ($CDCl_3$, δppm) : 1.36-1.62 (m, 6H), 1.73-1.92 (m, 6H), 2.34-2.39 (m, 6H), 2.48-2.57 (m, 2H), 2.88-3.07 (m, 4H), 3.52-3.66 (m, 6H), 4.15-4.26 (m, 1H).
APCIMS m/z: $[M+H]^+$ 425.
Melting point: 103-105˚C.

Example 226

[1-(1-Cyclopropylpiperidin-4-yl)-3-(3-piperidinopropyl)imidazolidin-2-ylidene]malononitrile (Compound 226)

A Compound obtained in Step 4 of Example 235, [1-(1-cyclopropylpiperidin-4-yl)-3-(3-methanesulfonyloxypropyl) imidazolidin-2-ylidene]malononitrile, (0.206 g, 0.52 mmol) was dissolved in 1,4-dioxane (1.2 mL) and the solution was added with piperidine (0.104 mL, 1.04 mmol) and potassium carbonate (0.145 g,1.04 mmol), followed by stirring at 80˚C overnight. The mixture was added with water and extracted with ethyl acetate. The organic layer was dried over anhydrous magnesium sulfate and the solvent was evaporated under reduced pressure. The residue was recrystallized from ethyl acetate-hexane to obtain the titled compound (0.166 g, 83.1%).
$^1$H NMR ($CDCl_3$, δppm) : 0.34-0.51 (m, 4H), 1.42-1.44 (m, 2H), 1.53-1.73 (m, 8H), 1.81-1.91 (m, 4H), 2.31-2.36 (m, 7H), 3.08-3.12 (m, 2H), 3.53-3.60 (m, 6H), 4.18-4.27 (m, 1H).
APCIMS m/z: $[M+H]^+$ 383.
Melting point: 104-105˚C.

Example 227

[1-(1-Allylpiperidin-4-yl)-3-(3-piperidinopropyl) imidazolidin-2-ylidene]malonanitrile (Compound 227)

[1-(Piperidin-4-yl)-3-(3-piperidinopropyl) imidazolidin-2-ylidene]malononitrile dihydrochloride (Compound 224) (0.300 g, 0.72 mmol) obtained in Example 224 was dissolved in. DMF (3.0 mL) and the solution was added with allyl bromide (0.0935 mL, 1.08 mmol) and potassium carbonate (0.398 g, 2.88 mmol), followed by stirring at room temperature overnight. The mixture was added with 1 mol/L hydrochloric acid and the aqueous layer was washed with ethyl acetate. Next, the aqueous layer was added with 10 mol/L aqueous sodium hydroxide solution and extracted with ethyl acetate. The organic layer was washed with saturated brine thrice, dried over anhydrous magnesium sulfate and the solvent was evaporated under reduced pressure to obtain the titled compound (0.113 g, 41.2 %).
$^1$H NMR ($CDCl_3$, δppm) : 1.43-1.44 (m, 2H), 1.53-1.59 (m, 4H), 1.75-1.78 (m, 2H), 1.84-1.91 (m, 4H), 2.08-2.15 (m, 2H), 2.32-2.37 (m, 6H), 2.99-3.02 (m, 4H), 3.51-3.63 (m, 6H), 4.16-4.26 (m, 1H), 5.14-5.21 (m, 2H), 5.79-5.88 (m, 1H).
APCIMS m/z: $[M+H]^+$ 383.

Example 228

{1-[1-(1,1-Dimethyl-2-propynyl)piperidin-4-yl]-3-(3-piperidinopropyl)imidazolidin-2-ylidene}malononitrile (Compound 228)

[1-(Piperidin-4-yl)-3-(3-piperidinopropyl) imidazolidin-2-ylidene]malononitrile dihydrochloride (Compound 224) (0.300 g, 0.72 mmol) obtained in Example 224 was suspended in THF (4.0 mL) and DMF (1.4 mL) and the suspension was added with 3-chloro-3-methyl-1-butyne (97 %, 0.110 mL, 0.95 mmol), triethylamine (0.269 mL, 1.93 mmol) and copper chloride (I) (0.0056 g, 0.056 mmol), followed by stirring at room temperature for 1.5 hours. The mixture was added with 1 mol/L hydrochloric acid and the aqueous layer was washed with ethyl acetate. The aqueous layer was added with saturated aqueous sodium hydrogencarbonate solution and extracted with ethyl acetate. The organic layer was washed with saturated brine thrice, dried over anhydrous magnesium sulfate and the solvent was evaporated under reduced pressure. The residue was purified by preparative TLC (chloroform/(2 mol/L ammonia-methanol) (4:1)) to obtain the titled compound (0.569 g, 19.3 %).
$^1$H NMR ($CDCl_3$, δppm) : 1.39 (s, 6H), 1.43-1.45 (m, 2H), 1.53-1.62 (m, 4H), 1.67 -1.92 (m, 7H), 2.28-2.38 (m, 8H), 3.17-3.21 (m, 2H), 3.53-3.63 (m, 6H), 4.13-4.23 (m, 1H).
APCIMS m/z: $[M+H]^+$ 409.
Melting point: 112-115˚C.

Example 229

[1-(1-Cyclopropylmethylpiperidin-4-yl)-3-(3-piperidinopropyl)imidazolidin-2-ylidene]malononitrile (Compound 229)

[1-(Piperidin-4-yl)-3-(3-piperidinopropyl) imidazolidin-2-ylidene]malononitrile dihydrochloride (Compound 224) (0.300 g,0.72 mmol) obtained in Example 224 was suspended in 1,4-dioxane (3.0 mL) and the suspension was added with (bromomethyl) cyclopropane (97 %, 0.0936 mL, 0.94 mmol) and potassium carbonate (0.398 g, 2.88 mmol), followed by stirring at 70˚C overnight. The mixture was concentrated under reduced pressure and the residue was dissolved in DMF (3.0 mL) and added with (bromomethyl)cyclopropane (97 %, 0.144 mL, 1.44 mmol), followed by stirring at 50˚C for 4 hours. The mixture was added with 1 mol/L hydrochloric acid and the aqueous layer was washed with ethyl acetate. The aqueous layer was added with 10 mol/L aqueous sodium hydroxide solution and extracted with ethyl acetate. The organic layer was washed with saturated brine thrice, dried over anhydrous magnesium sulfate and the solvent was evaporated under reduced pressure to obtain the titled compound (0.0648 g, 22.7 %).
$^1$H NMR ($CDCl_3$, δppm) : 0.09-0.13 (m, 2H), 0.50-0.53 (m, 2H), 0.79-0.86 (m, 1H), 1.43-1.45 (m, 2H), 1.54-1.61 (m, 4H), 1.76-1.89 (m, 6H), 2.10-2.18 (m, 2H), 2.27 (d, J = 6.6 Hz, 2H), 2.33-2.37 (m, 6H), 3.14-3.18 (m, 2H), 3.56-3.60 (m, 6H), 4.17-4.24 (m, 1H).
APCIMS m/z: $[M+H]^+$397.

Example 230

{3-[3-(3,3-Difluoropyrrolidin)-1-ylpropyl]-1-(1-isopropylpiperidin-4-yl)imidazolidin-2-ylidene} malononitrile (Compound 230)

(Step 1)

[Bis(methylthio)methylene]malononitrile (5.74 g, 33.7 mmol) was dissolved in THF (60 mL) and the solution was added with 4-aminopiperidine-1-carboxylic acid tert-butylester (6.75 g, 33.7 mmol) dissolved in THF (12 mL), followed by stirring at room temperature for 1.5 hours. Next, 2-aminoethanol (2.10 mL, 35.4 mmol) was added to the mixture and refluxed for 5 hours. The mixture was further added with 2-aminoethanol (0.30 mL, 5.0 mmol), refluxed for 24 hours and further added with 2-aminoethanol (0.40 mL, 6.6 mmol) and refluxed for 7 hours. After the mixture was cooled to room temperature, the solvent was evaporated under reduced pressure. The residue was purified by silica gel column chromatography (chloroform to chloroform/methanol (10:1)) to obtain ([1-(1-tert-butyloxycarbonyl)piperidin-4-ylamino]-3-(2-hydroxyethylamino)methylene}malononitrile (9.59 g, 84.8 %) as a white solid.
$^1$H NMR ($CDCl_3$, δppm) : 1.35-1.45 (m, 2H), 1.46 (s, 9H), 1.99-2.08 (m, 2H), 2.92-3.00 (m, 3H), 3.40 (td, J = 5.4, 4.4 Hz, 2H), 3.78-3.85 (m, 2H), 3.93-4.08 (m, 3H), 5.67-5.76 (m, 1H), 7.05-7.11 (m, 1H).

(Step 2)

{[1-(1-Tert-butyloxycarbonyl)piperidin-4-ylamino]-3-(2-hydroxyethylamino)methylene}malononitrile (9.59 g, 28.6 mmol) obtained in the Step 1 was dissolved in dichloromethane (90 mL) and the solution was added with triethylamine (15.9 mL, 114.4 mmol) and methanesulfonylchloride (3.32 mL, 42.9 mmol), followed by stirring at room temperature for 19 hours. The mixture was added with water. The precipitated solid was collected by filtration and washed with methanol to obtain {1-[1-(1-tert-butyloxycarbonyl)piperidin-4-yl] imidazolidin-2-ylidene}malononitrile (3.68 g, 40.5 %) as a white solid.
$^1$H NMR ($CDCl_3$, δppm) : 1.46 (s, 9H), 1.51-1.69 (m, 2H), 1.84-1.89 (m, 2H), 2.78-2.87 (m, 2H), 2.57-2.72 (m, 4H), 4.20-4.25 (m, 1H), 4.41 (td, J = 11.8, 3.8 Hz, 1H), 5.67-5.74 (m, 1H).

(Step 3)

{1-[1-(1-Tert-butyloxycarbonyl)piperidin-4-yl] imidazolidin-2-ylidene}malononitrile (3.68 g, 11.59 mmol) obtained in the Step 2 was dissolved in DMF (25 mL) and the solution was added with potassium carbonate (3.20 g, 23.18 mmol) and benzyl-3-bromopropylether (2.46 mL, 13.91 mmol), followed by stirring at 80˚C for 6.5 hours. Further, the mixture was added with potassium carbonate (1.60 g, 11.59 mmol) and benzyl-3-bromopropylether (1.72 mL, 5.80 mmol), followed by stirring at 80˚C for 14.5 hours. The mixture was added with saturated brine and extracted with ethyl acetate. The organic layer was washed with saturated brine, dried over anhydrous magnesium sulfate and the solvent was evaporated under reduced pressure. The residue was purified by silica gel column chromatography (chloroform to chloroform/methanol (20:1)) to obtain {3-(3-benzyloxypropyl)-1-[1-(1-tert-butyloxycarbonyl)piperidin-4-yl]imidazolidin-2-ylidene} malononitrile (4.84 g, 89.6 %) as a white solid.

$^1H$ NMR ($CDCl_3$, δppm) : 1.45 (s, 9H), 1.49-1.56 (m, 2H), 1.74-1.79 (m, 2H), 1.94-2.04 (m, 2H), 2.81 (brt, J = 12.3 Hz, 2H), 3.38-3.45 (m, 2H), 3.52-3.60 (m, 4H), 3.69 (t, J = 7.1 Hz, 2H), 4.18-4.35 (m, 3H), 4.49 (s, 2H), 7.26-7.38 (m, 5H).

(Step 4)

{3-(3-Benzyloxypropyl)-1-[1-(1-tert-butyloxycarbonyl)piperidin-4-yl]imidazolidin-2-ylidene} malononitrile (4.84 g, 10.39 mmol) obtained in the Step 3 was dissolved in dichloromethane (36 mL) and the solution was added with trifluoroacetic acid (12 mL), followed by stirring at 0˚C for 1 hour. The mixture was concentrated under reduced pressure and the residue (5.03g) was obtained.

The residue was dissolved in DMF (25 mL) and the solution was added with potassium carbonate (7.18 g, 52.0 mmol) and isopropyl iodide (3.11 mL, 31.2 mmol), followed by stirring at 40˚C for 15 hours. The mixture was added with saturated brine and extracted with ethyl acetate. The organic layer was washed with saturated brine, dried over anhydrous magnesium sulfate and the solvent was evaporated under reduced pressure. After the residue was purified by silica gel column chromatography (chloroform to chloroform/methanol (10:1)), the obtained solid was washed with diisopropylether to obtain [3-(3-benzyloxypropyl)-1-(1-isopropylpiperidin-4-yl)imidazolidin -2-ylidene]malononitrile (3.29 g, 77.7 %) as a white solid. $^1H$ NMR ($CDCl_3$, δppm) : 1.04 (d, J = 6.3 Hz, 6H), 1.64-1.83 (m, 6H), 1.94-2.03 (m, 2H), 2.30 (t, J = 11.5 Hz, 2H), 2.73-2.81 (m, 1H), 2.94 (brd, J = 12.2 Hz, 2H), 3.50-3.60 (m, 4H), 3.67 (t, J = 7.2 Hz, 2H), 4.09-4.20 (m, 1H), 4.49 (s, 2H), 7.26-7.38 (m, 5H).

(Step 5)

[3-(3-Benzyloxypropyl)-1-(1-isopropylpiperidin-4-yl) imidazolidin-2-ylidene]malononitrile (0.26 g, 0.49 mmol) obtained in the Step 4 was dissolved in dichloromethane (5 mL) and borane tribromide (1.0 mol/L dichloromethane solution; 1.48 mL, 1.48 mmol) was added dropwise to the solution at -78˚C, followed by stirring at -40˚C for 2 hours. After the mixture was added with saturated aqueous sodium hydrogencarbonate solution and 2 mol/L aqueous sodium hydroxide solution to adjust the pH to 10, the mixture was extracted with ethyl acetate. The organic layer was washed with saturated brine, dried over anhydrous magnesium sulfate and the solvent was evaporated under reduced pressure. The obtained solid was washed with diisopropylether to obtain [3-(3-hydroxypropyl)-1-(1-isopropylpiperidin-4-yl)imidazolidin-2-ylidene] malononitrile (0.12 g, 55.6 %) as a white solid.
$^1H$ NMR ($CDCl_3$, δppm) : 1.05 (d, J = 6.6 Hz, 6H), 1.72-1.98 (m, 6H), 2.28-2.35 (m, 2H), 2.73-2.81 (m, 1H), 2.97 (brd, J =, 11.9 Hz, 2H), 3.61 (s, 4H), 3.66-3.71 (m, 2H), 3.74 (t, J = 6.8 Hz, 2H), 4.13-4.23 (m, 1H) .

(Step 6)

[3-(3-Hydroxypropyl)-1-(1-isopropylpiperidin-4-yl) imidazolidin-2-ylidene]malononitrile (0.12 g, 0.28 mmol) obtained in the Step 5 was dissolved in dichloromethane (1.0 mL) and the solution was added with triethylamine (0.15 mL, 1.10 mmol) and methanesulfonylchloride (0.03 mL, 0.41 mmol), followed by stirring at room temperature for 1.5 hours. The mixture was added with saturated brine and extracted with ethyl acetate. The organic layer was washed with saturated brine, dried over anhydrous magnesium sulfate and the solvent was evaporated under reduced pressure. The obtained solid was washed with diisopropylether to obtain [1-(1-isopropylpiperidin-4-yl)-3-(3-methanesulfonyloxypropyl) imidazolidin-2-ylidene] malononitrile (0.09 g, 82.7 %) as a white solid.
$^1H$ NMR ($CDCl_3$, δppm) : 1.06 (d, J = 6.6 Hz, 6H), 1.75-1.80 (m, 4H), 2.13-2.21 (m, 2H), 2.30-2.37 (m, 2H), 2.75-2.84 (m, 1H), 2.99 (brd, J= 11.3 Hz, 2H), 3.05 (s, 3H), 3.62 (s, 4H), 3.72 (t, J = 7.2 Hz, 2H), 4.14-4.25 (m, 1H), 4.35 (t, J = 5.9 Hz, 2H).

(Step 7)

[1-(1-Isopropylpiperidin-4-yl)-3-(3-methanesulfonyloxypropyl)imidazolidin-2-ylidene] malononitrile (0.15 g, 0.38 mmol) obtained in the Step 6 was dissolved in 1,4-dioxane (1.5 ml) and the solution was added with a solution of potassium carbonate (0.08 g, 0.76 mmol) and 3,3-difluoropyrrolidine hydrochloride (0.083 g, 0.76 mmol) in a mixed solvent of 1,4-dioxane (0.5 mL) and 2 mol/L aqueous sodium hydroxide solution (0.3 mL, 0.80 mmol), followed by stirring at 80˚C for 16 hours. The mixture was added with saturated brine and extracted with ethyl acetate. The organic layer was washed with saturated brine, dried over anhydrous magnesium sulfate and the solvent was evaporated under reduced pressure. After the residue was purified by silica gel column chromatography (chloroform/methanol (3:1)), the obtained solid was washed with diisopropylether and recrystallized from n-hexane-ethyl acetate (20:1) to obtain the titled compound (0.069 g, 44.7 %) as a white solid.
$^1H$ NMR ($CDCl_3$, δppm) : 1.04 (d, J = 6.6 Hz, 6H), 1.60-1.89 (m, 6H), 2.23-2.34 (m, 4H), 2.54 (t, J = 6.8 Hz, 2H), 2.71-2.78 (m, 3H), 2.88 (t, J = 13.0 Hz, 2H), 2.92-2.97 (m, 2H), 3.58-3.64 (m, 6H), 4.13-4.24 (m, 1H).

APCIMS m/z: $[M+H]^+$407.
Melting point: 122-123°C.

Example 231

[1-(1-Isopropylpiperidin-4-yl)-3-(3-thiomorpholin-1-ylpropyl)imidazolidin-2-ylidene]malononitrile (Compound 231)

In a similar manner to Step 7 of Example 230, the titled compound (0.088 g, 57.5 %) was obtained as a white solid by using [1-(1-isopropylpiperidin-4-yl)-3-(3-methanesulfonyloxypropyl)imidazolidin-2-ylidene] malononitrile (0.15 g, 0.38 mmol) and thiomorpholine (0.06 mL, 0.76 mmol) in place of 3,3-difluoropyrrolidine hydrochloride.
$^1$H NMR ($CDCl_3$, δppm) : 1.04 (d, J = 6.6 Hz, 6H), 1.67-1.88 (m, 6H), 2.27-2.37 (m, 2H), 2.41 (t, J = 7.0 Hz, 2H), 2.65-2.79 (m, 9H), 2.94-2.98 (m, 2H), 3.54-3.60 (m, 6H), 4.13-4.24 (m, 1H).
APCIMS m/z: $[M+H]^+$403.
Melting point: 124-125°C.

Example 232

[3-(3-(4,4-Difluoropiperidine)-1-ylpropyl)-1-(1-isopropylpiperidin-4-yl)imidazolidin-2-ylidene] malononitrile (Compound 232)

In a similar manner to Step 7 of Example 230, the titled compound (0.097 g, 60.7 %) was obtained as a white solid by using [1-(1-isopropylpiperidin-4-yl)-3-(3-methanesulfonyloxypropyl)imidazolidin-2-ylidene] malononitrile (0.15 g, 0.38 mmol) and 4,4-difluoropiperidine hydrochloride (0.093 g, 0.76 mmol) in place of 3,3-difluoropyrrolidine hydrochloride.
$^1$H NMR ($CDCl_3$, δppm) : 1.04 (d, J = 6.6 Hz, 6H), 1.68-1.90 (m, 6H), 1.93-2.06 (m, 4H), 2.27-2.34 (m, 2H), 2.45 (t, J = 6.9 Hz, 2H), 2.54 (brt, J = 5.5 Hz, 4H), 2.72-2.80 (m, 1H), 2.95 (brt, J = 11.7 Hz, 2H), 3.57-3.62 (m, 6H), 4.13-4.23 (m, 1H).
APCIMS m/z: $[M+H]^+$421.
Melting point: 132-134°C.

Example 233

{1-(1-Isopropylpiperidin-4-yl)-3-[3-(4-oxopiperidino)propyl]imidazolidin-2-ylidene}malononitrile (Compound 233)

In a similar manner to Step 7 of Example 230, the titled compound (0.074 g, 48.9 %) was obtained as a white solid by using [1-(1-isopropylpiperidin-4-yl)-3-(3-methanesulfonyloxypropyl)imidazolidin-2-ylidene] malononitrile (0.15 g, 0.38 mmol) and 4-piperidone hydrochloride monohydrate (0.09 g, 0.76 mmol) in place of 3,3-difluoropyrrolidine hydrochloride.
$^1$H NMR ($CDCl_3$, δppm) : 1.04 (d, J = 6.6 Hz, 6H), 1.75 (dq, J= 3.2, 11.6 Hz, 2H), 1.87-1.99 (m, 4H), 2.27-2.34 (m, 2H), 2.46 (t, J = 6.0Hz, 4H), 2.53 (t, J = 6.8 Hz, 2H), 2.75 (t, J = 6.0 Hz, 5H), 2.96 (brd, J =11.7 Hz,2H), 3.60-3.67 (m, 6H), 4.13-4.24 (m, 1H).
APCIMS m/z: $[M+H]^+$399.
Melting point: 103-105°C.

Example 234

{3-[3-(Cis-2,6-dimethylmorpholino)propyl]-1-(1-isopropylpiperidin-4-yl)imidazolidin-2-ylidene} malononitrile (Compound 234)

In a similar manner to Step 7 of Example 230, the titled compound (0.07 g, 46.2 %) was obtained as a white solid by using [1-(1-isopropylpiperidin-4-yl)-3-(3-methanesulfonyloxypropyl)imidazolidin-2-ylidene]malononitrile (0.14 g, 0.36 mmol) and cis-2,6-dimethylmorpholine (0.09 ml, 0.73 mmol) in place of 3,3-difluoropyrrolidine hydrochloride.
$^1$H NMR ($CDCl_3$, δppm) : 1.04 (d, J = 6.6 Hz, 6H), 1.16 (d, J= 6.2 Hz, 6H), 1.68-1.77 (m, 4H), 1.81-1.91 (m, 4H), 2.27-2.39 (m.4H), 2.70-2.80 (m, 3H), 2.96 (brd, J = 12.1 Hz, 2H), 3.57-3.70 (m, 8H), 4.13-4.23 (m, 1H).
APCIMS m/z: $[M+H]^+$415.
Melting point: 117-118°C.

Example 235

[1-(1-Cyclopropylpiperidin-4-yl)-3-(3-morpholinopropyl)imidazolidin-2-ylidene]malononitrile (Compound 235)

(Step 1)

{1-[1-(1-Tert-butyloxycarbonyl)piperidin-4-yl] imidazolidin-2-ylidene}malononitrile (17.79 g, 56.0 mmol) obtained in Step 2 of Example 230 was dissolved in DMF (110 mL) and the solution was added with potassium carbonate (15.5 g, 112.0 mmol) and 3-(bromopropoxy)-tert-butyldimethylsilane (17.0 g, 67.18 mmol), followed by stirring at 60˚C for 20 hours. The mixture was added with saturated brine and extracted with ethyl acetate. The organic layer was washed with saturated brine, dried over anhydrous magnesium sulfate and the solvent was evaporated under reduced pressure. The obtained crude crystal was washed with diisopropylether/n-hexane (1:2) to obtain {3-(3-tert-butyldimethylsilyloxypropyl)-1-[1-(1-tert-butyloxycarbonyl)piperidin-4-yl]imidazolidin-2-ylidene} malononitrile (24.0 g, 87.5 %) as a white solid.
$^1$H NMR ($CDCl_3$, δppm) : 0.06 (s, 6H), 0.89 (s, 9H), 1.46 (s,9H), 1.59 (td, J = 12.3, 3.5 Hz, 2H), 1.82-1.94 (m, 4H), 2.79-1.88 (m, 2H), 3.48-3.55 (m, 2H), 3.59-3.71 (m, 6H), 4.19-4.25 (m, 2H), 4.30-4.40 (m, 1H).

(Step 2)

{3-(3- Tert- butyldimethylsilyloxypropyl)- 1-[1-(1- tert- butyloxycarbonyl) piperidin- 4- yl] imidazolidin- 2- ylidene} malononitrile (1.17 g, 2.39 mmol) obtained in the Step 1 was dissolved in ethyl acetate (10 mL) and the solution was added with 4 mol/L hydrochloric acid-ethyl acetate (5.98 mL, 11.95 mmol) at 0˚C., followed by stirring at room temperature for 4 hours. The obtained solid was collected by filtration and the filtrate was washed with ethyl acetate to obtain [1-(3-hydroxypropyl)-3-(piperidin-4-yl)imidazolidin-2-ylidene]malononitrile monohydrochloride (0.72 g, 96.7 %) as a white solid.
$^1$H NMR ($CD_3OD$, δppm) : 1.84-1.93 (m, 2H), 1.98-2.07 (m, 4H), 3.04-3.15 (m, 2H), 3.53-3.58 (m, 2H), 3.61-3.76 (m, 8H), 4.33-4.45 (m,1H).

(Step 3)

[1-(3-Hydroxypropyl)-3-(piperidin-4-yl)imidazolidin-2-ylidene]malononitrile monohydrochloride (0.72 g, 2.31 mmol) obtained in the Step 2 was dissolved in THF (35 mL) and the solution was added with methanol (0.36 ml), triethylamine (0.32 ml, 2.31 mmol), (1-ethoxycyclopropoxy)trimethylsilane (0.93 mL, 4.62 mmol), acetic acid (3.96 ml) and sodium cyanoborohydride (0.31 g, 4.62 mmol), followed by stirring at 60˚C for 3 hours. The mixture was concentrated under reduced pressure, and the residue was added with water (110 ml) and 2 mol/L hydrochloric acid (18ml), followed by washing with ethyl acetate. The pH of the obtained aqueous layer was adjusted to 7 by potassium carbonate and the mixture was extracted with ethyl acetate. The organic layer was washed with saturated brine, dried over anhydrous magnesium sulfate and the solvent was evaporated under reduced pressure. The obtained solid was washed with n-hexane/ethyl acetate (20:1) to obtain [1-(1-cyclopropylpiperidin-4-yl)-3-(3-hydroxypropyl) imidazolidin-2-ylidene] malononitrile (0.51 g, 70.1 %) as a white solid.
$^1$H NMR ($CDCl_3$, δppm) : 0.34-0.39 (m, 2H), 0.42-0.51 (m, 2H), 1.63-1.68 (m, 3H), 1.71-1.99 (m, 4H), 2.36 (td, J = 11.9, 2.5 Hz, 2H), 3.11 (brd, J = 11.8 Hz, 2H), 3.54-3.60 (m, 4H), 3.63-3.76 (m, 4H), 4.13-4.28 (m, 1H).

(Step 4)

[1-(1-Cyclopropylpiperidin-4-yl)-3-(3-hydroxypropyl) imidazolidin-2-ylidene]malononitrile (0.50 g, 1.60 mmol) obtained in the Step 3 was dissolved in dichloromethane (6 mL) and the solution was added with triethylamine (0.89 mL, 6.41 mmol) and methanesulfonylchloride (0.19 mL, 2.41 mmol), followed by stirring at room temperature for 2 hours. The mixture was added with saturated brine and extracted with ethyl acetate. The organic layer was washed with saturated brine, dried over anhydrous magnesium sulfate and the solvent was evaporated under reduced pressure to obtain [1-(1-cyclopropylpiperidin-4-yl)-3-(3-methanesulfonyloxypropyl)imidazolidin-2-ylidene] malononitrile (0.52 g, 82.5 %) as a white solid.
$^1$H NMR ($CDCl_3$, δppm): 0.41-0.50 (m, 4H), 1.64-1.86 (m, 4H), 2.12-2.22 (m, 2H), 2.38 (brt, J = 11.1 1 Hz, 2H), 3.05 (s, 3H), 3.06-3.15 (m, 2H), 3.57-3.67 (m, 5H), 3.72 (t, J = 7.2 Hz, 2H), 4.15-4.29 (m, 1H), 4.34 (t, J = 5.8Hz, 2H).

(Step 5)

[1-(1-Cyclopropylpiperidin-4-yl)-3-(3-methanesulfonyloxypropyl)imidazolidin-2-ylidene] malononitrile (0.15 g, 0.38 mmol) obtained in the Step 4 was dissolved in 1,4-dioxane (2 mL) and the solution was added with potassium

carbonate (0.11 g, 0.76 mmol) and morpholine (0.066 mL, 0.76 mmol), followed by stirring at 80˚C for 12 hours. The mixture was added with saturated brine and extracted with ethyl acetate. The organic layer was washed with saturated brine, dried over anhydrous magnesium sulfate and the solvent was evaporated under reduced pressure. The residue was purified by silica gel column chromatography (chloroform/methanol (9:1)) and the obtained solid was washed with diisopropylether to obtain the titled compound (0.086 g, 58.9 %) as a white solid.
$^1$H NMR ($CDCl_3$, δppm) : 0.34-0.41 (m, 2H), 0.43-0.51 (m, 2H), 1.65-1.75 (m, 3H), 1.81-1.91 (m, 4H), 2.30-2.45 (m, 8H), 3.11 (brd, J =12.0Hz, 2H), 3.52-3.63 (m, 6H), 3.70 (t, J = 4.6 Hz, 4H), 4.17-4.29 (m, 1H).
APCIMS m/z: $[M+H]^+$385.
Melting point: 104-106˚C.

Example 236

{1-(1-Cyclopropylpiperidin-4-yl)-3-[3-(thiomorpholin-1-yl)propyl]imidazolidin-2-ylidene} malononitrile (Compound 236)

In a similar manner to Step 5 of Example 235, the titled compound (0.088 g, 28.9 %) was obtained as a white solid by using [1-(1-cyclopropylpiperidin-4-yl)-3-(3-methanesulfonyloxypropyl)imidazolidin-2-ylidene] malononitrile (0.15 g, 0.38 mmol) and thiomorpholine (0.076 ml, 0.76 mmol) in place of morpholine.
$^1$H NMR ($CDCl_3$, δppm) : 0.36-0.41 (m, 2H), 0.43-0.51 (m, 2H), 1.62-1.75 (m, 5H), 1.81-1.90 (m, 4H), 2.31-2.44 (m, 4H), 2.65-2.72 (m, 6H), 3.11 (brd, J = 11.7 Hz, 2H), 3.51-3.60 (m, 6H), 4.17-4.27 (m, 1H).
APCIMS m/z: $[M+H]^+$401.
Melting point: 129-130˚C.

Example 237

[1-(1-Benzylpiperidin-4-yl)-3-(3-piperidinopropyl) tetrahydropyrimidin-2-ylidene]malononitrile (Compound 237)

(Step 1)

[Bis(methylthio)methylene]malononitrile (2.01 g, 11.8 8 mmol) was dissolved in THF (60 mL) and the solution was added with 4-amino-1-benzylpiperidine (2.41ml,11.8 mmol) dissolved in THF (2 mL), followed by stirring at room temperature for 2.5 hours. Then the mixture was added with 3-aminopropanol (0.96 mL, 12.4 mmol) and stirred at 60˚C for 8 hours. Further, the mixture was added with 3-aminopropanol (0.45 mL, 5.9 mmol) and stirred at 60˚C for 8 hours. After the mixture was cooled to room temperature, the solvent was evaporated under reduced pressure and the residue was purified by silica gel column chromatography (chloroform/methanol (100:1)) to obtain [1-(1-benzylpiperidin-4-ylamino)-2-(3-hydroxypropylamino) methylene]malononitrile (3.98 g, 99.5 %).
$^1$H NMR ($CDCl_3$, δppm) : 1.50-1.65 (m, 2H), 1.72-1.87 (m, 2H), 1.96-2.02 (m, 2H), 2.10-2.17 (m, 2H), 2.81-2.86 (m, 2H), 3.46-3.53 (m, 4H), 3.62-3.74 (m, 1H), 3.83 (t, J = 5.5 Hz, 2H), 5.69 (brd, J = 11.1 Hz, 1H), 5.98 (s, 1H), 7.27-7.35 (m, 5H).

(Step 2)

[1-(1-Benzylpiperidin-4-ylamino)-2-(3-hydroxypropylamino)methylene]malononitrile (3.98 g, 11.74 mmol) obtained in the Step 1 was dissolved in dichloromethane (33 mL) and the solution was added with triethylamine (8.18 mL, 58.7 mmol) and methanesulfonylchloride (1.36 mL, 17.61 mmol), followed by stirring at room temperature for 18 hours. The mixture was added with water and extracted with ethyl acetate. The organic layer was washed with saturated brine, dried over anhydrous magnesium sulfate and the solvent was evaporated under reduced pressure to obtain [1-(1-benzylpiperidin-4-ylamino)-2-(3-methanesulfonyloxypropylamino)methylene]malononitrile (2.5 g, 51.3 %) .
$^1$H NMR ($CDCl_3$, δppm) : 1.60 (qd, J = 10.9, 3.5 Hz, 2H), 1.94-1.98 (m, 2H), 2.07-2.14 (m, 4H), 2.85 (brd, J = 12.1 Hz, 2H), 3.08 (s, 3H), 3.50 (s, 2H), 3.52-3.62 (m, 3H), 4.37 (t, J = 5.5 Hz, 2H), 5.12 (d, J =8.4 Hz, 1H), 5.61-5.65 (m, 1H), 7.23-7.34 (m, 5H).

(Step 3)

[1-(1-Benzylpiperidin-4-ylamino)-2-(3-methanesulfonyloxypropylamino)methylene]malononitrile (2.43 g, 5.85 mmol) obtained in the Step 2 was dissolved in DMF (30 mL) and the solution was added with potassium carbonate (0.97 g, 7.02 mmol), followed by stirring at 110˚C for 1 hour. The mixture was added with water and extracted with ethyl acetate. The organic layer was washed with saturated brine, dried over anhydrous magnesium sulfate and the solvent was evaporated under reduced pressure. The obtained solid was washed with water to obtain [1-(1-benzylpiperidin-4-yl) tetrahydropyrimidin-2-ylidene]malononitrile (1.42 g, 76.1 %) as a white solid.

$^1H$ NMR ($CDCl_3$, δppm): 1.78-1.88 (m, 4H), 1.91-2.02 (m, 2H), 2.16 (td, J = 11.1, 3.1 Hz, 2H), 2.96 (brd, J = 11.5 Hz, 2H), 3.26-3.37 (m, 4H), 3.50 (s, 2H), 4.11-4.23 (m, 1H), 5.85 (brs, 1H), 7.23-7.34 (m, 5H).

(Step 4)

[1-(1-Benzylpiperidin-4-yl)tetrahydropyrimidin-2-ylidene]malononitrile (1.41 g, 4.42 mmol) obtained in the Step 3 was dissolved in DMF (10 mL) and the solution was added with potassium carbonate (1.22 g, 8.84 mmol) and 1,3-dibromopropane (2.24 mL, 22.1 mmol), followed by stirring at room temperature for 24.5 hours. Further, the mixture was added with potassium carbonate (0.61 g, 4.42 mmol) and 1,3-dibromopropane (1.12 mL, 11.1 mmol), followed by stirring at room temperature for 12.5 hours. The mixture was added with saturated brine and extracted with ethyl acetate. The organic layer was washed with saturated brine and dried over anhydrous magnesium sulfate. The solvent was evaporated under reduced pressure and the residue (0.72g) was obtained.

The residue (0.72 g) was dissolved in 1,4-dioxane (10 mL) and the solution was added with potassium carbonate (1.22 g, 8.84 mmol), potassium iodide (0.73g,4.42 mmol) and piperidine (1.31 ml, 13.3 mmol), followed by stirring at 40˚C for 16 hours. The mixture was added with saturated brine and extracted with ethyl acetate. The organic layer was washed with saturated brine, dried over anhydrous magnesium sulfate and the solvent was evaporated under reduced pressure. The residue was purified by silica gel column chromatography (chloroform to chloroform/methanol (10:1)) to obtain the titled compound (0.10 g, 5.3 %).
$^1H$ NMR ($CDCl_3$, δppm) : 1.40-1.45 (m, 2H), 1.56-1.72 (m, 4H), 1.74-2.06 (m, 8H), 2.16 (td, J = 11.3, 3.3 Hz, 2H), 2.32-2.38 (m, 6H), 2.97 (brd, J = 11.9 Hz, 2H), 3.19 (t, J = 6.0 Hz, 2H), 3.25 (t, J = 7.0 Hz, 2H), 3.50 (s, 2H), 3.60 (t, J = 7.8 Hz, 2H), 4.12-4.20 (m, 1H), 7.27-7.34 (m,5H).

Example 238

[1-(1-Isopropylpiperidin-4-yl)-3-(3-piperidinopropyl)tetrahydropyrimidin-2-ylidene] malononitrile (Compound 238)

[1-(1-Benzylpiperidin-4-yl)-3-(3-piperidinopropyl) tetrahydropyrimidin-2-ylidene]malononitrile (Compound 237) (0.10 g, 0.23 mmol) obtained in Example 237 was dissolved in dichloroethane (1.3 mL) and the solution was added with 1-chloroethyl chloroformate (0.03 mL, 0.28 mmol), followed by refluxing for 4.5 hours. The mixture was concentrated under reduced pressure and the residue was dissolved in methanol (2 mL) and refluxed for 1 hour. The mixture was concentrated under reduced pressure and the residue (0.10g) was triturated with diisopropylether to obtain a solid.

The obtained solid was dissolved in DMF (1 ml) and the solution was added with potassium carbonate (0.16 g, 1.15 mmol) and propyl iodide (0.07 ml, 0.69 mmol), followed by stirring at room temperature for 13 hours. The mixture was added with saturated brine and extracted with ethyl acetate. The organic layer was washed with saturated brine, dried over anhydrous magnesium sulfate and the solvent was evaporated under reduced pressure. After the residue was purified by silica gel column chromatography (chloroform/methanol (3:1)), the obtained solid was washed with diisopropylether/n-hexane (2:1) to obtain the titled compound (0.01 g, 11.0 %) as a clear and colorless oily substance.
$^1H$ NMR ($CDCl_3$, δppm) : 1.04 (d, J = 6.2 Hz, 6H), 1.42-1.46 (m, 2H), 1.56-1.61 (m, 4H), 1.76-2.02 (m, 9H), 2.28-2.39 (m, 7H), 2.72-2.79 (m, 1H), 2.88-2.99 (m, 2H), 3.20 (t, J = 5.8 Hz, 2H), 3.25 (t, J = 6.8Hz, 2H), 3.57-3.62 (m, 2H), 4.07-4.20 (m, 1H).
APCIMS m/z: $[M+H]^+$399.

Example 239

[Bis(1-benzylpiperidin-4-ylamino)methylene] malononitrile (Compound 239)

[Bis(methylthio)methylene]malononitrile (485 mg, 2.85 mmol) was added with 1-benzyl-4-aminopiperidine (5.80 mL, 28.4 mmol) and the mixture was stirred at room temperature for 3 hours, followed by further stirring at 60˚C for 4 hours. After cooling, the mixture was added with diisopropylether/hexane (1:1) and stirred to remove the supernatant. The residue was triturated with n-hexane/ethyl acetate (1:1) to obtain the titled compound (532 mg, 41.1 %) as a white solid.
$^1H$ NMR ($CDCl_3$, δppm) : 1.49-1.66 (m, 4H), 1.98 (d, J=11.2Hz, 4H), 2.15 (t, J = 11.6 Hz, 4H), 2.82 (d, J = 11.6 Hz, 4H), 3.50 (s,4H), 3.53-3.65 (m, 2H), 4.71 (d, J = 7.9 Hz, 2H), 7.22-7.36 (m, 10H).
Melting point: 147-148˚C.
APCIMS m/z: $[M+H]^+$455.

Example 240

[Bis(1-tert-butoxycarbonylpiperidin-4-ylamino) methylene]malononitrile (Compound 240)

[Bis(methylthio)methylene]malononitrile (0.88 g, 5.17 mmol) was added with 1-tert-butoxycarbonyl-4-aminopiperidine (4.80 g, 24.0 mmol) and the mixture was stirred at 100˚C for 2 hours. After cooling, the reaction mixture was purified by silica gel column chromatography (n-hexane/ethyl acetate (1:1)) to obtain the titled compound (1.00 g, 40.8 %) as a white solid.
$^1$H NMR ($CDCl_3$, δppm): 1.38-1.43 (m, 4H), 1.46 (s, 18H), 1.95-2.04 (m, 4H), 2.88 (t, J = 12.7 Hz, 4H), 3.69-3.77 (m, 2H), 4.08 (d, J = 12.7 Hz, 4H), 4.78 (d, J = 8.3 Hz, 2H).

Example 241

[Bis(1-isopropylpiperidin-4-ylamino)methylene] malononitrile (Compound 241)

[Bis(1-tert-butoxycarbonylpiperidin-4-ylamino) methylene]malononitrile (Compound 240) (1.00 g, 2.11 mmol) obtained in Example 240 was dissolved in ethyl acetate (10 mL) and the solution was added with 4 mol/L hydrochloric acid-ethyl acetate (10.0 mL, 40.0 mmol), followed by stirring at room temperature for 2 hours. The precipitated solid was collected by filtration and washed with ethyl acetate to obtain a white solid (0.88 g).
The obtained solid, potassium carbonate (2.90 g, 21.0 mmol) and isopropyl iodide (1.10 mL, 11.4 mmol) were dissolved in 1,4-dioxane (20 mL) and the solution was stirred at 60˚C for 17 hours. The mixture was further added with isopropyl iodide (1.10 mL, 11.4 mmol) and stirred at 60˚C for 2 hours. After cooling, the mixture was added with water and extracted with chloroform. The organic layer was washed with saturated brine, dried over anhydrous magnesium sulfate and the solvent was evaporated under reduced pressure. The residue was purified by NH silica gel column chromatography (chloroform/methanol (9:1)) to obtain the titled compound (0.225 g, 29.8 %) as a clear oily substance.
$^1$H NMR ($CDCl_3$, δppm) : 1.03 (d, J = 6.6 Hz, 12H), 1.47-1.65 (m, 4H), 1.97-2.08 (m, 4H), 2.21-2.35 (m, 4H), 2.75 (sept, J = 6.6 Hz, 2H), 2.78-2.89 (m, 4H), 3.50-3.64 (m, 2H), 4.83 (d, J = 8.3 Hz, 2H).
APCIMS m/z: $[M+H]^+$359.

Example 242

{3-[2-(2-Methylpyrrolidin-1-yl)ethyl]-1-[3-(2-methylpyrrolidin-1-yl)propyl]imidazolidin-2-ylidene} benzenesulfonylacetonitrile (Compound 242)

(Step 1)

Potassium hydroxide (7.30 g, 130 mmol) was dissolved in ethanol (60 mL) and the solution was added with benzenesulfonylacetonitrile (10.0 g, 55.2 mmol), dissolved at 50˚, and then carbon disulfide (4.65 mL,77.3 mmol) was added thereto dropwise, followed by stirring for 1 hour. After cooling the mixture, the precipitated solid was collected by filtration and washed with ethanol to obtain a white solid (9.17 g).
The obtained solid (9.17 g) was dissolved in DMF (35 ml) and the solution was added with methyl iodide (6.85 mL, 110 mmol), followed by stirring at 50˚C for 2 hours. After cooling the mixture, the solvent was evaporated under reduced pressure. The residue was added with water and extracted with ethyl acetate. The organic layer was washed with saturated brine, dried over anhydrous magnesium sulfate and the solvent was evaporated under reduced pressure. The obtained solid was washed with n-hexane-ethyl acetate and then with acetone to obtain 2-benzenesulfonyl-3,3-bis (methylsulfanyl)acrylonitrile (2.86 g, 18.2 %) as a yellow solid.
$^1$H NMR (DMSO-$d_6$, δppm) : 2.49 (s, 3H), 2.68 (s, 3H), 7.63-7.69 (m, 2H), 7.74-7.79 (m, 1H), 7.91-7.94 (m, 2H).

(Step 2)

2-Benzenesulfonyl-3,3-bis(methylsulfanyl) acrylonitrile (0.70 g, 2.45 mmol) obtained in the Step 1 was dissolved in THF (3 mL) and the solution was added with N-(2-hydroxyethyl)ethylenediamine (0.31 g, 2.57 mmol), followed by stirring at room temperature for 1 hour. The mixture was added with saturated brine and extracted with ethyl acetate. The organic layer was washed with saturated brine, dried over anhydrous magnesium sulfate and the solvent was evaporated under reduced pressure to obtain the residue.
The residue (0.72 g) was dissolved in DMF (4 mL) and the solution was added with potassium carbonate (0.67 g, 4.85 mmol) and 3-(bromopropoxy)-tert-butyldimethylsilane (0.9 mL, 3.68 mmol), followed by stirring at 80˚C for 23 hours. Further, the mixture was added with potassium carbonate (0.34 g, 2.45 mmol), 3-(bromopropoxy)-tert-butyldimethylsilane (0.6 mL, 2.45 mmol) and sodium iodide (0.37 g, 2.45 mmol), followed by stirring at 80˚C for 4.5 hours. The mixture was cooled, added with saturated brine and extracted with ethyl acetate. The organic layer was washed with saturated brine and the solvent was evaporated under reduced pressure. The residue was purified by silica gel chromatography (chloroform/methanol (100:1 to 10:1)) to obtain {1-[3-(tert-butyldimethylsilyloxy)propyl] -3-(2-hydroxyethyl) imidazolidin-2-ylidene} benzenesulfonylacetonitrile (1.2 g, 84.5 %).
$^1$H NMR ($CDCl_3$, δppm) : 0.07 (s, 6H), 0.85 (s, 9H), 1.72-1.79 (m, 2H), 3.40-3.52 (m, 4H), 3.62-3.66 (m, 2H), 3.71-3.79 (m, 4H), 3.80-3.87 (m, 2H), 7.25-7.52 (m, 3H), 7.89-7.93 (m, 2H).

(Step 3)

{1-[3-(Tert-butyldimethylsilyloxy)propyl]-3-(2-hydroxyethyl)imidazolidin-2-ylidene} benzenesulfonylacetonitrile (1.20 g, 2.07 mmol) obtained in the Step 2 was dissolved in dichloromethane (20 mL) and the solution was added with triethylamine (1.2 mL, 8.28 mmol) and benzenesulfonyl chloride (0.32 mL, 4.14 mmol), followed by stirring at room temperature for 2 hours. The mixture was added with saturated brine and extracted with chloroform. The organic layer was washed with saturated brine and the solvent was evaporated under reduced pressure to obtain the residue.

The residue (1.13 g) was dissolved in 1,4-dioxane (20 mL) and the solution was added with potassium carbonate (0.75 g, 5.43 mmol), 2-methylpyrrolidine (0.38 mL, 3.72 mmol) and sodium iodide (0.27 g, 1.80 mmol), followed by stirring at 80˚C for 24 hours. After cooling, the mixture was added with saturated brine and extracted with ethyl acetate. The organic layer was washed with saturated brine, dried over anhydrous magnesium sulfate and the solvent was evaporated under reduced pressure. The residue was purified by silica gel column chromatography (chloroform/methanol (10:1)) to obtain {1-[3-(tert-butyldimethylsilyloxy)propyl]-3-[2-(2-methylpyrrolidin-1-yl)ethyl]imidazolidin-2-ylidene} benzenesulfonylacetonitrile (0.18 g, 16.4 %).
$^1$H NMR ($CDCl_3$, δppm) : 0.02 (s, 6H), 0.85 (s, 9H), 1.02 (d,J= 6.0 Hz, 3H), 1.32-1.39 (m, 1H), 1.66-1.98 (m, 5H), 2.17-2.33 (m, 2H), 2.96-3.07 (m, 2H), 3.42-3.53 (m, 6H), 3.55-3.79 (m, 4H), 3.80-3.90 (m, 1H), 7.46-7.51 (m, 3H), 7.90-7.94 (m, 2H).

(Step 4)

{1-[3-(Tert-butyldimethylsilyloxy)propyl]-3-[2-(2-methylpyrrolidin-1-yl)ethyl]imidazolidin-2-ylidene} benzenesulfonylacetonitrile (0.18 g, 0.34 mmol) obtained in the Step 3 was dissolved in ethyl acetate (5 mL) and the solution was added with 4 mol/L hydrochloric acid-ethyl acetate (1 mL), followed by stirring at room temperature for 2 hours. The mixture was concentrated under reduced pressure and 2 mol/L aqueous sodium hydroxide solution was added dropwise to the residue to adjust the pH to 10. Then, the mixture was extracted with chloroform. The organic layer was washed with saturated brine, dried over anhydrous magnesium sulfate and the solvent was evaporated under reduced pressure to obtain the residue.
The residue (0.11 g) was dissolved in dichloromethane (5 mL) and the solution was added with triethylamine (0.14 mL, 1.04 mmol) and methanesulfonylchloride (0.04 mL, 0.52 mmol), followed by stirring at room temperature for 1 hour. The mixture was added with saturated aqueous sodium hydrogencarbonate solution and extracted with chloroform. The organic layer was washed with saturated brine and the solvent was evaporated under reduced pressure to obtain {1-(3-methanesulfonyloxypropyl)-3-[2-(2-methylpyrropydin-1-yl) ethyl]imidazolidin-2-ylidene}benzenesulfonylacetonitrile (0.14 g, 76.5 %).
$^1$H NMR ($CDCl_3$, δppm) : 1.08 (d, J = 5.90 Hz, 3H), 1.34-1.43 (m, 2H), 1.64-1.79 (m, 2H), 1.87-1.98 (m, 1H), 2.08-2.15 (m, 2H), 2.26-2.44 (m, 1H), 3.03 (s, 3H), 3.00-3.09 (m, 3H), 3.60-3.89 (m, 8H), 4.24 (t, J= 5.9Hz, 2H), 7.47-7.53 (m, 3H), 7.91-7.95 (m, 2H).

(Step 5)

{1-(3-Methanesulfonyloxypropyl)-3-[2-(2-methylpyrropydin-1-yl)ethyl]imidazolidin-2-ylidene} benzenesulfonylacetonitrile (0.14 g, 0.26 mmol) obtained in the Step 4 was dissolved in 1,4-dioxane (5 mL) and the solution was added with potassium carbonate (0.11 g, 0.78 mmol) and 2-methylpyrrolidine (0.05 mL, 0.52 mmol), followed by stirring at 40˚C for 24 hours. After cooling, the mixture was added with saturated brine and extracted with chloroform. The organic layer was washed with saturated brine, dried over anhydrous magnesium sulfate and the solvent was evaporated under reduced pressure. The residue was purified by silica gel column chromatography (chloroform/(2 mol/L ammonia-methanol) (10:1) to obtain the titled compound (0.05 g, 39.2 %) as a clear and colorless oily substance.
$^1$H NMR ($CDCl_3$, δppm) : 1.03 (d, J = 6.2 Hz, 3H), 1.05 (d, J=5.9 Hz, 3H), 1.27-1.46 (m, 2H), 1.63-2.08 (m, 12H), 2.17-2.34 (m, 2H), 2.59-2.69 (m, 1H), 2.98-3.09 (m, 3H), 3.36-3.50 (m, 3H), 3.61-3.79 (m, 4H), 3.82-3.92 (m, 1H), 7.43-7.64 (m, 3H), 7.93-7.99 (m, 2H).
APCIMS m/z: $[M+H]^+$ 486.

Example 243

{3-[2-((R)-2-methylpyrrolidin-1-yl)ethyl]-1-[3-((S) -2-methylpyrrolidin-1-yl)propy]imidazolidin-2-ylidene} benzenesulfonylacetonitrile (Compound 243)

In a similar manner to Steps 3 to 5 of Example 242, the titled compound (0.005 g, 3.0 %) was obtained as a clear and colorless oily substance by using {1-[3-(tert-butyldimethylsilyloxy)propyl]-3-(2-hydroxyethyl) imidazolidin-2-ylidene}benzenesulfonylacetonitrile (0.62 g, 0.79 mmol), (R)-2-methylpyrrolidine hydrobromate (0.50 g, 3.02 mmol) in place of 2-methylpyrrolidine of Step 3, and (S)-2-methylpyrrolidine hydrobromate (0.13 g, 0.77 mmol) in place of 2-methylpyrrolidine of Step 5.
$^1$H NMR ($CDCl_3$, δppm) : 1.04 (d, J = 5.9 Hz, 6H), 1.31-1.43 (m, 2H), 1.65-2.08 (m, 10H), 2.18-2.35 (m, 3H), 2.63 (td, J = 7.9, 11.9 Hz, 2H), 2.98-3.08 (m, 3H), 3.39-3.53 (m, 3H), 3.59-3.75 (m, 4H), 3.82-3.92 (m, 1H), 7.44-7.52 (m, 3H), 7.93-7.97 (m, 2H).
APCIMS m/z: $[M+H]^+$486.

Example 244

{1,3-Bis[3-(2-methylpyrrolidin-1-yl)propyl] imidazolidin-2-ylidene}benzenesulfonylacetonitrile (Compound 244)

(Step 1)

2-Benzenesulfonyl-3,3-bis(methylsulfanyl) acrylonitrile (1.02 g, 3.58 mmol) obtained in Step 1 of Example 242 was dissolved in THF (4 mL) and the solution was added with N-(3-hydroxypropyl)ethylenediamine (0.44g, 3.75 mmol) dissolved in THF (3 mL), followed by stirring at room temperature for 2 hours. The mixture was added with saturated brine and extracted with ethyl acetate. The organic layer was washed with saturated brine, dried over anhydrous magnesium sulfate and the solvent was evaporated under reduced pressure to obtain [1-(3-hydroxypropyl)imidazolidin-2-ylidene] benzenesulfonylacetonitrile (1.09 g, 99 %) as a clear and colorless oily substance.
$^1$H NMR ($CDCl_3$, δppm): 1.84-1.92 (m, 2H), 3.61-3.74 (m, 8H), 7.38 (brs, 1H), 7.47-7.59 (m, 3H), 7.89-7.93 (m, 2H).

(Step 2)

[1-(3-Hydroxypropyl)imidazolidin-2-ylidene] benzenesulfonylacetonitrile (0.62 g, 2.02 mmol) obtained in the Step 1 was dissolved in DMF (6mL) and the solution was added with potassium hydroxide (0.14 g, 2.42 mmol) and 1,3-dibromopropane (0.62 mL, 6.06 mmol), followed by stirring at room temperature for 2.5 hours. The mixture was added with saturated brine and extracted with ethyl acetate. The organic layer was washed with saturated brine and the solvent was evaporated under reduced pressure to obtain the residue.

The residue (0.72 g) was dissolved in dichloromethane (4.5 mL) and the solution was added with triethylamine (0.93 mL, 6.70 mmol) and methanesulfonylchloride (0.94 mL, 2.51 mmol), followed by stirring at room temperature for 1 hour. The mixture was added with saturated brine and extracted with chloroform.

The organic layer was washed with saturated brine and the solvent was evaporated under reduced pressure. The residue was purified by silica gel chromatography (chloroform/methanol (100:1 to 10:1)) to obtain [3-(3-bromopropyl)-1-(3-methanesulfonyloxypropyl)imidazolidin-2-ylidene]benzenesulfonylacetonitrile (0.70 g, 82.0 %).
$^1$H NMR ($CDCl_3$, δppm) : 2.08-2.17 (m, 4H), 3.04 (s, 3H), 3.26 (t, J = 6.3 Hz, 2H), 3.49-3.54 (m, 2H), 3.63-3.74 (m, 6H), 4.23-4.32 (m, 2H), 7.48-7.58 (m, 3H), 7.89-7.95 (m, 2H).

(Step 3)

[3-(3-Bromopropyl)-1-(3-methanesulfonyloxypropyl) imidazolidin-2-ylidene]benzenesulfonylacetonitrile (0.69 g, 1.36 mmol) obtained in the Step 2 was dissolved in 1,4-dioxane(4 mL) and the solution was added with potassium carbonate (0.75 g, 5.44 mmol), 2-methylpyrrolidine (0.56 mL, 5.44 mmol) and sodium iodide (0.20 g, 1.36 mmol), followed by stirring at 40°C for 17 hours. After cooling, the mixture was added with saturated brine and extracted with ethyl acetate. The organic layer was washed with saturated brine, dried over anhydrous magnesium sulfate and the solvent was evaporated under reduced pressure. The residue was purified by silica gel column chromatography (chloroform/methanol (3:1)) to obtain the titled compound (0.12 g, 17.4 %) as a clear and colorless oily substance.
$^1$H NMR ($CDCl_3$, δppm) : 1.04 (t, J = 6.3 Hz, 6H), 1.31-1.44 (m, 2H), 1.63-2.07 (m, 14H), 2.20-2.30 (m, 2H), 2.63 (td, J = 8.0,11.9 Hz, 2H), 3.05 (td, J = 8.0, 3.3 Hz, 2H), 3.35-3.59 (m, 4H), 3.64-3.77 (m, 4H), 7.43-7.53 (m, 3H), 7.94-7.97 (m, 2H).
APCIMS m/z: $[M+H]^+$500.

Example 245

{1,3-Bis[3-((R)-2-methylpyrrolidin-1-yl)propyl] imidazolidin-2-ylidene}benzenesulfonylacetonitrile (Compound 245)

(Step 1)

[1-(3-Hydroxypropyl)imidazolidin-2-ylidene] benzenesulfonylacetonitrile (2.15 g,7.01 mmol) obtained in Step 1 of Example 244 was dissolved in DMF (10 mL) and the solution was added with potassium carbonate (2.9 g, 21.03 mmol) and 3-bromopropoxy-tert-butyldimethylsilane (3.2 mL, 14.02 mmol), followed by stirring at 80˚C for 3 hours. Further, the mixture was added with potassium carbonate (1.9 g, 14.02 mmol), 3-bromopropoxy-tert-butyldimethylsilane (1.6 mL, 7.01 mmol) and sodium iodide (1.1 g, 7.01 mmol), followed by stirring at 80˚C for 18 hours. After cooling, the mixture was added with saturated brine and extracted with ethyl acetate. The organic layer was washed with saturated brine and the solvent was evaporated under reduced pressure. The residue was purified by silica gel chromatography (chloroform/methanol (100:1 to 10:1)) to obtain {1-[3-(tert-butyldimethylsilyloxy)propyl]-3-(3-hydroxypropyl) imidazolidin-2-ylidene}benzenesulfonylacetonitrile (2.44 g, 72.6 %).
$^1$H NMR ($CDCl_3$, δppm) : 0.05 (s, 6H), 0.87 (s, 9H), 1.64-1.70 (m, 2H), 1.72-1.87 (m, 2H), 1.87-1.97 (m, 2H), 2.15-2.19 (m, 1H), 3.32-3.45 (m, 4H), 3.51-3.70 (m, 6H), 7.38-7.47 (m, 3H), 7.85-7.89 (m, 2H).

(Step 2)

{1-[3-(Tert-butyldimethylsilyloxy)propyl]-3-(3-hydroxypropyl)imidazolidin-2-ylidene} benzenesulfonylacetonitrile (2.44 g, 5.09 mmol) obtained in the Step 1 was dissolved in dichloromethane (40 mL) and the solution was added with triethylamine (2.8 mL, 20.36 mmol) and methanesulfonylchloride (0.8 mL, 10.18 mmol), followed by stirring at room temperature for 1 hour. The mixture was added with saturated brine and extracted with chloroform. The organic layer was washed with saturated brine and the solvent was evaporated under reduced pressure. The residue was purified by silica gel chromatography (chloroform/methanol (100:1 to 10:1)) to obtain {1-[3-(tert-butyldimethylsilyloxy)propyl]-3-(3-methanesulfonyloxypropyl)imidazolidin-2-ylidene} benzenesulfonylacetonitrile (1.60 g, 56.4 %).
$^1$H NMR ($CDCl_3$, δppm) : 0.01 (s, 6H), 0.86 (s, 9H), 1.61-1.78 (m, 2H), 2.07-2.16 (m, 2H), 3.03 (s, 3H), 3.42 (t, J = 7.9 Hz, 2H), 3.49 (t, J =6.0 Hz, 2H), 3.61-3.75 (m, 6H), 4.25 (t, J = 6.0 Hz, 2H), 7.45-7.55 (m, 2H), 7.90-7.94 (m, 3H).

(Step 3)

{1-[3-(Tert-butyldimethylsilyloxy)propyl]-3-(3-methanesulfonyloxypropyl)imidazolidin-2-ylidene} benzenesulfonylacetonitrile (1.00 g, 1.80 mmol) obtained in the Step 2 was dissolved in 1,4-dioxane (10 mL) and the solution was added with a solution of (R)-2-methylpyrrolidine hydrobromate (0.54 g, 3.24 mmol) in a mixed solvent of 1,4-dioxane (10 mL) and 2 mol/L aqueous sodium hydroxide solution (1.7 mL, 3.42 mmol). Furhter, the mixture was added with potassium carbonate (0.75 g, 5.4 mmol) and stirred at 80˚C overnight. After cooling, the mixture was added with saturated brine and extracted with ethyl acetate. The organic layer was washed with saturated brine, dried over anhydrous magnesium sulfate and the solvent was evaporated under reduced pressure. The residue was purified by silica gel column chromatography (chloroform/methanol (100:1 to 10:1)) to obtain {1-[3-(tert-butyldimethylsilyloxy)propyl]-3-[3-((R)-2-methylpyrrolidin-1-yl)propyl]imidazolidin-2-ylidene}benzenesulfonylacetonitrile (0.70 g, 71.2 %).
$^1$H NMR ($CDCl_3$, δppm) : 0.02 (s, 6H), 0.86 (m, 9H), 1.03 (d,J = 5.9 Hz, 3H), 1.34-1.41 (m, 1H), 1.62-2.02 (m, 9H), 2.20-2.28 (m, 1H), 2.58-2.68 (m, 1H), 3.04-3.09 (m, 1H), 3.44-3.54 (m, 6H), 3.64-3.68 (m, 4H), 7.41-7.49 (m, 3H), 7.91-7.94 (m, 2H).

(Step 4)

{1-[3-(Tert-butyldimethylsilyloxy)propyl]-3-[3-((R)-2-methylpyrrolidin-1-yl)propyl]imidazolidin-2-ylidene} benzenesulfonylacetonitrile (0.70 g, 1.28 mmol) obtained in the Step 3 was dissolved in ethyl acetate (2 mL) and the solution was added with 4 mol/L hydrochloric acid-ethyl acetate (1 mL), followed by stirring at room temperature for 2 hours. The mixture was concentrated under reduced pressure and 2 mol/L aqueous sodium hydroxide solution wasa added dropwise to the residue to adjust the pH to 10. Then, the mixture was extracted with ethyl acetate. The organic layer was washed with saturated brine, dried over anhydrous magnesium sulfate and the solvent was evaporated under reduced pressure to obtain the residue.

The residue (0.55 g) was dissolved in dichloromethane (10 mL) and the solution was added with triethylamine (0.70 mL, 5.08 mmol) and methanesulfonylchloride (0.20 mL, 2.54 mmol), followed by stirring at room temperature for 1 hour. The mixture was added with saturated aqueous sodium hydrogencarbonate solution and extracted with chloroform. The organic layer was washed with saturated brine and the solvent was evaporated under reduced pressure to

obtain [1-(3-methanesulfonyloxypropyl)-3-[3-((R)-2-methylpyrrolidin-1-yl)propyl]imidazolidin-2-ylidene) benzenesulfonylacetonitrile (0.59 g, 94.8 %).
$^1$H NMR ($CDCl_3$, δppm) : 0.98 (d, J = 5.9 Hz, 3H), 1.29-1.39 (m, 1H), 1.61-1.73 (m, 6H), 1.76-2.01 (m, 2H), 2.05-2.12 (m, 2H), 2.50-2.60 (m, 1H), 2.94-2.98 (m, 1H), 2.99 (s, 3H), 3.34 (dt, J = 3.4, 7.7 Hz, 2H), 3.59-3.68 (m, 6H), 4.22 (t, J = 5.9 Hz, 2H), 7.41-7.48 (m, 2H), 7.87-7.91 (m, 3H).

(Step 5)

{1-(3-Methanesulfonyloxypropyl)-3-[3-((R)-2-methylpyrrolidin-1-yl)propyl]imidazolidin-2-ylidene} benzenesulfonylacetonitrile (0.29 g, 0.59 mmol) obtained in the Step 4 was dissolved in 1,4-dioxane (5 mL) and the solution was added with a solution of (R)-2-methylpyrrolidine hydrobromate (0.18 g, 1.06 mmol) in a mixed solvent of 1,4-dioxane (5 mL) and 2 mol/L aqueous sodium hydroxide solution (0.6 mL, 1.12 mmol). The mixture was further added with potassium carbonate (0.24 g, 1.77 mmol) and stirred at 80˚C overnight. After cooling, the mixture was added with saturated brine and extracted with ethyl acetate. The organic layer was washed with saturated brine, dried over anhydrous magnesium sulfate and the solvent was evaporated under reduced pressure. The residue was purified by silica gel column chromatography (chloroform/(2 mol/L ammonia-methanol) (3:1)) to obtain the titled compound (0.029 g, 9.9 %) as a clear and colorless oily substance.
$^1$H NMR ($CDCl_3$, δppm) : 1.04 (d, J = 5.8 Hz, 6H), 1.39-1.44 (m, 2H), 1.64-2.08 (m, 14H), 2.17-2.29 (m, 2H), 2.64 (dt, J = 11.0, 8.0 Hz, 2H), 3.07 (dt, J = 2.5, 8.0 Hz, 2H), 3.36-3.52 (m, 4H), 3.64-3.73 (m, 4H), 7.45-7.51 (m, 3H), 7.96 (dd, J = 1.5, 7.5 Hz, 2H).
APCIMS m/z: $[M+H]^+$500.

Example 246

{1-[3-((S)-2-methylpyrrolidin-1-yl)propyl]-3-[3-((R)-2-methylpyrrolidin-1-yl)propyl]imidazolidin-2-ylidene}benzenesulfonylacetonitrile (Compound 246)

In a similar manner to step 5 of Example 245, the titled compound (0.078 g, 17.2 %) was obtained as a clear and colorless oily substance by using {1-(3-methanesulfonyloxypropyl)-3-[3-((R)-2-methylpyrrolidin-1-yl)propyl]imidazolidin-2-ylidene} benzenesulfonylacetonitrile (0.29 g, 0.59 mmol), (S)-2-methylpyrrolidine hydrobromate (0.18 g, 1.06 mmol) in place of (R)-2-methylpyrrolidine hydrobromate.
$^1$H NMR ($CDCl_3$, δppm) : 1.04 (d, J = 6.2 Hz, 6H), 1.34-1.44 (m, 2H), 1.62-2.09 (m, 14H), 2.17-2.29 (m, 2H), 2.64 (dt, J = 11.7, 7.9 Hz, 2H), 3.07 (dt, J = 2.9, 8.3 Hz, 2H), 3.36-3.54 (m, 4H), 3.64-3.73 (m, 4H), 7.44-7.51 (m, 3H), 7.94-7.97 (m, 2H).
APCIMS m/z: $[M+H]^+$500.

Example 247

{1,3-Bis[3-((S)-2-methylpyrrolidin-1-yl)propyl] imidazolidin-2-ylidene}benzenesulfonylacetonitrile monohydrochloride (Compound 247)

In a similar manner to Steps 3 to 5 of Example 245, {1,3-bis[3-((S)-2-methylpyrrolidin-1-yl)propyl] imidazolidin-2-ylidene}benzenesulfonylacetonitrile (0.095 g, 0.19 mmol) was obtained by using {1-[3-(tert-butyldimethylsilyloxy)propyl]-3-(3-methanesulfonyloxypropyl)imidazolidin-2-ylidene} benzenesulfonylacetonitrile (0.45 g, 0.81 mmol), (S)-methylpyrrolidine hydrobromate (0.24 g, 1.46 mmol) in place of (R)-methylpyrrolidine hydrobromate of Step 3 and (S)-methylpyrrolidine hydrobromate (0.10 g, 0.63 mmol) in place of (R)-methylpyrrolidine hydrobromate of Step 5.
The obtained {1,3-bis[3-((S)-2-methylpyrrolidin-1-yl)propyl]imidazolidin-2-ylidene} benzenesulfonylacetonitrile (0.095 g) was dissolved in ethyl acetate (5 mL) and the solution was added with 4 mol/L hydrochloric acid-ethyl acetate (0.2 mL), followed by stirring at room temperature for 1 hour. The precipitated solid was collected by filtration and washed with ethyl acetate to obtain the titled compound (0.063 g, 57.6 %) as a white amorphous.
$^1$H NMR (DMSO-$d_6$, δppm) : 1.34 (d, J = 6.4 Hz, 6H), 1.53-1.66 (m, 2H), 1.84-2.08 (m, 11H), 2.11-2.21 (m, 2H), 2.72-2.85 (m, 3H), 2.92-3.04 (m, 2H), 3.06-3.17 (m, 2H), 3.36-3.43 (m, 2H), 3.47-3.57 (m, 2H), 3.75 (s, 4H), 7.58-7.63 (m, 3H), 7.78-7.81 (m, 2H).
APCIMS m/z: $[M+H]^+$500.

Example 248

[1-(1-Benzylpiperidin-4-yl)-3-(3-piperidinopropyl) imidazolidin-2-ylidene]methanesulfonylacetonitrile

(Compound 248)

In a similar manner to Example 126, the titled compound (0.19 g, 30.0 %) was obtained as a clear oily substance by using 2-methanesulfonyl-3,3-bis (methylsulfanyl)acrylonitrile (1.99 g, 8.93 mmol) obtained in Step 1 of Example 136 in place of [bis(methylthio) methylene]malononitrile of Step 1.
$^1$H NMR ($CDCl_3$, δppm) : 1.41-1.44 (m, 2H), 1.54-1.61 (m, 4H), 1.71-1.91 (m, 6H), 2.13 (dt, J = 2.0, 11.6 Hz, 2H), 2.30-2.36 (m, 6H), 2.94-2.99 (m, 2H), 3.15 (s, 3H), 3.50 (s, 2H), 3.54 (t, J = 7.6 Hz, 2H), 3.64 (s, 4H), 4.00-4.11 (m, 1H), 7.26-7.34 (m, 5H).

Example 249

[1-(Piperidin-4-yl)-3-(3-piperidinopropyl) imidazolidin-2-ylidene]methanesulfonylacetonitrile dihydrochloride (Compound 249)

In a similar manner to Example 185, the titled compound (0.17 g, 99 %) was obtained as a brown solid by using [1-(1-benzylpiperidin-4-yl)-3-(3-piperidinopropyl) imidazolidin-2-ylidene]methanesulfonylacetonitrile (Compound 248) (0.17 g, 0.36 mmol) obtained in Example 248.
$^1$H NMR ($CD_3OD$, δppm) : 1.77-1.86 (m, 3H), 1.93-2.01 (m, 3H), 2.06-2.21 (m, 6H), 2.95 (t, J = 12.4 Hz, 2H), 3.12-3.18 (m, 7H), 3.51-3.65 (m, 6H), 3.80 (s, 4H), 4.25-4.33 (m, 1H).

Example 250

2-[1-(1-Isopropylpiperidin-4-yl)-3-(3-piperidinopropyl)imidazolidin-2-ylidene] methanesulfonylacetonitrile (Compound 250)

In a similar manner to Example 130, the titled compound (0.02 g, 12.7 %) was obtained as a white solid by using [1-(piperidin-4-yl)-3-(3-piperidinopropyl) imidazolidin-2-ylidene]methanesulfonylacetonitrile dihydrochloride (Compound 249) (0.17 g, 0.36 mmol) obtained in Example 249.
$^1$H NMR ($CDCl_3$, δppm) : 1.05 (d, J = 6.3 Hz, 6H), 1.41-1.48 (m, 2H), 1.54-1.67 (m, 4H), 1.71-1.96 (m, 6H), 2.26-2.39 (m, 8H), 2.72-2.81 (m, 1H), 2.96 (brd, J = 2.2 Hz, 2H), 3.16 (s, 3H), 3.55 (t, J = 7.6 Hz, 2H), 3.66 (s, 4H), 3.97-4.06 (m, 1H).
APCIMS m/z: $[M+H]^+$437.
Melting point: 98-99˚C.

Reference example 1: 4-(N-methyl-N-phenylamino) piperidine dihydrochloride

(Step 1)

1-(Tert-butyloxycarbonyl)piperidine-4-one (980 mg, 4.92 mmol) was dissolved in 1,2-dichloroethane (10 mL), and the solution was added with aniline (0.449 mL, 4.92 mmol), acetic acid (0.847 mL, 14.8 mmol) and triacetoxy sodium borohydride (1.15 g, 5.41 mmol) under ice-cooling, followed by stirring at room temperature for 4 hours. Then, triacetoxy sodium borohydride (1.15 g, 5.41 mmol) was further added thereto, followed by stirring at room temperature for 12 hours. To the resulting reaction mixture, a saturated aqueous sodium hydrogen carbonate solution was added, and the reaction mixture was extracted with ethyl acetate. The resulting organic layer was washed with a saturated brine and dried over anhydrous magnesium sulfate, and then, the solvent was evaporated under reduced pressure. The residue was purified by silica gel column chromatography (n-hexane/ethyl acetate (9:1) to obtain 1-(tert-butyloxycarbonyl)-4-(phenylamino) piperidine (695 mg, 51%) as a pale yellow solid.
$^1$H NMR ($CDCl_3$, δppm) : 1.22-1.44 (m, 2H), 1.47 (s, 9H), 2.04 (d, J = 13.3 Hz, 2H), 2.93 (t, J = 12.8 8 Hz, 2H), 3.43 (m, 1H), 3.49 (br s, 1H), 4.04 (d, J = 12.8 Hz, 2H), 6.61 (d, J = 7.7 Hz, 2H), 6.70 (t, J = 7.7 Hz, 1H), 7.17 (t, J = 7.7 Hz, 2H)

(Step 2)

1-(Tert-butyloxycarbonyl)-4-(phenylamino)piperidine (690 mg, 2.50 mmol) obtained in the Step 1 was dissolved in DMF (7 ml), and the solution was added with potassium carbonate (691 mg, 5.00 mmol) and methyl iodide (0.235 mL, 3.75 mmol), followed by stirring at 60˚C for 6 hours. Then, potassium carbonate (346 mg, 2.50 mmol) and methyl iodide (0.156 mL, 2.50 mmol) were further added thereto, followed by stirring at 60˚C for 1.5 hours. After cooling the reaction mixture, a saturated brine was added thereto, and the reaction mixture was extracted with ethyl acetate. The organic layer was washed with a saturated brine and dried over anhydrous magnesium sulfate, and then, the solvent was evaporated under reduced pressure. The residue was purified by silica gel column chromatography (n-hexane/ethyl acetate (9:1) to obtain 1-(tert-butyloxy carbonyl)-4-(N-methyl-N-phenylamino)piperidine (332 mg, 46%) was obtained as a pale yellow solid.
$^1H$ NMR ($CDCl_3$, δppm) : 1.47 (s, 9H), 1.60-1.77 (m, 4H), 2.69-2.84 (m, 5H), 3.71 (m, 1H), 4.23 (d, J = 12.4 Hz, 2H), 6.73 (t, J = 7.7 Hz, 1H), 6.81 (d, J = 7.7 Hz, 2H), 7.24 (t, J = 7.7 Hz, 2H)

(Step 3)

1-(Tert-butyloxycarbonyl)-4-(N-methyl-N-phenylamino) piperidine (330 mg, 1.14 mmol) obtained in the above-described Step 2 was dissolved in ethyl acetate (6 mL), and a 4 mol/L hydrogen chloride/ethyl acetate solution (0.860 mL, 3.44 mmol) was added thereto, followed by stirring at room temperature for 4 hours. Then, the solvent was evaporated under reduced pressure to obtain the titled compound (300 mg, 100%)as a brown crystal.

Reference Example 2:4-Piperidinomethylpiperidine dihydrochloride

(Step 1)

1-(Tert-butyloxycarbonyl)-4-(hydroxymethyl) piperidine (5.04 g, 23.4 mmol) was dissolved in dichloromethane (60 mL) and the solution was added with sodium acetate (3.80 g, 46.3 mmol), pyridinium chlorochromate (10.1 g, 46.9 mmol) and silica gel (10 g) under ice bath, followed by stirring at room temperature for 1 hour. The solvent was evaporated under reduced pressure and the residue was purified by silica gel column chromatography (hexane/ethyl acetate (9:1)) to obtain 1-(tert-butyloxycarbonyl)-4-formylpiperidine (4.28 g, 86 %) as a clear oily substance.
$^1H$ NMR ($CDCl_3$, δppm) : 1.46 (s, 9H), 1.49-1.73 (m, 2H), 1.82-1.97 (m, 2H), 2.35-2.53 (m, 1H), 2.88-2.98 (m, 2H), 3.90-4.07 (m, 2H), 9.66 (s, 1H).

(Step 2)

1-(Tert-butyloxycarbonyl)-4-formylpiperidine (3.81 g, 17.9 mmol) obtained in the Step 1 was dissolved in dichloromethane (40 mL) and the solution was added with piperidine (2.70 mL, 27.3 mmol) and triacetoxy sodium borohydride (5.30 g, 25.0 mmol) under ice bath, followed by stirring at room temperature for 1 hour. The reaction mixture was added with saturated aqueous sodium hydrogencarbonate solution and extracted with chloroform. After washing with saturated brine, the organic layer was dried over anhydrous magnesium sulfate and the solvent was evaporated under reduced pressure. The residue was purified by silica gel column chromatography (hexane to chloroform/methanol (9:1)) to obtain 1-(tert-butyloxycarbonyl)-4-piperidinomethylpiperidine (2.88 g, 57 %) as a clear oily substance.
$^1H$ NMR ($CDCl_3$, δppm) : 1.36-1.48 (m, 12H), 1.48-1.76 (m, 8H), 2.11 (d, J = 6.6 Hz, 2H), 2.25-2.37 (m, 4H), 2.67 (t, J = 12.3 Hz, 2H), 4.06 (d, J = 12.3 Hz, 2H).

(Step 3)

1-(Tert-butyloxycarbonyl)-4-piperidinomethyl piperidine (2.88 g, 10.2 mmol) obtained in the Step 2 was dissolved in ethyl acetate (30 mL) and the solution was added with 4 mol/L hydrogen chloride-ethyl acetate solution (20.0 mL, 80.0 mmol), followed by stirring at room temperature for 2 hours. The solvent was evaporated under reduced pressure and the obtained solid was washed with acetone/diisopropylether (1:1) to obtain the titled compound (2.20 g, 84 %) as a clear crystal.
$^1H$ NMR (DMSO-d6, δppm) : 1.29-1.55 (m, 3H), 1.63-1.83 (m, 3H), 1.83-2.07 (m, 4H), 2.07-2.24 (m, 1H), 2.74-3.03 (m, 6H), 3.26 (d, J = 12.6Hz, 2H), 3.43 (d, J = 12.7 Hz, 2H), 8.78-9.26 (m, 2H).

Reference Example 3:4-(2-Methylpyrrolidin-1-ylmethyl) piperidine dihydrochloride

(Step 1)

In a similar manner to Step 2 of Reference Example 2, 1-(tert-butyloxycarbonyl)-4-(2-methylpyrrolidin-1-yl) methylpiperidine (2.22 g, 39 %) was obtained as a clear oily substance by using 1-(tert-butyloxycarbonyl)-4-formylpiperidine (4.28 g, 20.1 mmol) obtained in Step 1 of Reference Example 2 and 2-methylpyrrolidine (3.10 mL, 30.4 mmol) in place of piperidine.
$^1H$ NMR ($CDCl_3$, δppm) : 1.04 (d, J = 6.2 Hz, 3H), 1.06-1.14 (m, 2H), 1.33-1.43 (m, 1H), 1.45 (s, 9H), 1.50-1.80 (m, 4H), 1.82-2.09 (m, 4H), 2.18-2.27 (m, 1H), 2.51 (dd, J = 9.3, 11.9 Hz, 1H), 2.63-2.75 (m, 2H), 3.11 (td, J = 2.8, 8.4 Hz, 1H), 4.07 (d, J = 11.7 Hz, 2H).

(Step 2)

In a similar manner to Step 3 of Reference Example 2, the titled compound (1.45 g, 73 %) was obtained as a clear crystal by using 1-(tert-butyloxycarbonyl)-4-(2-methylpyrrolidin-1-yl)methylpiperidine (2.22 g, 7.79 mmol) obtained in the Step 1 in place of 1-(tert-butyloxycarbonyl)-4-piperidinomethylpiperidine.
$^1$H NMR (DMSO-$d_6$, δppm) : 1.33-1.56 (m, 5H), 1.65-1.82 (m, 1H), 1.82-2.34 (m, 6H), 2.77-2.99 (m, 4H), 2.99-3.32 (m, 4H), 3.59-3.74 (m, 1H), 8.75-9.14 (m, 2H), 10.24 (br s, 1H).

Reference Example 4: (R)-1-(2-aminoethyl)-2-methylpyrrolidine

(Step 1)

(R)-2-methylpyrrolidine hydrochloride (1.66 g, 13.7 mmol) prepared in a similar manner to the method described in US 2004/0171845 was dissolved in methanol (20 mL) and the solution was added with triethylamine (9.55 mL, 68.5 mmol) and bromoacetonitrile (2.86 mL, 41.1 mmol) under ice bath, followed by stirring at room temperature for 1 hour. The solvent was evaporated under reduced pressure and the residue was added with saturated brine and extracted with chloroform. After washing with saturated brine, the organic layer was dried over anhydrous magnesium sulfate and the solvent was evaporated under reduced pressure. The residue was purified by silica gel column chromatography (hexane/ethyl acetate (7:3)) to obtain (R)-1-cyanomethyl-2-methylpyrrolidine (1.70 g, 100 %) as a clear oily substance.
$^1$H NMR ($CDCl_3$, δppm) : 1.10 (d, J = 6.0 Hz, 3H), 1.39-1.53 (m, 1H), 1.71-1.91 (m, 2H), 1.94-2.08 (m, 1H), 2.53-2.66 (m, 2H), 3.03 (td, J =3.2 Hz, 8.4 Hz, 1H), 3.68 (s, 2H).

(Step 2)

Lithium aluminum hydroxide (774 mg, 20.4 mmol) was added with THF (20 mL) under ice bath and the mixture was added with a solution of (R)-1-cyanomethyl-2-methylpyrrolidine (1.70 g, 13.7 mmol) obtained in the Step 2 in THF (10 mL). After stirring at room temperature for 0.5 hour, water (0.77 mL), 15% aqueous sodium hydroxide solution (0.77 mL) and water (2.3 mL) were sequentially added under ice bath, followed by stirring at room temperature for 1 hour. The reaction mixture was diluted with ethyl acetate and filtered using sellite. The obtained filtrate was dried over anhydrous magnesium sulfate and the solvent was evaporated under reduced pressure to obtain the titled compound (1.17 g, 67 %) as a yellow oily substance.
$^1$H NMR ($CDCl_3$, δppm) : 1.08 (d, J = 6.2 Hz, 3H), 1.32-1.60 (m, 3H), 1.60-1.83 (m, 2H), 1.83-2.00 (m, 1H), 2.02-2.21 (m, 2H), 2.24-2.37 (m, 1H), 2.75-2.88 (m, 3H), 3.13 (td, J = 3.1, 8.4 Hz, 1H).

Reference Example 5: (S)-1-(2-aminoethyl)-2-methylpyrrolidine

In a similar manner to Reference Example 4, the titled compound (980 mg, 51 % (2 steps)) was obtained as a yellow oily substance by using (S)-2-methylpyrrolidine hydrobromate (2.49 g, 15.0 mmol) prepared in a similar manner to the method described in J. Org. Chem., Vol. 54, p.209-216 (1989) in place of (R)-2-methylpyrrolidine hydrochloride.
$^1$H NMR ($CDCl_3$, δppm): 1.08 (d, J = 6.2 Hz, 3H), 1.32-1.60 (m, 3H), 1.60-1.83 (m, 2H), 1.83-2.00 (m, 1H), 2.02-2.21 (m, 2H), 2.24-2.37 (m, 1H), 2.75-2.88 (m, 3H), 3.13 (td, J = 3.1, 8.4 Hz, 1H).

Reference Example 6: 4-(2-Methylpyrrolidin-1-yl)piperidine

(Step 1)

In a similar manner to Step 1 of Reference Example 1, 1-(tert-butyloxycarbonyl)-4-(2-methylpyrrolidin-1-yl) piperidine (1.21 g, 90 %) was obtained as a clear oily substance by using 1-(tert-butyloxycarbonyl)piperidine-4-one (1.00 g, 5.02 mmol) and 2-methylpyrrolidine (0.440 mL, 5.05 mmol) .
$^1$H NMR ($CDCl_3$, δppm) : 1.08 (d, J = 6.3 Hz, 3H), 1.32-1.59 (m, 10H), 1.61-2.00 (m, 7H), 2.52-2.80 (m, 4H), 2.86-2.99 (m, 2H), 4.05-4.22 (m, 2H).

(Step 2)

1-(Tert-butyloxycarbonyl)-4-(2-methylpyrrolidin-1-yl)piperidine (1.21 g, 4.51 mmol) obtained in the Step 1 was dissolved in ethyl acetate (4 mL) and the solution was added with 4 mol/L hydrogen chloride-ethyl acetate solution (2.00 mL, 8.00 mmol), followed by stirring at room temperature for 2 hours. The solvent was evaporated under reduced pressure, added with 2 mol/L aqueous sodium hydroxide solution and extracted with chloroform. After washing with

saturated brine, the organic layer was dried over anhydrous magnesium sulfate and the solvent was evaporated under reduced pressure to obtain the titled compound (330 mg, 43 %) as a yellow oily substance.
$^1H$ NMR ($CDCl_3$, δppm) : 1.08 (d, J = 6.3 Hz, 3H), 1.39-1.58 (m, 1H), 1.58-1.78 (m, 3H), 1.78-2.01 (m, 4H), 2.55-2.80 (m, 4H), 2.84-3.04 (m, 2H), 3.24 (dt, J = 3.3, 12.6 Hz, 2H), 4.11 (br s, 1H).

Reference Example 7: 1-(3-Aminopropyl)-2-methylpyrrolidine

N-(3-bromopropyl)phthalimide (5.00 g, 18.6 mmol) was dissolved in toluene (10 mL) and the solution was added with 2-methylpyrrolidine (3.80 mL, 37.2 mmol), followed by stirring at 110°C for 1 hour. After cooling, the reaction mixture was added with saturated brine and extracted with ethyl acetate. After washing with saturated brine, the organic layer was dried over anhydrous magnesium sulfate and the solvent was evaporated under reduced pressure. The obtained orange oily substance (5.63 g) was used to the next reaction as it is.

The orange oily substance (5.63 g) obtained above was dissolved in water (1 mL) and the solution was added with concentrated hydrochloric acid (5.6 mL), followed by stirring at 100°C for 12 hours. After cooling, the reaction mixture was added with ice water and an insoluble matter was filtered. The obtained filtrate was washed with diethylether and then with ethyl acetate and added with 2 mol/L aqueous sodium hydroxide solution to adjust the pH to about 10. Then, the mixture was extracted with chloroform. The organic layer was dried over anhydrous magnesium sulfate and the solvent was evaporated under reduced pressure to obtain the titled compound (1.05 g, 40 %) as a yellow oily substance.
$^1H$ NMR ($CDCl_3$, δppm) : 1.09 (d, J = 6.3 Hz, 3H), 1.29-1.49 (m, 3H), 1.58-1.82 (m, 4H), 1.84-1.97 (m, 1H), 1.99-2.12 (m, 2H), 2.17-2.31 (m, 1H), 2.76 (t, J = 6.9 Hz, 2H), 2.79-2.91 (m, 1H), 3.16 (td, J=3.0, 8.4 Hz,1H).

## Claims

**1.** A diamine derivative represented by the general formula (I):

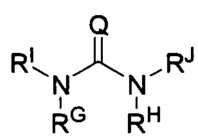

(I)

{wherein Q represents an oxygen atom, $=C(CN)_2$, $=CHNO_2$, =NCN, $=NSO_2NH_2$ or $=C (CN) (SO_2R^1)$ (wherein $R^1$ represents substituted or unsubstituted lower alkyl or substituted or unsubstituted aryl),
$R^G$ represents a hydrogen atom or substituted or unsubstituted lower alkyl, or $R^G$ and $R^H$ are combined to represent substituted or unsubstituted alkylene or substituted or unsubstituted phenylene, and

(i) when $R^G$ is a hydrogen atom or substituted or unsubstituted lower alkyl,
$R^I$ represents:

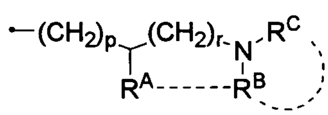

(wherein p and r may be the same or different, and each represents 0, 1, 2 or 3,
$R^A$ represents a hydrogen atom or substituted or unsubstituted lower alkyl, and
$R^B$ and $R^C$ may be the same or different, and each represents a hydrogen atom, substituted or unsubstituted lower alkyl, substituted or unsubstituted lower alkenyl, substituted or unsubstituted lower alkynyl, substituted or unsubstituted cycloalkyl, substituted or unsubstituted lower alkanoyl, substituted or unsubstituted cycloalkylcarbonyl, substituted or unsubstituted lower alkoxycarbonyl, substituted or unsubstituted aroyl, substituted or unsubstituted aryl, a substituted or unsubstituted aliphatic heterocyclic group or a substituted or unsubstituted aromatic heterocyclic group, or
$R^B$ and $R^C$ are combined together with the adjacent nitrogen atom thereto to form a substituted or unsubstituted nitrogen-containing heterocyclic group, or

$R^A$ and $R^B$ are combined to represent substituted or unsubstituted alkylene, and
$R^C$ represents a hydrogen atom, substituted or unsubstituted lower alkyl, substituted or unsubstituted lower alkenyl, substituted or unsubstituted lower alkynyl, substituted or unsubstituted cycloalkyl, substituted or unsubstituted lower alkanoyl, substituted or unsubstituted cycloalkylcarbonyl, substituted or unsubstituted lower alkoxycarbonyl, substituted or unsubstituted aroyl, substituted or unsubstituted aryl, a substituted or unsubstituted aliphatic heterocyclic group or a substituted or unsubstituted aromatic heterocyclic group),
$R^H$ represents a hydrogen atom or substituted or unsubstituted lower alkyl, and
$R^J$ represents:

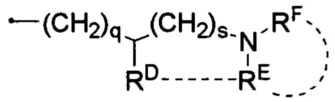

(wherein q and s may be the same or different, and each represents 0, 1, 2 or 3,
$R^D$ represents a hydrogen atom or substituted or unsubstituted lower alkyl, and
$R^E$ and $R^F$ may be the same or different, and each represents a hydrogen atom, substituted or unsubstituted lower alkyl, substituted or unsubstituted lower alkenyl, substituted or unsubstituted lower alkynyl, substituted or unsubstituted cycloalkyl, substituted or unsubstituted lower alkanoyl, substituted or unsubstituted cycloalkylcarbonyl, substituted or unsubstituted lower alkoxycarbonyl, substituted or unsubstituted aroyl, substituted or unsubstituted aryl, a substituted or unsubstituted aliphatic heterocyclic group or a substituted or unsubstituted aromatic heterocyclic group, or
$R^E$ and $R^F$ are combined together with the adjacent nitrogen atom thereto to form a substituted or unsubstituted nitrogen-containing heterocyclic group, or
$R^D$ and $R^E$ are combined to represent substituted or unsubstituted alkylene, and
$R^F$ represents a hydrogen atom, substituted or unsubstituted lower alkyl, substituted or unsubstituted lower alkenyl, substituted or unsubstituted lower alkynyl, substituted or unsubstituted cycloalkyl, substituted or unsubstituted lower alkanoyl, substituted or unsubstituted cycloalkylcarbonyl, substituted or unsubstituted lower alkoxycarbonyl, substituted or unsubstituted aroyl, substituted or unsubstituted aryl, a substituted or unsubstituted aliphatic heterocyclic group or a substituted or unsubstituted aromatic heterocyclic group), or
$R^H$ and $R^J$ are combined to represent $-(CH_2)_t-X-(CH_2)_u-$[wherein t represents 0, 1, 2 or 3, u represents 1, 2 or 3, and
X represents:

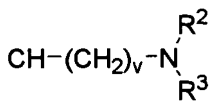

(wherein v represents 0 or 1, and
$R^2$ and $R^3$ may be the same or different, and each represents a hydrogen atom, substituted or unsubstituted lower alkyl, substituted or unsubstituted lower alkenyl, substituted or unsubstituted lower alkynyl, substituted or unsubstituted cycloalkyl, substituted or unsubstituted lower alkanoyl, substituted or unsubstituted cycloalkylcarbonyl, substituted or unsubstituted lower alkoxycarbonyl, substituted or unsubstituted aroyl, substituted or unsubstituted aryl, a substituted or unsubstituted aliphatic heterocyclic group or a substituted or unsubstituted aromatic heterocyclic group, or
$R^2$ and $R^3$ are combined together with the adjacent nitrogen atom thereto to form a substituted or unsubstituted nitrogen-containing heterocyclic group), or
$N-R^4$ (wherein $R^4$ represents a hydrogen atom, substituted or unsubstituted lower alkyl, substituted or unsubstituted lower alkenyl, substituted or unsubstituted lower alkynyl, substituted or unsubstituted cycloalkyl, substituted or unsubstituted lower alkanoyl, substituted or unsubstituted cycloalkylcarbonyl, substituted or unsubstituted lower alkoxycarbonyl, substituted or unsubstituted aroyl, substituted or unsubstituted aryl, a substituted or unsubstituted aliphatic heterocyclic group or a substituted or unsubstituted aromatic heterocyclic group)], and
(ii) when $R^H$ and $R^I$ are combined to form substituted or unsubstituted alkylene or substituted or unsubstituted phenylene,

$R^I$ is:

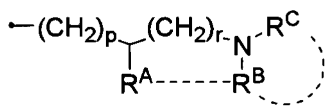

(wherein p, r, $R^A$, $R^B$ and $R^C$ have the same definitions as described above, respectively), and $R^J$ is:

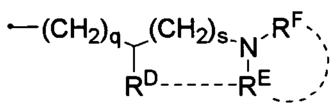

(wherein q, s, $R^D$, $R^E$ and $R^F$ have the same definitions as described above, respectively), with the proviso that when Q is an oxygen atom, at least $R^A$ and $R^B$, and/or $R^D$ and $R^E$ are respectively combined to represent substituted or unsubstituted alkylene, or $R^B$ and $R^C$, and/or $R^E$ and $R^F$ are respectively combined together with the adjacent nitrogen atom thereto to form a substituted or unsubstituted nitrogen-containing heterocyclic group, and

when Q is $=CHNO_2$, at least $R^G$ and $R^H$ are combined to represent substituted or unsubstituted alkylene or substituted or unsubstituted phenylene}, or a pharmaceutically acceptable salt thereof.

**2.** The diamine derivative or the pharmaceutically acceptable salt thereof according to claim 1, wherein Q is an oxygen atom, $=C(CN)_2$, $=CHNO_2$, =NCN, $=NSO_2NH_2$ or $=C (CN) (SO_2R^{1A})$ (wherein $R^{1A}$ represents substituted or unsubstituted $C_{1-10}$ alkyl or substituted or unsubstituted $C_{6-14}$ aryl),
$R^G$ is a hydrogen atom or substituted or unsubstituted $C_{1-10}$ alkyl, or $R^G$ and $R^H$ are combined to form substituted or unsubstituted $C_{1-10}$ alkylene or substituted or unsubstituted phenylene, and

(i) when $R^G$ is a hydrogen atom or substituted or unsubstituted $C_{1-10}$ alkyl,
$R^I$ is:

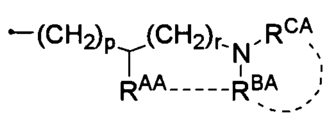

(wherein p and r have the same definitions as described above, respectively,
$R^{AA}$ represents a hydrogen atom or substituted or unsubstituted $C_{1-10}$ alkyl, and
$R^{BA}$ and $R^{CA}$ may be the same or different, and each represents a hydrogen atom, substituted or unsubstituted $C_{1-10}$ alkyl, substituted or unsubstituted $C_{2-10}$ alkenyl, substituted or unsubstituted $C_{2-10}$ alkynyl, substituted or unsubstituted $C_{3-8}$ cycloalkyl, substituted or unsubstituted $C_{2-11}$ alkanoyl, substituted or unsubstituted $C_{3-8}$ cycloalkylcarbonyl, substituted or unsubstituted $C_{1-10}$ alkoxycarbonyl, substituted or unsubstituted $C_{7-15}$ aroyl, substituted or unsubstituted $C_{6-14}$ aryl, a substituted or unsubstituted aliphatic heterocyclic group or a substituted or unsubstituted aromatic heterocyclic group, or
$R^{BA}$ and $R^{CA}$ are combined together with the adjacent nitrogen atom thereto to form a substituted or unsubstituted nitrogen-containing heterocyclic group, or
$R^{AA}$ and $R^{BA}$ are combined to represent substituted or unsubstituted $C_{1-10}$ alkylene, and
$R^{CA}$ represents a hydrogen atom, substituted or unsubstituted $C_{1-10}$ alkyl, substituted or unsubstituted $C_{2-10}$ alkenyl, substituted or unsubstituted $C_{2-10}$ alkynyl, substituted or unsubstituted $C_{3-8}$ cycloalkyl, substituted or unsubstituted $C_{2-11}$ alkanoyl, substituted or unsubstituted $C_{3-8}$ cycloalkylcarbonyl, substituted or unsubstituted $C_{1-10}$ alkoxycarbonyl, substituted or unsubstituted $C_{7-15}$ aroyl, substituted or unsubstituted $C_{6-14}$ aryl, a substituted or unsubstituted aliphatic heterocyclic group or a substituted or unsubstituted aromatic heterocyclic group),
$R^H$ is a hydrogen atom or substituted or unsubstituted $C_{1-10}$ alkyl, and
$R^J$ is:

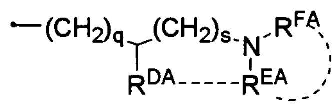

(wherein q and s have the same definitions as described above, respectively,
$R^{DA}$ represents a hydrogen atom or substituted or unsubstituted $C_{1-10}$ alkyl, and
$R^{EA}$ and $R^{FA}$ may be the same or different, and each represents a hydrogen atom, substituted or unsubstituted $C_{1-10}$ alkyl, substituted or unsubstituted $C_{2-10}$ alkenyl, substituted or unsubstituted $C_{2-10}$ alkynyl, substituted or unsubstituted $C_{3-8}$ cycloalkyl, substituted or unsubstituted $C_{2-11}$ alkanoyl, substituted or unsubstituted $C_{3-8}$ cycloalkylcarbonyl, substituted or unsubstituted $C_{1-10}$ alkoxycarbonyl, substituted or unsubstituted $C_{7-15}$ aroyl, substituted or unsubstituted $C_{6-14}$ aryl, a substituted or unsubstituted aliphatic heterocyclic group or a substituted or unsubstituted aromatic heterocyclic group, or
$R^{EA}$ and $R^{FA}$ are combined together with the adjacent nitrogen atom thereto to form a substituted or unsubstituted nitrogen-containing heterocyclic group, or
$R^{DA}$ and $R^{EA}$ are combined to represent substituted or unsubstituted $C_{1-10}$ alkylene, and
$R^{FA}$ represents a hydrogen atom, substituted or unsubstituted $C_{1-10}$ alkyl, substituted or unsubstituted $C_{2-10}$ alkenyl, substituted or unsubstituted $C_{2-10}$ alkynyl, substituted or unsubstituted $C_{3-8}$ cycloalkyl, substituted or unsubstituted $C_{2-11}$ alkanoyl, substituted or unsubstituted $C_{3-8}$ cycloalkylcarbonyl, substituted or unsubstituted $C_{1-10}$ alkoxycarbonyl, substituted or unsubstituted $C_{7-15}$ aroyl, substituted or unsubstituted $C_{6-14}$ aryl, a substituted or unsubstituted aliphatic heterocyclic group or a substituted or unsubstituted aromatic heterocyclic group), or
$R^H$ and $R^J$ are combined to form $-(CH_2)_t-X_A-(CH_2)_u-$[wherein t and u have the same definitions as described above, respectively, and
$X_A$ is:

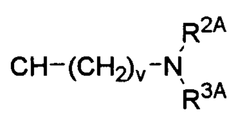

(wherein v represents 0 or 1, and
$R^{2A}$ and $R^{3A}$ may be the same or different, and each represents a hydrogen atom, substituted or unsubstituted $C_{1-10}$ alkyl, substituted or unsubstituted $C_{2-10}$ alkenyl, substituted or unsubstituted $C_{2-10}$ alkynyl, substituted or unsubstituted $C_{3-8}$ cycloalkyl, substituted or unsubstituted $C_{2-11}$ alkanoyl, substituted or unsubstituted $C_{3-8}$ cycloalkylcarbonyl, substituted or unsubstituted $C_{1-10}$ alkoxycarbonyl, substituted or unsubstituted $C_{7-15}$ aroyl, substituted or unsubstituted $C_{6-14}$ aryl, a substituted or unsubstituted aliphatic heterocyclic group or a substituted or unsubstituted aromatic heterocyclic group, or
$R^{2A}$ and $R^{3A}$ are combined together with the adjacent nitrogen atom thereto to form a substituted or unsubstituted nitrogen-containing heterocyclic group), or
$N-R^{4A}$ (wherein $R^{4A}$ represents a hydrogen atom, substituted or unsubstituted $C_{1-10}$ alkyl, substituted or unsubstituted $C_{2-10}$ alkenyl, substituted or unsubstituted $C_{2-10}$ alkynyl, substituted or unsubstituted $C_{3-8}$ cycloalkyl, substituted or unsubstituted $C_{2-11}$ alkanoyl, substituted or unsubstituted $C_{3-8}$ cycloalkylcarbonyl, substituted or unsubstituted $C_{1-10}$ alkoxycarbonyl, substituted or unsubstituted $C_{7-15}$ aroyl, substituted or unsubstituted $C_{4-14}$ aryl, a substituted or unsubstituted aliphatic heterocyclic group or a substituted or unsubstituted aromatic heterocyclic group)], and
(ii) when $R^G$ and $R^H$ are combined to form substituted or unsubstituted $C_{1-10}$ alkylene or substituted or unsubstituted phenylene,
$R^I$ is:

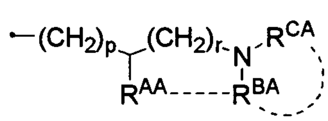

(wherein p, r, $R^{AA}$, $R^{BA}$ and $R^{CA}$ have the same definitions as described above, respectively), and $R^J$ is:

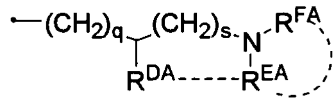

(wherein q, s, $R^{DA}$, $R^{EA}$ and $R^{FA}$ have the same definitions as described above, respectively).

**3.** A diamine derivative represented by the general formula (II):

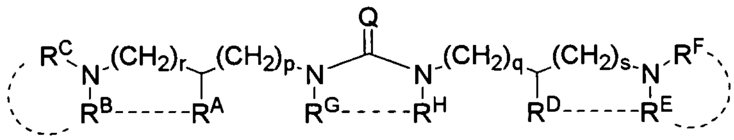

(II)

[wherein p, q, r and s may be the same or different, and each represents 0, 1, 2 or 3,
$R^A$ and $R^D$ may be the same or different, and each represents a hydrogen atom or substituted or unsubstituted lower alkyl,
$R^B$ and $R^E$ may be the same or different, and each represents a hydrogen atom, substituted or unsubstituted lower alkyl, substituted or unsubstituted lower alkenyl, substituted or unsubstituted lower alkynyl, substituted or unsubstituted cycloalkyl, substituted or unsubstituted lower alkanoyl, substituted or unsubstituted cycloalkylcarbonyl, substituted or unsubstituted lower alkoxycarbonyl, substituted or unsubstituted aroyl, substituted or unsubstituted aryl, a substituted or unsubstituted aliphatic heterocyclic group or a substituted or unsubstituted aromatic heterocyclic group, or
$R^A$ and $R^B$, and/or $R^D$ and $R^E$ are respectively combined to represent substituted or unsubstituted alkylene,
$R^C$ and $R^F$ may be the same or different, and each represents a hydrogen atom, substituted or unsubstituted lower alkyl, substituted or unsubstituted lower alkenyl, substituted or unsubstituted lower alkynyl, substituted or unsubstituted cycloalkyl, substituted or unsubstituted lower alkanoyl, substituted or unsubstituted cycloalkylcarbonyl, substituted or unsubstituted lower alkoxycarbonyl, substituted or unsubstituted aroyl, substituted or unsubstituted aryl, a substituted or unsubstituted aliphatic heterocyclic group or a substituted or unsubstituted aromatic heterocyclic group, or
$R^B$ and $R^C$, and/or $R^E$ and $R^F$ are respectively combined together with the adjacent nitrogen atom thereto to form a substituted or unsubstituted nitrogen-containing heterocyclic group,
$R^G$ and $R^H$ may be the same or different, and each represents a hydrogen atom or substituted or unsubstituted lower alkyl, or
$R^G$ and $R^H$ are combined to represent substituted or unsubstituted alkylene or substituted or unsubstituted phenylene, and
Q represents an oxygen atom, $=C(CN)_2$, $=CHNO_2$, =NCN, $=NSO_2NH_2$ or $=C(CN)(SO_2R^1)$ (wherein $R^1$ represents substituted or unsubstituted lower alkyl or substituted or unsubstituted aryl), with the proviso that
when Q is an oxygen atom, at least $R^A$ and $R^B$, and/or
$R^D$ and $R^E$ are respectively combined to represent substituted or unsubstituted alkylene, or $R^B$ and $R^C$, and/or $R^E$ and $R^F$ are respectively combined together with the adjacent nitrogen atom thereto to form a substituted or unsubstituted nitrogen-containing heterocyclic group, and
when Q is $=CHNO_2$, at least $R^G$ and $R^H$ are combined to represent substituted or unsubstituted alkylene or substituted or unsubstituted phenylene], or a pharmaceutically acceptable salt thereof.

**4.** The diamine derivative or the pharmaceutically acceptable salt thereof according to claim 2, wherein Q is $=C(CN)_2$, =NCN or $=C(CN)SO_2R^{1A}$ (wherein $R^{1A}$ has the same definition as described above).

**5.** The diamine derivative or the pharmaceutically acceptable salt thereof according to any one of claims 1 to 3, wherein Q is $=C(CN)_2$.

**6.** The diamine derivative or the pharmaceutically acceptable salt thereof according to any one of claims 1 to 5, wherein

p and q are 0.

**7.** The diamine derivative or the pharmaceutically acceptable salt thereof according to any one of claims 1 to 6, wherein r and s may be the same or different, and each is 1 or 2.

**8.** The diamine derivative or the pharmaceutically acceptable salt thereof according to any one of claims 1 to 7, wherein $R^G$ and $R^H$ are combined to form substituted or unsubstituted $C_{1-10}$ alkylene.

**9.** The diamine derivative or the pharmaceutically acceptable salt thereof according to claim 8, wherein the substituted or unsubstituted $C_{1-10}$ alkylene is substituted or unsubstituted methylene, substituted or unsubstituted ethylene or substituted or unsubstituted trimethylene.

**10.** The diamine derivative or the pharmaceutically acceptable salt thereof according to claim 8, wherein the substituted or unsubstituted $C_{1-10}$ alkylene is ethylene.

**11.** The diamine derivative or the pharmaceutically acceptable salt thereof according to any one of claims 1 to 7, wherein $R^G$ and $R^H$ are combined to form substituted or unsubstituted 1,2-phenylene.

**12.** The diamine derivative or the pharmaceutically acceptable salt thereof according to any one of claims 1 to 11, wherein $R^B$ and $R^C$, and/or $R^E$ and $R^F$ are respectively combined together with the adjacent nitrogen atom thereto to form a substituted or unsubstituted nitrogen-containing heterocyclic group.

**13.** The diamine derivative or a pharmaceutically acceptable salt thereof according to any one of claims 1, 2 and 4 to 5, wherein $R^H$ and $R^J$ are combined to form $-(CH_2)_t-X_A-(CH_2)_u-$ (wherein t, u, and $X_A$ have the same definitions as described above, respectively).

**14.** The diamine derivative or the pharmaceutically acceptable salt thereof according to claim 13, wherein $X_A$ is:

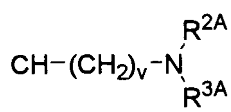

(wherein v, $R^{2A}$ and $R^{3A}$ have the same definitions as described above, respectively).

**15.** The diamine derivative or the pharmaceutically acceptable salt thereof according to claim 13 or 14, wherein p is 0.

**16.** The diamine derivative or the pharmaceutically acceptable salt thereof according to any one of claims 13 to 15, wherein r is 1 or 2.

**17.** The diamine derivative or the pharmaceutically acceptable salt thereof according to any one of claims 1 to 5, wherein $R^A$ or $R^{AA}$ is a hydrogen atom and $R^G$ and $R^H$ are combined to form substituted or unsubstituted $C_{1-10}$ alkylene.

**18.** The diamine derivative or the pharmaceutically acceptable salt thereof according to any one of claims 1 to 5 and 17, wherein $R^B$ and $R^c$, or $R^{BA}$ and $R^{CA}$ are combined together with the adjacent nitrogen atom thereto to form a substituted or unsubstituted nitrogen-containing heterocyclic group.

**19.** The diamine derivative or the pharmaceutically acceptable salt thereof according to claim 17 or 18, wherein $R^D$ or $R^{DA}$ is a hydrogen atom.

**20.** The diamine derivative or the pharmaceutically acceptable salt thereof according to any one of claims 17 to 19, wherein $R^E$ and $R^E$, or $R^{EA}$ and $R^{EA}$ are combined together with the adjacent nitrogen atom thereto to form a substituted or unsubstituted nitrogen-containing heterocyclic group.

**21.** The diamine derivative or the pharmaceutically acceptable salt thereof according to any one of claims 1 to 5, 17 and 18, wherein $R^D$ and $R^E$, or $R^{DA}$ and $R^{EA}$ are combined to form substituted or unsubstituted $C_{1-10}$ alkylene and q is 0.

**22.** The diamine derivative or the pharmaceutically acceptable salt thereof according to claim 21, wherein s is 2, and $R^D$ and $R^E$, or $R^{DA}$ and $R^{EA}$ are combined to form ethylene.

**23.** A pharmaceutical composition which comprises, as an active ingredient, the diamine derivative or the pharmaceutically acceptable salt thereof recited in any one of claims 1 to 22.

**24.** A histamine $H_3$ receptor antagonist and/or inverse agonist which comprise, as an active ingredient, the diamine derivative or the pharmaceutically acceptable salt thereof recited in any one of claims 1 to 22.

**25.** Use of the diamine derivative or the pharmaceutically acceptable salt thereof recited in any one of claims 1 to 22 for manufacture of a pharmaceutical composition.

**26.** A method of antagonizing and/or inversely agonizing a histamine $H_3$ receptor, which comprises administering the diamine derivative or the pharmaceutically acceptable salt thereof recited in any one of claims 1 to 22 as an active ingredient.

**27.** Use of the diamine derivative or the pharmaceutically acceptable salt thereof recited in any one of claims 1 to 22 for manufacture of a histamine $H_3$ receptor antagonist and/or inverse agonist.

**INTERNATIONAL SEARCH REPORT**

International application No.
PCT/JP2006/301527

A. CLASSIFICATION OF SUBJECT MATTER
***C07D211/58***(2006.01), ***A61K31/4178***(2006.01), ***A61K31/4184***(2006.01), ***A61K31/427***
(2006.01), ***A61K31/444***(2006.01), ***A61K31/4453***(2006.01), ***A61K31/4468***(2006.01), ***A61K31/4545***(2006.01), ***A61K31/4725***(2006.01), ***A61K31/496***(2006.01),

B. FIELDS SEARCHED

Minimum documentation searched (classification system followed by classification symbols)
A61K31/4178, A61K31/4184, A61K31/427, A61K31/444, A61K31/4453, A61K31/4468, A61K31/4545, A61K31/4725, A61K31/496, A61K31/506, A61K31/5375, A61K31/5377, A61K31/541, A61K31/55, A61P1/04, A61P3/04, A61P9/00, A61P9/12, A61P25/04,

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2006 |
| Kokai Jitsuyo Shinan Koho | 1971-2006 | Toroku Jitsuyo Shinan Koho | 1994-2006 |

Electronic data base consulted during the international search (name of data base and, where practicable, search terms used)
CAPLUS(STN), REGISTRY(STN), MEDLINE(STN), BIOSIS(STN), EMBASE(STN)

C. DOCUMENTS CONSIDERED TO BE RELEVANT

| Category* | Citation of document, with indication, where appropriate, of the relevant passages | Relevant to claim No. |
|---|---|---|
| X<br><br>A | WO 02/085357 A1 (EURO-CELTIQUE, S.A.), 31 October, 2002 (31.10.02), Particularly, page 35 & EP 1379246 A1 & US 2003/069249 A1 | 1-4,7,11, 21-25,27<br>5,6,8-10, 12-20 |
| X | WO 02/100861 A1 (AKZONOBEL N.V.), 19 December, 2002 (19.12.02), Particularly, examples 13, 14 (Family: none) | 1-3,7,11, 21-25,27 |
| X | WO 02/24661 A2 (BOEHRINGER INGELHEIM PHARMA KG), 28 March, 2002 (28.03.02), Full text & US 2003/027823 A1 & EP 1322622 A2 & JP 2004-509872 A | 1-3,7,11,12, 18-20,23-25, 27 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| | |
|---|---|
| Date of the actual completion of the international search<br>13 March, 2006 (13.03.06) | Date of mailing of the international search report<br>20 March, 2006 (20.03.06) |
| Name and mailing address of the ISA/<br>Japanese Patent Office<br>Facsimile No. | Authorized officer<br><br>Telephone No. |

Form PCT/ISA/210 (second sheet) (April 2005)

**INTERNATIONAL SEARCH REPORT**

International application No.
PCT/JP2006/301527

C (Continuation). DOCUMENTS CONSIDERED TO BE RELEVANT

| Category* | Citation of document, with indication, where appropriate, of the relevant passages | Relevant to claim No. |
|---|---|---|
| X | WO 03/037890 A2 (ICAGEN, INC.), 08 May, 2003 (08.05.03), Particularly, compounds 190, 191, 194 & EP 1451173 A2 & US 2003/171360 A1 | 1-3,7,11,12, 17,18,21-25, 27 |
| X | WO 03/037271 A2 (MILLENNIUM PHARMACEUTICALS, INC.), 08 May, 2003 (08.05.03), Particularly, table 5, No.9-15, 9-16 & US 2005/143372 A1 | 1-3,7,11,12, 17,18,21-25, 27 |

Form PCT/ISA/210 (continuation of second sheet) (April 2005)

**INTERNATIONAL SEARCH REPORT**

International application No.
PCT/JP2006/301527

**Box No. II Observations where certain claims were found unsearchable (Continuation of item 2 of first sheet)**

This international search report has not been established in respect of certain claims under Article 17(2)(a) for the following reasons:

1. ☒ Claims Nos.: 26
because they relate to subject matter not required to be searched by this Authority, namely:
Claim 26 pertains to methods for treatment of the human body by therapy and thus relates to a subject matter which this International Searching Authority is not required, under the provisions of Article 17(2)(a)(i) of the PCT and Rule 39.1(iv) of the Regulations under the PCT, to search.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**Box No. III Observations where unity of invention is lacking (Continuation of item 3 of first sheet)**

This International Searching Authority found multiple inventions in this international application, as follows:

1. ☐ As all required additional search fees were timely paid by the applicant, this international search report covers all searchable claims.

2. ☐ As all searchable claims could be searched without effort justifying an additional fee, this Authority did not invite payment of any additional fee.

3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest** the

☐ The additional search fees were accompanied by the applicant's protest and, where applicable, payment of a protest fee..

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet (2)) (April 2005)

**INTERNATIONAL SEARCH REPORT**

International application No.
PCT/JP2006/301527

Although the general formula (I) of claim 1 includes a very large number of compounds, only a few compounds among those represented by the formula (I) are disclosed within the meaning of PCT Article 5. Consequently, claim 1 is not fully supported within the meaning of PCT Article 6.

Form PCT/ISA/210 (extra sheet) (April 2005)

**INTERNATIONAL SEARCH REPORT**

International application No.
PCT/JP2006/301527

Continuation of A. CLASSIFICATION OF SUBJECT MATTER
(International Patent Classification (IPC))

***A61K31/506***(2006.01), ***A61K31/5375***(2006.01), ***A61K31/5377***(2006.01), ***A61K31/541***(2006.01), ***A61K31/55***(2006.01), ***A61P1/04***(2006.01), ***A61P3/04***(2006.01), ***A61P9/00***(2006.01), ***A61P9/12***(2006.01), ***A61P25/04***(2006.01), ***A61P25/08***(2006.01), ***A61P25/20***(2006.01), ***A61P25/28***(2006.01), ***A61P29/00***(2006.01), ***A61P37/08***(2006.01), ***A61P43/00***(2006.01), ***C07D233/24***(2006.01), ***C07D233/44***(2006.01), ***C07D235/14***(2006.01), ***C07D239/06***(2006.01), ***C07D295/12***(2006.01), ***C07D401/06***(2006.01), ***C07D401/14***(2006.01), ***C07D403/06***(2006.01), ***C07D403/14***(2006.01), ***C07D413/06***(2006.01)

(According to International Patent Classification (IPC) or to both national classification and IPC)

Continuation of B. FIELDS SEARCHED
Minimum documentation searched (International Patent Classification (IPC))

A61P25/08, A61P25/20, A61P25/28, A61P29/00, A61P37/08, A61P43/00, C07D211/58, C07D233/24, C07D233/44, C07D235/14, C07D239/06, C07D295/12, C07D401/06, C07D401/14, C07D403/06, C07D403/14, C07D413/06

Minimum documentation searched (classification system followed by classification symbols)

Form PCT/ISA/210 (extra sheet) (April 2005)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2002079168 A
- WO 2002072570 A
- WO 0006254 A
- WO 9924405 A
- WO 9638141 A
- WO 9924421 A
- WO 200489410 A
- JP 4226167 A
- JP 3054654 B
- JP 7014916 B
- JP 5017471 A
- DE 2628347
- EP 159533 A
- US 20040171845 A


### Non-patent literature cited in the description


- *Molecular Pharmacology,* 2001, vol. 59, 415-419
- *Trends in Pharmacological Science,* 1998, vol. 19, 177-183
- *Annual Report in Medicinal Chemistry,* 1998, vol. 33, 31-39
- *British Journal of Pharmacology,* 1994, vol. 111, 1269-1279
- *Pharmacology, Biochemistry and Behavior,* 2002, vol. 72, 751-760
- *Archiv der Pharmazie,* 1980, vol. 313, 471-476
- Protective Groups in Organic Synthesis. John Wiley & Sons Inc, 1999
- Jikken Kagaku Koza. Maruzen Co., Ltd, 1992, vol. 20, 279-372
- Jikken Kagaku Koza. Maruzen Co., Ltd, 1992, vol. 19, 363-482
- Jikken Kagaku Koza. Maruzen Co., Ltd, 1992, vol. 20, 1-110279-372
- *J. Am. Chem. Soc.,* 1950, vol. 72, 1814-1815
- *Jikken Kagaku Koza,* 1992, vol. 20, 279-372
- *J. Org. Chem.,* 1986, vol. 51, 713-717
- Protective Groups in Organic Synthesis. John Wiley & Sons Inc, 1999
- *Journal of Pharmacology and Experimental Therapeutics,* 1992, vol. 263, 304-310
- *Journal of Pharmacology and Experimental Therapeutics,* 2003, vol. 305, 887-896
- *J. Org. Chem.,* 1989, vol. 54, 209
- *Bioorg. Med. Chem. Lett,* 1998, vol. 8, 1595
- *Tetrahedoron,* 1965, vol. 21, 1
- *J. Org. Chem.,* 1989, vol. 54, 209-216